# EUROPÄISCHE PATENTSCHRIFT

(11) **EP 1 487 821 B1**
(45) Veröffentlichungstag und Bekanntmachung des Hinweises auf die Patenterteilung: **21.03.2007**
(21) Anmeldenummer: 03708202.1
(22) Anmeldetag: 10.03.2003
(51) Int. Cl.: C07D 401/14, C07D 471/04, A61K 31/5513, A61P 3/10

(54) **BENZODIAZEPIN-SUBSTITUIERTE PIPERDINE ZUR VERWENDUNG IN DER BEHANDLUNG VON CARDIOVASKULÄREN ERKRANKUNGEN**
BENZODIAZEPINE-SUBSTITUTED PIPERIDINES FOR UTILIZATION IN THE TREATMENT OF CARDIOVASCULAR DISEASES
PIPERIDINES SUBSTITUEES PAR BENZODIAZEPINE A UTILISER DANS LE TRAITEMENT DE MALADIES CARDIOVASCULAIRES

(30) Priorität: 14.03.2002 DE 10211770
(43) Veröffentlichungstag der Anmeldung: 22.12.2004
(73) Patentinhaber: Boehringer Ingelheim Pharma GmbH & Co.KG, 55216 Ingelheim am Rhein (DE)
(72) Erfinder: HURNAUS, Rudolf, 88400 Biberach (DE); RUDOLF, Klaus, 88447 Warthausen (DE); MÜLLER, Stephan, Georg, 88447 Warthausen (DE); STENKAMP, Dirk, 88400 Biberach (DE); LUSTENBERGER, Philipp, 4056 Basel (CH); DREYER, Alexander, 88484 Gutenzell-Hürbel (DE); GERLACH, Kai, 88400 Biberach (DE); SCHINDLER, Marcus, 88400 Biberach (DE); ARNDT, Kirsten, 88400 Biberach (DE); BAUER, Eckhart, 88400 Biberach (DE)
(86) Internationale Anmeldenummer: PCT/EP2003/002417
(87) Internationale Veröffentlichungsnummer: WO 2003/076432

(56) Entgegenhaltungen:
- WO-A-01/32649
- WO-A-01/49676

## Beschreibung

Gegenstand der vorliegenden Erfindung sind neue substituierte Piperidine der allgemeinen Formel 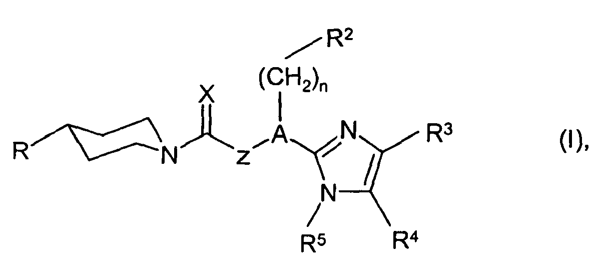 deren Tautomere, deren Diastereomere, deren Enantiomere, deren Gemische und deren Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

In der PCT/EP00/10463 werden bereits Arylalkane, Arylalkene und Aryl-Azaalkane mit CGRP-antagonistischen Eigenschaften beschrieben, die sich strukturell jedoch wesentlich von den Verbindungen der vorliegenden Anmeldung unterscheiden.

In der obigen allgemeinen Formel (I) bedeuten
R einen gesättigten, einlach ungesättigten oder, im Fall der 6- und 7-gliedrigen Heterocyclen, auch zweifach ungesättigten 5- bis 7-gliedrigen Aza-, Diaza-, Triaza-, Oxaza-, Thiaza-, Thiadiaza- oder S,S-Dioxido-thiadiaza-Heterocyclus,
wobei die vorstehend erwähnten Heterocyclen über ein Kohlenstoff- oder Stickstoffatom verknüpft sind und
ein oder zwei Carbonylgruppen enthalten, die mit mindestens einem Stickstoffatom verknüpft sind,
an einem der Stickstoffatome durch eine Alkylgruppe substituiert sein können,
an einem oder an zwei Kohlenstoffatomen jeweils durch eine Alkyl-, Phenyl-, Phenylmethyl-, Naphthyl-, Biphenylyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolylgruppe substituiert sein können, wobei die Substituenten gleich oder verschieden sein können,
   und wobei eine Doppelbindung eines der vorstehend erwähnten ungesättigten Heterocyclen mit einem Benzol-, Pyridin-, Diazin-, 1,3-Oxazol-, Thiophen-, Furan-, Thiazol-, Pyrrol-, N-Methyl-pyrrol- oder Chinolin-Ring, mit einem gegebenenfalls am Stickstoffatom durch eine Alkylgruppe substituierten 2(1*H*)-Oxo-chinolin-Ring oder mit einem Imidazol- oder *N*-Methyl-imidazol-Ring kondensiert sein kann oder auch zwei olefinische Doppelbindungen eines der vorstehend erwähnten ungesättigten Heterocyclen jeweils mit einem Benzolring kondensiert sein können,
   wobei die in R enthaltenen Phenyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolylgruppen sowie benzo-, thieno-, pyrido- und diazinokondensierten Heterocyclen im Kohlenstoffgerüst zusätzlich durch Fluor-, Chlor-, Brom- oder lodatome, durch Alkyl-, Alkoxy-, Nitro-, Alkylthio-, Alkylsulfinyl-, Alkylsulfonyl-, Alkylsulfonylamino-, Phenyl-, Trifluormethyl-, Difluormethyl-, Difluormethoxy-, Alkoxycarbonyl-, Carboxy-, Dialkylamino-, Hydroxy-, Amino-, Acetylamino-, Propionylamino-, Aminocarbonyl-, Alkylaminocarbonyl-, Dialkylaminocarbonyl-, (4-Morpholinyl)carbonyl-, (1-Pyrrolidinyl)carbonyl-, (1-Piperidinyl)carbonyl-, (Hexahydro-1-azepinyl)carbonyl-, (4-Methyl-1-piperazinyl)carbonyl-, Methylendioxy-, Aminocarbonylamino-, Alkanoyl-, Cyan-, Trifluormethoxy-, Trifluormethylthio-, Trifluormethylsulfinyl-oder Trifluormethylsulfonylgruppen mono-, di- oder trisubstituiert sein können, wobei die Substituenten gleich oder verschieden sein können,
X ein Sauerstoffatom oder, sofern Z die Gruppe -NR¹. bedeutet, auch eine der Gruppen =N-CN oder =N-SO₂-R⁶, worin
R⁶ einen Alkylrest mit 1 bis 4 Kohlenstoffatomen oder einen gegebenenfalls durch ein Halogenatom, eine Methyl- oder eine Methoxygruppe substituierten Phenylrest darstellt,
Z eine der Gruppen -CH₂-, in der die Wasserstoffatome unabhängig voneinander durch ein Fluoratom oder eine C₁₋₃-Alkylgruppe ersetzt sein können, oder -NR¹-, in der
R¹ das Wasserstoffatom, eine Alkylgruppe, die im Alkylteil mit Ausnahme der Position 1 durch eine Amino-, C₁₋₃-Alkyl-amino- oder Di-(C₁-₃-alkyl)-aminogruppe substituiert sein kann, oder eine Phenylalkylgruppe, die im Phenylteil durch Fluor-, Chlor-, Brom- oder lodatome, durch C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃- alkyl)-amino-, Carboxy-, C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe mono- oder disubstituiert sein kann, wobei die Substituenten gleich oder verschieden sein können, darstellt,
A ein durch ein Wasserstoffatom oder durch eine C₁₋₃-Alkylgruppe substituiertes Kohlenstoffatom
n die Zahl 1 oder 2,
R² die Gruppe 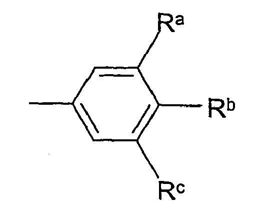 in der
eine der Gruppen R^{a}, R^{b} und. R^{c} das Wasserstoff-, Fluor-, Chlor-, Brom- oder lodatom, eine verzweigte oder unverzweigte Alkylgruppe, eine Hydroxy-, Alkoxy-, Trifluormethyl-, Difluormethyl-, Trifluormethoxy-, Difluormethoxy-, Trifluormethylthio-, Amino-, Acetylamino-, Dialkylaminoalkyl-, Dialkylaminoalkoxy-, Nitro-, Methylsulfonyloxy-, Aminocarbonyl-, Alkylaminocarbonyl-, Dialkylaminocarbonyl-, Alkanoyl-, Cyan-, Trifluormethylsulfinyl- oder Trifluormethylsulfonylgruppe,
eine zweite der Gruppen R^{a}, R^{b} und R^{c} das Wasserstoff-, Fluor-, Chlor-, Brom-oder lodatom, eine verzweigte oder unverzweigte Alkylgruppe, eine Hydroxy-, Alkoxy-, Trifluormethyl-, Difluormethyl-, Trifluormethoxy-, Difluormethoxy-, Trifluormethylthio-, Amino-, Acetylamino-, Alkanoyl-, Aminocarbonyl-, Alkylaminocarbonyl- oder Dialkylaminocarbonylgruppe und
die dritte der Gruppen R^{a}, R^{b} und R^{c} das Wasserstoff-, Fluor-, Chlor-, Brom- oder lodatom, eine verzweigte oder unverzweigte Alkylgruppe, eine Hydroxy-, Alkoxy-, Trifluormethyl-, Difluormethyl-, Difluormethoxy-, oder Trifluormethoxygruppe darstellen, wobei die Substituenten gleich oder verschieden sein können,
R³ und R⁴, die gleich oder verschieden sein können, jeweils das Wasserstoffatom, eine C₁₋₄-Alkyl-, Amino-C₁₋₃-alkyl-, C₁₋₃-Alkyl-amino-C₁₋₃-alkyl oder Di-(C₁₋₃-alkyl)-amino-C₁₋₃-alkylgruppe,
eine Phenyl- oder Naphthylgruppe, die jeweils durch R^{d} mono- oder disubstituiert sein können, wobei die Substituenten gleich oder verschieden sein können und R^{d} ein Fluor-, Chlor-, Brom- oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe oder eine 4- bis 7-gliedrige Cycloalkylenimino-carbonylgruppe, in der
ein oder zwei Wasserstoffatome jeweils durch eine C₁₋₃-Alkylgruppe ersetzt sein können oder/und
jeweils die Methylengruppe in Position 4 eines 6- oder 7-gliedrigen Cycloalkyleniminoteils durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)-gruppe ersetzt sein kann, darstellt,
eine im Kohlenstoffgerüst gegebenenfalls durch eine C₁₋₃-Alkyl-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl oder Di-(C₁₋₃-alkyl)-amino-C₁₋₃-alkylgruppe substituierte monocyclische 5- oder 6-gliedrige Heteroarylgruppe, wobei
die 6-gliedrige Heteroarylgruppe ein, zwei oder drei Stickstoffatome enthält und gegebenenfalls zusätzlich durch ein Fluor-, Chlor-, Brom- oder lodatom, eine C₁₋₃-Alkyl-, C₁₋₃-Alkoxy-, Amino-, C₁₋₃-Alkylamino- oder Di-(C₁₋₃-alkyl)aminogruppe substituiert sein kann und
die 5-gliedrige Heteroarylgruppe eine gegebenenfalls durch eine C₁₋₃-Alkyl-, Acetyl-, Trifluoracetyl-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe, ein Sauerstoff- oder Schwefelatom oder
eine gegebenenfalls durch eine C₁₋₃-Alkyl-, Acetyl-, Trifluoracetyl-, C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl-, Acetyl-, Trifluoracetyl-, C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe und zwei Stickstoffatome enthält,
oder R³ und R⁴ zusammen eine 1,3-Butadien-1,4-ylengruppe, in der
eine, zwei oder drei Methingruppen jeweils durch ein Stickstoffatom ersetzt sein können,
ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Di-(C₁₋₃-alkyl)-amino-C₂₋₃-alkyl-aminocarbonyl-, Amino-C₁₋₃-alkyl-, C₁₋₃-Alkyl-amino-C₁₋₃-alkyl- oder Di-(C₁₋₃-alkyl)-amino-C₁-₃-alkylgruppe oder durch eine 4- bis 7-gliedrige Cycloalkylenimino-carbonylgruppe, in der
   ein oder zwei Wasserstoffatome jeweils durch eine C₁₋₃-Alkylgruppe ersetzt sein können oder/und
   jeweils die Methylengruppe in Position 4 eines 6- oder 7-gliedrigen Cycloalkyleniminoteils durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)-gruppe ersetzt sein kann,
und gegebenenfalls zusätzlich ein zweites Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy- oder Trifluormethoxygruppe ersetzt sein kann, und
R⁵ die Gruppe -(CH₂)ₘ-R^{e}, in der
m die Zahl 0 und
R^{e} ein Wasserstoffatom,
eine Phenyl- oder Naphthylgruppe, die jeweils durch R^{f} mono- oder disubstituiert sein können, wobei die Substituenten gleich oder verschieden sein können und R^{f} ein Fluor-, Chlor-, Brom- oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt, oder
eine im Kohlenstoffgerüst gegebenenfalls durch eine C₁₋₃-Alkylgruppe substituierte Pyridinyl-, Pyrazinyl-, Pyrimidinyl-, Pyridazinyl-, Pyrrolyl-, Furyl-, Thienyl-, Pyrazolyl-, Imidazolyl-, Oxazolyl-, lsoxazolyl-, Thiazolyl- oder lsothiazolylgruppe, in denen ein an ein Stickstoffatom gebundenes Wasserstoffatom durch eine C₁₋₃-Alkyl- oder Acetylgruppe ersetzt sein kann, oder
eine C₃₋₇-Cycloalkylgruppe, wobei
   ein Wasserstoffatom der C₃₋₇-Cycloalkylgruppe durch eine Amino-, C₁₋₃-Alkyl-amino-, Di-(C₁₋₃-alkyl)-amino-, Amino-C₁₋₃-alkyl-, C₁₋₃-Alkyl-amino-C₁₋₃-alkyl- oder Di-(C₁₋₃-alkyl)-amino-C₁₋₃-alkylgruppe ersetzt sein kann oder/und
   die Methylengruppe in Position 3 der Cyclopentylgruppe und die Methylengruppe in Position 4 der Cyclohexyl- und Cycloheptylgruppe jeweils durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)-gruppe ersetzt sein kann,
oder m eine der Zahlen 1 bis 5 und
R^{e} ein Wasserstoffatom, eine Aminomethylen-, C₁₋₃-Alkylaminomethylen-, Di-(C₁₋₃-alkyl)-aminomethylen-, Aminocarbonyl-, C₁₋₃-Alkylaminocarbonyl-, Di-(C₁₋₃-alkyl)-aminocarbonyl- oder C₃₋₇-Cycloalkylgruppe, wobei
   ein Wasserstoffatom der C₃₋₇-Cycloalkylgruppe durch eine Amino-, C₁₋₃-Alkyl-amino-, Di-(C₁₋₃-alkyl)-amino-, Amino-C₁₋₃-alkyl-, C₁₋₃-Alkyl-amino-C₁₋₃-alkyl- oder Di-(C₁₋₃-alkyl)-amino-C₁₋₃-alkylgruppe ersetzt sein kann oder/und
   die Methylengruppe in Position 3 der Cyclopentylgruppe und die Methylengruppe in Position 4 der Cyclohexyl- und Cycloheptylgruppe jeweils durch ein Sauerstoff- oder Schwefelatom, durch eine Sulfinyl-, Sulfonyl-, -NH-,
   -N(C₁₋₃-Alkyl)- oder -N(C₁₋₃-Alkyl-carbonyl)-gruppe ersetzt sein kann,
eine 4- bis 7-gliedrige Cycloalkyleniminogruppe, in der
   ein oder zwei Wasserstoffatome jeweils durch eine C₁₋₃-Alkylgruppe ersetzt sein können oder/und
   jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)-gruppe ersetzt sein kann,
eine Phenyl- oder Naphthylgruppe, die jeweils unabhängig voneinander durch R^{g} mono- oder disubstituiert sein können, wobei die Substituenten gleich oder verschieden sein können und R^{g} ein Fluor-, Chlor-, Brom- oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt,
oder eine im Kohlenstoffgerüst gegebenenfalls durch eine C₁₋₃-Alkyl-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte monocyclische 5- oder 6-gliedrige Heteroarylgruppe, wobei
   die 6-gliedrige Heteroarylgruppe ein, zwei oder drei Stickstoffatome und gegebenenfalls zusätzlich durch ein Fluor-, Chlor-, Brom- oder lodatom, eine C₁₋₃-Alkyl-, C₁₋₃-Alkoxy-, Amino-, C₁₋₃-Alkylamino- oder Di-(C₁₋₃-alkyl)aminogruppe substituiert sein kann und
   die 5-gliedrige Heteroarylgruppe eine gegebenenfalls durch eine C₁₋₃-Alkyl-, Acetyl-, Trifluoracetyl-, C₁₋₃-Alkoxy-carbonyl-, Aminocarbonyl-, C₁₋₃-Alkylaminocarbonyl-, Di-(C₁₋₃-alkyl)-aminocarbonyl-, Amino-C₁₋₃-alkyl-, C₁₋₃-Alkyl-amino-C₁₋₃-alkyl oder Di-(C₁₋₃-alkyl)-aminO-C₁₋₃-alkylgruppe substituierte Iminogruppe, ein Sauerstoff- oder Schwefelatom oder
   eine gegebenenfalls durch eine C₁₋₃-Alkyl-, Acetyl-, Trifluoracetyl-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe oder ein Sauerstoff-oder Schwefelatom und zusätzlich ein Stickstoffatom oder
   eine gegebenenfalls durch eine C₁₋₃-Alkyl-, Acetyl-, Trifluoracetyl-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁-₃-alkylgruppe substituierte Iminogruppe und zwei Stickstoffatome enthält,
darstellen, wobei die vorstehend genannten Alkylgruppen oder die in den vorstehend genannten Resten enthaltenen Alkylgruppen, sofern nichts anderes angegeben wurde, 1 bis 5 Kohlenstoffatome enthalten und verzweigt oder unverzweigt sein können.

Beispielsweise kommt für R die 4-(2-Oxo-4-phenyl-2,3-dihydro-imidazol-1-yl)-, 4-(2-Oxo-2,3-dihydro-benzimidazol-1-yl)-, 4-(2,4-Dioxo-1,4-dihydro-2*H*-chinazolin-3-yl)-, 4-(2-Oxo-1,2-dihydro-imidazo[4,5-b]pyridin-3-yl)-, 4-(2-Oxo-1,4-dihydro-2*H*-chinazolin-3-yl)-, 4-(2-Oxo-1,2,4,5-tetrahydro-1,3-benzodiazepin-3-yl)-, 4-(7-Methoxy-2-oxo-1,2,4,5-tetrahydro-1,3-benzodiazepin-3-yl)-, 4-(2-Oxo-1,2-dihydro-chinolin-3-yl)-, 4-(5-Oxo-3-phenyl-4,5-dihydro-1,2,4-triazol-1-yl)-, 4-(2-Oxo-1,2-dihydro-imidazo[4,5-c]-chinolin-3-yl)- Gruppe in Betracht.

Die vorliegende Erfindung betrifft Racemate, sofern die Verbindungen der allgemeinen Formel I ein Chiralitätselement besitzen. Die Anmeldung umfaßt jedoch auch die einzelnen diastereomeren Antipodenpaare oder deren Gemische, die dann vorliegen, wenn mehr als ein Chiralitätselement in den Verbindungen der allgemeinen Formel (I) vorhanden ist, sowie die einzelnen optisch aktiven Enantiomeren, aus denen sich die erwähnten Racemate zusammensetzen.

Die Verbindungen der allgemeinen Formel (1) weisen wertvolle pharmakologische Eigenschaften auf, die auf ihre CGRP-antagonistischen Eigenschaften zurückgehen. Ein weiterer Gegenstand der Erfindung sind diese Verbindungen enthaltende Arzneimittel, deren Verwendung und deren Herstellung.

Eine besonders zu erwähnende Untergruppe von Verbindungen der allgemeinen Forme I sind diejenigen, in denen R, X, Z, A, n, R², und R⁵ wie vorstehend erwähnt definier sind und
R³ das Wasserstoffatom oder eine C₁₋₄-Alkylgruppe und
R⁴ das Wasserstoffatom, eine C₁₋₄-Alkyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe,
eine Phenyl- oder Naphthylgruppe, die jeweils unabhängig voneinander durch R^{d} mono- oder disubstituiert sein können, wobei die Substituenten gleich oder verschieden sein können und R^{d} ein Fluor-, Chlor-, Brom- oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt, oder
eine im Kohlenstoffgerüst gegebenenfalls durch eine C₁₋₃-Alkyl-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte monocyclische 5- oder 6-gliedrige Heteroarylgruppe, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Acetyl-, Trifluoracetyl-, C₁₋₃-Alkoxy-carbonyl-, Aminocarbonyl-, C₁₋₃-Alkylaminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe, ein Sauerstoff- oder Schwefelatom oder
eine gegebenenfalls durch eine C₁₋₃-Alkyl-, Acetyl-, Trifluoracetyl- C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl-, Acetyl-, Trifluoracetyl- C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe und zwei Stickstoffatome enthält,
bedeuten, deren Tautomere, deren Diastereomere, deren Enantiomere, derer Gemische und deren Salze.

Eine zweite besonders zu erwähnende Untergruppe von Verbindungen der allgemeiner Formel I sind diejenigen, in denen R, X, Z, A, n, R², und R⁵ wie vorstehend erwähn definiert sind und
R³ und R⁴ zusammen eine 1,3-Butadien-1,4-ylengruppe, in der
eine, zwei oder drei Methingruppen jeweils durch ein Stickstoffatom ersetzt sein können,
ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Di(C₁₋₃-alkyl)-amino-C₂₋₃-alkyl-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe oder durch eine 4- bis 7-gliedrige Cycloalkylenimino-carbonylgruppe, in der
   ein oder zwei Wasserstoffatome jeweils durch eine C₁₋₃-Alkylgruppe ersetzt sein können oder/und
   jeweils die Methylengruppe in Position 4 eines 6- oder 7-gliedrigen Cycloalkyleniminoteils durch ein Sauerstoff- oder Schwefelatom, durch eine Sulfinyl-, Sulfonyl-, -NH-, -N(C₁₋₃-Alkyl)- oder-N(C₁₋₃-Alkyl-carbonyl)- Gruppe ersetzt sein kann,
und gegebenenfalls zusätzlich ein zweites Wasserstoffatom durch ein Fluor-, Chlor-, -Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy- oder Trifluormethoxygruppe ersetzt sein kann, darstellen.

Bevorzugte Verbindungen der obigen allgemeinen Formel I sind diejenigen, in denen
R einen gesättigten oder einfach ungesättigten 5- bis 7-gliedrigen Aza-, Diaza-, Oxaza- oder Thiaza-Heterocyclus,
wobei die vorstehend erwähnten Heterocyclen über ein Stickstoffatom verknüpft sind und
eine mit einem oder zwei Stickstoffatomen des Heterocyclus verknüpfte Carbonylgruppe enthalten,
an einem der Stickstoffatome durch eine Alkylgruppe substituiert sein können,
an einem der Kohlenstoffatome durch eine Alkyl-, Phenyl-, Pyridinyl-, Furyl-, Thienyl- oder Pyrrolylgruppe substituiert sein können,
und wobei eine Doppelbindung eines der vorstehend erwähnten ungesättigten Heterocyclen mit einem Benzol-, Pyridin- oder Thiophen-Ring kondensiert sein kann,
   wobei die in R enthaltenen Phenyl-, Pyridinyl-, Furyl-, Thienyl-, Pyrrolylgruppen sowie die benzo-, thieno- und pyridokondensierten Heterocyclen im Kohlenstoffgerüst zusätzlich durch Fluor-, Chlor-, Brom- oder lodatome, durch Alkyl-, Alkoxy-, Nitro-, Alkylthio-, Trifluormethyl-, Difluormethyl-, Alkoxycarbonyl-, Carboxy-, Dialkylamino-, Hydroxy-, Amino-, Acetylamino-, Aminocarbo-, nyl-, Alkylaminocarbonyl-, Alkanoyl-, Cyan- oder Trifluormethoxygruppen mono- oder disubstituiert sein können, wobei die Substituenten gleich oder verschieden sein können,
X ein Sauerstoffatom oder, sofern Z die Gruppe -NR¹- bedeutet, auch die Gruppe =N-CN,
Z eine der Gruppen -CH₂- oder -NR¹-, in der
R¹ das Wasserstoffatom oder eine Alkylgruppe darstellt,
A ein durch ein Wasserstoffatom oder durch eine C₁₋₃-Alkylgruppe substituiertes Kohlenstoffatom,
n die Zahl 1 oder 2,
R² die Gruppe 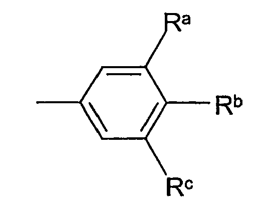 in der
eine der Gruppen R^{a}, R^{b} und R^{c} das Wasserstoff-, Fluor-, Chlor-, Brom- oder lodatom, eine verzweigte oder unverzweigte Alkylgruppe, eine Hydroxy-, Alkoxy-, Trifluormethyl-, Difluormethyl-, Trifluormethoxy-, Amino-, Acetylamino-, Dialkylaminoalkyl-, Dialkylaminoalkoxy-, Aminocarbonyl-, Alkylaminocarbonyl-, Dialkylaminocarbonyl-, Alkanoyl-, Cyan- oder Trifluormethylsulfonylgruppe,
eine zweite der Gruppen R^{a}, R^{b} und R^{c} das Wasserstoff-, Fluor-, Chlor-, Brom-oder lodatom, eine verzweigte oder unverzweigte Alkylgruppe, eine Alkoxy-, Trifluormethyl-, Amino-, Acetylamino- oder Alkanoylgruppe und
die dritte der Gruppen R^{a}, R^{b} und R^{c} das Wasserstoff-, Fluor-, Chlor-, Brom- oder lodatom oder eine verzweigte oder unverzweigte Alkylgruppe darstellen, wobei die Substituenten gleich oder verschieden sein können,
R³ das Wasserstoffatom oder eine C₁₋₃-Alkylgruppe,
R⁴ das Wasserstoffatom, eine C₁₋₄-Alkyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe,
eine Phenylgruppe, die durch R^{d} mono- oder disubstituiert sein kann, wobei die Substituenten gleich oder verschieden sein können und R^{d} ein Fluor-, Chlor-, Brom-oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Amino-, C₁₋₃-Alkylamino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt, oder
eine im Kohlenstoffgerüst gegebenenfalls durch eine C₁₋₃-Alkylgruppe substituierte Pyridinyl-, Pyrazinyl-, Pyrimidinyl-, Pyridazinyl-, Pyrrolyl-, Furyl-, Thienyl-, Pyrazolyl-, lmidazolyl-, Oxazolyl-, lsoxazolyl-, Thiazolyl- oder lsothiazolylgruppe, in denen ein an ein Stickstoffatom gebundenes Wasserstoffatom durch eine C₁₋₃-Alkyl- oder Acetylgruppe ersetzt sein kann,
oder R³ und R⁴ zusammen eine 1,3-Butadien-1,4-ylengruppe, in der
ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Di-(C₁₋₃-alkyl)-amino-C₂₋₃-alkyl-aminocarbonyl-, Amino-C₁₋₃-alkyl-, C₁₋₃-Alkyl-amino-C₁₋₃-alkyl-, Di-(C₁₋₃-alkyl)-amino-C₁₋₃-alkylgruppe oder durch eine 5- bis 7-gliedrige Cycloalkylenimino-carbonylgruppe, in der
   ein oder zwei Wasserstoffatome jeweils durch eine C₁₋₃-Alkylgruppe ersetzt sein können oder/und
   jeweils die Methylengruppe in Position 4 eines 6- oder 7-gliedrigen Cycloalkyleniminoteils durch ein Sauerstoff- oder Schwefelatom, durch eine -NH-, -N(C₁₋₃-Alkyl)- oder-N(C₁₋₃-Alkyl-carbonyl)- Gruppe ersetzt sein kann,
und gegebenenfalls zusätzlich ein zweites Wasserstoffatom durch ein Fluor-, Chlor-, Brom- oder lodatom, durch eine C₁₋₃-Alkyl- oder Trifluormethylgruppe ersetzt sein kann, und
R⁵ die Gruppe -(CH₂)ₘ-R^{e}, in der
m die Zahl 0 und
R^{e} ein Wasserstoffatom oder eine C₅₋₆-Cycloalkylgruppe, wobei
   die Methylengruppe in Position 3 der Cyclopentylgruppe und die Methylengruppe in Position 4 der Cyclohexylgruppe jeweils durch eine -NH-, -N(C₁₋₃-Alkyl)- oder -N(C₁₋₃-Alkyl-carbonyl)-gruppe ersetzt sein kann,
oder m eine der Zahlen 1 bis 5 und
R^{e} ein Wasserstoffatom, eine Aminomethylen-, C₁₋₃-Alkylaminomethylen-, Di-(C₁₋₃-alkyl)-aminomethylen-, Aminocarbonyl-, C₁₋₃-Alkyl-aminocarbonyl-, Di-(C₁₋₃-alkyl)-amino carbonyl- oder C₅₋₆-Cycloalkylgruppe, wobei
   die Methylengruppe in Position 3 der Cyclopentylgruppe und die Methylengruppe in Position 4 der Cyclohexylgruppe jeweils durch eine -NH-, -N(C₁₋₃-Alkyl)- oder -N(C₁₋₃-Alkyl-carbonyl)-gruppe ersetzt sein kann,
eine 5- bis 6-gliedrige Cycloalkyleniminogruppe, wobei
   die Methylengruppe in Position 4 einer 6-gliedrigen Cycloalkyleniminogruppe durch eine -NH-, -N(C₁₋₃-Alkyl)- oder -N(C₁₋₃-Alkyl-carbonyl)-gruppe ersetzt sein kann,
eine Phenylgruppe, die durch R⁹ mono- oder disubstituiert sein kann, wobei die Substituenten gleich oder verschieden sein können und R^{g} ein Fluor-, Chlor-, Brom- oder lodatom, eine C₁₋₃-Alkyl-, Trifluormethyl-, Difluormethyl-, Hydroxy-, C₁₋₃-Alkoxy-, Amino-, C₁₋₃-Alkyl-amino-, Di-(C₁₋₃-alkyl)-amino-, Aminocarbonyl-C₁₋₃-Alkyl-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt,
oder eine im Kohlenstoffgerüst gegebenenfalls durch eine C₁₋₃-Alkylgruppe substituierte Pyridinyl-, Pyrazinyl-, Pyrimidinyl-, Pyridazinyl-, Pyrrolyl-, Furyl-, Thienyl-, Pyrazolyl-, Imidazolyl-, Oxazolyl-, Isoxazolyl-, Thiazolyl- oder Isothiazolylgruppe, in denen ein an ein Stickstoffatom gebundenes Wasserstoffatom durch eine C₁₋₃-Alkyl- oder Acetylgruppe ersetzt sein kann, darstellen,
bedeuten, wobei die vorstehend genannten Alkylgruppen oder die in den vorstehend genannten Resten enthaltenen Alkylgruppen, sofern nichts anderes angegeben wurde, 1 bis 4 Kohlenstoffatome enthalten und verzweigt oder unverzweigt sein können,
deren Tautomere, deren Diastereomere, deren Enantiomere und deren Salze.

Eine besonders zu erwähnende Untergruppe von bevorzugten Verbindungen de allgemeinen Formel I sind diejenigen, in denen R, X, Z, A, n, R², und R⁵ wie vorstehen erwähnt definiert sind und
R³ das Wasserstoffatom oder eine C₁₋₃-Alkylgruppe und
R⁴ eine Phenylgruppe, die durch R^{d} mono- oder disubstituiert sein kann, wobei die Substituenten gleich oder verschieden sein können und R^{d} ein Fluor-, Chlor-, Brom-oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt, oder
eine im Kohlenstoffgerüst gegebenenfalls durch eine C₁₋₃-Alkylgruppe substituierte Pyridinyl-, Pyrazinyl-, Pyrimidinyl-, Pyridazinyl-, Pyrrolyl-, Furyl-, Thienyl-, Pyrazolyl-, Imidazolyl-, Oxazolyl-, Isoxazolyl-, Thiazolyl- oder Isothiazolylgruppe, in denen ein an ein Stickstoffatom gebundenes Wasserstoffatom durch eine C₁₋₃-Alkyl- oder Acetylgruppe ersetzt sein kann, bedeuten.

Eine zweite besonders zu erwähnende Untergruppe von bevorzugten Verbindungen de allgemeinen Formel I sind diejenigen, in denen R, X, Z, A, n, R², und R⁵ wie vorstehen erwähnt definiert sind und
R³ und R⁴ zusammen eine 1,3-Butadien-1,4-ylengruppe, in der
ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl- oder Di-(C₁₋₃-alkyl)-amino-C₂₋₃-alkyl-aminocarbonylgruppe oder durch eine 5- bis 7-gliedrige Cycloalkylenimino-carbonylgruppe, in der
   ein oder zwei Wasserstoffatome jeweils durch eine C₁-₃-Alkylgruppe ersetzt sein können oder/und
   jeweils die Methylengruppe in Position 4 eines 6- oder 7-gliedrigen Cycloalkyleniminoteils durch ein Sauerstoff- oder Schwefelatom, durch eine -NH-, -N(C₁₋₃-Alkyl)- oder-N(C₁₋₃-Alkyl-carbonyl)- gruppe ersetzt sein kann,
und gegebenenfalls zusätzlich ein zweites Wasserstoffatom durch ein Fluor-, Chlor-, Brom- oder lodatom, durch eine C₁₋₃-Alkyl- oder Trifluormethylgruppe ersetzt sein kann, bedeuten.

Besonders bevorzugte Verbindungen der obigen allgemeinen Formel I sind diejenigen, in denen
R einen einfach ungesättigten, über ein Stickstoffatom verknüpften 5- bis 7-gliedrigen Diaza-Heterocyclus,
wobei die vorstehend erwähnten Heterocyclen eine mit beiden Stickstoffatomen des Heterocyclus verknüpfte Carbonylgruppe enthalten,
an einem der Stickstoffatome und einem der gesättigten Kohlenstoffatome unabhängig voneinander jeweils durch eine C₁₋₃-Alkylgruppe substituiert sein können,
und wobei die Doppelbindung der vorstehend erwähnten ungesättigten Heterocyclen mit einem gegebenenfalls durch ein Fluor-, Chlor-, Brom- oder lodatom, durch eine C₁₋₃-Alkyl-, C₁₋₃-Alkoxy-, Trifluormethyl-, Carboxy-, C₁₋₃-Alkoxycarbonyl-, Amino-, Acetylamino-, Hydroxy-, Aminocarbonyl- oder Alkanoylgruppe substituierten Benzolring kondensiert ist,
X ein Sauerstoffatom,
Z eine der Gruppen -CH₂- oder-NR¹-, in der
R¹ das Wasserstoffatom oder eine C₁₋₃-Alkylgruppe darstellt,
A ein durch ein Wasserstoffatom oder durch eine C₁₋₃-Alkylgruppe substituiertes Kohlenstoffatom,
n die Zahl 1,
R² die Gruppe 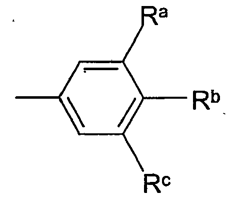 in der
eine der Gruppen R^{a}, R^{b} und R^{c} das Wasserstoff-, Fluor-, Chlor-, Brom- oder lodatom, eine C₁₋₃-Alkyl-, Trifluormethyl-, Hydroxy-, Alkoxy- oder Aminogruppe,
eine zweite der Gruppen R^{a}, R^{b} und R^{c} das Wasserstoff-, Fluor-, Chlor-, Brom-oder lodatom, eine C₁₋₃-Alkyl- oder Trifluormethylgruppe und
die dritte der Gruppen R^{a}, R^{b} und R^{c} das Wasserstoff-, Fluor-, Chlor-, Brom- oder lodatom darstellen, wobei die Substituenten gleich oder verschieden sein können,
R³ das Wasserstoffatom oder eine C₁₋₃-Alkylgruppe,
R⁴ eine gegebenenfalls durch ein Fluor-, Chlor-, Brom- oder lodatom, durch eine C₁₋₃-Alkyl-, Trifluormethyl-, Hydroxy-, C₁₋₃-Alkoxy-, Amino-, C₁₋₃-Alkylamino-, Di-(C₁₋₃-alkyl)-amino-, Carboxy- oder C₁₋₃-Alkoxy-carbonylgruppe substitiuierte Phenylgruppe,
oder R³ und R⁴ zusammen eine 1,3-Butadien-1,4-ylengruppe, in der
ein Wasserstoffatom durch ein Fluor-, Chlor-, Brom- oder lodatom, durch eine C₁₋₃-Alkyl-, Trifluormethyl-, Hydroxy-, C₁₋₃-Alkoxy-, Carboxy-, C₁₋₃-Alkoxycarbonyl-, Aminocarbonyl-, C₁₋₃-Alkyl-aminocarbonyl-, Di-(C₁₋₃-alkyl)-aminocarbonyl- oder Di-(C₁₋₃-alkyl)-amino-C₂₋₃-alkyl-aminocarbonylgruppe oder durch eine 5- bis 7-gliedrige Cycloalkylenimino-carbonylgruppe, wobei
   die Methylengruppe in Position 4 des 6-gliedrigen Cycloalkyleniminoteils durch eine -NH-, -N(C₁₋₃-Alkyl)- oder -N(C₁₋₃-Alkyl-carbonyl)- Gruppe ersetzt sein kann,
und gegebenenfalls zusätzlich ein zweites Wasserstoffatom durch ein Fluor-, Chlor-, Brom- oder lodatom oder durch eine C₁₋₃-Alkylgruppe ersetzt sein kann, und
R⁵ die Gruppe -(CH₂)ₘ-R^{e}, in der
m die Zahl 0 und
R^{e} ein Wasserstoffatom,
oder m eine der Zahlen 1 bis 3 und
R^{e} ein Wasserstoffatom, eine Aminomethylen-, C₁₋₃-Alkylaminomethylen- oder Di-(C₁₋₃-alkyl)-aminomethylengruppe darstellen,
bedeuten, wobei die vorstehend genannten Alkylgruppen oder die in den vorstehend genannten Resten enthaltenen Alkylgruppen, sofern nichts anderes angegeben wurde, verzweigt oder unverzweigt sein können,
deren Tautomere, deren Diastereomere, deren Enantiomere und deren Salze.

Eine besonders zu erwähnende Untergruppe von besonders bevorzugten Verbindungen der allgemeinen Formel I sind diejenigen, in denen R, X, Z, A, n, R², und R⁵ wie vor stehend erwähnt definiert sind und
R³ das Wasserstoffatom oder eine C₁₋₃-Alkylgruppe und
R⁴ eine gegebenenfalls durch ein Fluor-, Chlor-, Brom- oder lodatom, durch eine C₁₋₃-Alkyl-, Trifluormethyl-, Hydroxy-, C₁₋₃-Alkoxy-, Amino-, C₁₋₃-Alkylamino-, Di-(C₁₋₃-alkyl)-amino-, Carboxy- oder C₁₋₃-Alkoxy-carbonylgruppe substitiuierte Phenylgruppe bedeuten.

Eine zweite besonders zu erwähnende Untergruppe von besonders bevorzugter Verbindungen der allgemeinen Formel I sind diejenigen, in denen R, X, Z, A, n, R², und R⁵ wie vorstehend erwähnt definiert sind und
R³ und R⁴ zusammen eine 1,3-Butadien-1,4-ylengruppe, in der
ein Wasserstoffatom durch ein Fluor-, Chlor-, Brom- oder lodatom, durch eine C₁₋₃-Alkyl-, Trifluormethyl-, Hydroxy-, C₁₋₃-Alkoxy-, Carboxy-, C₁₋₃-Alkoxycarbonyl-, Aminocarbonyl-, C₁₋₃-Alkyl-aminocarbonyl-, Di-(C₁₋₃-alkyl)-aminocarbonyl- oder Di-(C₁₋₃-alkyl)-amino-C₂₋₃-alkyl-aminocarbonylgruppe oder durch eine 5- bis 7-gliedrige Cycloalkylenimino-carbonylgruppe, wobei
   die Methylengruppe in Position 4 des 6-gliedrigen Cycloalkyleniminoteils durch eine -NH-, -N(C₁₋₃-Alkyl)- oder-N(C₁₋₃-Alkyl-carbonyl)-gruppe ersetzt sein kann,
und gegebenenfalls zusätzlich ein zweites Wasserstoffatom durch ein Fluor-, Chlor-, Brom- oder lodatom oder durch eine C₁₋₃-Alkylgruppe ersetzt sein kann,
bedeuten.

Als besonders bevorzugte Verbindungen seien beispielsweise folgende genannt:
(1) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(3-dimethylamino-propyl)-1*H*-benzimidazol-2-yl]-ethyl}-amid,
(2) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(2-dimethylamino-ethy)-1*H*-benzimidazol-2-yl]-ethyl}-amid,
(3) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-methyl-1*H*-benzimidazol-2-yl)-ethyl]-amid,
(4) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1*H*-benzimidazol-2-yl)-ethyl]-amid,
(5) 2-((*R*)-2-(4-Amino-3,5-dibrom-phenyl)-1-{[4-(2-oxo-1,4-dihydro-3*H*-chinazolin-3-yl)-piperidin-1-carbonyl]-amino}-ethyl)-1*H*-benzimidazol-5-carbonsäuremethylester,
(6) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimidazol-5-carbonsäuremethylester,
(7) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimid azol-5-carbonsäure,
(8) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[6-(4-methyl-piperazin-1-carbonyl)-1*H-*benzimidazol-2-yl]-ethyl}-amid,
(9) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimidazol-5-carbonsäure-(3-dimethylamino-propyl)-amid,
(10) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimidazol-5-carbonsäure-(2-dimethylamino-ethyl)-amid,
(11) 4-(2-Oxo-1,4-dihydro-2*H*-chinazolin-3-yl)-piperidin-1-carbonsäure-{(*R*)-2-(4-amino-3,5-dibrom-phenyl)-1-[1-(3-dimethylamino-propyl)-5-phenyl-1*H*-imidazol-2-yl]-ethyl}-amid,
(12) 4-(2-Oxo-1,2,4,5-tetrahydro-1,3-benzodiazepin-3-yl)-piperidin-1-carbonsäure-[(*R*)-2-(4-amino-3,5-dibrom-phenyt)-1-(1-butyl-1*H*-benzimidazol-2-yl)-ethyl]-amid,
(13) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(3-pyrrolidin-1-yl-propyl)-1*H*-benzimidazol-2-yl]-ethyl}-amid,
(14) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-pyridin-3-ylmethyl-1*H*-benzimidazol-2-yl)-ethyl]-amid,
(15) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-benzyl-1*H*-benzimidazol-2-yl)-ethyl]-amid,
(16) 4-(2-Oxo-1,2,4,5-tetrahydro-1,3-benzodiazepin-3-yl)-piperidin-1-carbonsäure {(*R*)-2-(4-amino-3,5-dibrom-phenyl)-1-[1-(1-methyl-piperidin-4-ylmethyl)-1*H-*benzimidazol-2-yl]-ethyl}-amid,
(17) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(4-dimethylamino-butyl)-1*H*-benzimidazol-2-yl]-ethyl}-amid,
(18) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(1-methyl-piperidin-4-yl)-1*H*-benzimidazol-2-yl]-ethyl}-amid,
(19) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-cydohexyl-1*H*-benzimidazol-2-yl)-ethyl]-amid,
(20) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-cyclopentyl-1*H*-benzimidazol-2-yl)-ethyl]-amid,
(21) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(5-dimethylamino-pentyl)-1*H*-benzimidazol-2-yl]-ethyl}-amid,
(22) 4-[2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-benzimidazol-1-yl]-buttersäure-methylester,
(23) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[6-chlor-1-(3-dimethylamino-propyl)-1*H-*benzimidazol-2-yl]-ethyl}-amid,
(24) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(3-dimethylamino-propyl)-6-fluor-1*H-*benzimidazol-2-yl]-ethyl}-amid,
(25) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(3-dimethylamino-propyl)-5-fluor-1*H-*benzimidazol-2-yl]-ethyl}-amid,
(26) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[5,6-dichlor-1-(3-dimethylamino-propyl)-1*H*-benzimidazol-2-yl]-ethyl}-amid,
(27) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazol-5-carbonsäure-methylester,
(28) 2-(2-(4-Amino-3-chlor-5-trifluormethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazole-5-carbonsäure,
(29) 4-(2-Oxo-1,2,4,5-tetrahydro-1,3-benzodiazepin-3-yl)-piperidin-1-carbonsäure-{(*R*)-2-(3,5-dibrom-4-hydroxy-phenyl)-1-[1-(1-methyl-piperidin-4-ylmethyl)-1*H-*benzimidazol-2-yl]-ethyl}-amid,
(30) 4-(2-Oxo-1,2,4,5-tetrahydro-1,3-benzodiazepin-3-yl)-piperidin-1-carbonsäure-{(*R*)-2-(3,5-dibrom-4-hydroxy-phenyl)-1-[1-(1-methyl-piperidin-4-yl)-1*H*-benzimidazol-2-yl]-ethyl}-amid,
(31) 4-(2-Oxo-1,2,4,5-tetrahydro-1,3-benzodiazepin-3-yl)-piperidin-1-carbonsäure-{(*R*)-2-(3,5-dibrom-4-hydroxy-phenyl)-1-[1-(3-dimethylamino-propyl)-1*H*-benzimidazol-2-yl]-ethyl}-amid,
(32) 4-(2-Oxo-1,2,4,5-tetrahydro-1,3-benzodiazepin-3-yl)-piperidin-1-carbonsäure-{(*R*)-2-(3,5-dibrom-4-hydroxy-phenyl)-1-[1-(3-pyrrolidin-1-yl-propyl)-1*H*-benzimidazol-2-yl]-ethyl}-amid,
(33) 4-(2-Oxo-1,2,4,5-tetrahydro-1,3-benzodiazepin-3-yl)-piperidin-1-carbonsäure-[1-[6-chlor-1-(3-dimethylamino-propyl)-1*H*-benzimidazol-2-yl]-2-(3,4-dibromphenyl)-ethyl]-amid,
(34) 4-(2-Oxo-1,2,4,5-tetrahydro-1,3-benzodiazepin-3-yl)-piperidin-1-carbonsäure-{2-(3,4-dibrom-phenyl)-1-[5,6-dichlor-1-(3-dimethylamino-propyl)-1*H*-benzimidazol-2-yl]-ethyl}-amid,
(35) 4-(2-Oxo-1,2,4,5-tetrahydro-1,3-benzodiazepin-3-yl)-piperidin-1-carbonsäure-[1-[7-chlor-1-(3-dimethylamino-propyl)-1*H*-benzimidazol-2-yl]-2-(3,4-dibromphenyl)-ethyl]-amid,
(36) 4-(2-Oxo-1,2,4,5-tetrahydro-1,3-benzodiazepin-3-yl)-piperidin-1-carbonsäure-{2-(3,4-dibrom-phenyl)-1-[1-(3-dimethylamino-propyl)-6-fluor-1*H*-benzimidazol-2-yl]-ethyl}-amid,
(37) 4-(2-Oxo-1,2,4,5-tetrahydro-1,3-benzodiazepin-3-yl)-piperidin-1-carbonsäure-{2-(3,4-dibrom-phenyl)-1-[1-(1-methyl-piperidin-4-yl)-1*H*-benzimidazol-2-yl]-ethyl}-amid,
(38) 4-(2-Oxo-1,2,4,5-tetrahydro-1,3-benzodiazepin-3-yl)-piperidin-1 -carbonsäure-{2-(3,4-dibrom-phenyl)-1-[1-(3-pyrrolidin-1-yl-propyl)-1*H*-benzimidazol-2-yl]-ethyl}-amid,
(39) 4-(2-Oxo-1,2,4,5-tetrahydro-1,3-benzodiazepin-3-yl)-piperidin-1-carbonsäure-{2-(3,4-dibrom-phenyl)-1-[1-(1-methyl-piperidin-4-ylmethyl)-1*H*-benzimidazol-2-yl]-ethyl}-amid,
(40) 4-(2-Oxo-1,2,4,5-tetrahydro-1,3-benzodiazepin-3-yl)-piperidin-1-carbonsäure-[2-(3,4-dibrom-phenyl)-1-(1-pyridin-3-ylmethyl-1*H*-benzimidazol-2-yl)-ethyl]-amid,
(41) 4-(2-Oxo-1,2,4,5-tetrahydro-1,3-benzodiazepin-3-yl)-piperidin-1-carbonsäure-{2-(3,4-dibrom-phenyl)-1-[1-(3-dimethylamino-propyl)-1*H*-benzimidazol-2-yl]-ethyl}-amid,
(42) 4-(2-Oxo-1,2,4,5-tetrahydro-1,3-benzodiazepin-3-yl)-piperidin-1-carbonsäure-{2-(3,4-dibrom-phenyl)-1-[1-(4-dimethylamino-butyl)-1*H*-benzimidazol-2-yl]-ethyl}-amid,
(43) 4-(2-Oxo-1,2,4,5-tetrahydro-1,3-benzodiazepin-3-yl)-piperidin-1-carbonsäure-{2-(3,4-diethyl-phenyl)-1-[1-(3-dimethylamino-propyl)-1*H*-benzimidazol-2-yl]-ethyl}-amid,
(44) 4-(2-Oxo-1,2,4,5-tetrahydro-1,3-benzodiazepin-3-yl)-piperidin-1-carbonsäure-{2-(3,4-diethyl-phenyl)-1-[1-(4-dimethylamino-butyl)-1*H*-benzimidazol-2-yl]-ethyl}-amid,
(45) 4-(2-Oxo-1,2,4,5-tetrahydro-1,3-benzodiazepin-3-yl)-piperidin-1-carbonsäure-{2-(3,4-diethyl-phenyl)-1-[1-(1-methyl-piperidin-4-yl)-1*H*-benzimidazol-2-yl]-ethyl}-amid,
(46) 4-(2-Oxo-1,2,4,5-tetrahydro-1,3-benzodiazepin-3-yl)-piperidin-1-carbonsäure-{2-(3,4-diethyl-phenyl)-1-[1-(3-pyrrolidin-1-yl-propyl)-1*H*-benzimidazol-2-yl]-ethyl}-amid,
(47) 4-(2-Oxo-1,2,4,5-tetrahydro-1,3-benzodiazepin-3-yl)-piperidin-1-carbonsäure-{2-(3,4-diethyl-phenyl)-1-[1-(1-methyl-piperidin-4-ylmethyl)-1*H*-benzimidazol-2-yl]-ethyl}-amid,
(48) 4-(2-Oxo-1,2,4,5-tetrahydro-1,3-benzodiazepin-3-yl)-piperidin-1-carbonsäure-[2-(3,4-diethyl-phenyl)-1-(1-pyridin-3-ylmethyl-1*H*-benzimidazol-2-yl)-ethyl]-amid,
(49) 4-(2-Oxo-1,2,4,5-tetrahydro-1,3-benzodiazepin-3-yl)-piperidin-1-carbonsäure-{2-(3,5-bis-trifluormethyl-phenyl)-1-[1-(1-methyl-piperidin-4-yl)-1*H-*benzimidazol-2-yl]-ethyl}-amid,
(50) 4-(2-Oxo-1,2,4,5-tetrahydro-1,3-benzodiazepin-3-yl)-piperidin-1-carbonsäure-{2-(3,5-bis-trifluormethyl-phenyl)-1-[1-(3-pyrrolidin-1-yl-propyl)-1*H*-benzimidazol-2-yl]-ethyl}-amid,
(51) 4-(2-Oxo-1,2,4,5-tetrahydro-1,3-benzodiazepin-3-yl)-piperidin-1-carbonsäure-{2-(3,5-bis-trifluormethyl-phenyl)-1-[1-(1-methyl-piperidin-4-ylmethyl)-1*H*-benzimidazol-2-yl]-ethyl}-amid,
(52) 4-(2-Oxo-1,2,4,5-tetrahydro-1,3-benzodiazepin-3-yl)-piperidin-1-carbonsäure-{2-(3,5-bis-trifluormethyl-phenyl)-1-[1-(3-dimethylamino-propyl)-1*H*-benzimidazol-2-yl]-ethyl}-amid,
(53) 2-(2-(4-Amino-3-chlor-5-trifluormethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazole-5-carbonsäure-methylester,
(54) 2-(2-(4-Amino-3-chlor-5-trifluormethyl-phenyl)-1 -{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazole-5-carbonsäure,
(55) 4-(2-Oxo-1,2,4,5-tetrahydro-1,3-benzodiazepin-3-yl)-piperidin-1-carbonsäure-{2-(4-amino-3-chlor-5-trifluormethyl-phenyl)-1-[1-(1-methyl-piperidin-4-yl)-1*H-*benzimidazol-2-yl]-ethyl}-amid,
(56) 4-(2-Oxo-1,2,4,5-tetrahydro-1,3-benzodiazepin-3-yl)-piperidin-1-carbonsäure-{2-(4-amino-3-chlor-5-trifluormethyl-phenyl)-1-[1-(3-pyrrolidin-1-yl-propyl)-1*H-*benzimidazol-2-yl]-ethyl}-amid,
(57) 4-(2-Oxo-1,2,4,5-tetrahydro-1,3-benzodiazepin-3-yl)-piperidin-1 -carbonsäure-{2-(4-amino-3-chlor-5-trifluormethyl-phenyl)-1-[1-(1-methyl-piperidin-4-yl-methyl)-1*H*-benzimidazol-2-yl]-ethyl}-amid,
(58) 4-(2-Oxo-1,2,4,5-tetrahydro-1,3-benzodiazepin-3-yl)-piperidin-1-carbonsäure-[2-(4-amino-3-chlor-5-trifluormethyl-phenyl)-1-(1-pyridin-3-ylmethyl-1*H*-benzimidazol-2-yl)-ethyl]-amid,
(59) 4-(2-Oxo-1,2,4,5-tetrahydro-1,3-benzodiazepin-3-yl)-piperidin-1-carbonsäure-{2-(4-amino-3-chlor-5-trifluormethyl-phenyl)-1-[1-(3-dimethylamino-propyl)-1*H-*benzimidazol-2-yl]-ethyl}-amid,
(60) 3-(1-{4-(3,4-Diethyl-phenyl)-3-[1-(3-dimethylamino-propyl)-1*H*-benzimidazol-2-yl]-butyryl}-piperidin-4-yl)-1,3,4,5-tetrahydro-1,3-benzodiazepin-2-on,
(61) 3-(1-{4-(4-Amino-3-chlor-5-trifluormethyl-phenyl)-3-[1-(3-pyrrolidin-1-yl-propyl)-1*H*-benzimidazol-2-yl]-butyryl}-piperidin-4-yl)-1,3,4,5-tetrahydro-1,3-benzodiazepin-2-on,
(62) 3-(1-{4-(4-Amino-3-chlor-5-trifluormethyl-phenyl)-3-[1-(3-imidazol-1-yl-propyl)-1*H*-benzimidazol-2-yl]-butyryl}-piperidin-4-yl)-1,3,4,5-tetrahydro-1,3-benzodiazepin-2-on,
(63) 3-(1-{4-(4-Amino-3-chlor-5-trifluormethyl-phenyl)-3-[5-(3-dimethylaminopropyl)-1-ethyl-1*H*-benzimidazol-2-yl]-butyryl}-piperidin-4-yl)-1,3,4,5-tetrahydro-1,3-benzodiazepin-2-on,
(64) 3-(1-{4-(4-Amino-3-chlor-5-trifluormethyl-phenyl)-3-[1-(3-diethylamino-propyl)-1*H*-benzimidazol-2-yl]-butyryl}-piperidin-4-yl)-1,3,4,5-tetrahydro-1,3-benzodiazepin-2-on,
(65) 3-(1-{4-(4-Amino-3-chlor-5-trifluormethyl-phenyl)-3-[1-(4-hydroxy-butyl)-1*H-*benzimidazol-2-yl]-butyryl}-piperidin-4-yl)-1,3,4,5-tetrahydro-1,3-benzodiazepin-2-on,
(66) 3-(1-{4-(4-Amino-3-chlor-5-trifluormethyl-phenyl)-3-[1-(1-methyl-piperidin-3-ylmethyl)-1*H*-benzimidazol-2-yl]-butyryl}-piperidin-4-yl)-1,3,4,5-tetrahydro-1,3-benzodiazepin-2-on,
(67) 3-[1-(4-(4-Amino-3-chlor-5-trifluormethyl-phenyl)-3-{1-[2-(4-methyl-piperazin-1-yl)-ethyl]-1*H*-benzimidazol-2-yl}-butyryl)-piperidin-4-yl]-1,3,4,5-tetrahydro-1,3-benzodiazepin-2-on,
(68) 3-{1-[4-(4-Amino-3-chlor-5-trifluormethyl-phenyl)-3-(1-pyridin-4-ylmethyl-1*H-*benzimidazol-2-yl)-butyryl]-piperidin-4-yl}-1,3,4,5-tetrahydro-1,3-benzodiazepin-2-on,
(69) 3-{1-[4-(4-Amino-3-chlor-5-trifluormethyl-phenyl)-3-(1-piperidin-4-ylmethyl-1*H-*benzimidazol-2-yl)-butyryl]-piperidin-4-yl}-1,3,4,5-tetrahydro-1,3-benzodiazepin-2-on,
(70) 3-(1-{4-(4-Amino-3-chlor-5-trifluormethyl-phenyl)-3-[1-(3-dimethylamino-2,2-dimethyl-propyl)-1*H*-benzimidazol-2-yl]-butyryl}-piperidin-4-yl)-1,3,4,5-tetrahydro-1,3-benzodiazepin-2-on,
(71) 3-(1-{4-(4-Amino-3-chlor-5-trifluormethyl-phenyl)-3-[7-(3-dimethylaminopropoxy)-1*H*-benzimidazol-2-yl]-butyryl}-piperidin-4-yl)-1,3,4,5-tetrahydro-1,3-benzodiazepin-2-on,
(72) 3-{1-[4-(4-Amino-3-chlor-5-trifluormethyl-phenyl)-3-(1-piperidin-4-ylmethyl-1*H-*imidazo[4,5-c]pyridin-2-yl)-butyryl]-piperidin-4-yl}-1,3,4,5-tetrahydro-1,3-benzodiazepin-2-on,
(73) 3-(1-{4-(4-Amino-3-chlor-5-trifluormethyl-phenyl)-3-[3-(3-pyrrolidin-1-yl-propyl)-3*H*-imidazo[4,5-c]pyridin-2-yl]-butyryl}-piperidin-4-yl)-1,3,4,5-tetrahydro-1,3-benzodiazepin-2-on,
(74) 3-{1-[4-(4-Amino-3-chlor-5-trifluormethyl-phenyl)-3-(1-methyl-6-pyrrolidin-1-yl-methyl-1*H*-benzimidazol-2-yl)-butyryl]-piperidin-4-yl}-1,3,4,5-tetrahydro-1,3-berizodiazepin-2-on,
(75) Enantiomere von 3-(1-{4-(4-Amino-3-chlor-5-trifluormethyl-phenyl)-3-[1-(3-diethylamino-propyl)-1*H-*benzimidazol-2-yl]-butyryl}-piperidin-4-yl)-1,3,4,5-tetrahydro-1,3-benzodiazepin-2-on,
(76) Enantiomere von 3-[1-(4-(4-Amino-3-chlor-5-trifluormethyl-phenyl)-3-{1-[2-(4-methyl-piperazin-1-yl)-ethyl]-1*H*-benzimidazol-2-yl}-butyryl)-piperidin-4-yl]-1,3,4,5-tetrahydro-1,3-benzodiazepin-2-on,
deren Stereoisomere und Salze.

Die Verbindungen der allgemeinen Formel I werden nach prinzipiell bekannten Methoden hergestellt. Die folgenden Verfahren haben sich zur Herstellung der erfindungsgemäßen Verbindungen der allgemeinen Formel I besonders bewährt
a) Zur Herstellung von Verbindungen der allgemeinen Formel I, in der X das Sauerstoffatom und Z die -NR¹-Gruppe bedeuten, und wobei R¹, R³ und R⁴ und A wie eingangs erwähnt definiert sind
   Umsetzung von Aminen der allgemeinen Formel II, 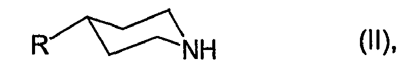 in der
   R wie eingangs erwähnt definiert ist, mit Kohlensäurederivaten der allgemeinen Formel III, 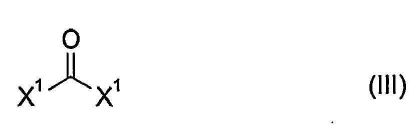 in der
   X¹ eine nucleofuge Gruppe, bevorzugt die 1*H*-Imidazol-1-yl-, 1*H*-1,2,4-Triazol-1-yl-, Trichlormethoxy- oder die 2,5-Dioxopyrrolidin-1-yloxy-gruppe, bedeutet,
   und mit Verbindungen der allgemeinen Formel IV, 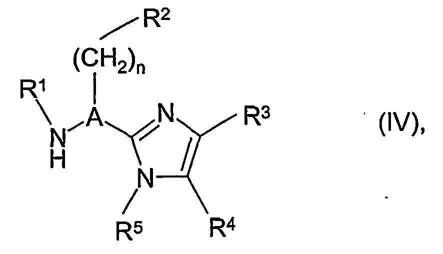 in der
   R¹, R², R³, R⁴, R⁵ und n wie eingangs erwähnt definiert sind,
   und, falls nötig, anschließende Abspaltung von Schutzgruppen oder Abwandlung von Präcursor-Funktionen nach den vorstehend beschriebenen Verfahren.
   Die im Prinzip zweistufigen Reaktionen werden in der Regel als Eintopfverfahren durchgeführt, und zwar bevorzugt in der Weise, daß man in der ersten Stufe eine der beiden Komponenten II oder IV mit äquimolaren Mengen des Kohlensäurederivats der allgemeinen Formel III in einem geeigneten Lösemittel bei tieferer Temperatur zur Reaktion bringt, anschließend wenigstens äquimolare Mengen der anderen Komponente IV oder II zugibt und die Umsetzung bei höherer Temperatur beendet. Die Umsetzungen mit Bis-(trichlormethyl)-carbonat werden bevorzugt in Gegenwart von wenigstens 2 Äquivalenten (bezogen auf Bis-(trichlormethyl)-carbonat) einer tertiären Base, beispielsweise Triethylamin, *N*-Ethyl-diisopropylamin, Pyridin, 1,5-Diazabicyclo[4,3,0]non-5-en, 1,4-Diazabicyclo[2,2,2]octan oder 1,8-Diazabicyclo[5,4,0]undec-7-en, durchgeführt. Als Lösemittel, die wasserfrei sein sollten, kommen beispielsweise Tetrahydrofuran, Dioxan, Dimethylformamid, Dimethylacetamid, *N*-Methyl-2-pyrrolidon, 1,3-Dimethyl-2-imidazolidinon oder Acetonitril in Betracht, bei Verwendung von Bis-(trichlormethyl)-carbonat als Carbonylkomponente werden wasserfreie Chlorkohlenwasserstoffe, beispielsweise Dichlormethan, 1,2-Dichlorethan oder Trichlorethylen, bevorzugt. Die Reaktionstemperaturen liegen für die erste Reaktionsstufe zwischen -30 und +25°C, bevorzugt -5 und +10°C, für die zweite Reaktionsstufe zwischen +15°C und der Siedetemperatur des verwendeten Lösemittels, bevorzugt zwischen +20°C und +70°C (Siehe auch: H. A. Staab und W. Rohr, "Synthesen mit heterocyclischen Amiden (Azoliden)", Neuere Methoden der Präparativen Organischen Chemie, Band V, S. 53 - 93, Verlag Chemie, Weinheim/Bergstr., 1967; P. Majer und R.S. Randad, J. Org. Chem. 59, 1937-1938 (1994); K. Takeda, Y. Akagi, A. Saiki, T. Sukahara und H. Ogura, Tetrahedron Letters 24 (42), 4569 - 4572 (1983)).
b) Zur Herstellung von Verbindungen der allgemeinen Formel I, in der R³ und R⁴ zusammen eine 1,3-Butadien-1,4-ylengruppe bedeuten, in der
   eine, zwei oder drei Methingruppen jeweils durch ein Stickstoffatom ersetzt sein können,
   ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Di-(C₁₋₃-alkyl)-amino-C₂₋₃-alkyl-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe oder durch eine 4- bis 7-gliedrige Cycloalkylenimino-carbonylgruppe, in der
      ein oder zwei Wasserstoffatome jeweils durch eine C₁₋₃-Alkylgruppe ersetzt sein können oder/und
      jeweils die Methylengruppe in Position 4 eines 6- oder 7-gliedrigen Cycloalkyleniminoteils durch ein Sauerstoff- oder Schwefelatom, durch eine Sulfinyl-, Sulfonyl-, -NH-, -N(C₁₋₃-Alkyl)- oder -*N*(C₁₋₃-Alkylcarbonyl)-gruppe ersetzt sein kann,
   und gegebenenfalls zusätzlich ein zweites Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy- oder Trifluormethoxygruppe ersetzt sein kann,und
   Ringkondensation eines Amids der allgemeinen Formel 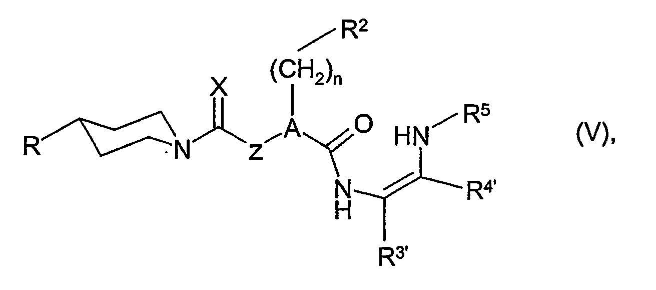 in der R, X, Z, A, n, R² und R⁵ wie eingangs erwähnt definiert sind, R^{3'} und R^{4'} zusammen eine 1,3-Butadien-1,4-ylengruppe bedeuten, in der
   eine, zwei oder drei Methingruppen jeweils durch ein Stickstoffatom ersetzt sein können,
   ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Di-(C₁₋₃-alkyl)-amino-C₂₋₃-alkyl-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe oder durch eine 4- bis 7-gliedrige Cycloalkyleniminocarbonylgruppe, in der
      ein oder zwei Wasserstoffatome jeweils durch eine C₁₋₃-Alkylgruppe ersetzt sein können oder/und
      jeweils die Methylengruppe in Position 4 eines 6- oder 7-gliedrigen Cyclo alkyleniminoteils durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)- Gruppe ersetzt sein kann,
   und gegebenenfalls zusätzlich ein zweites Wasserstoffatom durch ein Fluor-, Chlor-oder Bromatom, durch eine C₁₋₃-Alkyl-, Trifluormethyl-, Difluormethyl-, Cyclopropyl-, Hydroxy-, C₁₋₃-Alkoxy-, Difluormethoxy- oder Trifluormethoxygruppe ersetzt sein kann,und
   Die Umsetzung wird gegebenenfalls in einem Lösungsmittel oder Lösungsmittelgemisch wie Dichlormethan, Acetonitril, Dimethylformamid, Dimethylsulfoxid, Sulfolan, Benzol, Toluol, Chlorenzol, Tetrahydrofuran, Benzol/Tetrahydrofuran oder Dioxan zweckmäßigerweise in Gegenwart einer wasserfreien Säure wie Trifluoressigsäure, Methansulfonsäure, *p*-Toluolsulfonsäure oder Schwefelsäure oder in Gegenwart eines wasserentziehenden Mittels, z.B. in Gegenwart von Chlorameisensäureisobutylester, Thionylchlorid, Trimethylchlorsilan, Phosphortrichlorid, Phosphorpentoxid, *N,N'* -Dicyclohexylcarbodiimid, *N,N'*-Dicyclohexylcarbodiimid/N-Hydroxysuccinimid oder 1-Hydroxy-benztriazol, *N,N'*-Carbonyldiimidazol oder Triphenylphosphin/Tetrachlorkohlenstoff, bei Temperaturen zwischen -20 und 200°C, vorzugsweise jedoch bei Temperaturen zwischen -10 und 160°C, durchgeführt.
c) Zur Herstellung von Verbindungen der allgemeinen Formel I, in der X das Sauerstoffatom und Z die -CH₂-Gruppe darstellt:
   Kupplung einer Carbonsäure der allgemeinen Formel 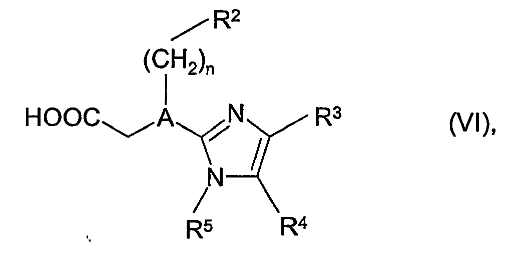 in der
   R² bis R⁵, A und n wie eingangs erwähnt definiert sind,
      mit einer Verbindung der allgemeinen Formel 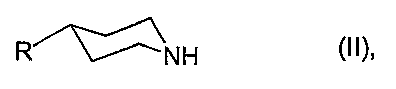 in der
   R wie eingangs erwähnt definiert ist.

   Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z. B. Dicyclohexylcarbodiimid (DCC), Diisopropylcarbodiimid (DIC) oder Ethyl-(3-dimethylaminopropyl)-carbodiimid, *O*-(1*H*-Benzotriazol-1-yl)-*N,N,N',N*'-tetramethyluroniumhexafluorphosphat (HBTU) oder -tetrafluorborat (TBTU) oder 1*H*-Benzotriazol-1-yl-oxy-tris-(dimethylamino)-phosphoniumhexafluorphosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann eine eventuelle Konfigurationsänderung gewünschtenfalls zusätzlich unterdrückt bzw. die Reaktionsgeschwindigkeit gesteigert werden. Die Kupplungen werden normalerweise mit äquimolaren Anteilen der Kupplungskomponenten sowie des Kupplungsreagenz in Lösemitteln wie Dichlormethan, Tetrahydrofuran, Acetonitril, Dimethylformamid (DMF), Dimethylacetamid (DMA), *N*-Methyl-pyrrolidon (NMP) oder Gemischen aus diesen und bei Temperaturen zwischen -30 und +30°C, bevorzugt -20 und +25°C, durchgeführt. Sofern erforderlich, wird als zusätzliche Hilfsbase *N*-Ethyl-diisopropylamin (DIEA) (Hünigsbase) bevorzugt. Als weiteres Kupplungsverfahren zur Synthese von Verbindungen der allgemeinen Formel I wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J.
   Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel II und dem Kohlensäuremonoisobutylester erhalten wird. Die Herstellung dieses gemischten Anhydrids und die Kupplung mit Aminen erfolgt im Eintopfverfahren, unter Verwendung der vorstehend genannten Lösemittel und bei Temperaturen zwischen -20 und +25°C, bevorzugt 0 und +25°C.
d) Zur Herstellung von Verbindungen der allgemeinen Formel I, in der X das Sauerstoffatom und Z die -CH₂-Gruppe darstellt:
   Kupplung einer Verbindung der allgemeinen Formel 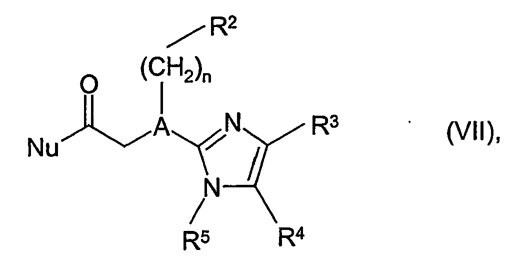 in der
   A, n, und R² bis R⁵ wie eingangs erwähnt definiert sind und Nu eine Austrittsgruppe, beispielsweise ein Halogenatom, wie das Chlor-oder Bromatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxy- oder Naphthylsulfonyloxygruppe, wobei die Substituenten gleich oder verschieden sein können, eine 1*H*-Imidazol-1-yl-, eine gegebenenfalls durch 1 oder 2 Methylgruppen im Kohlenstoffgerüst substituierte 1*H*-Pyrazol-1-yl-, 1*H*-1,2,4-Triazol-1-yl-, 1*H*-1,2,3-Triazol-1-yl- oder 1*H*-1,2,3,4-Tetrazol-1-yl-gruppe, eine Vinyl-, Propargyl-, *p*-Nitrophenyl-, 2,4-Dinitrophenyl-, Trichlorphenyl-, Pentachlorphenyl-, Pentafluorphenyl-, Pyranyl- oder Pyridinyl-, Dimethylaminyloxy-, 2(1*H*)-Oxopyridin-1-yloxy-, 2,5-Dioxopyrrolidin-1-yloxy-, Phthalimidyloxy-, 1*H*-Benzotriazol-1-yloxy- oder Azidgruppe bedeutet,
      mit einer Verbindung der allgemeinen Formel 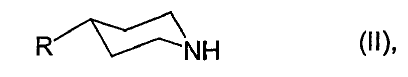 in der
   R wie eingangs erwähnt definiert ist.

   Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösemitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, *N*-Ethyl-diisopropylamin, *N*-Ethyl-dicyclohexylamin, 1,4-Diazabicyclo[2,2,2]octan oder 1,8-Diazabicyclo[5,4,0]undec-7-en, als Lösemittel beispielsweise Dichlormethan, Tetrahydrofuran, 1,4-Dioxan, Acetonitril, Dimethylformamid, Dimethylacetamid, *N*-Methyl-pyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.
e) Zur Herstellung von Verbindungen der allgemeinen Formel I, in der X das Sauerstoffatom und Z die Gruppe -NH- darstellt:
   Umsetzung von Isocyanaten der allgemeinen Formel VIII, 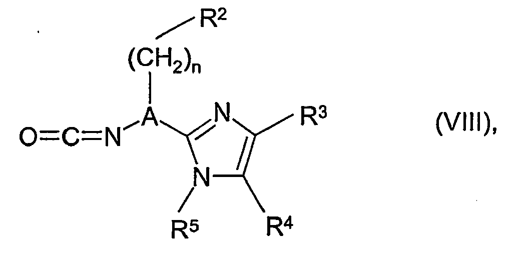 in der
   R² bis R⁵, A und n wie eingangs definiert sind
      mit Aminen der allgemeinen Formel II, 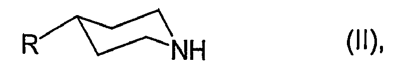 in der
   R wie eingangs erwähnt definiert ist, und, falls nötig, anschließende Abspaltung von Schutzgruppen oder Behandlung von Präcursor-Funktionen nach den oben beschriebenen Verfahren.

   Die Umsetzung wird bei Temperaturen zwischen 0°C und 150°C, bevorzugt zwischen 20°C und 100°C, und gegebenenfalls in Gegenwart wasserfreier Lösemittel, z.B. von Tetrahydrofuran, 1,4-Dioxan, Dimethylformamid, Dimethylacetamid, N-Methyl-2-pyrrolidon oder 1,3-Dimethyl-2-imidazolidinon oder Gemischen davon, durchgeführt.
f) Zur Herstellung von Verbindungen der allgemeinen Formel I, in der X eine der Gruppen =N-CN oder =N-SO₂-R⁶ und Z die Gruppe -NR¹- bedeutet, wobei R¹ und R⁶ wie eingangs erwähnt definiert sind:
   Umsetzung von Verbindungen der allgemeinen Formel 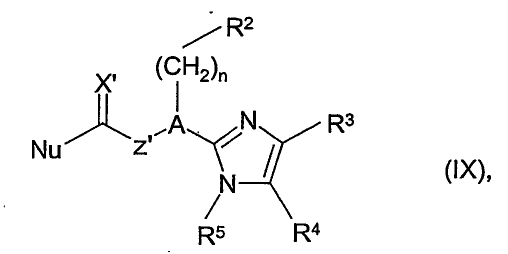 in der A, n und R² bis R⁵ wie eingangs definiert sind, X' eine der Gruppen =N-CN oder =N-SO₂-R⁶, Z' die Gruppe -NR¹-, wobei R¹ und R⁶ wie eingangs erwähnt definiert sind, bedeutet und Nu eine Austrittsgruppe ist, beispielsweise eine Alkoxy-, Aryloxy-, Alkylthio-, Alkylsulfinyl- oder Alkylsulfonylgruppe mit jeweils bis zu 10 Kohlenstoffatomen, z. B. die Methoxy-, Ethoxy-, Phenyloxy-, Methylthio-, Ethylthio-, Methylsulfinyl-, Ethylsulfinyl-, Propylsulfinyl-, Isopropylsulfinyl-, Methylsulfonyl- oder Ethylsulfonylgruppe, das Chlor- oder Bromatom, die SO₂H-, SO₃H- oder OPOCl₂-Gruppe, vorzugsweise jedoch die Phenoxygruppe,
      mit sekundären Aminen der allgemeinen Formel 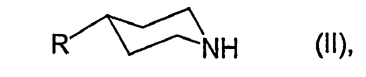 in der R wie eingangs definiert ist.

Die Umsetzungen werden in Analogie zu literaturbekannten Verfahren (siehe G. B. L. Smith, J. Amer. Chem. Soc. 51, 476 [1929]; B. Rathke, Chem. Ber. 17, 297 [1884]; R. Phillips und H. T. Clarke, J. Amer. Chem. Soc. 45, 1755 [1923]; S. J. Angyal und W. K. Warburton, J. Amer. Chem. Soc. 73, 2492 [1951]; H. Lecher und F. Graf, Chem. Ber. 56, 1326 [1923]; J. Wityak, S. J. Gould, S. J. Hein und D. A. Keszler, J. Org. Chem. 52, 2179 [1987]; T. Teraji, Y. Nakai, G. J. Durant, WO-A-81/00109, Chem. Abstr. 94, 192336z [1981]; C. A. Maryanoff, R. C. Stanzione, J. N. Plampin und J. E. Mills, J. Org. Chem. 51, 1882 - 1884 [1986]; A. E. Miller und J. J. Bischoff, Synthesis 1986, 777; R. A. B. Bannard, A. A. Casselman, W. F. Cockburn und G. M. Brown, Can. J. Chem. 36, 1541 [1958]; Aktieselskabet Grea, Kopenhagen, DE2826452-C2; K. Kim, Y. T. Lin und H. S. Mosher, Tetrah. Letters 29, 3183-3186 [1988]; H. B. Arzeno et al., Synth. Commun. 20, 3433-3437 [1990]; H. Bredereck und K. Bredereck, Chem. Ber. 94, 2278 [1961]; H. Eilingsfeld, G. Neubauer, M. Seefelder und H. Weidinger, Chem. Ber. 97, 1232 [1964]; P. Pruszynski, Can. J. Chem. 65, 626 [1987]; D. F. Gavin, W. J. Schnabel, E. Kober und M. A. Robinson, J. Org. Chem. 32, 2511 [1967]; N. K. Hart, S. R. Johns, J. A. Lamberton und R. l. Willing, Aust. J. Chem. 23, 1679 [1970]; CIBA Ltd., Belgisches Patent 655403; Chem. Abstr. 64, 17481 [1966]; J. P. Greenstein, J. Org. Chem. 2, 480 [1937]; F. L. Scott und J. Reilly, J. Amer. Chem. Soc. 74, 4562 [1952]; W. R. Roush und A. E. Walts, J. Amer. Chem. Soc. 106, 721 [1984]; M. S. Bernatowicz, Y. Wu und G. R. Matsueda, J. Org. Chem. 57, 2497-2502 [1992]; H. Tsunematsu, T. lmamura und S. Makisumi, J. Biochem. 94, 123-128 [1983]; R. Mohr, A. Buschauer und W. Schunack, Arch. Pharm. 321, 221-227 [1988]; K. Atwal, F. N. Ferrara und S. Z. Ahmed, Tetrah. Lett. 35, 8085-8088 [1994]; P. J. Garratt, C. J. Hobbs und R. Wrigglesworth, J. Org. Chem. 54, 1062-1069 [1989]; P. J. Garratt und S. N. Thorn, Tetrahedron 49, 6885-6898 [1993]) bei Temperaturen zwischen 0°C und +100°C, bevorzugt +40°C und +80°C, und unter Verwendung inerter Lösemittel, beispielsweise von Dichlormethan, Tetrahydrofuran, 1,4-Dioxan, Acetonitril, Dimethylformamid, 2-Pentanol, Dimethylacetamid, *N*-Methylpyrrolidon oder Gemischen davon und in der Regel in Gegenwart von Hilfsbasen, insbesondere von Alkalicarbonaten, wie Natrium- oder Kaliumcarbonat, oder von tertiären Aminen, bevorzugt *N*-Ethyl-diisopropylamin oder Triethylamin, durchgeführt.

Bei den vorstehend beschriebenen Umsetzungen können gegebenenfalls vorhandene reaktive Gruppen wie Hydroxy-, Carboxy-, Amino- oder Iminogruppen nach literaturbekannten Methoden während der Umsetzung durch übliche Schutzgruppen geschützt werden, welche nach der Umsetzung wieder abgespalten werden, insbesondere können die in der Peptidchemie üblichen Schutzgruppen verwendet werden. Diesbezügliche Angaben finden sich beispielsweise in der WO 98/11128.

Alternativ lassen sich Verbindungen vom Typ (I) auch wie folgt herstellen:

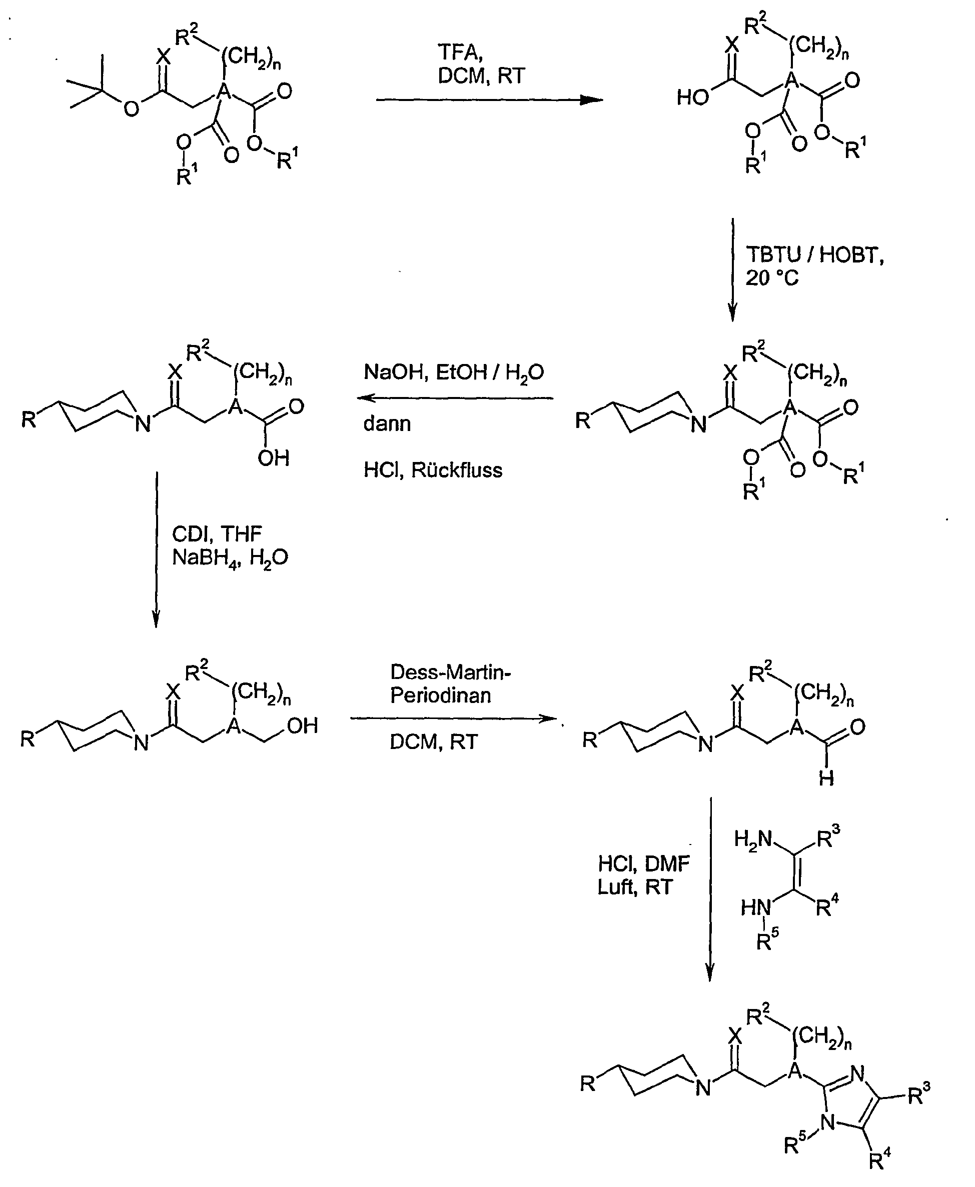

Stereoisomere Verbindungen der Formel (I) lassen sich prinzipiell nach üblichen Methoden trennen. Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel (I) fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)-Camphersulfonsäure, bzw. einer optisch aktiven Base, beispielsweise mit (R)-(+)-1-Phenyl-ethylamin, (S)-(-)-1-Phenylethylamin oder (S)-Brucin, entstehen.

Nach einem üblichen Verfahren zur Isomerentrennung wird das Racemat einer Verbindung der allgemeinen Formel (I) mit einer der vorstehend angegebenen optisch aktiven Säuren bzw. Basen in äquimolarer Menge in einem Lösungsmittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden MeOH, Ethanol oder deren Gemische, beispielsweise im Volumenverhältnis 50:50, verwendet.

Sodann wird jedes der optisch aktiven Salze in Wasser gelöst, mit einer Base, wie Natriumcarbonat oder Kaliumcarbonat, Natronlauge oder Kalilauge neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel I fallender diastereomerer Verbindungen wird auch dadurch erhalten, daß man die oben beschriebenen Synthesen mit jeweils einer geeigneten (R)- bzw. (S)-konfigurierten Reaktionskomponente durchführt.

Die Ausgangsverbindungen der allgemeinen Formeln II und III sind käuflich oder werden nach literaturbekannten Verfahren hergestellt. Ausgangsverbindungen der allgemeinen Formel (II) können entsprechend den in der WO 98/11128 , WO 00/55154 und DE 199 52 146 angegebenen Verfahren erhalten werden.

Isocyanate der allgemeinen Formel VIII lassen sich leicht aus entsprechenden α-Aminosäurederivaten bzw. aus deren Hydrochloriden durch Umsetzung mit Phosgen, Diphosgen oder Triphosgen in Gegenwart von Pyridin (siehe auch: J.S. Nowick, N.A. Powell, T.M. Nguyen und G. Noronha, J. Org. Chem. 57, 7364-7366 [1992]) herstellen.

Ausgangsverbindungen der allgemeinen Formel IV erhält man beispielsweise nach folgendem Syntheseschema, in dem A ein durch ein Wasserstoffatom oder durch eine C₁₋₃-Alkylgruppe substituiertes Kohlenstoffatom bedeutet:

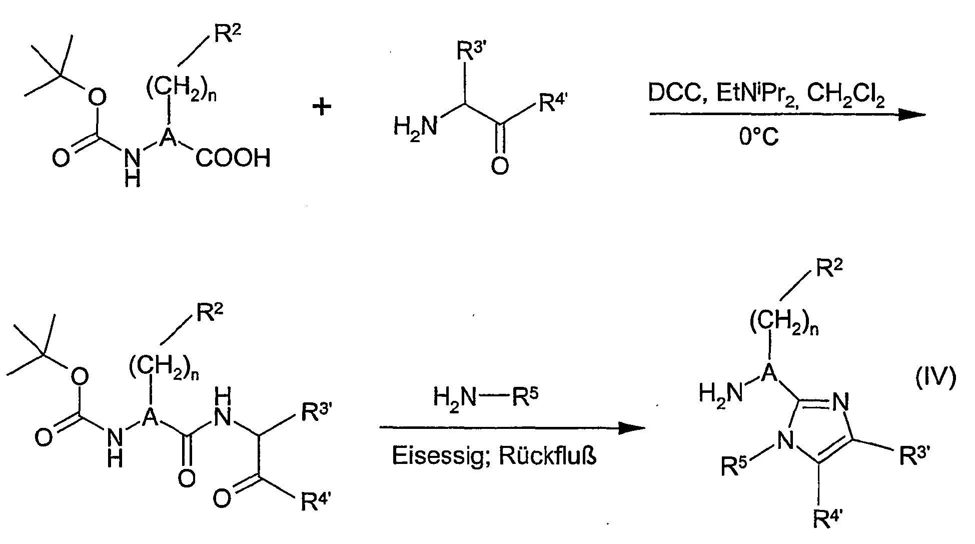

Ausgangsverbindungen der allgemeinen Formel V erhält man beispielsweise nach folgendem Syntheseschema:

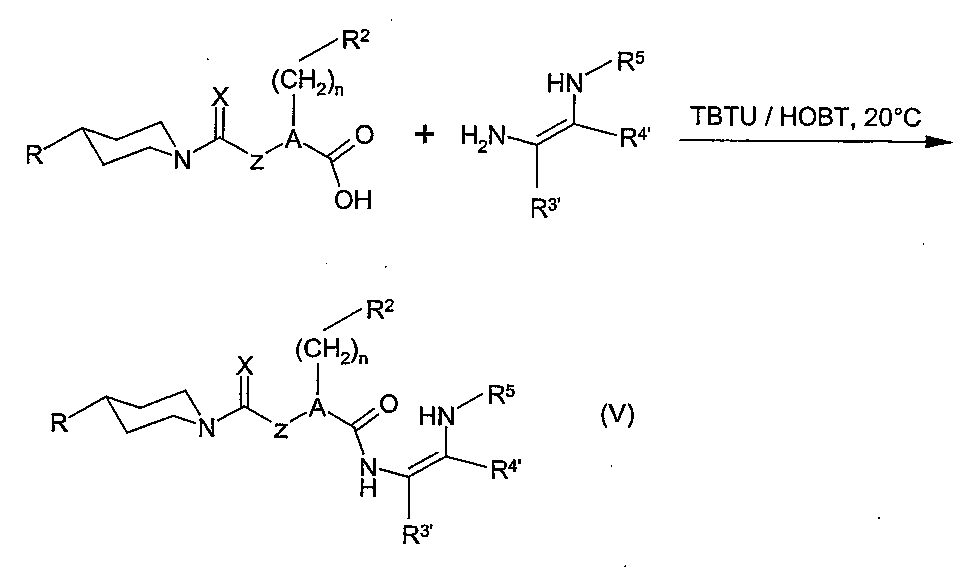

Die benötigten Ausgangsmaterialien sind entweder in WO 98/11128 beschrieben oder werden ausgehend von 1-Fluor-2-nitro-aryl- oder Heteroarylverbindungen durch Umsetzung mit entsprechenden Aminen und anschließende Reduktion der Nitro- zur Aminogruppe, gewonnen.

Verbindungen des Typ VI erhält man beispielsweise nach folgendem Syntheseschema, wobei R³ und R⁴ zusammen nicht eine 1,3-Butadien-1,4-ylengruppe ausbilden dürfen.

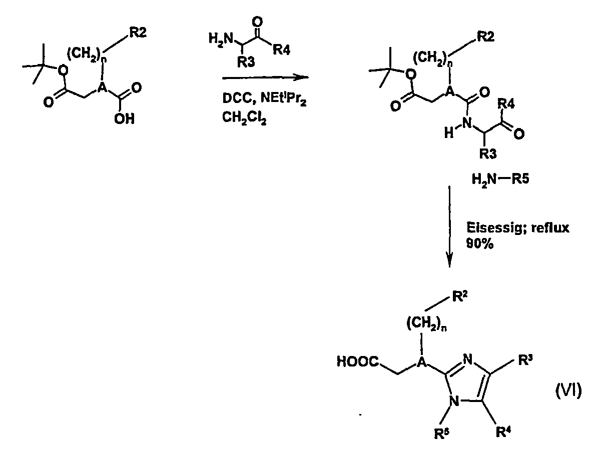

Verbindungen des Typ VII erhält man durch literaturbekannte Umsetzungen ausgehend von den entsprechenden Carbonsäuren (VI).

Verbindungen des Typ IX erhält man beispielsweise durch Umsetzung der Amine der allgemeinen Formel 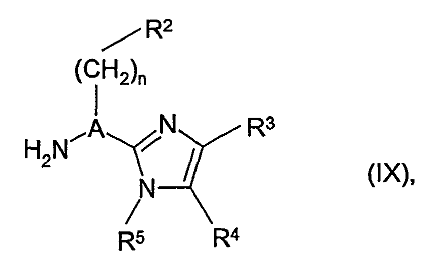 in der A, n, R² bis R⁵ wie eingangs definiert sind mit Iminocarbonaten der allgemeinen Formel

Nu-X'-Nu"

In der X' eine der oben erwähnten Gruppen =N-CN oder =N-SO₂-R⁶, Nu eine Austrittsgruppe ist beispielsweise eine Alkoxy-, Aryloxy-, Alkylthio-, Alkylsulfinyl- oder Alkylsulfonylgruppe mit jeweils bis zu 10 Kohlenstoffatomen, z.B. die Methoxy-, Ethoxy-, Phenyloxy-, Methlythio-, Methylsulfinyl-, Methylsulfonyl, das Chlor- oder Bromatom, die SO₂H-, SO₃H- oder OPOCl₂-Gruppe und Nu", das von Nu verschieden oder auch gleich wie Nu sein kann, die gleichen Bedeutungen wie Nu annehmen kann.

Die erhaltenen Verbindungen der allgemeinen Formel (1) können, sofern sie geeignete basische Funktionen enthalten, insbesondere für pharmazeutische Anwendungen in ihre physiologisch verträglichen Salze mit anorganischen oder organischen Säuren übergeführt werden. Als Säuren kommen hierfür beispielsweise Salzsäure, Bromwasserstoffsäure, Phosphorsäure, Salpetersäure, Schwefelsäure, Methansulfonsäure, Ethansulfonsäure, Benzolsulfonsäure, *p*-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Außerdem lassen sich die neuen Verbindungen der Formel (1), falls sie eine saure Funktion, beispielsweise eine Carboxygruppe enthalten, gewünschtenfalls in ihre Additionssalze mit anorganischen oder organischen Basen, insbesondere für die pharmazeutische Anwendung in ihre physiologisch verträglichen Additionssalze überführen. Als Basen kommen hierbei beispielsweise Natriumhydroxid, Kaliumhydroxid, Ammoniak, Cyclohexylamin, Dicyclohexylamin, Ethanolamin, Diethanolamin und Triethanolamin in Betracht.

Die neuen Verbindungen der allgemeinen Formel I und deren physiologisch verträglichen Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität von Verbindungen der allgemeinen Formel I zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 mL "Balanced Salts Solution" [BSS (in mM): NaCl 120, KCl 5.4, NaHCO₃ 16.2, MgSO₄ 0.8, NaHPO₄ 1.0, CaCl₂ 1.8, D-Glucose 5.5, HEPES 30, pH7.40] werden die Zellen zweimal bei 100 x g zentrifugiert und in BSS resuspendiert. Nach Bestimmung der Zellzahl werden die Zellen mit Hilfe eines Ultra-Turrax homogenisiert und 10 Minuten lang bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40), angereichert mit 1% Rinderserum-Albumin und 0.1 % Bacitracin) rezentrifugiert und resuspendiert (1 mL / 1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden 180 Minuten lang bei RT mit 50 pM ¹²⁵I-Iodtyrosyl-Calcitonine-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1 %) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität in Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die Verbindungen der allgemeinen Formel I zeigen in dem beschriebenen Test lC₅₀-Werte ≤ 10000 nM.

### B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-lsobutyl-1-methylxanthin, 1% BSA, pH 7.4) gewaschen und bei 37°C 15 Minuten lang vorinkubiert. Nach Zugabe von CGRP (10 µl) als Agonist in steigenden Konzentrationen (10⁻¹¹ bis 10⁻⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C, 15 Minuten lang) extrahiert. Die Überstände werden in flüssigem Stickstoff eingefroren und bei -20°C gelagert.

Die cAMP-Gehalte der Proben werden mittels Radioimmunassay (Fa. Amersham) bestimmt und die pA₂-Werte antagonistisch wirkender Substanzen graphisch ermittelt.

Die Verbindungen der allgemeinen Formel I zeigen in dem beschriebenen in-vitro-Testmodell CGRP-antagonistische Eigenschaften in einem Dosisbereich zwischen 10⁻¹¹ bis 10⁻⁵ M.

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne- bzw. Cluster-Kopfschmerz. Weiterhin beeinflussen die erfindungsgemäßen Verbindungen auch die folgenden Erkrankungen positiv: "Complex regional pain syndrome", nicht-insulinabhängigen Diabetes mellitus ("NIDDM"), cardiovaskuläre Erkrankungen, Morphintoleranz, Clostritiumtoxinbedingte Durchfallerkrankungen, Erkrankungen der Haut, insbesondere thermische und strahlenbedingte Hautschäden inklusive Sonnenbrand, entzündliche Erkrankungen, z.B. entzündliche Gelenkerkrankungen (Arthritis), neurogene Entzündungen der oralen Mucosa, entzündliche Lungenerkrankungen, allergische Rhinitis, Asthma, Erkrankungen, die mit einer überschießenden Gefäßerweiterung und dadurch bedingter verringerter Gewebedurchblutung einhergehen, z.B. Schock und Sepsis. Darüber hinaus zeigen die erfindungsgemäßen Verbindungen eine lindernde Wirkung auf Schmerzzustände im allgemeinen.

Die Symptomatik menopausaler, durch Gefäßerweiterung und erhöhten Blutfluß verursachter Hitzewallungen östrogendefizienter Frauen sowie hormonbehandelter Prostatakarzinompatienten wird durch die CGRP-Antagonisten der vorliegenden Anwendung präventiv und akut-therapeutisch günstig beeinflußt, wobei sich dieser Therapieansatz vor der Hormonsubstitution durch Nebenwirkungsarmut auszeichnet.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils 1 bis 3 x täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Die erfindungsgemäß hergestellten Verbindungen können entweder alleine oder gegebenenfalls in Kombination mit anderen Wirksubstanzen zur Behandlung von Migräne intravenös, subkutan, intramuskulär, intrarektal, intranasal, durch Inhalation, transdermal oder oral erfolgen, wobei zur Inhalation insbesondere Aerosolformulierungen geeignet sind. Die Kombinationen können entweder simultan oder sequentiell verabreicht werden.

Als Kombinationspartner denkbare Wirkstoffklassen sind z.B. Antiemetica, Prokinetica, Neuroleptica, Antidepressiva, Neurokinin-Antagonisten, Anticonvulsiva, Histamin-H1-Rezeptorantagonisten, Antimuscarinika, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Acclofenac, Acemetacin, Acetylsalicylsäure, Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib und Celecoxib, in Betracht.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Topiramat, Riboflavin, Montelukast, Lisinopril, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

Ein weiterer Gegenstand der Erfindung ist die Verwendung der Verbindungen der allgemeinen Formel I als wertvolle Hilfsmittel zur Erzeugung und Reinigung (Affinitätschromatographie) von Antikörpern sowie, nach geeigneter radioaktiver Markierung, beispielsweise durch direkte Markierung mit ¹²⁵I oder ¹³¹I oder durch Tritiierung geeigneter Vorstufen, beispielsweise durch Ersatz von Halogenatomen durch Tritium, in RIA- und ELISA-Assays und als diagnostische bzw. analytische Hilfsmittel in der Neurotransmitter-Forschung.

Die nachfolgenden Beispiele sollen die Erfindung näher erläutern:

### Vorbemerkungen:

Für die hergestellten Verbindungen liegen in der Regel Schmelzpunkte, IR-, UV-, ¹H-NMR und/oder Massenspekten vor. Wenn nicht anders angegeben, wurden R_{f}-Werte unter Verwendung von DC-Fertigplatten Kieselgel 60 F₂₅₄ (E. Merck, Darmstadt, Artikel-Nr. 1.05714) ohne Kammersättigung bestimmt. Die unter der Bezeichnung Alox ermittelten R_{f}-Werte wurden unter Verwendung von DC-Fertigplatten Aluminiumoxid 60 F₂₅₄ (E. Merck, Darmstadt, Artikel-Nr. 1.05713) ohne Kammersättigung bestimmt. Die bei den Fliessmitteln angegebenen Verhältnisse beziehen sich auf Volumeneinheiten der jeweiligen Lösungsmittel. Zu chromatographischen Reinigungen wurde Kieselgel der Firma Millipore (MATREX*^{™}*, 35-70 my) verwendet. Falls nähere Angaben zur Konfiguration fehlen, bleibt offen, ob es sich um reine Enantiomere handelt oder ob partielle oder gar völlige Racemisierung eingetreten ist.

In der Versuchsbeschreibung werden die folgenden Abkürzungen verwendet:
- abs.: absolutiert
- Alox: Aluminiumoxid (neutral oder basisch)
- Boc: *tert*.-Butoxycarbonyl
- CDI: *N,N'*-Carbonyldiimidazol
- CDT: *N,N*'-Carbonylditriazol
- DMF: *N,N*-Dimethylformamid
- Ether: Diethylether
- EtOAc: Essigsäureethylester
- EtOH: Ethanol
- ges.: gesättigt
- halbkonz.: halbkonzentriert
- HCl: Salzsäure
- HOAc: Essigsäure
- HOBt: 1-Hydroxybenzotriazol-hydrat
- Hünigsbase: *N,N*-Diisopropyl-ethylamin
- i. vac.: in vacuo (im Vakuum)
- KOH: Kaliumhydroxid
- konz.: konzentriert
- MeOH: Methanol
- MTBE: Methyl-*tert*.-butylether
- NaCl: Natriumchlorid
- NaOH: Natriumhydroxid
- org.: organisch
- RT: Raumtemperatur
- TBTU: 2-(1*H*-Benzotriazol-1-yl)-1,1,3,3-tetramethyluronium-Tetrafluorborat
- TFA: Trifluoressigsäure
- THF: Tetrahydrofuran

Die Benennung der Verbindungen erfolgt in Anlehnung an die englische Nomenklatur. Beispielsweise ist unter "4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(3-dimethylaminopropyl)-1*H*-benzimidazol-2-yl]-ethyl}-amid" die Verbindung der folgenden Formel zu verstehen:

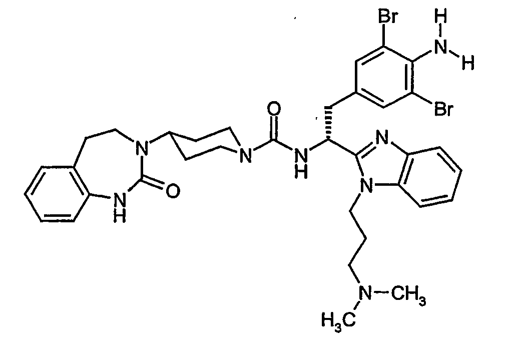

### Herstellung von Zwischenverbindungen

### Zwischenprodukt 1

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[2-(3-dimethylamino-propylamino)-phenylcarbamoyl]-ethyl}-amid

Zu der eisgekühlten Suspension aus 1.0 g (1.64 mmol) (*R*)-3-(4-Amino-3,5-dibromphenyl)-2-{[4-(2-oxo-1,2,4,5-tetrahydro-benzo[d][1,3]diazepin-3-yl)-piperidin-1-carbonyl]-amino}-propionsäure, 0.305 g (1.70 mmol), *N*-(3-Dimethylamino-propyl)-phenyl-1,2-diamin und 0.9 mL-(8.1 mmol) *N*-Methylmorpholin in 50 mL Dichlormethan wurde unter Rühren 4.8 mL Propanphosphonsäurecycloanhydrid gegeben. Die Lösung wurde anschließend 2 Stunden bei 0 °C und 16 Stunden bei RT gerührt. Das Lösemittel wurde i. vac. entfernt und das entstandene Produkt (1.0 g; Ausbeute: 78% der Theorie) roh weiter umgesetzt.

### Zwischenprodukt 2

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[2-(2-dimethylamino-ethylamino)-phenylcarbamoyl]-ethyl}-amid

Das Zwischenprodukt wurde analog zu Zwischenprodukt 1 ausgehend von (R)-3-(4-Amino-3,5-dibrom-phenyl)-2-{[4-(2-oxo-1,2,4,5-tetrahydro-benzo[d][1,3]diazepin-3-yl)-piperidin-1-carbonyl]-amino}-propionsäure und N-(2-Dimethylamino-ethyl)-benzol-1,2-diamin erhalten.

### Zwischenprodukt 3

### 4-(2-Oxo-1,2,4,5-tetrahydro-1,3-benzodiazepin-3-yl)-piperidin-1-carbonsäure-[(R)-2-(4-amino-3,5-dibrom-phenyl)-1-(2-methylamino-phenylcarbamoyl)-ethyl]-amid

Das Zwischenprodukt wurde analog zu Zwischenprodukt 1 ausgehend von (R)-3-(4-Amino-3,5-dibrom-phenyl)-2-{[4-(2-oxo-1,2,4,5-tetrahydro-benzo[d][1,3]diazepin-3-yl)-piperidin-1-carbonyl]-amino}-propionsäure und *N*-Methyl-benzol-1,2-diamin erhalten.

### Zwischenprodukt 4

### 4-(2-Oxo-1,2,4,5-tetrahydro-1,3-benzodiazepin-3-yl)-piperidin-1-carbonsäure-[(R)-2-(4-amino-3,5-dibrom-phenyl)-1-(2-amino-phenylcarbamoyl)-ethyl]-amid

Das Zwischenprodukt wurde analog zu Zwischenprodukt 1 ausgehend von (*R*)-3-(4-Amino-3,5-dibrom-phenyl)-2-{[4-(2-oxo-1,2,4,5-tetrahydro-benzo[d][1,3]diazepin-3-yl)-piperidin-1-carbonyl]-amino}-propionsäure und Benzol-1,2-diamin erhalten.

### Zwischenprodukt 5

### 4-Amino-3-((R)-3-(4-amino-3,5-dibrom-phenyl)-2-{[4-(2-oxo-1,4-dihydro-2H-chinazolin-3-yl)-piperidin-1-carbonyl]-amino}-propionylamino)-benzoesäuremethylester

Das Zwischenprodukt wurde analog zu Zwischenprodukt 1 ausgehend von (*R*)-3-(4-Amino-3,5-dibrom-phenyl)-2-{[4-(2-oxo-1,4-dihydro-2*H*-chinazolin-3-yl)-piperidin-1 - carbonyl]-amino}-propionsäure und 3,4-Diamino-benzoesäure-methylester erhalten.

### Zwischenprodukt 6

### 4-Amino-3-((R)-3-(4-amino-3,5-d ibrom-phenyl)-2-{[4-(2-oxo-1,2,4,5-tetrahydro-1,3-benzodiazepin-3-yl)-piperidin-1-carbonyl]-amino}-propionylamino)-berizoesäure-methylester

Das Zwischenprodukt wurde analog zu Zwischenprodukt 1 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und 3,4-Diaminobenzoesäure-methylester erhalten.

### Zwischenprodukt 7

### 4-(2-Oxo-1,2,4,5-tetrahydro-1,3-benzodiazepin-3-yl)-piperidin-1-carbonsäure-[(R)-2-(4-amino-3,5-dibrom-phenyl)-1-(2-butylamino-phenylcarbamoyl)-ethyl]-amid

Das Zwischenprodukt wurde analog zu Zwischenprodukt 1 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und *N*-Butyl-benzol-1,2-diamin erhalten.

### Zwischenprodukt 8

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[2-(3-pyrrolidin-1-yl-propylamino)-phenylcarbamoyl]-ethyl}-amid

Das Zwischenprodukt wurde analog zu Zwischenprodukt 1 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und *N*-(3-Pyrrolidin-1-yl-propyl)-benzol-1,2-diamin (Zwischenprodukt 15) erhalten.

### Zwischenprodukt 9

### 4-(2-Oxo-1,2,4,5-tetrahydro-1,3-benzodiazepin-3-yl)-piperidin-1-carbonsäure-((R)-2-(4-amino-3,5-dibrom-phenyl)-1-{2-[(pyridin-3-ylmethyl)-amino]-phenylcarbamoyl}-ethyl)-amid

Das Zwischenprodukt wurde analog zu Zwischenprodukt 1 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und *N*-Pyridin-3-ylmethyl-benzol-1,2-diamin (Zwischenprodukt 16) erhalten.

### Zwischenprodukt 10

### 4-(2-Oxo-1,2,4,5-tetrahydro-1,3-benzodiazepin-3-yl)-piperidin-1-carbonsäure-[(R)-2-(4-amino-3,5-dibrom-phenyl)-1-(2-benzylamino-phenylcarbamoyl)-ethyl]-amid

Das Zwischenprodukt wurde analog zu Zwischenprodukt 1 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und *N*-Benzyl-benzol-1,2-diamin erhalten.

### Zwischenprodukt 11

### 4-(2-Oxo-1,2,4,5-tetrahydro-1,3-benzodiazepin-3-yl)-piperidin-1-carbonsäure-((R)-2-(4-amino-3,5-dibrom-phenyl)-1-{2-[(1-methyl-piperidin-4-ylmethyl)-amino]-phenylcarbamoyl}-ethyl)-amid

Das Zwischenprodukt wurde analog zu Zwischenprodukt 1 ausgehend von (R)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und *N*-(1-Methyl-piperidin-4-ylmethyl)-benzol-1,2-diamin (Zwischenprodukt 17) erhalten.

### Zwischenprodukt 12

### [(R)-2-(4-Amino-3,5-dibrom-phenyl)-1-(2-oxo-2-phenyl-ethylcarbamoyl)-ethyl]-carbaminsäure-tert.-butylester

Zu einer eisgekühlten Mischung aus 1.752 g (4.0 mmol) (*R*)-3-(4-Amino-3,5-dibromphenyl)-2-*tert*.-butoxycarbonylamino-propionsäure, 0.8 g (4.7 mmol) 2-Amino-1-phenyl-ethanon-hydrochlorid und 50 mL Dichlormethan wurden 0.92 g (4.8 mmol) 1-(3-Dimethylaminpropyl)-3-ethylcarbodiimid-hydrochlorid und 0.822 mL (4.8 mmol) Hünigsbase gegeben. Die Reaktionsmischung wurde 1 Woche bei RT gerührt, mit ges. wässeriger Natriumbicarbonat-Lösung versetzt und wiederholt mit Dichlormethan extrahiert. Die vereinigten organischen Extrakte wurden mit Wasser, ges. wässeriger NaCI-Lösung gewaschen und über Magnesiumsulfat getrocknet. Nach Entfernen des Lösemittels erhielt man 2.35 g eines Feststoffes der säulenchromatographisch an Kieselgel unter Verwendung von Petrolether / EtOAc gereinigt wurde. Man erhielt 0.54 g (24% der Theorie) schwach gelbe Kristalle.
ESI-MS: (M-H)⁻ = 552 /554 / 556 (Br₂)

### Zwischenprodukt 13

### {(R)-2-(4-Amino-3,5-dibrom-phenyl)-1-[1-(3-dimethylamino-propyl)-5-phenyl-1H-imidazol-2-yl]-ethyl}-carbaminsäure-tert.-butylester

Ein Gemisch von 3.37 g (6.1 mmol) [(*R*)-2-(4-Amino-3,5-dibrom-phenyl)-1-(2-oxo-2-phenyl-ethylcarbamoyl)-ethyl]-carbaminsäure-*tert*.-butylester (Zwischenprodukt 12), 2.17 g (21.2 mmol) 3-Dimethylamino-1-propylamin und 35 mL Xylol wurde mit 3.5 mL (61.2 mmol) HOAc versetzt und 50 Minuten am Wasserabscheider unter Rückfluss erhitzt. Nach nochmaliger Zugabe von 2.17 g (21.2 mmol) 3-Dimethylamino-1-propylamin und 3.5 mL (61.2 mmol) HOAc wurde weitere 50 Minuten am Wasserabscheider unter Rückfluss erhitzt und das Reaktionsgemisch mit EtOAc und 30 mL ges. wässeriger Natriumcarbonat-Lösung versetzt. Die organische Phase wurde abgetrennt, mit ges. wässeriger NaCl-Lösung gewaschen, über Magnesiumsulfat getrocknet und i. vac. eingedampft. Der Rückstand wurde säulenchromatographisch an Kieselgel unter Verwendung von Petrolether /EtOAc (1/1 v/v) und Dichlormethan / MeOH (9/1 v/v) gereinigt. Man erhielt 0.54 g (14% der Theorie) des gewünschten Produktes.
ESI-MS: (M-H)⁻ = 619/ 621/ 623 (Br₂)

### Zwischenprodukt 14

### 3-((R)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionylamino)-4-(3-pyrrolidin-1-yl-propylamino)-benzoesäure-methylester

Zu einer Lösung von 800 mg (1.313 mmol) (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure in 70 mL DMF wurde bei RT 450 mg (1.401 mmol) TBTU und 0.245 mL (1.406 mmol) Hünigsbase zugegeben und das Gemisch 30 Minuten bei RT gerührt. 370 mg (1.334 mmol) 3-Amino-4-(3-pyrrolidin-1-yl-propylamino)-benzoesäure-methylester (Zwischenprodukt 26) wurde zugegeben und das Gemisch 16 Stunden bei RT gerührt. Das Lösungsmittel wurde i. vac. eingedampft, der Rückstand mit 15% wässeriger Kaliumcarbonat-Lösung versetzt. Der gebildete Niederschlag wurde abfiltriert und i. vac. getrocknet.
Ausbeute: 1.10 g (96% der Theorie)
R_{f} = 0.22 (Kieselgel, Dichlormethan / MeOH / Cyclohexan / konz. wässeriger Ammoniak 70 / 15 / 15 / 2 v/v/v/v)
ESI-MS: (M-H)⁻ = 867/869/871 (Br₂)

### Zwischenprodukte 15

### N-(3-Pyrrolidin-1-yl-propyl)-benzol-1,2-diamin

Zu einer Lösung von 3.700 g (28.857 mmol) 3-Pyrrolidin-1-yl-propylamin und 4.000 g (28.941 mmol) Kaliumcarbonat in 20 mL DMF wurde 1-Fluor-2-nitro-benzol zugetropft und das Gemisch 16 Stunden bei RT gerührt. Das Reaktionsgemisch wurde über basisches Alox filtriert und i. vac. eingeengt. Der Rückstand wurde in EtOAc gelöst, die org. Phase mit Wasser gewaschen, über Natriumsulfat getrocknet und i. vac eingeengt. Das Rohprodukt wurde ohne weitere Reinigung in den nachfolgenden Reaktionsschritt eingesetzt (7.080 g, quantitative Ausbeute).
Zu einer Suspension von 0.700 g Pd/C (10%) in 50 mL THF wurde das Rohprodukte zugegeben und das Gemisch bei 50 °C und 3 bar H₂-Druck 1.5 Stunden hydriert. Der Katalysator wurde abfiltriert und das Filtrat i. vac. eingeengt und unter Argon bei -20 °C gelagert.
Ausbeute: 6.08 g (99% der Theorie)
R_{f} = 0.68 (Kieselgel, Petrolether / EtOAc 4/ 1 v/v)
ESI-MS: (M+H)⁺ = 250

### Zwischenprodukt 16

### N-Pyridin-3-ylmethyl-benzol-1,2-diamin

Das Produkte wurde analog zu Zwischenprodukt 15 ausgehend von C-Pyridin-3-yl-methylamin und 1-Fluor-2-nitro-benzol erhalten. Die Hydrierung wurde mit Raney-Nickel bei 50 °C und 3 bar H₂-Druck in 4 Stunden durchgeführt.
Ausbeute: 71 % der Theorie
ESI-MS: (M+H)⁺ = 200

### Zwischenprodukt 17

### N-(1-Methyl-piperidin-4-ylmethyl)-benzol-1,2-diamin

Das Produkte wurde analog zu Zwischenprodukt 15 ausgehend von C-(1-Methylpiperidin-4-yl)-methylamin und 1-Fluor-2-nitro-benzol erhalten.
Ausbeute: 86% der Theorie
R_{f} = 0.09 (Alox, Dichlormethan / MeOH / konz. wässeriger Ammoniak 90 / 10 / 1 v/v/v)
ESI-MS: (M+H)⁺ = 220

### Zwischenprodukt 18

### N-(4-Dimethylamino-butyl)-benzol-1,2-diamin

Das Produkte wurde analog zu Zwischenprodukt 15 ausgehend von *N*-(4-Dimethyl-amino-butyl)-benzol-1,2-diamin und 1-Fluor-2-nitro-benzol erhalten.
Ausbeute: 97% der Theorie
R_{f} = 0.09 (Kieselgel, Dichlormethan / MeOH / konz. wässeriger Ammoniak 90 / 10 / 1 v/v/v)
ESI-MS: (M+H)⁺ = 208

### Zwischenprodukt 19

### N-(1-Methyl-piperidin-4-yl)-benzol-1,2-diamin

Das Produkte wurde analog zu Zwischenprodukt 15 ausgehend von 1-Methylpiperidin-4-ylamin und 1-Fluor-2-nitro-benzol erhalten.
Ausbeute: 96% der Theorie
R_{f} = 0.08 (Kieselgel, Dichlormethan / MeOH / konz. wässeriger Ammoniak 90 / 10 / 1 v/v/v)
ESI-MS: (M+H)⁺ = 206

### Zwischenprodukt 20

### N-(5-Dimethylamino-pentyl)-benzol-1,2-diamin

Das Produkte wurde analog zu Zwischenprodukt 15 ausgehend von N-(4-Dimethyl-amino-pentyl)-benzol-1,2-diamin und 1-Fluor-2-nitro-benzol erhalten.
Ausbeute: 97% der Theorie
R_{f} = 0.05 (Kieselgel, Dichlormethan / MeOH / konz. wässeriger Ammoniak 90 / 10 / 1 v/v/v)
ESI-MS: (M+H)⁺ = 222

### Zwischenprodukt 21

### 4-(2-Amino-phenylamino)-buttersäure-methylester

Das Produkte wurde analog zu Zwischenprodukt 15 ausgehend von 4-Aminobuttersäure-methylester-hydrochlorid und 1-Fluor-2-nitro-benzol erhalten.
Ausbeute: 80% der Theorie
R_{f} = 0.74 (Kieselgel, Dichlormethan / MeOH / konz. wässeriger Ammoniak 90 / 10 / 1 v/v/v)
ESI-MS: (M+H)⁺ = 209

### Zwischenprodukt 22

### 4-Chlor-N²-(3-dimethylamino-propyl)-benzol-1,2-diamin

Zu einer Lösung von 3.500 mL (27.814 mmol) *N*¹,*N*¹-Dimethyl-propan-1,3-diamin und 4.000 g (28.941 mmol) Kaliumcarbonat in 40 mL DMF wurde 4.800g (27.343 mmol) 2,5-Difluor-1-nitro-benzol zugegeben und das Gemisch 16 Stunden bei RT gerührt. Das Reaktionsgemisch wurde über basisches Alox filtriert und i. vac. eingeengt. Der Rückstand wurde in EtOAc gelöst, die org. Phase mit Wasser gewaschen, über Natriumsulfat getrocknet und i. vac eingeengt. Das Rohprodukt wurde ohne weitere Reinigung in den nachfolgenden Reaktionsschritt eingesetzt (6.900 g, 98% der Theorie).
Zu einer Suspension von 0.700 g Raney-Nickel in 50 mL THF wurde das Rohprodukte zugegeben und das Gemisch bei 50 °C und 50 psi H₂-Druck 2 Stunden hydriert. Der Katalysator wurde abfiltriert und das Filtrat i. vac. eingeengt und unter Argon bei -20°C gelagert.
Ausbeute: quantitativ
R_{f} = 0.25 (Kieselgel, Dichlormethan / MeOH / konz. wässeriger Ammoniak 90 / 10 / 1 v/v/v)
ESI-MS: (M+H)⁺ = 228/230 (Cl)

### Zwischenprodukt 23

### N²-(3-Dimethylamino-propyl)-4-fluor-benzol-1,2-diamin

Das Produkte wurde analog zu Zwischenprodukt 22 ausgehend von *N*¹,*N*¹-Dimethylpropan-1,3-diamin und 2,4-Difluor-1-nitro-benzol erhalten.
Ausbeute: 81 % der Theorie
R_{f} = 0.13 (Kieselgel, Dichlormethan / MeOH / konz. wässeriger Ammoniak 90 /10/ 1 v/v/v)
ESI-MS: (M+H)⁺ = 212 (Cl)

### Zwischenprodukt 24

### N¹-(3-Dimethylamino-propyl)-4-fluor-benzol-1,2-diamin

Das Produkte wurde analog zu Zwischenprodukt 22 ausgehend von *N*¹,*N*¹-Dimethylpropan-1,3-diamin und 2,5-Difluor-1-nitro-benzol erhalten.
Ausbeute: 97% der Theorie
R_{f} = 0.13 (Kieselgel, Dichlormethan / MeOH / konz. wässeriger Ammoniak 90/10/1 v/v/v)
ESI-MS: (M+H)⁺ = 212

### Zwischenprodukt 25

### 4,5-Dichlor-N-(3-dimethylamino-propyl)-benzol-1,2-diamin

Das Produkte wurde analog zu Zwischenprodukt 22 ausgehend von *N*¹,*N*¹-Dimethylpropan-1,3-diamin und 4,5-Dichlor-2-fluor-phenylamin erhalten.
Ausbeute: quantitativ
R_{f} = 0.21 (Kieselgel, Dichlormethan / MeOH / konz. wässeriger Ammoniak 90 / 10 / 1 v/v/v)
ESI-MS: (M+H)⁺ = 262/264/266 (Cl₂)

### Zwischenprodukt 26

### 3-Amino-4-(3-pyrrolidin-1-yl-propylamino)-benzoesäure-methylester

Das Produkte wurde analog zu Zwischenprodukt 22 ausgehend von 3-Pyrrolidin-1-yl-propylamin und 4-Fluor-3-nitro-benzoesäure-methylester erhalten.
Ausbeute: 79% der Theorie
R_{f} = 0.21 (Kieselgel, Dichlormethan / MeOH / konz. wässeriger Ammoniak 90 / 10 / 1 v/v/v)
ESI-MS: (M+H)⁺ = 278

### Zwischenprodukt 27

### 3-Chlor-N²-(3-dimethylamino-propyl)-benzol-1,2-diamin

Das Produkte wurde analog zu Zwischenprodukt 22 ausgehend von *N*¹,*N*¹-Dimethylpropan-1,3-diamin und 1-Chlor-2-fluor-3-nitro-benzol erhalten.
Ausbeute: 98% der Theorie
R_{f} = 0.29 (Kieselgel, Dichlormethan / MeOH / konz. wässeriger Ammoniak 90 / 10 / 1 v/v/v)
ESI-MS: (M+H)⁺ = 228/230 (Cl)

### Zwischenprodukt 28

### N-(3-Imidazol-1-yl-propyl)-benzol-1,2-diamin-trihydrochlorid

Das Produkte wurde analog zu Zwischenprodukt 22 ausgehend von 3-Imidazol-1-yl-propylamin und 1-Fluor-2-nitro-benzol erhalten und nach Zugabe von 20 mL 5 M HCl in Isopropanol als Trihydrochlorid-Salz isoliert.
Ausbeute: 98% der Theorie
ESI-MS: (M+H)⁺ = 217

### Zwischenprodukt 29

### 4-(3-Dimethylamino-propyl)-N¹-ethyl-benzol-1,2-diamin

Zu einer Lösung von 2.500 g (9.912 mmol) 3-(4-Acetylamino-3-nitro-phenyl)-propionsäure und 3.400 g (10.589 mmol) TBTU in 50 mL THF wurde 4.40 mL (25.007 mmol) Hünigsbase zugegeben und das Gemisch 30 Minuten bei RT gerührt. 1.000 g (12.263 mmol) Dimethylamin-hydrochlorid wurde zugegeben und das Gemisch weitere 16 Stunden bei RT gerührt. Das Reaktionsgemisch wurde i. vac. eingeengt, der Rückstand mit 15% wässeriger Kaliumcarbonat-Lösung versetzt und mit Dichlormethan erschöpfend extrahiert. Die vereinigten org. Phasen wurden über Natriumsulfat getrocknet und i. vac. eingeengt. Das Rohprodukt wurde mittels Säulenchromatographie (Kieselgel, Dichlormethan / MeOH / konz. wässeriger Ammoniak 90 / 10 / 3 v/v/v) gereinigt (2.600 g, 94% der Theorie).

Das Zwischenprodukt wurde in 50 mL THF gelöst, 10.00 mL (78.821 mmol) Trichlorsilan bei RT zugetropft und die Suspension 30 Minuten gerührt. 1.400 g (61.009 mmol) Lithiumborhydrid wurde portionenweise zugegeben und das Gemisch 1 Stunde bei RT gerührt und 2 Stunden unter Rückfluss erhitzt. 10 mL halbkonz. HCI in 40 mL Wasser wurde langsam zugetropft und das Gemisch erneut 1 Stunde unter Rückfluss erhitzt. Das Gemisch wurde 16 Stunden stehen lassen; mit EtOAc versetzt und stark gerührt. Die org. Phase wurde abgetrennt, die wässerige Phase mit konz. wässerigem Ammoniak basisch gestellt und mit EtOAc erschöpfend extrahiert. Die vereinigten org. Extrakte wurden über Magnesiumsulfat getrocknet und i. vac. eingeengt. Der Rückstand wurde mittels Säulenchromatographie (Kieselgel, Dichlormethan / MeOH / konz. wässeriger Ammoniak 90 / 10 / 3 v/v/v) gereinigt.
Ausbeute: 0.350 g (16% der Theorie)
R_{f} = 0.49 (Kieselgel, Dichlormethan / Cyclohexan / MeOH / konz. wässeriger Ammoniak 70 / 15 / 15 / 1 v/v/v/v)
EI-MS: M⁺ = 221

### Zwischenprodukt 30

### 4-(2-Amino-phenylamino)-butan-1-ol

Das Produkte wurde analog zu Zwischenprodukt 22 ausgehend von 4-Amino-butanol und 1-Fluoro-2-nitro-benzol erhalten und nach Zugabe von 20 mL 5 M HCl in Isopropanol als Dihydrochlorid-Salz isoliert.
Ausbeute: 73% der Theorie
R_{f} = 0.49 (Kieselgel, Dichlormethan / MeOH 50 / 1 v/v)
ESI-MS: (M+H)⁺ = 211

### Zwischenprodukt 31

### N-(1-Methyl-piperidin-3-ylmethyl)-benzol-1,2-diamin

Das Produkte wurde analog zu Zwischenprodukt 22 ausgehend von C-(1-Methylpiperidin-3-yl)-methylamin und 1-Fluor-2-nitro-benzol erhalten und nach Zugabe von 3 mL 5 M HCl in Isopropanol als Trihydrochlorid-Salz isoliert.
Ausbeute: 19% der Theorie
R_{f} = 0.70 (Alox, Dichlormethan / MeOH 50/ 1 v/v)
ESI-MS: (M+H)⁺ = 220

### Zwischenprodukt 32

### N-[2-(4-Methyl-piperazin-1-yl)-ethyl]-benzol-1,2-diamin

Das Produkte wurde analog zu Zwischenprodukt 22 ausgehend von 2-(4-Methylpiperazin-1-yl)-ethylamin und 1-Fluor-2-nitro-benzol erhalten und nach Zugabe von 15 mL 5 M HCl in Isopropanol als Trihydrochlorid-Salz isoliert.
Ausbeute: 72% der Theorie
ESI-MS: (M+H)⁺ = 235

### Zwischenprodukt 33

### N-Pyridin-4-ylmethyl-benzol-1,2-diamin

Zu einer Suspension von 7.000 g (50.650 mmol) Kaliumcarbonat in 80 mL DMF wurde bei RT 4.000 g (24.380 mmol) 4-Chlormethyl-pyridin-hydrochlorid und 8.000 g (44.180 mmol) Benzol-1,2-diamin zugegeben und das Gemisch 48 Stunden bei RT gerührt. Das Reaktionsgemisch wurde i. vac. eingeengt, 100 mL Wasser zugegeben und mit EtOAc erschöpfend extrahiert. Die vereinigten org. Phasen wurden über Natriumsulfat getrocknet und i. vac. eingeengt. Das Rohprodukt wurde mittels Säulenchromatographie (Kieselgel, Gradient EtOAc / MeOH 10:0 → 10:1 v/v) gereinigt. Das Produkt wurde durch Lösen in MeOH, Zugabe von 4 mL 5 M HCl in Isopropanol und Trocknen i. vac. als Trihydrochlorid-Salz isoliert.
Ausbeute: 2.320g (31% der Theorie)
ESI-MS: (M+H)⁺ = 200

### Zwischenprodukt 34

### 4-[(2-Amino-phenylamino)-methyl]-piperidin-1-carbonsäure-tert.-butylester

Das Produkte wurde analog zu Zwischenprodukt 22 ausgehend von 4-Aminomethyl-piperidin-1-carbonsäure-*tert*.-butylester und 1-Fluor-2-nitro-benzol erhalten.
Ausbeute: 88% der Theorie
R_{f} = 0.51 (Alox, Petrolether / EtOAc 1 / 1 v/v)
ESI-MS: (M+H)⁺ = 306

### Zwischenprodukt 35

### 3-{1-[4-(4-Amino-3-chlor-5-trifluormethyl-phenyl)-3-hydroxymethyl-butyryl]-piperidin-4-yl}-1,3,4,5-tetrahydro-1,3-benzodiazepin-2-on

Zu einer Lösung von 3.00 g (5.425 mmol) 2-(4-Amino-3-chlor-5-trifluormethylbenzyl)-4-oxo-4-[4-(2-oxo-1,2,4,5-tetrahydro-1,3-benzodiazepin-3-yl)-piperidin-1-yl]-buttersäure (Zwischenprodukt 68) in 100 mL THF wurde 1.054 g (6.500 mmol) CDI zugegeben und das Gemisch 2 Stunden bei RT gerührt. Diese Lösung wurde unter einer Stickstoffatmosphäre innerhalb von 5 Minuten zu einer eisgekühlten Lösung von 0.728 g (19.250 mmol) Natriumborhydrid in 50 mL Wasser zugetropft und das Gemisch 16 Stunden bei RT gerührt. Das Reaktionsgemisch wurde mit 1 N wässeriger HCl auf pH 2 angesäuert und mit EtOAc erschöpfend extrahiert. Die vereinigten org. Phasen wurden mit ges. wässeriger NaCl-Lösung gewaschen, über Natriumsulfat getrocknet und i. vac. eingeengt.
Ausbeute: 2.900 g (quantitativ)
ESI-MS: (M+H)⁺ = 539/541 (CI).

### Zwischenprodukt 36

### 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-butyraldehyd

Zu einer Lösung von 0.539 g (1.000 mmol) 3-{1-[4-(4-Amino-3-chlor-5-trifluormethylphenyl)-3-hydroxymethyl-butyryl]-piperidin-4-yl}-1,3,4,5-tetrahydro-1,3-benzodiazepin-2-on (Zwischenprodukt 35) in 10 mL Dichlormethan wurde 0.933 g (2.200 mmol) Dess-Martin-Periodinan zugegeben und das Gemisch 3 Stunden bei RT gerührt. Das Reaktionsgemisch wurde mit ges. wässeriger Natriumthiosulfat-Lösung und ges. wässeriger NaCl-Lösung gewaschen, über Natriumsulfat getrocknet und i. vac. eingeengt. Das Rohprodukt wurde mittels Säulenchromatographie (Kieselgel, Gradient Dichlormethan / MeOH 50:1 → 25:1 v/v) gereinigt.
Ausbeute: 0.320 g (60% der Theorie)
ESI-MS: (M+H)⁺ = 537/539 (Cl).

### Zwischenprodukt 37

### Dimethylamino-2,2-dimethyl-propyl)-benzol-1,2-diamin

Das Produkte wurde analog zu Zwischenprodukt 22 ausgehend von 2,2,*N*¹,*N*¹-Tetramethyl-propan-1,3-diamin und 1-Fluor-2-nitro-benzol erhalten und nach Zugabe von 25 mL 5 M HCl in Isopropanol als Trihydrochlorid-Salz isoliert.
Ausbeute: 89% der Theorie
ESI-MS: (M+H)⁺ = 222

### Zwischenprodukt 38

### 3-(3-Dimethylamino-propoxy)-benzol-1,2-diamin

Das Produkte wurde analog zu Zwischenprodukt 22 ausgehend von (3-Chlor-propyl)-dimethyl-amin und 2-Amino-3-nitro-phenol erhalten und nach Zugabe von 5 mL 5 M HCl in Isopropanol als Trihydrochlorid-Salz isoliert.
Ausbeute: 74% der Theorie
ESI-MS: (M+H)⁺ = 210

### Zwischenprodukt 39

### N-(3-Diethylamino-propyl)-benzol-1,2-diamin

Das Produkte wurde analog zu Zwischenprodukt 22 ausgehend von *N*¹,*N*¹-Diethyl-propan-1,3-diamin und 1-Fluor-2-nitro-benzol erhalten und nach Zugabe von 28 mL 5 M HCl in Isopropanol als Trihydrochlorid-Salz isoliert.
Ausbeute: 96% der Theorie
ESI-MS: (M+H)⁺ = 222

### Zwischenprodukt 40

### 4-[(3-Amino-pyridin-4-ylamino)-methyl]-piperidin-1-carbonsäure-tert.-butylester

Eine Lösung von 643 mg (3.00 mmol) 4-Aminomethyl-piperidin-1-carbonsäure-*tert*.-butylester und 471 mg (3.00 mmol) 4-Methoxy-3-nitro-pyridin in 5 mL MeOH wurde 2 Stunden unter Rückfluss erhitzt und anschließend i. vac. eingeengt. Das Rohprodukt wurde mittels Säulenchromatographie (Kieselgel, Gradient Dichlormethan / MeOH 100:0 → 19:1 v/v) gereinigt (Ausbeute: 1.010 g, quantitativ)
Zu einer Suspension von 500 mg Raney-Nickel in 30 mL MeOH wurde das Zwischenprodukt zugegeben und das Gemisch 6 Stunden bei 50 °C und 50 psi H₂-Druck hydriert. Der Katalysator wurde abfiltriert und Filtrat i. vac. eingeengt. Das Rohprodukt wurde mittels Säulenchromatographie (Alox (neutral, Aktivität II-III), Gradient Dichlormethan / MeOH 99:1 → 19:1 v/v) gereinigt.
Ausbeute: 0.480 g (53% der Theorie)
R_{f} = 0.15 (Kieselgel, Dichlormethan / MeOH 19 / 1 v/v)
ESI-MS: (M+H)⁺ = 307

### Zwischenprodukt 41

### N³-(3-Pyrrolidin-1-yl-propyl)-pyridine-3,4-diamin

Zu einer Suspension von 0.820 g (5.940 mmol) Kaliumcarbonat in 20 mL DMF wurde 1.300 g (5.940 mmol) 3-Brom-4-nitro-pyridin-1-oxid und 0.980 g (7.430 mmol) 3-Pyrrolidin-1-yl-propylamin zugegeben und das Gemisch 3 Stunden bei 90 °C gerührt. Das Lösungsmittel wurde i. vac. abgedampft und der Rückstand mittels Säulenchromatographie (Alox (neutral, Aktivität II-III), Gradient Dichlormethan / MeOH 100:0 → 95:5 v/v) gereinigt (0.950 g, 60% der Theorie).
Zu einer Suspension von 300 mg Pd/C in 10 mL EtOH wurde 0.900 g (3.380 mmol) des Zwischenprodukts zugegeben und das Gemisch 4.5 Stunden bei RT und 50 psi H₂-Druck hydriert. Der Katalysator wurde abfiltriert und das Filtrat i. vac. eingeengt. Das Produkt liegt im Gemisch mit dem entsprechenden Pyridin-*N*-oxid vor und wurde ohne weitere Reinigung in den nachfolgenden Reaktionsschritt eingesetzt.
Ausbeute: 35% der Theorie
ESI-MS: (M+H)⁺ = 221

### Zwischenprodukt 42

### N²-Methyl-4-pyrrolidin-1-ylmethyl-benzol-1,2-diamin

Zu einer Lösung von 0.700 g (3.569 mmol) 3-Methylamino-4-nitro-benzoesäure und 0.58 mL (7.000 mmol) Pyrrolidin in 40 mL THF wurde 1.156 g (3.600 mmol) TBTU zugegeben und das Gemisch 3 Stunden bei RT gerührt. Das Reaktionsgemisch wurde mit EtOAc verdünnt, die org. Phase mit halbges. wässeriger Natriumbicarbonat-Lösung gewaschen, über Magnesiumsulfat getrocknet und i. vac. eingeengt (0.700 g, 79% der Theorie).
Unter einer Stickstoffatmosphäre wurde zu einer Suspension von 76 mg (2.000 mmol) Lithiumaluminiumhydrid in 15 mL THF wurde eine Lösung des Zwischenprodukts in 15 mL THF langsam zugetropft und das Gemisch 16 Stunden unter Rückfluss erhitzt. Nach dem Abkühlen wurden erneut 35 mg Lithiumaluminiumhydrid zugegeben und das Gemisch weitere 8 Stunden unter Rückfluss erhitzt. Überschüssiges Lithiumaluminiumhydrid wurde nach der Fieser-Methode mit Wasser und wässeriger NaOH-Lösung zersetzt und abfiltriert. Das Filtrat wurde mit EtOAc verdünnt, die org. Phase mit ges. wässeriger NaCl-Lösung gewaschen, über Magnesiumsulfat getrocknet und i. vac. eingeengt. Das Rohprodukt wurde mittels Säulenchromatographie (Kieselgel, Gradient Dichlormethan / MeOH / ges. wässeriger Ammoniak 100:0:0 → 90:10:1 v/v/v) gereinigt.
Ausbeute: 60 mg (21 % der Theorie)
R_{f} = 0.20 (Kieselgel, Dichlormethan / MeOH / ges. wässeriger Ammoniak 90 / 10 / 1 v/v/v)
ESI-MS: (M+H)⁺ = 206

### Zwischenprodukt 43

### 2-Amino-3-(3,4-dibrom-phenyl)-propionsäure-ethylester

Zu einer Suspension von 14.00 g (101.30 mmol) Kaliumcarbonat in 300 mL Acetonitril und 2.7 mL Wasser wurde 10.69 g (40.00 mmol) (Benzhydryliden-amino)-essigsäure-ethylester, 1.87 g (5.80 mmol) Tetrabutylammoniumbromid und 20.00 g (45.12 mmol) 1,2-Dibrom-4-brommethyl-benzol zugegeben und das Gemisch 20 Stunden unter Rückfluss erhitzt. Das Reaktionsgemisch wurde abgekühlt, filtriert und das Filtrat i. vac. eingeengt. Der Rückstand wurde in 120 mL Ether aufgenommen, mit halbkonz. wässeriger HCl versetzt und das Gemisch 1 Stunden stark gerührt. Die etherische Phase wurde abgetrennt und die wässerige Phase mit Ether gewaschen. Die Wasserphase wurde mit ges. wässeriger Natriumbicarbonat-Lösung neutralisiert und mit EtOAc erschöpfend extrahiert. Die vereinigten org. Extrakte wurden über Natriumsulfat getrocknet und i. vac. eingeengt. Das Rohprodukt wurde mittels Säulenchromatographie (Kieselgel, EtOAc) gereinigt.
Ausbeute: 8.27 g (59% der Theorie)
R_{f} = 0.59 (Kieselgel, EtOAc)

### Zwischenprodukt 44

### 3-(3,4-Dibrom-phenyl)-2-[4-(2-oxo-1,2,4,5-tetrahydro-1,3-benzodiazepin-3-yl)-piperidin-1-yl]-propionsäure-ethylester

Zu einer Lösung von 4.20 g (24.31 mmol) CDT in 50 mL DMF wurde bei 0 °C eine Lösung von 8.27 g (23.56 mmol) 2-Amino-3-(3,4-dibrom-phenyl)-propionsäure-ethylester (Zwischenprodukt 43) in 30 mL DMF langsam zugetropft und das Gemisch 30 Minuten bei 0 °C und 30 Minuten bei RT gerührt. 5.90 g (24.05 mmol) 3-Piperidin-4-yl-1,3,4,5-tetrahydro-1,3-benzodiazepin-2-on wurde zugegeben und das Gemisch 16 Stunden bei RT gerührt. Das Reaktionsgemisch wurde auf 250 mL Eiswasser gegossen, 150 mL EtOAc zugegeben und 1 Stunde stark gerührt. Der gebildete Niederschlag wurde abfiltriert und an Luft getrocknet.
Ausbeute: 10.20 g (68% der Theorie)
R_{f} = 0.45 (Kieselgel, EtOAc)

### Zwischenprodukt 45

### 3-(3,4-Dibrom-phenyl)-2-[4-(2-oxo-1,2,4,5-tetrahydro-1,3-benzodiazepin-3-yl)-piperidin-1-yl]-propionsäure

Zu einer Lösung von 11.62 g (18.67 mmol) 3-(3,4-Dibrom-phenyl)-2-[4-(2-oxo-1,2,4,5-tetrahydro-1,3-benzodiazepin-3-yl)-piperidin-1-yl]-propionsäure-ethylester (Zwischenprodukt 44) in 100 mL EtOH wurde eine Lösung von 5.00 g (125.00 mmol) Natriumhydroxid in 50 mL Wasser zugegeben und das Gemisch 1 Stunde unter Rückfluss erhitzt. Ethanol wurde i. vac. abgedampft und der Rückstand mit 50 mL Wasser verdünnt. 65 mL 2 M wässrige HCl wurde langsam zugetropft, der gebildete Niederschlag abfiltriert und i. vac. getrocknet.
Ausbeute: 11.09 g (quantitative Ausbeute)
ESI-MS: (M-H)⁻ = 591/593/595 (Br₂)

### Zwischenprodukt 46

### 2-Acetylamino-2-(3,4-diethyl-benzyl)-malonsäurediethylester

Unter Stickstoffatmosphäre wurden zu 200 mL abs. EtOH portionenweise 8.14 g (354 mmol) Natrium gegeben und bis zum vollständigen Auflösen nachgerührt. Zu dieser Lösung wurden dann 76.9 g (354 mmol) 2-Acetylaminomalonsäurediethylester zugegeben, wobei das gebildete Natriumsalz ausfiel. Nach Zugabe von 150 mL 1,4-Dioxan wurde zu dieser Suspension eine Lösung von 80 g (352 mmol) 4-Brommethyl-1,2-diethyl-benzol in 500 mL 1,4-Dioxan zugetropft. Die Reaktionslösung wurde 2 Stunden bei 50°C gehalten und anschließend 16 Stunden bei RT nachgerührt. Das Lösungsmittel wurde i. vac. abdestilliert, der ölige Rückstand mit Wasser versetzt, wobei das Produkt in Form weißer Kristalle anfiel. Diese wurden abgesaugt, mit Wasser gewaschen und ohne weitere Reinigung umgesetzt.
R_{f} = 0.35 (Kieselgel, Petrolether / EtOAc 2 / 1 v/v)

### Zwischenprodukt 47

### 2-Amino-3-(3,4-diethyl-phenyl)-propionsäure

Das rohe Zwischenprodukt 46 wurde in 250 mL AcOH gelöst und mit 250 mL konz. wässeriger HCl und 150 mL Wasser versetzt. Die Reaktionslösung wurde 3 Stunden unter Rückfluss erhitzt, die Lösungsmittel i. vac. eingedampft, der Rückstand in EtOH aufgenommen, der entstandene Niederschlag abgesaugt und mit Diethylether nachgewaschen.
Ausbeute: 45 g (57% der Theorie)
R_{f} = 0.35 (Kieselgel, EtOAc / MeOH / AcOH 90 / 10 / 3 v/v/v)
ESI-MS: (M+H)⁺ = 222

### Zwischenprodukt 48

### 2-Amino-3-(3,4-diethyl-phenyl)-propionsäuremethylester

41 g (159 mmol) 2-Amino-3-(3,4-diethyl-phenyl)-propionsäure (Zwischenprodukt 47) wurden mit 300 mL HCl-ges. MeOH versetzt und 16 Stunden bei RT stehen gelassen, wobei das gewünschte Hydrochlorid ausfiel. Man erwärmte auf 50°C, wobei HCl entwich und das Produkt wieder in Lösung ging. Die Lösung i. vac. auf 1/3 des ursprünglichen Volumens eingeengt, das ausgefallene Produkt mit Ether verrührt, abgesaugt und zweimal mit Ether nachgewaschen. Das Rohprodukt wurde ohne weitere Reinigung umgesetzt.
Ausbeute: 42 g (97% der Theorie)
R_{f} = 0.7 (Kieselgel, MeOH)
ESI-MS: (M+H)⁺ = 236

### Zwischenprodukt 49

### 3-(3,4-Diethyl-phenyl)-2-{[4-(2-oxo-1,2,4,5-tetrahydro-1,3-benzodlazepin-3-yl)-piperidin-1-carbonyl]-amino}-propionsäure-methylester

Zu einer auf 0 °C gekühlten Lösung von 10.5 g (44.6 mmol) 2-Amino-3-(3,4-diethylphenyl)-propionsäure-methylester (Zwischenprodukt 48) in 250 mL THF wurden 7.4 g (45 mmol) CDT gegeben und weitere 30 Minuten bei dieser Temperatur gerührt. Anschließend erfolgte die Zugabe von 10.9 g (44.6 mmol) 3-Piperidin-4-yl-1,3,4,5-tetrahydro-1,3-benzodiazepin-2-on, wobei die Reaktionslösung weitere 20 Minuten bei dieser Temperatur gehalten wurde, ehe für 30 Minuten unter Rückfluss erhitzt wurde. Das Lösungsmittel wurde im Vakuum entfernt, der Rückstand in ges. Natriumbicarbonat-Lösung aufgenommen, erschöpfend mit Ether / EtOAc (1:1) extrahiert und über Magnesiumsulfat getrocknet. Nach Entfernen des Trocken- und Lösungsmittels wurde das Rohprodukt ohne weitere Reinigung umgesetzt.
Ausbeute: quantitativ
R_{f} = 0.6 (Kieselgel, EtOAc / Petrolether 6 / 4 v/v)

### Zwischenprodukt 50

### 3-(3,4-Diethyl-phenyl)-2-{[4-(2-oxo-1,2,4,5-tetrahydro-1,3-benzodiazepin-3-yl)-piperidin-1-carbonyl]-amino}-propionsäure

26 g des rohen Zwischenprodukts 49 wurden in 200 mL EtOH gelöst, mit 2.3 g (55 mmol) Lithiumhydroxid versetzt und 16. Stunden gerührt. Die Reaktionslösung wurde i. vac. eingedampft, der Rückstand mit Wasser aufgenommen, mit Ether extrahiert, mit 15% wässeriger Zitronensäure-Lösung angesäuert und mit EtOAc erschöpfend extrahiert. Die vereinigten org. Phasen wurden über Magnesiumsulfat getrocknet und nach Entfernen des Trocken- und Lösungsmittels ohne weitere Reinigung umgesetzt.
Ausbeute: 19 g (75% der Theorie)
R_{f} = 0.1 (Kieselgel, EtOAc / Petrolether 6 / 4 v/v)

### Zwischenprodukt 51

### 2-Amino-3-(3,5-bis-trifluormethyl-phenyl)-propionsäure-ethylester

Das Zwischenprodukt wurde analog zu Zwischenprodukt 43 ausgehend von (Benzhydryliden-amino)-essigsäure-ethylester und 1-Brommethyl-3,5-bis-trifluormethyl-benzol als Hydrochlorid-Salz erhalten.
Ausbeute: 91 % der Theorie
ESI-MS: (M+H)⁺ = 330

### Zwischenprodukt 52

### 3-(3,5-Bis-trifluormethyl-phenyl)-2-{[4-(2-oxo-1,2,4,5-tetrahydro-1,3-benzodiazepin-3-yl)-piperidine-1-carbonyl]-amino}-propionsäure-ethylester

Das Zwischenprodukt wurde analog zu Zwischenprodukt 44 ausgehend von 2-Amino-3-(3,5-bis-trifluormethyl-phenyl)-propionsäure-ethylester (Zwischenprodukt 51), 1 Äquivalent Hünigsbase und 3-Piperidin-4-yl-1,3,4,5-tetrahydro-1,3-benzodiazepin-2-on erhalten. Das Rohprodukt wurde mittels Säulenchromatographie (Kieselgel, EtOAc / Cyclohexan 4/ 1 v/v) gereinigt.
Ausbeute: 19% der Theorie
R_{f} = 0.63 (Kieselgel, EtOAc)
ESI-MS: (M+H)⁺ = 601

### Zwischenprodukt 53

### 3-(3,5-Bis-trifluormethyl-phenyl)-2-{[4-(2-oxo-1,2,4,5-tetrahydro-1,3-benzodiazepin-3-yl)-piperidine-1-carbonyl]-amino}-propionsäure

Das Zwischenprodukt wurde analog zu Zwischenprodukt 45 ausgehend von 3-(3,5-Bis-trifluormethyl-phenyl)-2-{[4-(2-oxo-1,2,4,5-tetrahydro-1,3-benzodiazepin-3-yl)-piperidine-1-carbonyl]-amino}-propionsäure-ethylester (Zwischenprodukt 52) erhalten.
Ausbeute: quantitativ
ESI-MS: (M+H)⁺ = 573

### Zwischenprodukt 54

### (4-Amino-3-chlor-5-trifluormethyl-phenyl)-methanol

Zu einer Lösung von 10.0 g (41.7 mmol) 4-Amino-3-chlor-5-trifluormethyl-benzoesäure (Arzneim. Forsch. 1984, 1612-1624.) in 100 mL THF wurde 7.13 g (44.0 mmol) CDI zugegeben und die Mischung bis zum Ende der Gasentwicklung bei 40 °C gerührt. Unter Kühlung wurde 4.64 g (120 mmol) Natriumborhydrid in 300 mL Wasser zugegeben und die Mischung anschließend 2 Stunden bei RT gerührt. Das Reaktionsgemisch wurde mit 10% wässriger HCI angesäuert, 1 Stunde gerührt, mit ges. wässriger Natriumbicarbonat-Lösung neutralisiert und mit EtOAc erschöpfend extrahiert. Die vereinigten org. Extrakte wurden mit ges. wässriger Natriumbicarbonat-Lösung gewaschen, über Natriumsulfat getrocknet und i. vac. eingeengt. Säulenchromatographie (Kieselgel, Gradient Dichlormethan / MeOH / konz. wässriger Ammoniak 100/0/0 → 87/10/3 v/v/v) ergab das Produkt.
Ausbeute: 5.4 g (57% der Theorie)

### Zwischenprodukt 55

### 2-Chlor-4-chlormethyl-6-trifluormethyl-phenylamin

Zu einer Lösung von 1.00 g (4.43 mmol) (4-Amino-3-chlor-5-trifluormethyl-phenyl)-methanol (Zwischenprodukt 54) in 50 mL Dichlormethan wurde bei RT 0.94 mL (13.00 mmol) Thionylchlorid zugegeben und das Gemisch 3 Stunden bei RT gerührt. Das Reaktionsgemisch wurde auf Eis gegossen und die wässrige Phase mit Dichlormethan erschöpfen extrahiert. Die vereinigten org. Phasen wurden mit eiskalter Natriumbicarbonat-Lösung gewaschen, über Natriumsulfat getrocknet, über Aktivkohle filtriert und i. vac. eingeengt. Das Rohprodukt wurde ohne weitere Reinigung im nachfolgenden Reaktionsschritt eingesetzt.
Ausbeute: 1.08 g (quantitative Ausbeute)
EI-MS: M⁺ = 243/245/247 (Cl₂)
R_{f} = 0.81 (Kieselgel, Petrolether / EtOAc 2/1 v/v/)

### Zwischenprodukt 56

### 2-Acetylamino-2-(4-amino-3-chlor-5-trifluormethyl-benzyl)-malonsäure-diethylester

Zu einer frisch zubereiteten Lösung von 2.55 g (0.11 mol) Natrium in 200 mL abs. EtOH wurde unter Stickstoffatmosphäre 24.11 g (0.11 mol) 2-Acetylaminomalonsäure-diethylester zugegeben und das Gemisch 15 Minuten bei RT gerührt. Eine Lösung von 27.00 g (0.11 mol) 2-Chlor-4-chlormethyl-6-trifluormethyl-phenylamin (Zwischenprodukt 55) in 100 mL 1,4-Dioxan wurde schnell zugetropft und das Gemisch 4 Stunden bei RT gerührt. 500 mL Wasser wurden zugegeben und das Gemisch weitere 16 Stunden gerührt. Der gebildete Niederschlag wurde abfiltriert, mit Wasser gewaschen und i. vac. getrocknet.
Ausbeute: 40.0 g (84% der Theorie)
R_{f} = 0.14 (Kieselgel, Petrolether / EtOAc 2 / 1 v/v)

### Zwischenprodukt 57

### 2-Amino-3-(4-amino-3-chlor-5-trifluormethyl-phenyl)-propionsäure-hydrochlorid

Zu einer Lösung von 40.0 g (94.16 mmol) 2-Acetylamino-2-(4-amino-3-chlor-5-trifluormethyl-benzyl)-malonsäure-diethylester (Zwischenprodukt 56) in 110 mL AcOH und 150 mL Wasser wurde 50 mL konz. wässrige HCl zugegeben und das Reaktionsgemisch wurde 4 Stunden auf 140 °C erhitzt. Der gebildete Niederschlag wurde abfiltriert und verworfen. Das Filtrat wurde i. vac. eingeengt, mit 100 mL EtOH versetzt und 15 Minuten bei RT gerührt. Der gebildete Niederschlag wurde abfiltriert, mit EtOH gewaschen und i. vac. getrocknet. Das Rohprodukt wurde ohne weitere Reinigung im nachfolgenden Reaktionsschritt eingesetzt.
Ausbeute: 16 g (53% der Theorie)
ESI-MS: (M-H)⁻ = 281 / 283 (Cl)

### Zwischenprodukt 58

### 2-Amino-3-(4-amino-3-chlor-5-trifluormethyl-phenyl)-propionsäure-ethylester

16 g (50.14 mmol) 2-Amino-3-(4-amino-3-chlor-5-trifluormethyl-phenyl)-propionsäure-hydrochlorid (Zwischenprodukt 57) wurden in 350 mL HCl (12 M in EtOH) gelöst und 5 Stunden bei RT gerührt. Das Reaktionsgemisch wurde i. vac. auf 100 mL eingeengt und mit 200 mL Ether versetzt. Der gebildete Niederschlag wurde abfiltriert, mit Ether gewaschen und i. vac. getrocknet.
Ausbeute: 12.2 g (70% der Theorie)
ESI-MS: (M+H)⁺ = 311/313 (Cl)

### Zwischenprodukt 59

### 3-(4-Amino-3-chlor-5-trifluormethyl-phenyl)-2-{[4-(2-oxo-1,2,4,5-tetrahydro-1,3-benzodiazepin-3-yl)-piperidin-1-carbonyl]-amino}-propionsäure-ethylester

Zu einer Suspension von 8.00 g (23.04 mmol) 2-Amino-3-(4-amino-3-chlor-5-trifluormethyl-phenyl)-propionsäure-ethylester (Zwischenprodukt 58) und 16.0 mL (115.00 mmol) Triethylamin in 100 mL DMF wurde bei 0 °C 4.15 g (23.04 mmol) CDT zugegeben und das Gemisch wurde 1.5 Stunden bei 0 °C gerührt. Eine Lösung von 5.64 g (23.00 mmol) 3-Piperidin-4-yl-1,3,4,5-tetrahydro-1,3-benzodiazepin-2-on in 200 mL DMF wurde zugegeben und das Gemisch 2 Stunden auf 100 °C erhitzt. Das Reaktionsgemisch wurde auf RT gekühlt, mit 1.5 L Wasser verdünnt und weitere 10 Minuten gerührt. Der gebildete Niederschlag wurde abfiltriert, mit Wasser gewaschen und i. vac. getrocknet.
Ausbeute: 13.0 g (97% der Theorie)
ESI-MS: (M+H)⁺ = 582 / 584 (Cl)

### Zwischenprodukt 60

### 3-(4-Amino-3-chlor-5-trifluormethyl-phenyl)-2-{[4-(2-oxo-1,2,4,5-tetrahydro-1,3-benzodiazepin-3-yl)-piperidin-1-carbonyl]-amino}-propionsäure

Zu einer Lösung von 13.00 g (22.34 mmol) 3-(4-Amino-3-chlor-5-trifluormethylphenyl)-2-{[4-(2-oxo-1,2,4,5-tetrahydro-1,3-benzodiazepin-3-yl)-piperidin-1-carbonyl]-amino}-propionsäure-ethylester (Zwischenprodukt 59) in 100 mL EtOH wurden 45 mL wässrige NaOH (1 M) zugegeben und das Gemisch 16 Stunden bei RT gerührt. EtOH wurde i. vac. abgedampft, 45 mL wässrige HCl (1M) zugegeben und 15 Minuten gerührt. Der gebildete Niederschlag wurde abfiltriert, mit Wasser gewaschen und i. vac. bei 75 °C getrocknet.
Ausbeute: 10.5 g (85% der Theorie)
ESI-MS: (M-H)⁻ = 552 / 554 (Cl)

### Zwischenprodukt 61

### 4-(3,4-Diethyl-phenyl)-3,3-bis-ethoxycarbonyl-buttersäure-tert.-butylester

Zu einer Lösung von 14.70 g (53.59 mmol) 3-Ethoxycarbonyl-bernsteinsäure-1-tert.-butylester-4-ethylester in 100 mL abs. THF wurde unter Stickstoffatmosphäre und

Eiskühlung portionsweise 2.40 g (55.00 mmol) NaH (55% in Mineralöl) zugegeben und die Mischung 1 Stunde bei RT gerührt. 11.00 g (48.43 mmol) 4-Brommethyl-1,2-diethyl-benzol, gelöst in 50 mL abs. THF, wurde zugetropft und das Gemisch 3 Stunden bei RT gerührt. Das Reaktionsgemisch wurde filtriert und das Filtrat i. vac. eingeengt. Der Rückstand wurde mit Wasser versetzt und die wässrige Phase mit EtOAc erschöpfend extrahiert. Die org. Phase wurde mit 1 M wässeriger Kaliumhydrogensulfat-Lösung und Wasser gewaschen, über MgSO₄ getrocknet und i. vac. eingeengt. Das Rohprodukt wurde ohne weitere Reinigung im nachfolgenden Reaktionsschritt eingesetzt.
Ausbeute: 20.30 g (quantitative Ausbeute)
ESI-MS: (M+Na)⁺ = 423
R_{f} = 0.48 (Fliessmittel: Petrolether / EtOAc 4 / 1 v/v)

### Zwischenprodukt 62

### 4-(3,4-Diethyl-phenyl)-3,3-bis-ethoxycarbonyl-buttersäure

Zu einer Lösung von 20.30 g (48.27 mmol) 4-(3,4-Diethyl-phenyl)-3,3-bis-ethoxycarbonyl-buttersäure-*tert*.-butylester (Zwischenprodukt 61) in 100 mL CH₂Cl₂ wurde unter Eiskühlung 40 mL TFA zugegeben und die Mischung 1 Stunde unter Rückfluss erhitzt. Das Reaktionsgemisch wurde i. vac. eingeengt, der Rückstand mit Wasser versetzt und die wässerige Phase mit EtOAc erschöpfend extrahiert. Die vereinigten org. Extrakte wurden mit ges. wässeriger NaCl-Lösung gewaschen, über Natriumsulfat getrocknet und i. vac. eingeengt.
Ausbeute: 17.10 g (97% der Theorie)
ESI-MS: (M-H)⁻ = 363

### Zwischenprodukt 63

### 2-(3,4-Diethyl-benzyl)-2-{2-oxo-2-[4-(2-oxo-1,2,4,5-tetrahydro-1,3-benzodiazepin-3-yl)-piperidin-1-yl]-ethyl}-malonsäure-diethylester

Zu einer Lösung von 17.00 g (46.65 mmol) 4-(3,4-Diethyl-phenyl)-3,3-bis-ethoxycarbonyl-buttersäure (Zwischenprodukt 62), 12.00 g (48.91 mmol) 3-Piperidin-4-yl-1,3,4,5-tetrahydro-1,3-benzodiazepin-2-on, 16.00 g (49.84 mmol) TBTU und 6.50 g (47.14 mmol) HOBT in 300 mL THF wurde 10.00 mL (56.83 mmol) Hünigsbase zugetropft und die Mischung 5 Stunde bei RT gerührt. Das Reaktionsgemisch wurde i. vac. eingeengt, der Rückstand mit ges. wässeriger Natriumbicarbonat-Lösung versetzt und die wässerige Phase mit Dichlormethan erschöpfend extrahiert. Die vereinigten org. Extrakte wurden mit Wasser gewaschen, über Natriumsulfat getrocknet und i. vac. eingeengt.
Ausbeute: 14.00 g (51 % der Theorie)
ESI-MS: (M+H)⁺ = 592

### Zwischenprodukt 64

Zu einer Lösung von 14.00 g (23.66 mmol) 2-(3,4-Diethyl-benzyl)-2-{2-oxo-2-[4-(2-oxo-1,2,4,5-tetrahydro-1,3-benzodiazepin-3-yl)-piperidin-1-yl]-ethyl}-malonsäurediethylester (Zwischenprodukt 63) in 150 mL EtOH wurde 6.30 g (95.62 mmol) KOH, gelöst in 100 mL Wasser, zugegeben und das Gemisch 16 Stunden unter Rückfluss erhitzt. EtOH wurde i. vac. abgedampft, das Reaktionsgemisch mit konz. wässriger HCl auf pH 4 angesäuert und 1 Stunde bei RT. Der gebildete Niederschlag wurde abfiltriert, mit Wasser und Diisopropylether gewaschen und i. vac. getrocknet.
Ausbeute: 10.90 g (94% der Theorie)
ESI-MS: (M+H)⁺ = 492

### Zwischenprodukt 65

### 4-(4-Amino-3-chlor-5-trifluormethyl-phenyl)-3,3-bis-ethoxycarbonyl-buttersäure-tert.-butylester

Zu einer Lösung von 1.20 g (4.43 mmol) 3-Ethoxycarbonyl-bernsteinsäure-1-*tert*.-butylester-4-ethylester in 50 mL abs. THF wurde unter Stickstoffatmosphäre und Eiskühlung portionsweise 193 mg (4.43 mmol) NaH (55% in Mineralöl) zugegeben und die Mischung 1 Stunde bei RT gerührt. 1.1 g (4.43 mmol) 2-Chlor-4-chlormethyl-6-trifluormethyl-phenylamin, gelöst in 10 mL abs. THF, wurde zugetropft und das Gemisch 16 Stunden bei RT gerührt. Das Reaktionsgemisch wurde mit Wasser verdünnt und die wässrige Phase mit EtOAc extrahiert. Die org. Phase wurde über Magnesiumsulfat getrocknet und i. vac. eingeengt. Das Rohprodukt wurde ohne weitere Reinigung im nachfolgenden Reaktionsschritt eingesetzt.
Ausbeute: 2.1 g (98% der Theorie)
ESI-MS: (M+H)⁺ = 482/484 (Cl)
R_{f} = 0.48 (Kieselgel, Petrolether / EtOAc 4 / 1 v/v)

### Zwischenprodukt 66

### 4-(4-Amino-3-chlor-5-trifluormethyl-phenyl)-3,3-bis-ethoxycarbonyl-buttersäure

Zu einer Lösung von 30.0 g (62.25 mmol) 4-(4-Amino-3-chlor-5-trifluormethylphenyl)-3,3-bis-ethoxycarbonyl-buttersäure-*tert*.-butylester (Zwischenprodukt 65) in 200 mL CH₂Cl₂ wurde unter Eiskühlung 20 mL TFA zugegeben und die Mischung 16 Stunden bei RT gerührt. Das Reaktionsgemisch wurde i. vac. eingeengt und der Rückstand aus Petrolether umkristallisiert. Der Niederschlag wurde abfiltriert, mit Petrolether gewaschen und getrocknet.
Ausbeute: 23.6 g (89% der Theorie)
ESI-MS: (M-H)⁻ = 424/426 (Cl)

### Zwischenprodukt 67

### 2-(4-Amino-3-chlor-5-trifluormethyl-benzyl)-2-{2-oxo-2-[4-(2-oxo-1,2,4,5-tetrahydro-1,3-benzodiazepin-3-yl)-piperidin-1-yl]-ethyl}-malonsäure-diethylester

Zu einer Lösung von 13.00 g (30.53 mmol) 4-(4-Amino-3-chlor-5-trifluormethylphenyl)-3,3-bis-ethoxycarbonyl-buttersäure (Zwischenprodukt 66) und 9.7 mL (70.00 mmol) Triethylamin in 70 mL DMF wurde bei 0 °C 10.60 g (33.00 mmol) TBTU zugegeben und das Gemisch 1.5 Stunden bei 0 °C gerührt. Eine Lösung 7.482 g (30.50 mmol) 3-Piperidin-4-yl-1,3,4,5-tetrahydro-1,3-benzodiazepin-2-on in 50 mL DMF wurde zugegeben und das Gemisch 16 Stunden bei RT gerührt. Das Reaktionsgemisch wurde i. vac. eingeengt und Rückstand mit ges wässeriger Natriumbicarbonat-Lösung und EtOAc versetzt. Der gebildete Niederschlag wurde abfiltriert, in Aceton suspendiert, erneut abfiltriert, mit Aceton gewaschen und i. vac. getrocknet.
Ausbeute: 17.90 g (90% der Theorie)
ESI-MS: (M+H)⁺ = 653/655 (Cl)

### Zwischenprodukt 68

### 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-buttersäure

Zu einer Lösung von 17.90 g (27.41 mmol) 2-(4-Amino-3-chlor-5-trifluormethylbenzy)-2-{2-oxo-2-[4-(2-oxo-1,2,4,5-tetrahydro-1,3-benzodiazepin-3-yl)-piperidin-1 - yl]-ethyl}-malonsäure-diethylester (Zwischenprodukt 67) in 1200 mL EtOH wurden 5.48 g (137.00 mmol) NaOH in 500 mL Wasser zugegeben und das Gemisch 4 Stunden unter Rückfluss erhitzt. EtOH wurde i. vac. abgedampft und die wässerige Phase mit konz. wässeriger HCl auf pH 2 angesäuert. Der gebildete Niederschlag wurde abfiltriert, mit Wasser gewaschen und i. vac. getrocknet.
Ausbeute: 14.80 g (98% der Theorie)
ESI-MS: (M-H)⁻ = 551/553 (Cl)

### Herstellung von Endverbindungen:

### Beispiel 1

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[1-(3-dimethylamino-propyl)-1H-benzimidazol-2-yl]-ethyl}-amid

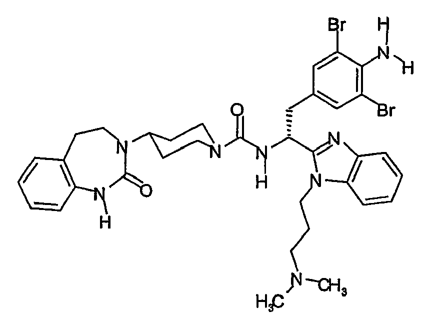

Die Lösung, von 1.0 g (1.30 mmol) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[2-(3-dimethyl-amino-propylamino)-phenylcarbamoyl]-ethyl}-amid (Zwischenprodukt 1) in 60 mL 1,4-Dioxan wurde mit 200 mg *p*-Toluolsulfonsäure versetzt und 15 Minuten unter Rückfluss erhitzt. Das Lösemittel wurde i. vac. entfernt, der Rückstand in Wasser aufgenommen und durch Zugabe von wässeriger Natriumbicarbonat Lösung alkalisch gestellt. Der entstehende Niederschlag wurde abfiltriert, mit Aceton verrieben und getrocknet. Man erhielt 0.35 g (36% der Theorie) an farblosen Kristallen vom R_{f} = 0.55 (Kieslegel, Dichlormethan / MeOH / Cyclohexan / konz. wässeriger Ammoniak 70 / 15 / 15 / 2 v/v/v/v).
ESI-MS: (M+H)⁺ = 751/753/755 (Br₂).

### Beispiel 2

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[1-(2-dimethylamino-ethyl)-1H-benzimidazol-2-yl]-ethyl}-amid

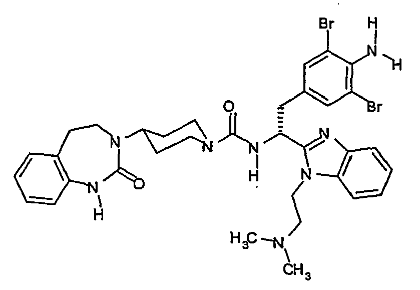

Das Produkte wurde analog zu Beispiel 1 aus 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[2-(2-dimethylamino-ethylamino)-phenylcarbamoyl]-ethyl}-amid (Zwischenprodukt 2) erhalten.
Ausbeute: 36% der Theorie
R_{f} = 0.55 (Kieselgel, Dichlormethan / MeOH / Cyclohexan / konz. wässeriger Ammoniak 70 /15 / 15 / 2 v/v/v/v)
ESI-MS: (M+H)⁺ = 751/753/755 (Br₂)

### Beispiel 3

### 4-(2-Oxo-1,2,4,5-tetrahydro-1,3-benzodiazepin-3-yl)-piperidin-1-carbonsäure-[(R)-2-(4-amino-3,5-dibrom-phenyl)-1-(1-methyl-1H-benzimidazol-2-yl)-ethyl]-amid

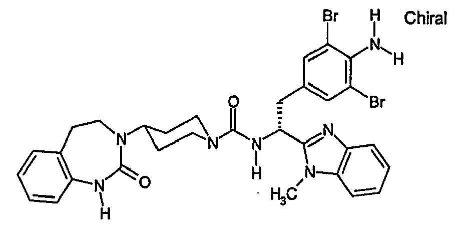

Das Produkte wurde analog zu Beispiel 1 aus 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(2-methylamino-phenylcarbamoyl)-ethyl]-amid (Zwischenprodukt 3) erhalten.
Ausbeute: 46% der Theorie
R_{f} = 0.64 (Kieselgel, Dichlormethan / MeOH / Cyclohexan / konz. wässeriger Ammoniak 70 / 15 / 15 / 2 v/v/v/v)
ESI-MS: (M+H)⁺ = 694/696/698 (Br₂)

### Beispiel 4

### 4-(2-Oxo-1,2,4,5-tetrahydro-1,3-benzodiazepin-3-yl)-piperidin-1-carbonsäure-[(R)-2-(4-amino-3,5-dibrom-phenyl)-1-(1H-benzimidazol-2-yl)-ethyl]-amid

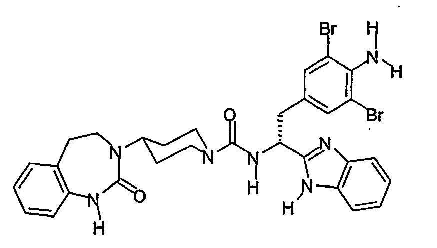

Das Produkte wurde analog zu Beispiel 1 aus 4-(2-Oxo-1,2,4,5-tetrahydro-1,3-benzodiazepin-3-yl)-piperidin-1-carbonsäure-[(*R*)-2-(4-amino-3,5-d ibrom-phenyl)-1-(2-amino-phenylcarbamoyl)-ethyl]-amid (Zwischenprodukt 4) erhalten.
Ausbeute: 27% der Theorie
R_{f} = 0.69 (Kieselgel, Dichlormethan / MeOH / Cyclohexan / konz. wässeriger Ammoniak 70 / 15 / 15 / 2 v/v/v/v)
ESI-MS: (M+H)⁺ = 680/682/684 (Br₂)

### Beispiel 5

### 2-((R)-2-(4-Amino-3,5-dibrom-phenyl)-1-{[4-(2-oxo-1,4-dihydro-2H-chinazolin-3-yl)-piperidin-1-carbonyl]-amino}-ethyl)-1H-benzimidazol-5-carbonsäuremethylester

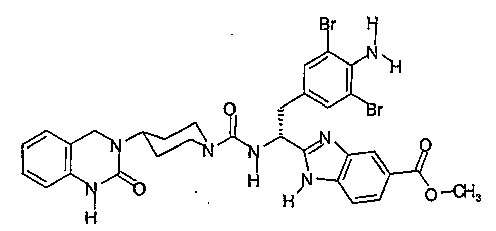

Das Produkte wurde analog zu Beispiel 1 aus 4-Amino-3-((*R*)-3-(4-amino-3,5-dibrom-phenyl)-2-{[4-(2-oxo-1,4-dihydro-2*H*-chinazolin-3-yl)-piperidin-1-carbonyl]-amino}-propionylamino)-benzoesäuremethylester (Zwischenprodukt 5) erhalten.
Ausbeute: 95% der Theorie
ESI-MS: (M+H)⁺ = 724/726/728 (Br₂)

### Beispiel 6

### 2-((R)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1H-benzimidazol-5-carbonsäuremethylester

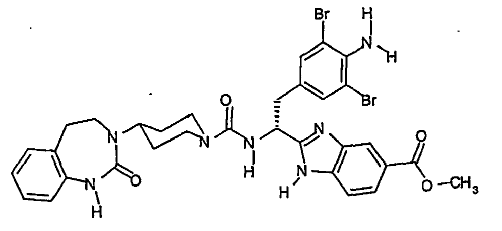

Das Produkte wurde analog zu Beispiel 1 aus 4-Amino-3-((*R*)-3-(4-amino-3,5-dibrom-phenyl)-2-{[4-(2-oxo-1,2,4,5-tetrahydro-1,3-benzodiazepin-3-yl)-piperidin-1-carbonyl]-amino}-propionylamino)-benzoesäuremethylester (Zwischenprodukt 6) erhalten.
Ausbeute: 85% der Theorie
ESI-MS: (M+H)⁺ = 738/740/742 (Br₂)

### Beispiel 7

### 4-(2-Oxo-1,2,4,5-tetrahydro-1,3-benzodiazepin-3-yl)-piperidin-1-carbonsäure-[(R)-2-(4-amino-3,5-dibrom-phenyl)-1-(1-butyl-1H-benzimidazol-2-yl)-ethyl]-amid

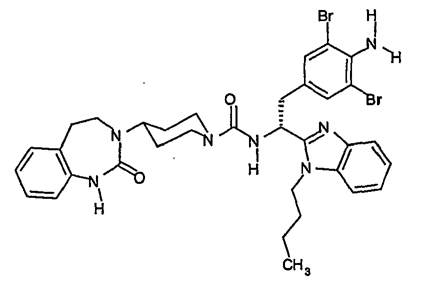

Das Produkte wurde analog zu Beispiel 1 aus 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(2-butylamino-phenylcarbamoyl)-ethyl]-amid (Zwischenprodukt 7) erhalten.
Ausbeute: 20% der Theorie
ESI-MS: (M+H)⁺ = 736/738/740 (Br₂)

### Beispiel 8

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[1-(3-pyrrolidin-1-yl-propyl)-1H-benzimidazol-2-yl]-ethyl}-amid

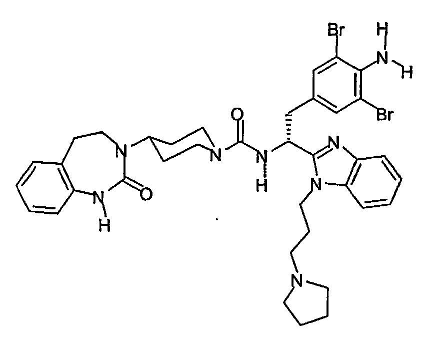

Das Produkte wurde analog zu Beispiel 1 aus 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[2-(3-pyrrolidin-1-yl-propylamino)-phenylcarbamoyl]-ethyl}-amid (Zwischenprodukt 8) erhalten.
Ausbeute: 9% der Theorie
ESI-MS: (M+H)⁺ = 791/793/795 (Br₂)

### Beispiel 9

### 4-(2-Oxo-1,2,4,5-tetrahydro-1,3-benzodiazepin-3-yl)-piperidin-1-carbonsäure-[(R)-2-(4-amino-3,5-dibrom-phenyl)-1-(1-pyridin-3-ylmethyl-1H-benzimidazol-2-yl)-ethyl]-amid

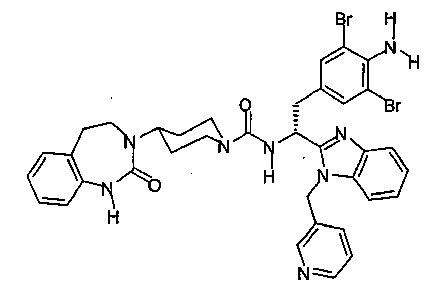

Das Produkte wurde analog zu Beispiel 1 aus 4-(2-Oxo-1,2,4,5-tetrahydro-1,3-benzodiazepin-3-yl)-piperidin-1-carbonsäure-((*R*)-2-(4-amino-3,5-dibrom-phenyl)-1-{2-[(pyridin-3-ylmethyl)-amino]-phenylcarbamoyl}-ethyl)-amid (Zwischenprodukt 9) erhalten.
Ausbeute: 31 % der Theorie
ESI-MS: (M+H) + = 771/773/775 (Br₂)

### Beispiel 10

### 4-(2-Oxo-1,2,4,5-tetrahydro-1,3-benzodiazepin-3-yl)-piperidin-1-carbonsaure-[(R)-2-(4-amino-3,5-dibrom-phenyl)-1-(1-benzyl-1H-benzimidazol-2-yl)-ethyl]-amid

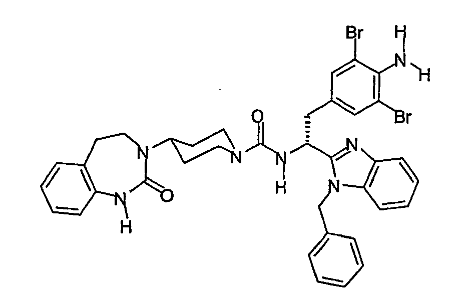

Das Produkte wurde analog zu Beispiel 1 aus 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(2-benzylamino-phenylcarbamoyl)-ethyl]-amid (Zwischenprodukt 10) erhalten.
Ausbeute: 47% der Theorie
ESI-MS: (M+H)⁺ = 770/772/774 (Br₂)

### Beispiel 11

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[1-(1-methyl-piperidin-4-ylmethyl)-1H-benzimidazol-2-yl]-ethyl}-amid

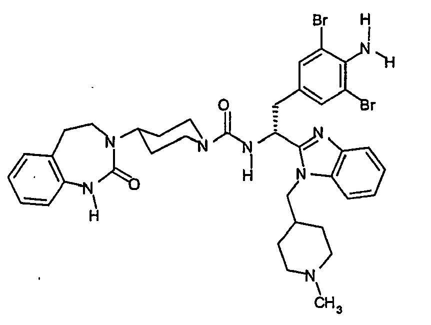

Das Produkte wurde analog zu Beispiel 1 aus 4-(2-Oxo-1,2,4,5-tetrahydro-1,3-benzodiazepin-3-yl)-piperidin-1-carbonsäure-((R)-2-(4-amino-3,5-dibrom-phenyl)-1-{2-[(1=methyl-piperidin-4-ylmethyl)-amino]-phenylcarbamoyl}-ethyl)-amid (Zwischenprodukt 11) erhalten.
Ausbeute: 25% der Theorie
ESI-MS: (M+H)⁺ = 791/793/795 (Br₂)

### Beispiel 12

### 2-((R)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1H-benzimidazol-5-carbonsäure

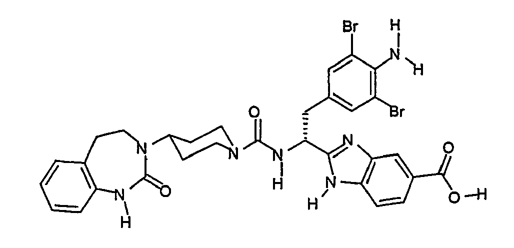

Zu einer Lösung von 1.5 g (2.03 mmol) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimidazol-5-carbonsäuremethylester in 100 mL MeOH wurde eine Lösung von 0.4 g Lithiumhydroxid in 50 mL Wasser zugegeben und 2 Tage bei RT gerührt. Das organische Lösemittel wurde i. vac. entfernt und der wässerige Rückstand dreimal mit jeweils 20 mL Dichlormethan extrahiert. Die Wasserphase wurde anschließend durch Zugabe von 25 mL einer 1 N Salzsäure sauer gestellt und das ausgefallene Produkt abfiltriert und getrocknet. Man erhielt 1.1 g (75% der Theorie) an farblosen Kristallen vom Rf = 0.22 (Kieselgel, Dichlormethan / MeOH /Cyclohexan / konz. wässeriger Ammoniak 70 / 15 / 15 / 2 v/v/v/v).
ESI-MS: (M+H)⁺ = 724/726/728 (Br₂).

### Beispiel 13

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[6-(4-methyl-piperazin-1-carbonyl)-1H-benzimidazol-2-yl]-ethyl}-amid

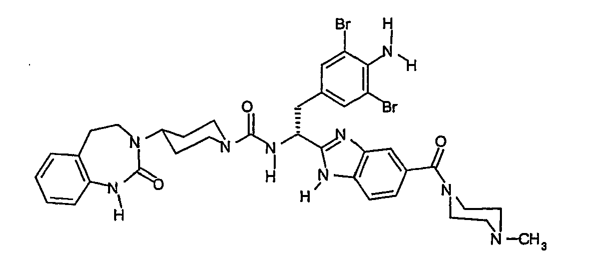

Zu einer Lösung von 0.20 g (0.276 mmol) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimidazol-5-carbonsäure in 50 mL THF wurde 0.10 g (0.311 mmol) TBTU, 0.042 g (0.305 mmol) HOBt und 0.2 mL Hünigsbase zugegeben und diese Mischung 20 Minuten bei RT gerührt. Zu dieser Mischung wurde 34 µL (0.30 mmol) 1-Methylpiperazin zugegeben und die Mischung 2 Tage bei RT gerührt. Das Lösemittel wurde i. vac. entfernt, der Rückstand mit ges. wässeriger Natriumbicarbonat-Lösung versetzt und erschöpfend mit Dichlormethan extrahiert. Die vereinigten Dichlormethan-Extrakte wurde mit wässeriger Kaliumhydrogensulfatlösung versetzt und der entstehende Niederschlag abfiltriert und getrocknet. Man erhielt 0.12 g (54% der Theorie) an farblosem Feststoff vom R_{f} = 0.40 (Kieselgel, Dichlormethan / MeÖH / Cyclohexan / konz. wässeriger Ammoniak 70 / 15 / 15 / 2 v/v/v/v).
ESI-MS: (M+H)⁺ = 806/808/810 (Br₂)

### Beispiel 14

### 2-((R)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1H-benzimidazol-5-carbonsäure-(3-dimethylamino-propyl)-amid

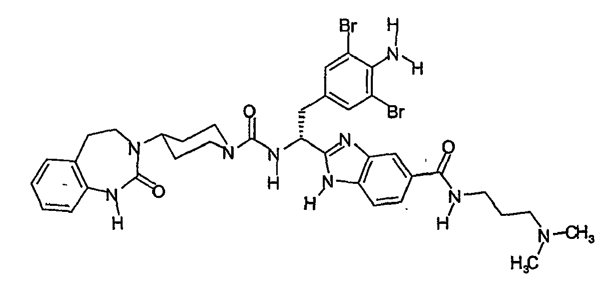

Zu einem Gemisch von 0.30 g (0.414 mmol) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimidazole-5-carbonsäure in 50 mL THF wurde 0.16 g (0.498 mmol) TBTU, 0.065 g (0.47 mmol) HOBt und 0.2 mL Hünigsbase zugegeben und diese Mischung 30 Minuten bei RT gerührt. Der nicht gelöste Anteil dieser Mischung wurde durch Zugabe von DMF in Lösung gebracht, tropfte dann 55 µL (0.433 mmol) 3-Dimethylamino-1-propylamin zu und rührte 16 Stunden. Das Lösemittel wurde i. vac. entfernt, der Rückstand in ges. wässeriger Natriumbicarbonat-Lösung aufgenommen und erschöpfend mit Dichlormethan extrahiert. Die vereinigten Extrakte wurden mit Wasser gewaschen, über Natriumsulfat getrocknet und i. vac. eingeengt. Der Rückstand wurde säulenchromatographisch an Kieselgel unter Verwendung von Dichlormethan / MeOH gereinigt. Man erhielt 0.11 g (33% der Theorie) farblose Kristalle vom R_{f} = 0.22 (Kieselgel, Dichlormethan / MeOH / Cyclohexan / konz. wässeriger Ammoniak 70 / 15 / 15 / 2 v/v/v/v).
ESI-MS: (M-H) = 806/808/810 (Br₂).

### Beispiel 15

### 2-((R)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1H-benzimidazol-5-carbonsäure-(2-dimethylamino-ethyl)-amid

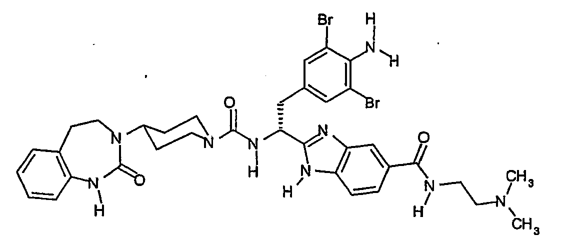

Das Produkt wurde analog zu Beispiel 14 ausgehend von 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimidazole-5-carbonsäure und *N*¹,*N*¹-Dimethyl-ethan-1,2-diamin hergestellt.
Ausbeute: 24% der Theorie
R_{f} = 0.36 (Kieselgel, Dichlormethan / MeOH / Cyclohexan / konz. wässeriger Ammoniak 70 / 15 / 15 / 2 v/v/v/v)
ESI-MS: (M+H)⁺ = 794/796/798 (Br₂)

### Beispiel 16

### 4-(2-Oxo-1,4-dihydro-2H-chinazolin-3-yl)-piperidin-1-carbonsäure-{(R)-2-(4-amino-3,5-dibrom-phenyl)-1-[1-(3-dimethylamino-propyl)-5-phenyl-1H-imidazol-2-yl]-ethyl}-amid

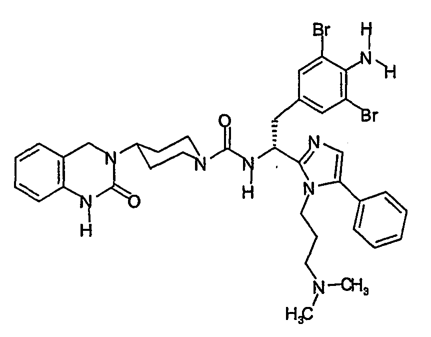

Eine Mischung aus 0.185 g (0.30 mmol) {(*R*)-2-(4-Amino-3,5-dibrom-phenyl)-1-[1-(3-dimethylamino-propyl)-5-phenyl-1*H*-imidazol-2-yl]-ethyl}-carbaminsäure-*tert*.-butylester (Zwischenprodukt 13), 0.065 g (0.60 mmol) Anisol und 3 mL Dichlormethan wurde mit 0.58 mL (7.45 mmol) TFA versetzt und 2.5 Stunden bei RT gerührt. Die Lösemittel wurden i. vac. entfernt, das entstandene, rohe 4-{(*R*)-2-Amino-2-[1-(3-dimethylamino-propyl)-5-phenyl-1*H*-imidazol-2-yl]-ethyl}-2,6-dibrom-phenylamin in 5 mL THF aufgenommen und unter Rühren und Eisbadkühlung 0.153 mL (0.89 mmol) Hünigsbase und 0.054 g (0.328 mmol) CDT zugegeben. Die Mischung wurde 30 Minuten unter Eiskühlung und 30 Minuten bei RT gerührt, mit 0.69 g (0.30 mmol) 3-Piperidin-4-yl-3,4-dihydro-1*H*-chinazolin-2-on, 15 mL THF und 2 mL DMF versetzt und 2.5 Stunden unter Rückfluss erhitzt. Das Reaktionsgemisch wurde i. vac. eingeengt, der Rückstand in EtOAc gelöst, wiederholt mit Wasser und ges. wässeriger NaCl-Lösung gewaschen und über Magnesiumsulfat getrocknet. Nach Entfernen des Lösemittels verblieb ein öliger Rückstand der säulenchromatographisch an Kieselgel unter Verwendung von Dichlormethan / MeOH / konz. wässeriger Ammoniak 90 / 9 / 1 gereinigt wurde. Man erhielt 0.03 g (13% der Theorie) des gewünschten Produktes.
ESI-MS: (M-H) = 777/779/781 (Br₂).

### Beispiel 17

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[1-(4-dimethylamino-butyl)-1H-benzimidazol-2-yl]-ethyl}-amid

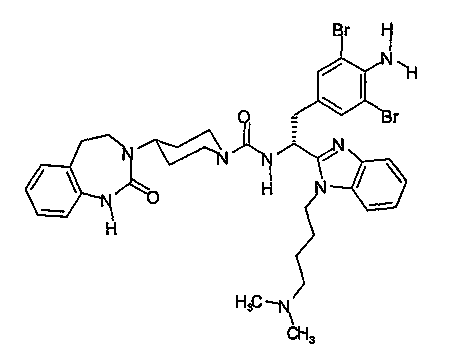

Zu einer Lösung von 200 mg (0.609 mmol) (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure in 10 mL DMF/THF (2:1 v/v) wurde bei RT 116 mg (0.361 mmol) TBTU und 65 µL (0.369 mmol) Hünigsbase zugegeben und das Gemisch 20 Minuten gerührt. 75 mg (0.362 mmol) *N*-(4-Dimethylamino-butyl)-benzol-1,2-diamin (Zwischenprodukt 18) wurde zugegeben und 16 Stunden bei RT gerührt. Das Gemisch wurde über basisches Alox filtriert, mit 10 mL DMF nachgewaschen und i. vac. eingeengt. Der Rückstand wurde in 20 mL Dioxan gelöst, mit 100 mg p-Toluolsulfonsäure versetzt und 45 Minuten unter Rückfluss erhitzt. Das Lösungsmittel wurde i. vac. abgedampft und der Rückstand in Dichlormethan aufgenommen. Die org. Phase wurde mit 15% wässeriger Kaliumcarbonat-Lösung gewaschen, über Natriumsulfat getrocknet und i. vac. eingeengt. Das Rohprodukt wurde mittels HPLC-MS (Zorbax Bonus C18 Amid-Phase 5 µm, Gradient 0.15% Ameisensäure in Wasser / Acetonitril 10/90 → 90 / 10 v/v) gereinigt und anschließend lyophilisiert.
Ausbeute: 10 mg (4% der Theorie)
R_{f} = 0.43 (Kieselgel, Dichlormethan / MeOH / Cyclohexan / konz. wässeriger Ammoniak 70/15/15/2 v/v/v/v)
ESI-MS: (M+H)⁺ = 779/781/783 (Br₂)

### Beispiel 18

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[1-(1-methyl-piperidin-4-yl)-1H-benzimidazol-2-yl]-ethyl}-amid

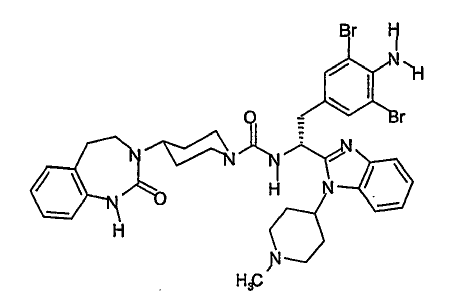

Das Produkt wurde analog zu Beispiel 17 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und *N*-(1-Methyl-piperidin-4-yl)-benzol-1,2-diamin (Zwischenprodukt 19) erhalten.
Ausbeute: 9% der Theorie
R_{f} = 0.50 (Kieselgel, Dichlormethan / MeOH / Cyclohexan / konz. wässeriger Ammoniak 70 / 15 / 15 / 2 v/v/v/v)
ESI-MS: (M+H)⁺ = 777/779/781 (Br₂)

### Beispiel 19

### 4-(2-Oxo-1,2,4,5-tetrahydro-1,3-benzodiazepin-3-yl)-piperidin-1-carbonsäure-[(R)-2-(4-amino-3,5-dibrom-phenyl)-1-(1-cyclohexyl-1H-benzimidazol-2-yl)-ethyl]-amid

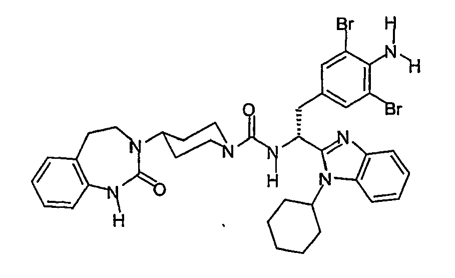

Das Produkt wurde analog zu Beispiel 17 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure *N*-Cyclohexyl-benzol-1,2-diamin erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Diisopropylether verrieben.
Ausbeute: 21% der Theorie
R_{f} = 0.81 (Kieselgel, Dichlormethan / MeOH / Cyclohexan / konz. wässeriger Ammoniak 70 / 15 / 15 / 2 v/v/v/v)
ESI-MS: (M+H)⁺ = 762/764/766 (Br₂)

### Beispiel 20

### 4-(2-Oxo-1,2,4,5-tetrahydro-1,3-benzodiazepin-3-yl)-piperidin-1-carbonsäure-[(R)-2-(4-amino-3,5-dibrom-phenyl)-1-(1-cyclopentyl-1H-benzimidazol-2-yl)-ethyl]-amid

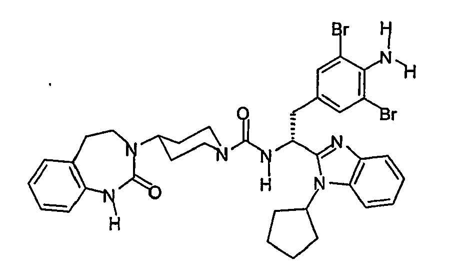

Das Produkt wurde analog zu Beispiel 17 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und *N*-Cyclopentyl-benzol-1,2-diamin erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Diisopropylether verrieben.
Ausbeute: 31 % der Theorie
R_{f} = 0.78 (Kieselgel, Dichlormethan / MeOH / Cyclohexan / konz. wässeriger Ammoniak 70 / 15 / 15 / 2 v/v/v/v)
ESI-MS: (M+H)⁺ = 748/750/752 (Br₂)

### Beispiel 21

### 4-(2-Oxo-1,2,4,5-tetrahydro-1,3-benzodiazepin-3-yl)-piperidin-1-carbonsäure{(R)-2-(4-amino-3,5-dibrom-phenyl)-1 -[l -(5-dimethylamino-pentyl)-1H-benzimidazol-2-yl]-ethyl}-amid

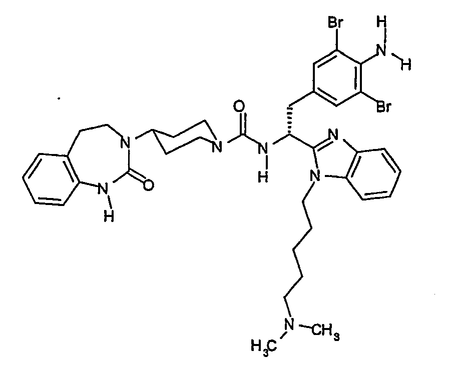

Das Produkt wurde analog zu Beispiel 17 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und *N*-(5-Dimethylamino-pentyl)-benzol-1,2-diamin (Zwischenprodukt 20) erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Diisopropylether verrieben.
Ausbeute: 67% der Theorie
R_{f} = 0.55 (Kieselgel, Dichlormethan / MeOH / Cyclohexan / konz. wässeriger Ammoniak 70 / 15 / 15 / 2 v/v/v/v)
ESI-MS: (M+H)⁺ = 793/795/797 (Br₂)

### Beispiel 22

### 4-[2-((R)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-benzimidazol-1-yl]-buttersäure-methylester

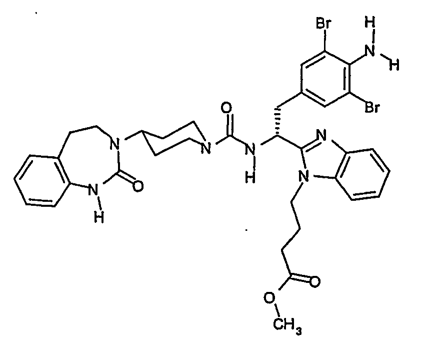

Das Produkt wurde analog zu Beispiel 17 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und 4-(2-Aminophenylamino)-buttersäure-methylester (Zwischenprodukt 21) erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Diisopropylether verrieben.
Ausbeute: 55% der Theorie
R_{f} = 0.80 (Kieselgel, Dichlormethan / MeOH / Cyclohexan / konz. wässeriger Ammoniak 70 / 15 / 15 / 2 v/v/v/v)
ESI-MS: (M+H)⁺ = 780/782/784 (Br₂)

### Beispiel 23

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[6-chlor-1-(3-dimethylamino-propyl)-1H-benzimidazol-2-yl]-ethyl}-amid

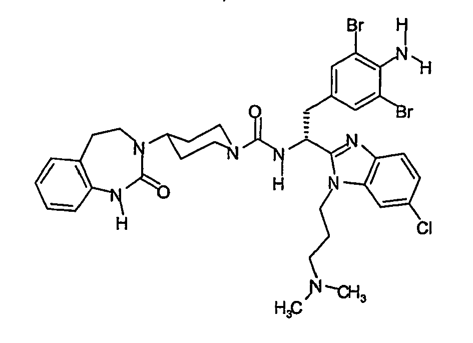

Das Produkt wurde analog zu Beispiel 17 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und 4-Chlor-*N*²-(3-dimethylamino-propyl)-benzol-1,2-diamin (Zwischenprodukt 22) erhalten. Das Rohprodukt wurde nach Reinigung mittels Säulenchromatographie (Kieselgel, Dichlormethan / MeOH / konz. wässeriger Ammoniak 90 / 10 / v/v/v) und Verreiben mit Diisopropylether als p-Toluolsulfonsäure-Salz isoliert.
Ausbeute: 27% der Theorie
R_{f} = 0.61 (Kieselgel, Dichlormethan / MeOH / Cyclohexan / konz. wässeriger Ammoniak 70 / 15 / 15 / 2 v/v/v/v)
ESI-MS: (M+H)⁺ = 799/781/783/785 (Br₂Cl)

### Beispiel 24

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[1-(3-dimethylamino-propyl)-6-fluor-1H-benzimidazol-2-yl]-ethyl}-amid

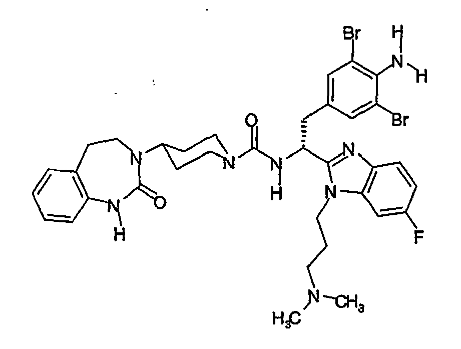

Das Produkt wurde analog zu Beispiel 17 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und *N*²-(3-Dimethylamino-propyl)-4-fluor-benzol-1,2-diamin (Zwischenprodukt 23) erhalten. Das Produkt wurde nach Reinigung mittels Säulenchromatographie (Kieselgel, Dichlormethan / MeOH / konz. wässeriger Ammoniak 90 / 10 / 1 v/v/v) und Verreiben mit Diisopropylether als p-Toluolsulfonsäure-Salz isoliert.
Ausbeute: 13% der Theorie
R_{f} = 0.18 (Kieselgel, Dichlormethan / MeOH / Cyclohexan / konz. wässeriger Ammoniak 70 / 15 / 15 / 2 v/v/v/v)
ESI-MS: (M+H)⁺ = 783/785/787 (Br₂)

### Beispiel 25

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[1-(3-dimethylamino-propyl)-5-fluor-1H-benzimidazol-2-yl]-ethyl}-amid

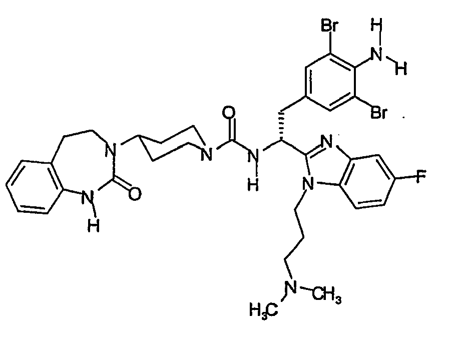

Das Produkt wurde analog zu Beispiel 17 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und *N*¹-(3-Dimethylamino-propyl)-4-fluor-benzol-1,2-diamin (Zwischenprodukt 24) erhalten. Das Produkt wurde nach Reinigung mittels Säulenchromatographie (Kieselgel, Dichlormethan / MeOH / konz. wässeriger Ammoniak 90 / 10 / 1 v/v/v) und Verreiben mit Diisopropylether als p-Toluolsulfonsäure-Salz isoliert.
Ausbeute: 57% der Theorie
R_{f} = 0.45 (Kieselgel, Dichlormethan / MeOH / Cyclohexan / konz. wässeriger Ammoniak 70 / 15 / 15 / 2 v/v/v/v)
ESI-MS: (M+H)⁺ = 783/785/787 (Br₂)

### Beispiel 26

### 4-(2-Oxo-1,2,4,5-tetrahydro-1,3-benzodiazepin-3-yl)-piperidin-1-carbonsäure-{(R)-2-(4-amino-3,5-dibrom-phenyl)-1-[5,6-dichlor-1-(3-dimethylamino-propyl)-1H-benzimidazol-2-yl]-ethyl}-amid

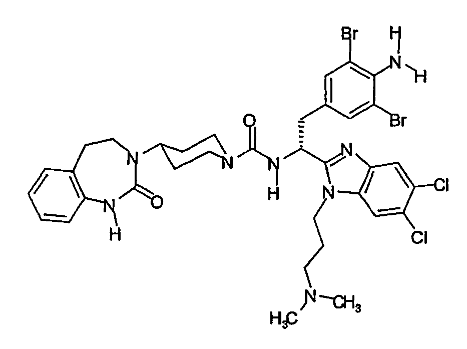

Das Produkt wurde analog zu Beispiel 17 ausgehend von (*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionsäure und 4,5-Dichlor-N-(3-dimethylamino-propyl)-benzol-1,2-diamin (Zwischenprodukt 25) erhalten. Das Produkt wurde nach Reinigung mittels Säulenchromatographie (Kieselgel, Dichlormethan / MeOH / konz. wässeriger Ammoniak 90 / 10 / 1 v/v/v) und Verreiben mit Diisopropylether als p-Toluolsulfonsäure-Salz isoliert.
Ausbeute: 18% der Theorie
R_{f} = 0.31 (Kieselgel, Dichlormethan / MeOH / Cyclohexan / konz. wässeriger Ammoniak 70 / 15 / 15 / 2 v/v/v/v)
ESI-MS: (M+H)⁺ = 833/835/837/839/841 (Br₂Cl₂)

### Beispiel 27

### 2-((R)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1H-benzimidazol-5-carbonsäure-methylester

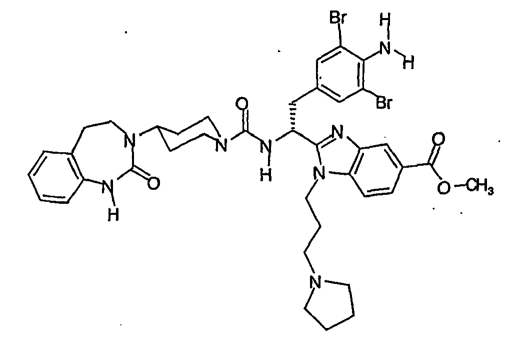

Eine Lösung von 1.00 g (1.151 mmol) 3-((*R*)-3-(4-Amino-3,5-dibrom-phenyl)-2-{[4-(2-oxo-1,2,4,5-tetrahydro-1,3-benzodiazepin-3-yl)-piperidin-1-carbonyl]-amino}-propionylamino)-4-(3-pyrrolidin-1-yl-propylamino)-benzoesäure-methylester (Zwischenprodukt 14) in 20 mL Eisessig wurde 2 Stunden unter Rückfluss erhitzt und anschließend das Lösungsmittel i. vac. abgedampft. Das Rohprodukt wurde mittels Säulenchromatographie (Kieselgel, Dichlormethan / MeOH / konz. wässeriger Ammoniak 90 / 10 / 1 v/v/v) gereinigt und mit Diisopropylether verrieben.
Ausbeute: 410 mg (42% der Theorie)
R_{f} = 0.39 (Kieselgel, Dichlormethan / MeOH / Cyclohexan / konz. wässeriger Ammoniak 70 / 15 / 15 / 2 v/v/v/v)
ESI-MS: (M+H)⁺ = 849/851/853 (Br₂)

### Beispiel 28

### 2-(2-(4-Amino-3-chlor-5-trifluormethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1H-benzimidazole-5-carbonsäure

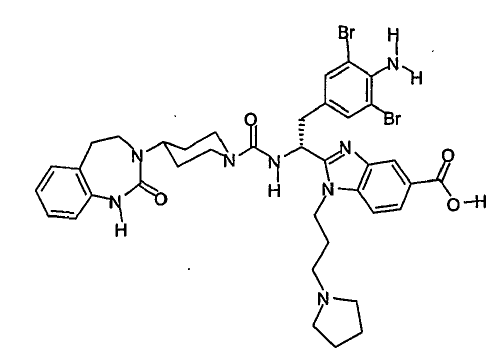

Zu einer Lösung von 320 mg (0.376 mmol) 2-((*R*)-2-(4-Amino-3,5-dibrom-phenyl)-1-{[4-(2-oxo-1 ,2,4,5-tetrahydro-1,3-benzodiazepin-3-yl)-piperidin-1-carbonyl]-amino}-ethyl)-1-(3-pyrrolidin-1-yl-propyl)-1*H*-benzimidazol-5-carbonsäure-methylester (Beispiel 27) in 20 mL THF wurde bei RT eine Lösung von 64 mg (1.525 mmol) Lithiumhydroxid-monohydrat in 2 mL Wasser zugegeben und das Gemisch 2 Tage bei RT gerührt. Das organische Lösemittel wurde i. vac. abgedampft und der wässerige Rückstand mit Dichlormethan gewaschen. Die wässerige Phase wurde mit 1 M wässeriger HCI auf pH 2 angesäuert, das ausgefallene Produkt abfiltriert und i. vac. getrocknet.
Ausbeute: 240 mg (76% der Theorie)
R_{f} = 0.28 (Kieselgel, Dichlormethan / MeOH / Cyclohexan / konz. wässeriger Ammoniak 70 / 15 / 15 / 2 v/v/v/v)
ESI-MS: (M+H)⁺ = 835/837/839 (Br₂)

### Beispiel 29

### 4-(2-Oxo-1,2,4,5-tetrahydro-1,3-benzodiazepin-3-yl)-piperidin-1-carbonsäure-{(R)-2-(3,5-dibrom-4-hydroxy-phenyl)-1-[1-(1-methyl-piperidin-4-ylmethyl)-1H-benzimidazol-2-yl]-ethyl}-amid

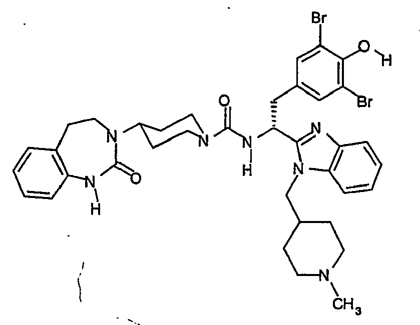

Zu einer Lösung von 500 mg (0.819 mmol) (R)-3-(3,5-Dibrom-4-hydroxy-phenyl)-2-{[4-(2-oxo-1,2,4,5-tetrahydro-1,3-benzodiazepin-3-yl)-piperidin-1-carbonyl]-amino}-propionsäure, 321 mg (1.000 mmol) TBTU und 0.277 mL (2.000 mmol) Triethylamin in 10 mL DMF wurde 197 mg (0.900 mmol) *N*-(1-Methyl-piperidin-4-ylmethyl)-benzol-1,2-diamin (Zwischenprodukt 17) zugegeben und das Gemisch 16 Stunden bei RT gerührt. Das Reaktionsgemisch wurde auf 150 mL ges. wässerige Natriumbicarbonat-Lösung gegossen und 15 Minuten bei RT gerührt. Der gebildete Niederschlag wurde abfiltriert, mit Wasser gewaschen und i. vac. getrocknet. Das Zwischenprodukt wurde in 30 mL Dioxan und 5 mL Isopropanol gelöst, 10 mg *p*-Toluolsulfonsäure zugegeben und das Gemisch 40 Minuten auf 115 °C erhitzt. Das Lösungsmittel wurde i. vac. abgedampft, der Rückstand in Dichlormethan aufgenommen und die org. Phase mit 15% wässeriger Kaliumcarbonat-Lösung gewaschen. Die org. Phase wurde über Natriumsulfat getrocknet und i. vac. eingeengt. Der Rückstand wurde mittels HPLC-MS (Zorbax Bonus C18 Amid-Phase 5 µm, Gradient 0.15% Ameisensäure in Wasser / Acetonitril 10 / 90 → 90 / 10 v/v) gereinigt und das Produkt lyophilisiert.
Ausbeute: 36 mg (6% der Theorie)
EI-MS: M⁺ = 792/794/796 (Br₂)

### Beispiel 30

### 4-(2-Oxo-1,2,4,5-tetrahydro-1,3-benzodiazepin-3-yl)-piperidin-1-carbonsäure-{(R)-2-(3,5-dibrom-4-hydroxy-phenyl)-1-[1-(1-methyl-piperidin-4-yl)-1H-benzimidazol-2-yl]-ethyl}-amid

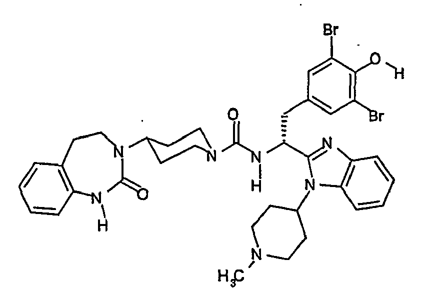

Das Produkt wurde analog zu Beispiel 29 ausgehend von (*R*)-3-(3,5-Dibrom-4-hydroxy-phenyl)-2-{[4-(2-oxo-1,2,4,5-tetrahydro-1,3-benzodiazepin-3-yl)-piperidin-1-carbonyl]-amino}-propionsäure und *N*-(1-Methyl-piperidin-4-yl)-benzol-1,2-diamin (Zwischenprodukt 19) erhalten.
Ausbeute: 24 mg (4% der Theorie)
EI-MS: M⁺ = 778/780/782 (Br₂)

### Beispiel 31

### 4-(2-Oxo-1,2,4,5-tetrahydro-1,3-benzodiazepin-3-yl)-piperidin-1-carbonsäure-{(R)-2-(3,5-dibrom-4-hydroxy-phenyl)-1-[1-(3-dimethylamino-propyl)-1H-benzimidazol-2-yl]-ethyl}-amid

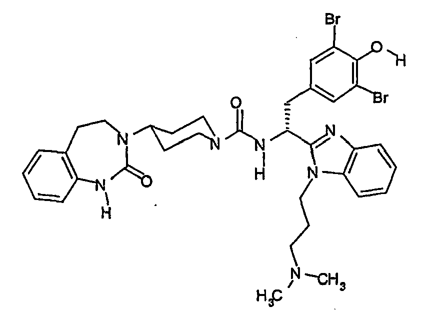

Das Produkt wurde analog zu Beispiel 29 ausgehend von (*R*)-3-(3,5-Dibrom-4-hydroxy-phenyl)-2-{[4-(2-oxo-1,2,4,5-tetrahydro-1,3-benzodiazepin-3-yl)-piperidin-1-carbonyl]-amino}-propionsäure und *N*-(3-Dimethylamino-propyl)-benzol-1,2-diamin erhalten.
Ausbeute: 42 mg (7% der Theorie)
El-MS: M⁺ = 766/768/770 (Br₂)

### Beispiel 32

### 4-(2-Oxo-1,2,4,5-tetrahydro-1,3-benzodiäzepin-3-yl)-piperidin-1-carbonsäure-{(R)-2-(3,5-dibrom-4-hydroxy-phenyl)-1-[1-(3-pyrrolidin-1-yl-propyl)-1H-benzimidazol-2-yl]-ethyl}-amid

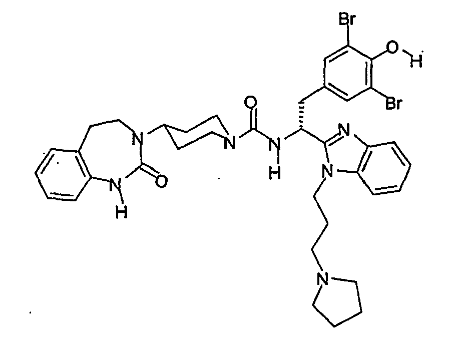

Das Produkt wurde analog zu Beispiel 29 ausgehend von (*R*)-3-(3,5-Dibrom-4-hydroxy-phenyl)-2-{[4-(2-oxo-1,2,4,5-tetrahydro-1,3-benzodiazepin-3-yl)-piperidin-1-carbonyl]-amino}-propionsäure und *N*-(3-Pyrrolidin-1-yl-propyl)-benzol-1,2-diamin (Zwischenprodukt 15) erhalten.
Ausbeute: 21 mg (3% der Theorie)
EI-MS: M⁺ = 792/794/796 (Br₂)

### Beispiel 33

### 4-(2-Oxo-1,2,4,5-tetrahydro-1,3-benzodiazepin-3-yl)-piperidin-1-carbonsäure-[1-[6-chlor-1-(3-dimethylamino-propyl)-1H-benzimidazol-2-yl]-2-(3,4-dibrom-phenyl)-ethyl]-amid

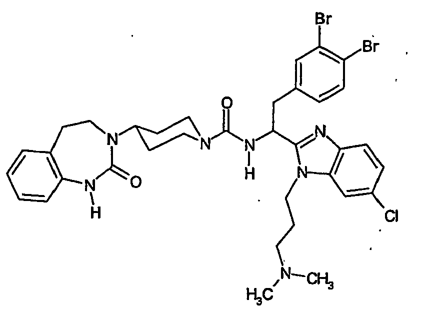

Das Produkt wurde analog zu Beispiel 17 ausgehend von 3-(3,4-Dibrom-phenyl)-2-{[4-(2-oxo-1,2,4,5-tetrahydro-1,3-benzodiazepin-3-yl)-piperidin-1-carbonyl]-amino}-propionsäure (Zwischenprodukt 45) und 4-Chlor-*N*²-(3-dimethylamino-propyl)-benzol-1,2-diamin (Zwischenprodukt 22) erhalten. Das Rohprodukt wurde mittels Säulenchromatographie (Kieselgel, Dichlormethan / MeOH / konz. wässeriger Ammoniak 90/10/1 v/v/v) gereinigt und mit Diisopropylether verrieben.
Ausbeute: 30% der Theorie
ESI-MS: (M+H)⁺ = 784/786/788 (Br₂)

### Beispiel 34

### 4-(2-Oxo-1,2,4,5-tetrahydro-1,3-benzodiazepin-3-yl)-piperidin-1-carbonsäure-{2-(3,4-dibrom-phenyl)-1-[5,6-dichlor-1-(3-dimethylamino-propyl)-1H-benzimidazol-2-yl]-ethyl}-amid

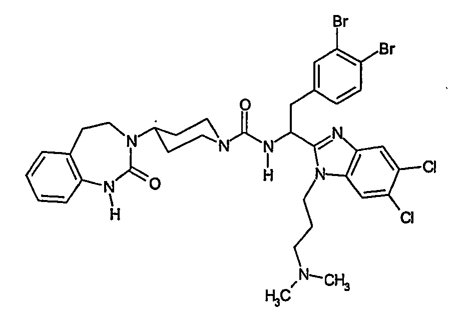

Das Produkt wurde analog zu Beispiel 17 ausgehend von 3-(3,4-Dibrom-phenyl)-2-{[4-(2-oxo-1,2,4,5-tetrahydro-1,3-benzodiazepin-3-yl)-piperidin-1-carbonyl]-amino}-propionsäure (Zwischenprodukt 45) und 4,5-Dichlor-N-(3-dimethylamino-propyl)-benzol-1,2-diamin (Zwischenprodukt 25) erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Diisopropylether verrieben.
Ausbeute: 7% der Theorie
ESI-MS: (M+H)⁺ = 818/820/822/824/826 (Br₂Cl₂)

### Beispiel 35

### 4-(2-Oxo-1,2,4,5-tetrahydro-1,3-benzodiazepin-3-yl)-piperidin-1-carbonsäure-[1-[7-chlor-1-(3-dimethylamino-propyl)-1H-benzimidazol-2-yl]-2-(3,4-dibrom-phenyl)-ethyl]-amid

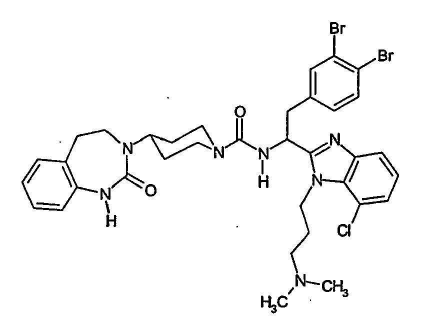

Das Produkt wurde analog zu Beispiel 17 ausgehend von 3-(3,4-Dibrom-phenyl)-2-{[4-(2-oxo-1,2,4,5-tetrahydro-1,3-benzodiazepin-3-yl)-piperidin-1-carbonyl]-amino}-propionsäure (Zwischenprodukt 45) und 3-Chlor-*N*²-(3-dimethylamino-propyl)-benzol-1,2-diamin (Zwischenprodukt 27) und erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Diisopropylether verrieben.
Ausbeute: 15% der Theorie
ESI-MS: (M+H)⁺ = 784/786/788/790 (Br₂Cl)

### Beispiel 36

### 4-(2-Oxo-1,2,4,5-tetrahydro-1,3-benzodiazepin-3-yl)-piperidin-1-carbonsäure-{2-(3,4-dibrom-phenyl)-1-[1-(3-dimethylamino-propyl)-6-fluor-1H-benzimidazol-2-yl]-ethyl}-amid

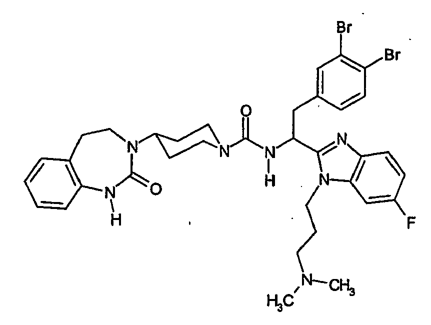

Das Produkt wurde analog zu Beispiel 17 ausgehend von 3-(3,4-Dibrom-phenyl)-2-{[4-(2-oxo-1,2,4,5-tetrahydro-1,3-benzodiazepin-3-yl)-piperidin-1-carbonyl]-amino}-propionsäure (Zwischenprodukt 45) und *N*²-(3-Dimethylamino-propyl)-4-fluor-benzol-1,2-diamin (Zwischenprodukt 23) erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Diisopropylether verrieben. Ausbeute: 19% der Theorie
ESI-MS: (M+H)⁺ = 768/770/72 (Br₂)

### Beispiel 37

### 4-(2-Oxo-1,2,4,5-tetrahydro-1,3-benzodiazepin-3-yl)-piperidin-1-carbonsäure-{2-(3,4-dibrom-phenyl)-1-[1-(1-methyl-piperidin-4-yl)-1H-benzimidazol-2-yl]-ethyl}-amid

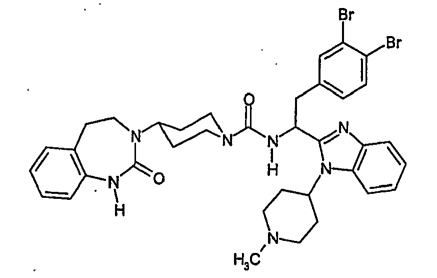

Das Produkt wurde analog zu Beispiel 17 ausgehend von 3-(3,4-Dibrom-phenyl)-2-{[4-(2-oxo-1 ,2,4,5-tetrahydro-1 ,3-benzodiazepin-3-yl)-piperidin-1-carbonyl]-amino}-propionsäure (Zwischenprodukt 45) und *N*-(1-Methyl-piperidin-4-yl)-benzol-1,2-diamin (Zwischenprodukt 19) erhalten. Das Rohprodukt wurde mittels Säulenchromatographie (Kieselgel, Dichlormethan / MeOH / konz. wässeriger Ammoniak 90 /10 / 1 v/v/v) gereinigt und mit Diisopropylether verrieben.
Ausbeute: 19% der Theorie
ESI-MS: (M+H)⁺ = 762/764/766 (Br₂)

### Beispiel 38

### 4-(2-Oxo-1,2,4,5-tetrahydro-1,3-benzodiazepin-3-yl)-piperidin-1-carbonsäure-{2-(3,4-dibrom-phenyl)-1-[1-(3-pyrrolidin-1-yl-propyl)-1H benzimidazol-2-yl]-ethyl}-amid

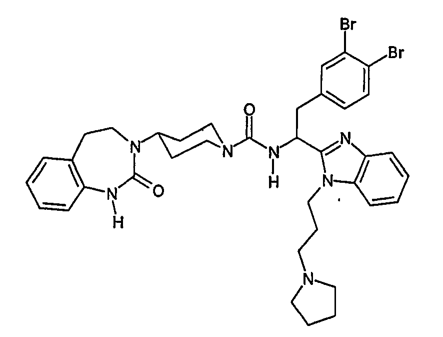

Das Produkt wurde analog zu Beispiel 17 ausgehend von 3-(3,4-Dibrom-phenyl)-2-{[4-(2-oxo-1,2,4,5-tetrahydro-1,3-benzodiazepin-3-yl)-piperidin-1-carbonyl]-amino}-propionsäure (Zwischenprodukt 45) und *N*-(3-Pyrrolidin-1-yl-propyl)-benzol-1,2-diamin (Zwischenprodukt 15) erhalten. Das Rohprodukt wurde mittels Säulenchromatographie (Kieselgel, Dichlormethan / MeOH / konz. wässeriger Ammoniak 90 /10 / 1 v/v/v) gereinigt und mit Diisopropylether verrieben.
Ausbeute: 19% der Theorie
ESI-MS: (M+H)⁺ = 776/778/780 (Br₂)

### Beispiel 39

### 4-(2-Oxo-1,2,4,5-tetrahydro-1,3-benzodiazepin-3-yl)-piperidin-1-carbonsäure-{2-(3,4-dibrom-phenyl)-1-[1-(1-methyl-piperidin-4-yl methyl)-1H-benzimidazol-2-yl]-ethyl}-amid

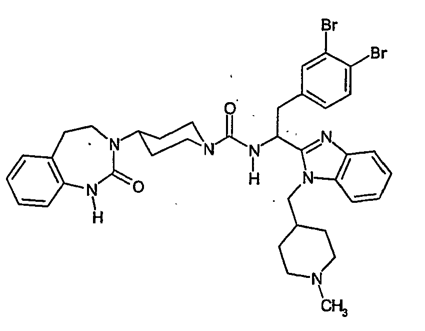

Das Produkt wurde analog zu Beispiel 17 ausgehend von 3-(3,4-Dibrom-phenyl)-2-{[4-(2-oxo-1,2,4,5-tetrahydro-1,3-benzod iazepin-3-yl)-piperidin-1-carbonyl]-amino}-propionsäure (Zwischenprodukt 45) und *N*-(1-Methyl-piperidin-4-ylmethyl)-benzol-1,2-diamin (Zwischenprodukt 19) erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Diisopropylether verrieben.
Ausbeute: 11 % der Theorie
ESI-MS: (M+H)⁺ = 776/778/780 (Br₂)

### Beispiel 40

### 4-(2-Oxo-1,2,4,5-tetrahydro-1,3-benzodiazepin-3-yl)-piperidin-1 -carbonsäure-[2-(3,4-dibrom-phenyl)-1-(1-pyridin-3-ylmethyl-1H-benzimidazol-2-yl)-ethyl]-amid

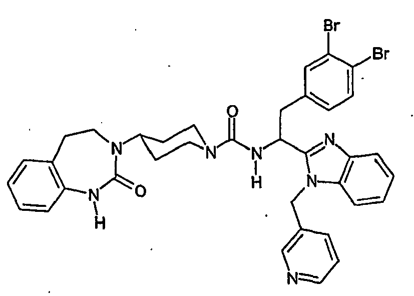

Das Produkt wurde analog zu Beispiel 17 ausgehend von 3-(3,4-Dibrom-phenyl)-2-{[4-(2-oxo-1,2,4,5-tetrahydro-1,3-benzodiazepin-3-yl)-piperidin-1-carbonyl]-amino}-propionsäure (Zwischenprodukt 45) und *N*-Pyridin-3-ylmethyl-benzol-1,2-diamin (Zwischenprodukt 16) erhalten. Das Rohprodukt wurde mittels Säulenchromatographie (Kieselgel, Dichlormethan / MeOH / konz. wässeriger Ammoniak 90/10/ 1 v/v/v) gereinigt und mit Diisopropylether verrieben.
Ausbeute: 12% der Theorie
ESI-MS: (M+H)⁺ = 756/758/760 (Br₂)

### Beispiel 41

### 4-(2-Oxo-1,2,4,5-tetrahydro-1,3-benzodiazepin-3-yl)-piperidin-1-carbonsäure-{2-(3,4-dibrom-phenyl)-1-[1-(3-dimethylamino-propyl)-1H-benzimidazol-2-yl]-ethyl}-amid

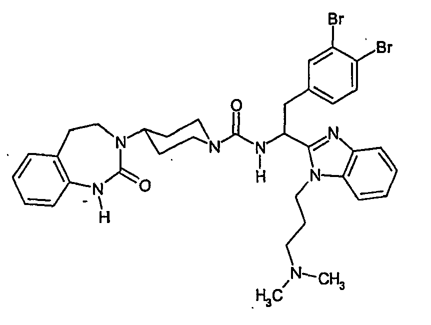

Das Produkt wurde analog zu Beispiel 17 ausgehend von 3-(3,4-Dibrom-phenyl)-2-{[4-(2-oxo-1,2,4,5-tetrahydro-1,3-benzodiazepin-3-yl)-piperidin-1-carbonyl]-amino}-propionsäure (Zwischenprodukt 45) und *N*-(3-Dimethylamino-propyl)-benzol-1,2-diamin erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Diisopropylether verrieben.
Ausbeute: 20% der Theorie
ESI-MS: (M+H)⁺ = 750/752/754 (Br₂)

### Beispiel 42

### 4-(2-Oxo-1,2,4,5-tetrahydro-1,3-benzodiazepin-3-yl)-piperidin-1-carbonsäure-{2-(3,4-dibrom-phenyl)-1-[1-(4-dimethylamino-butyl)-1H-benzimidazol-2-yl]-ethyl}-amid

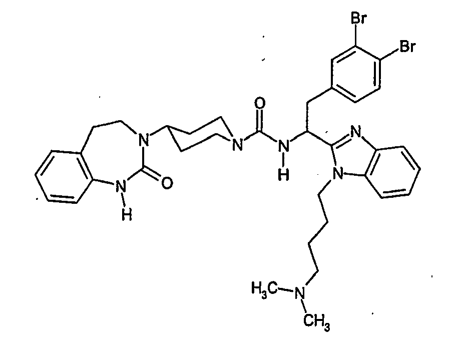

Das Produkt wurde analog zu Beispiel 17 ausgehend von 3-(3,4-Dibrom-phenyl)-2-{[4-(2-oxo-1,2,4,5-tetrahydro-1,3-benzodiazepin-3-yl)-piperidin-1-carbonyl]-amino}-propionsäure (Zwischenprodukt 45) und *N*-(3-Dimethylamino-butyl)-benzol-1,2-diamin (Zwischenprodukt 18) erhalten. Das Rohprodukt wurde mittels Säulenchromatographie (Kieselgel, Dichlormethan / MeOH / konz. wässeriger Ammoniak 90 /10 / 1 v/v/v) gereinigt und mit Diisopropylether verrieben.
Ausbeute: 16% der Theorie
ESI-MS: (M+H)⁺ = 764/766/768 (Br₂)

### Beispiel 43

### 4-(2-Oxo-1,2,4,5-tetrahydro-1,3-benzodiazepin-3-yl)-piperidin-1-carbonsäure-{2-(3,4-diethyl-phenyl)-1-[1-(3-dimethylamino-propyl)-1H-benzimidazol-2-yl]-ethyl}-amid

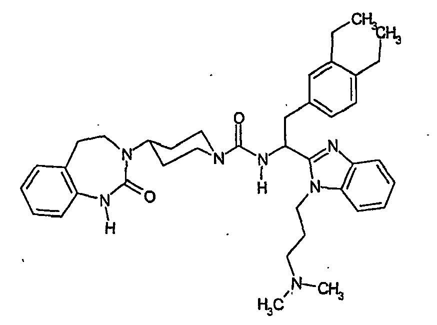

Zu einer Lösung von 150 mg (0.304 mmol) 3-(3,4-Diethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 50), 105 mg (0.327 mmol) TBTU und 44 mg (0.326 mmol) HOBt in 50 mL THF wurde 58 µL (0.327 mmol) Hünigsbase zugegeben und das Gemisch 30 Minuten bei RT gerührt. 60 mg (0.310 mmol) *N*-(3-Dimethylamino-propyl)-benzol-1,2-diamin wurde zugegeben und das Gemisch 16 Stunden bei RT gerührt. Das Lösungsmittel wurde i. vac. eingedampft, der Rückstand in Dichlormethan aufgenommen und die org. Phase mit ges. wässeriger Natriumbicarbonat-Lösung gewaschen. Die org. Phase wurde über Natriumsulfat getrocknet und i. vac. eingeengt. 30 mL Dioxan und 100 mg *p*-Toluolsulfonsäure wurde zugegeben und das Gemisch 15 Minuten unter Rückfluss erhitzt. Das Lösungsmittel wurde i. vac. abgedampft und Dichlormethan zugegeben. Die org. Phase wurde mit ges. wässeriger Natriumbicarbonat-Lösung gewaschen, über Natriumsulfat getrocknet und i. vac. eingeengt. Reinigung mittels HPLC-MS (Zorbax Bonus C18 Amid-Phase 5 µm, Gradient 0.15% Ameisensäure in Wasser / Acetonitril 10 / 90 → 90 / 10 v/v) ergab das Produkt.
Ausbeute: 80 mg (40% der Theorie)
R_{f} = 0.51 (Kieselgel, Dichlormethan / MeOH / Cyclohexan / konz. wässeriger Ammoniak 70/15/15/2 v/v/v/v)
ESI-MS: (M+H)⁺ = 650

### Beispiel 44

### 4-(2-Oxo-1,2,4,5-tetrahydro-1,3-benzodiazepin-3-yl)-piperidin-1-carbonsäure-{2-(3,4-diethyl-phenyl)-1-[1-(4-dimethylamino-butyl)-1H-benzimidazol-2-yl]-ethyl}-amid

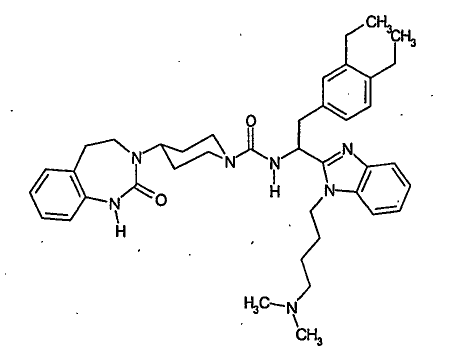

Das Produkt wurde analog zu Beispiel 17 ausgehend von 3-(3,4-Diethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 50) und *N*-(3-Dimethylamino-butyl)-benzol-1,2-diamin (Zwischenprodukt 18) erhalten. Das Rohprodukt wurde mittels Säulenchromatographie - (Kieselgel, Dichlormethan / MeOH / konz. wässeriger Ammoniak 90 / 10 / 1 v/v/v) gereinigt und mit Diisopropylether verrieben.
Ausbeute: 29% der Theorie
ESI-MS: (M+H)⁺ = 664

### Beispiel 45

### 4-(2-Oxo-1,2,4,5-tetrahydro-1,3-benzodiazepin-3-yl)-piperidin-1-carbonsäure-{2-(3,4-diethyl-phenyl)-1-[1-(1-methyl-piperidin-4-yl)-1H-benzimidazol-2-yl]-ethyl}-amid

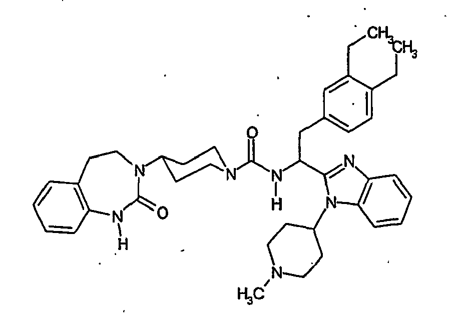

Das Produkt wurde analog zu Beispiel 17 ausgehend von 3-(3,4-Diethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 50) und *N*-(1-Methyl-piperidin-4-yl)-benzol-1,2-diamin (Zwischenprodukt 19) erhalten. Das Rohprodukt wurde mittels Säulenchromatographie (Kieselgel, Dichlormethan / MeOH / konz. wässeriger Ammoniak 90/10/ 1 v/v/v) gereinigt und mit Diisopropylether verrieben.
Ausbeute: 6% der Theorie
ESI-MS: (M+H)⁺ = 662

### Beispiel 46

### 4-(2-Oxo-1,2,4,5-tetrahydro-1,3-benzodiazepin-3-yl)-piperidin-1-carbonsäure-{2-(3,4-diethyl-phenyl)-1-[1-(3-pyrrolidin-1-yl-propyl)-1H-benzimidazol-2-yl]-ethyl}-amid

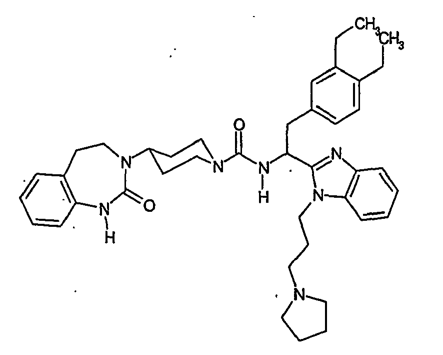

Das Produkt wurde analog zu Beispiel 17 ausgehend von 3-(3,4-Diethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 50) und *N*-(3-Pyrrolidin-1-yl-propyl)-benzol-1,2-diamin (Zwischenprodukt 15) erhalten. - Das Rohprodukt wurde mittels Säulenchromatographie (Kieselgel, Dichlormethan / MeOH / konz. wässeriger Ammoniak 90/10/1 v/v/v) gereinigt und mit Diisopropylether verrieben.
Ausbeute: 16% der Theorie
ESI-MS: (M+H)⁺ = 676

### Beispiel 47

### 4-(2-Oxo-1,2,4,5-tetrahydro-1,3-benzodiazepin-3-yl)-piperidin-1-carbonsäure-{2-(3,4-diethyl-phenyl)-1-[1-(1-methyl-piperidin-4-ylmethyl)-1H-benzimidazol-2-yl]-ethyl}-amid

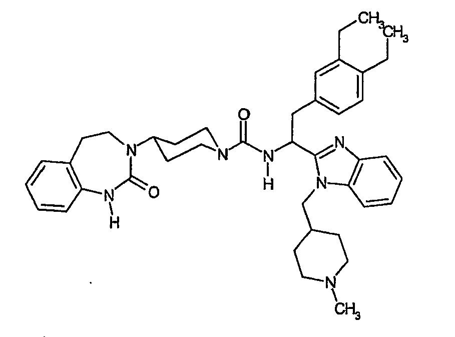

Das Produkt wurde analog zu Beispiel 17 ausgehend von 3-(3,4-Diethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 50) und *N*-(1-Methyl-piperidin-4-ylmethyl)-benzol-1,2-diamin (Zwischenprodukt 17) erhalten. Das Rohprodukt wurde mittels Säulenchromatographie (Kieselgel, Dichlormethan / MeOH / konz. wässeriger Ammoniak 90/10/1 v/v/v) gereinigt und mit Diisopropylether verrieben.
Ausbeute: 28% der Theorie
ESI-MS: (M+H)⁺ = 676

### Beispiel 48

### 4-(2-Oxo-1,2,4,5-tetrahydro-1,3-benzod iazepin-3-yl)-piperidin-1-carbonsäure-[2-(3,4-diethyl-phenyl)-1-(1-pyridin-3-ylmethyl-1H-benzimidazol-2-yl)-ethyl]-amid

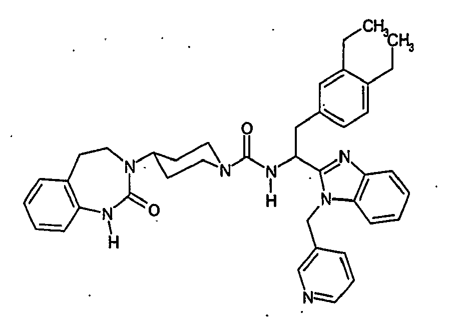

Das Produkt wurde analog zu Beispiel 17 ausgehend, von 3-(3,4-Diethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 50) und *N*-Pyridin-3-ylmethyl-benzol-1,2-diamin (Zwischenprodukt 16) erhalten. Das Rohprodukt wurde mittels Säulenchromatographie (Kieselgel, Dichlormethan / MeOH / konz. wässeriger Ammoniak 90/10/1 v/v/v) gereinigt und mit Diisopropylether verrieben.
Ausbeute: 17% der Theorie
ESI-MS: (M+H)⁺ = 656

### Beispiel 49

### 4-(2-Oxo-1,2,4,5-tetrahydro-1,3-benzodiazepin-3-yl)-piperidin-1-carbonsäure-{2-(3,5-bis-trifluormethyl-phenyl)-1-[1-(1-methyl-piperidin-4-yl)-1H-benzimidazol-2-yl]-ethyl}-amid

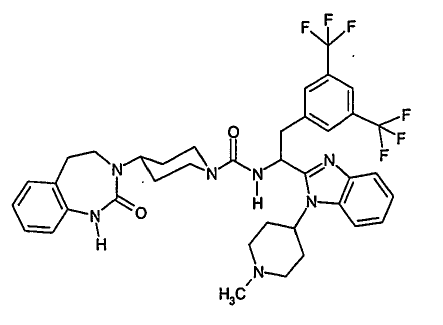

Das Produkt wurde analog zu Beispiel 17 ausgehend von 3-(3,5-Bis-trifluormethylphenyl)-2-{[4-(2-oxo-1,2,4,5-tetrahydro-1,3-benzodiazepin-3-yl)-piperidin-1-carbonyl]-amino}-propionsäure (Zwischenprodukt 53) und *N*-(1-Methyl-piperidin-4-yl)-benzol-1,2-diamin (Zwischenprodukt 19) erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Petrolether verrieben.
Ausbeute: 8% der Theorie
ESI-MS: (M+H)⁺ = 742

### Beispiel 50

### 4-(2-Oxo-1,2,4,5-tetrahydro-1,3-benzodiazepin-3-yl)-piperidin-1-carbonsäure-{2-(3,5-bis-trifluormethyl-phenyl)-1-[1-(3-pyrrolidin-1-yl-propyl)-1H-benzimidazol-2-yl]-ethyl}-amid

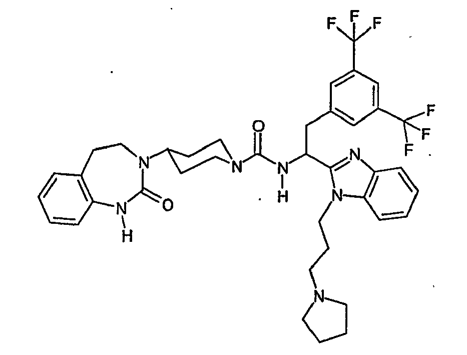

Das Produkt wurde analog zu Beispiel 17 ausgehend von 3-(3,5-Bis-trifluormethylphenyl)-2-{[4-(2-oxo-1,2,4,5-tetrahydro-1,3-benzodiazepin-3-yl)-piperidin-1-carbonyl]-amino}-propionsäure (Zwischenprodukt 53) und *N*-(3-Pyrrolidin-1-yl-propyl)-benzol-1,2-diamin (Zwischenprodukt 15) erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Petrolether verrieben.
Ausbeute: 11 % der Theorie
ESI-MS: (M+,H)⁺ = 756

### Beispiel 51

### 4-(2-Oxo-1,2,4,5-tetrahydro-1,3-benzodiazepin-3-yl)-piperidin-1-carbonsäure-{2-(3,5bis-trifluormethyl-phenyl)-1-[1-(1-methyl-piperidin-4-ylmethyl)-1H-benzimidazol-2-yl]-ethyl}-amid

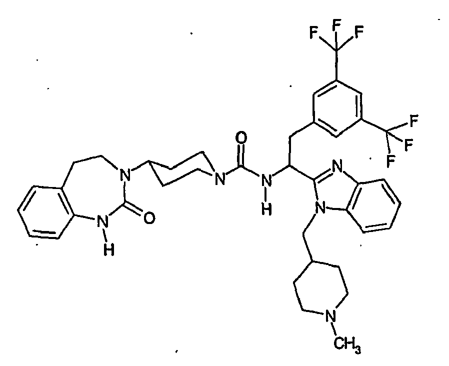

Das Produkt wurde analog zu Beispiel 17 ausgehend von 3-(3,5-Bis-trifluormethylphenyl)-2-{[4-(2-oxo-1,2,4,5-tetrahydro-1,3-benzodiazepin-3-yl)-piperidin-1-carbonyl]-amino}-propionsäure (Zwischenprodukt 53) und *N*-(1-Methyl-piperidin-4-ylmethyl)-benzol-1,2-diamin (Zwischenprodukt 17) erhalten. Das Rohprodukt wurde mittels Säulenchromatographie (Kieselgel, Dichlormethan / MeOH / konz. wässeriger Ammoniak 90/10/1 v/v/v) gereinigt und mit Petrolether verrieben.
Ausbeute: 23% der Theorie
ESI-MS: (M+H)⁺ = 756

### Beispiel 52

### 4-(2-Oxo-1,2,4,5-tetrahydro-1,3-benzodiazepin-3-yl)-piperidin-1 -carbonsäure-{2-(3,5-bis-trifluormethyl-phenyl)-1-[1-(3-dimethylamino-propyl)-1H-benzimidazol-2-yl]-ethyl}-amid

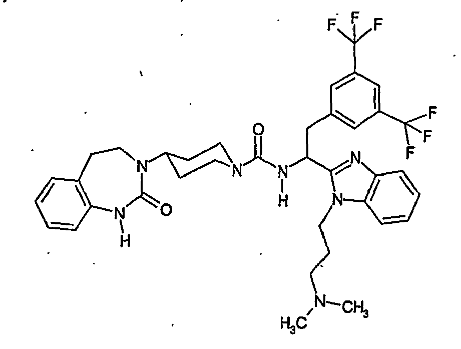

Das Produkt wurde analog zu Beispiel 17 ausgehend von 3-(3,5-Bis-trifluormethylphenyl)-2-{[4-(2-oxo-1,2,4,5-tetrahydro-1,3-benzodiazepin-3-yl)-piperidin-1-carbonyl]-amino}-propionsäure (Zwischenprodukt 53) und *N*-(3-Dimethylamino-propyl)-benzol-1,2-diamin erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Petrolether verrieben.
Ausbeute: 35% der Theorie
ESI-MS: (M+H)⁺ = 730

### Beispiel 53

### 2-(2-(4-Amino-3-chlor-5-trifluormethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1H-benzimidazole-5-carbonsäure-methylester

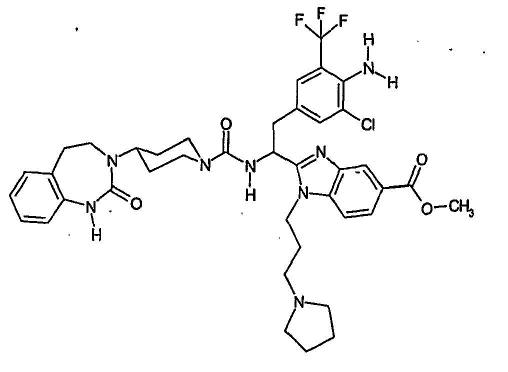

Zu einer Lösung von 800 mg (1.444 mmol) 3-(4-Amino-3-chlor-5-trifluormethylphenyl)-2-{[4-(2-oxo-1,2,4,5-tetrahydro-1,3-benzodiazepin-3-yl)-piperidin-1-carbonyl]-amino}-propionsäure (Zwischenprodukt 60) und 500 mg (1.557 mmol) TBTU in 70 mL DMF wurde 0.27 mL (1.550 mmol) Hünigsbase zugegeben und das Gemisch 30 Minuten bei RT gerührt. 410 mg (1.478 mmol) 3-Amino-4-(3-pyrrolidin-1-yl-propylamino)-benzoesäure-methylester (Zwischenprodukt 26) wurde zugegeben und das Gemisch16 Stunden bei RT gerührt. Das Reaktionsgemisch wurde i. vac. eingeengt und der Rückstand mit 15% wässeriger Kaliumcarbonat-Lösung versetzt. Der gebildete Niederschlag wurde abfiltriert und i. vac. getrocknet. Der Niederschlag wurde in 20 mL Eisessig gelöst und 2 Stunden unter Rückfluss erhitzt. Das Lösungsmittel wurde i. vac. abgedampft und das Rohprodukt mittels Säulenchromatographie (Kieselgel, Dichlormethan / MeOH / konz. wässeriger Ammoniak 90 / 10 / 1 v/v/v) gereinigt und mit Diisopropylether verrieben.
Ausbeute: 430 mg (41 % der Theorie).
R_{f} = 0.36 (Kieselgel, Dichlormethan / MeOH / Cyclohexan / konz. wässeriger Ammoniak 70 / 15 / 15 / 2 v/v/v/v)
ESI-MS: (M+H)⁺ = 795/797 (Cl)

### Beispiel 54

### 2-(2-(4-Amino-3-chlor-5-trifluormethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1H-benzimidazole-5-carbonsäure

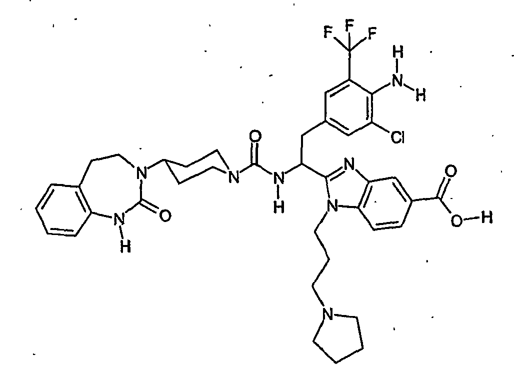

Zu einer Lösung von 310 mg (0.390 mmol) 2-(2-(4-Amino-3-chlor-5-trifluormethylphenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazole-5-carbonsäure-methylester (Beispiel 53) in 20 mL THF wurde eine Lösung von 72 mg (1.716 mmol) Lithiumhydroxid-monohydrat in 5 mL Wasser zugegeben und das Gemisch 2 Tage bei RT gerührt. THF wurde i. vac. abgedampft, der Rückstand in Wasser gelöst und 1.7 mL 1 M wässerige HCl zugegeben. Der gebildete Niederschlag wurde abfiltriert und i. vac. getrocknet.
Ausbeute: 190 mg (62% der Theorie)
R_{f} = 0.22 (Kieselgel, Dichlormethan / MeOH / Cyclohexan / konz. wässeriger Ammoniak 70 / 15 / 15 / 2 v/v/v/v)
ESI-MS: (M+H)⁺ = 781/783 (Cl)

### Beispiel 55

### 4-(2-Oxo-1,2,4,5-tetrahydro-1,3-benzodiazepin-3-yl)-piperidin-1-carbonsäure-{2-(4-amino-3-chlor-5-trifluormethyl-phenyl)-1-[1-(1-methyl-piperidin-4-yl)-1H-benzimidazol-2-yl]-ethyl}-amid

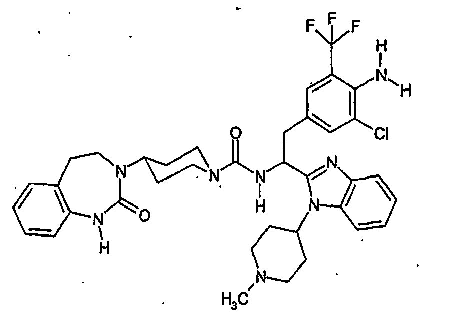

Das Produkt wurde analog zu Beispiel 17 ausgehend von 3-(4-Amino-3-chlor-5-trifluormethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 60) und *N*-(1-Methylpiperidin-4-yl)-benzol-1,2-diamin (Zwischenprodukt 19) erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Petrolether verrieben.
Ausbeute: 2% der Theorie
ESI-MS: (M+H)⁺ = 723/725 (Cl)

### Beispiel 56

### 4-(2-Oxo-1,2,4,5-tetrahydro-1,3-benzodiazepin-3-yl)-piperidin-1-carbonsäure-{2-(4-amino-3-chlor-5-trifluormethyl-phenyl)-1-[1-(3-pyrrolidin-1-yl-propyl)-1H-benzimidazol-2-yl]-ethyl}-amid

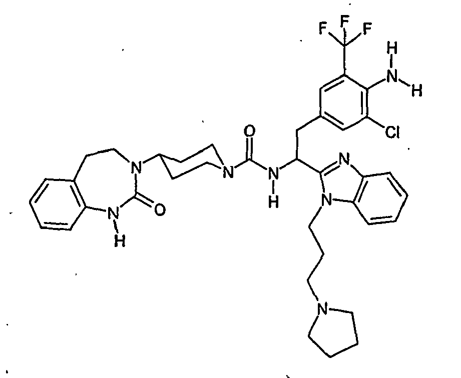

Das Produkt wurde analog zu Beispiel 17 ausgehend von 3-(4-Amino-3-chlor-5-trifluormethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 60) und *N*-(3-Pyrrolidin-1-yl-propyl)-benzol-1,2-diamin (Zwischenprodukt 15) erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Petrolether verrieben.
Ausbeute: 19% der Theorie
ESI-MS: (M+H)⁺ = 737/739 (Cl)

### Beispiel 57

### 4-(2-Oxo-1,2,4,5-tetrahydro-1,3-benzodiazepin-3-yl)-piperidin-1-carbonsäure-{2-(4-amino-3-chlor-5-trifluormethyl-phenyl)-1-[1-(1-methyl-piperidin-4-ylmethyl)-1H-benzimidazol-2-yl]-ethyl}-amid

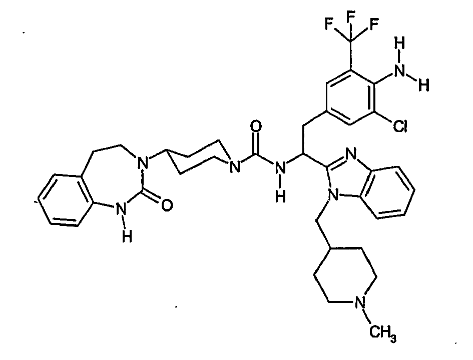

Das Produkt wurde analog zu Beispiel 17 ausgehend von 3-(4-Amino-3-chlor-5-trifluormethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 60) und *N*-(1-Methylpiperidin-4-ylmethyl)-benzol-1,2-diamin (Zwischenprodukt 17) erhalten. Das Rohprodukt wurde mittels Säulenchromatographie (Kieselgel, Dichlormethan / MeOH / konz. wässeriger Ammoniak 90/10/ 1 v/v/v) gereinigt und mit Petrolether verrieben.
Ausbeute: 26% der Theorie
ESI-MS: (M+H)⁺ = 737/739 (Cl)

### Beispiel 58

### 4-(2-Oxo-1,2,4,5-tetrahydro-1,3-benzodiazepin-3-yl)-piperidin-1-carbonsäure-[2-(4-amino-3-chlor-5-trifluormethyl-phenyl)-1-(1-pyridin-3-ylmethyl-1H-benzimidazol-2-yl)-ethyl]-amid

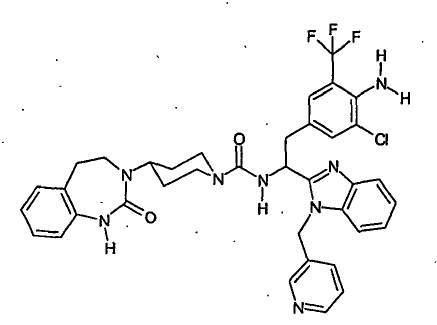

Das Produkt wurde analog zu Beispiel 17 ausgehend von 3-(4-Amino-3-chlor-5-trifluormethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 60) und *N*-Pyridin-3-ylmethyl-benzol-1,2-diamin (Zwischenprodukt 16) erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Petrolether verrieben.
Ausbeute: 6% der Theorie
ESI-MS: (M+H)⁺ = 717/719 (Cl)

### Beispiel 59

### 4-(2-Oxo-1,2,4,5-tetrahydro-1,3-benzodiazepin-3-yl)-piperidin-1-carbonsäure-{2-(4-amino-3-chlor-5-trifluormethyl-phenyl)-1-[1-(3-dimethylamino-propyl)-1H-benzimidazol-2-yl]-ethyl}-amid

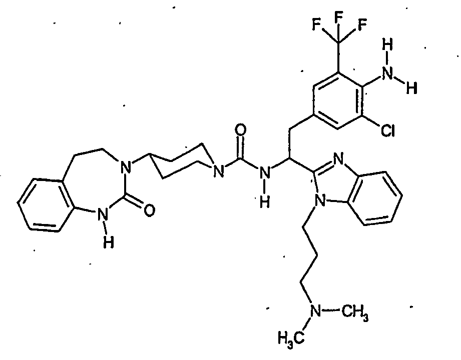

Das Produkt wurde analog zu Beispiel 17 ausgehend von 3-(4-Amino-3-chlor-5-trifluormethyl-phenyl)-2-{[4-(2-oxo-1,2,4,5-tetrahydro-1,3-benzodiazepin-3-yl)-piperidin-1-carbonyl]-amino}-propionsäure (Zwischenprodukt 60) und *N*-(3-Dimethylaminopropyl)-benzol-1,2-diamin erhalten. Das Rohprodukt wurde mittels Säulenchromatographie (Kieselgel, Dichlormethan / MeOH / konz. wässeriger Ammoniak 90 / 10 / 1 v/v/v) gereinigt und mit Petrolether verrieben.
Ausbeute: 12% der Theorie
ESI-MS: (M+H)⁺ = 711/713 (CI)

### Beispiel 60

### 3-(1-{4-(3,4-Diethyl-phenyl)-3-[1-(3-dimethylamino-propyl)-1H-benzimidazol-2-yl]-butyryl}-piperidin-4-yl)-1,3,4,5-tetrahydro-1,3-benzodiazepin-2-on

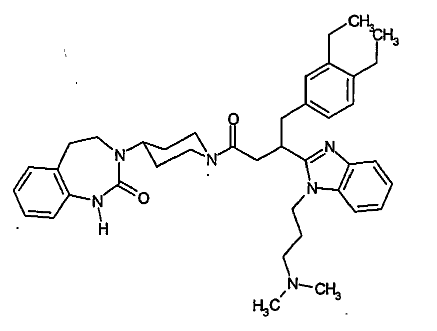

Das Produkt wurde analog zu Beispiel 43 ausgehend von 2-(3,4-Diethyl-benzyl)-4-oxo-4-[4-(2-oxo-1,2,4,5-tetrahydro-1,3-benzodiazepin-3-yl)-piperidin-1-yl]-buttersäure (Zwischenprodukt 64) und *N*-(3-Dimethylamino-propyl)-benzol-1,2-diamin erhalten. Das Rohprodukt wurde mittels HPLC-MS (Zorbax Bonus C18 Amid-Phase 5 µm, Gradient 0.15% Ameisensäure in Wasser / Acetonitril 10 / 90 → 90 / 10 v/v) gereinigt und anschließend lyophilisiert.
Ausbeute: 5% der Theorie)
R_{f} = 0.61 (Kieselgel, Dichlormethan / MeOH / Cyclohexan / konz. wässeriger Ammoniak 70 /15 /15 / 2 v/v/v/v)
ESI-MS: (M+H)⁺ = 649

### Beispiel 61

### 3-(1-{4-(4-Amino-3-chlor-5-trifluormethyl-phenyl)-3-[1-(3-pyrrolidin-1-yl-propyl)-1H-benzimidazol-2-yl]-butyryl}-piperidin-4-yl)-1,3,4,5-tetrahydro-1,3-benzodiazepin-2-on

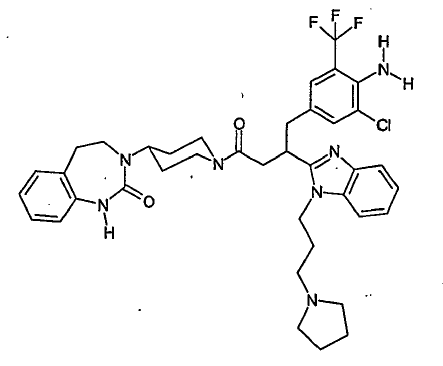

Zu einer Lösung von 380 mg (0.708 mmol) 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-butyraldehyd (Zwischenprodukt 36) in 7.5 mL DMF und 0.5 mL Wasser wurde 233 mg (0.710 mmol) *N*-(3-Pyrrolidin-1-yl-propyl)-benzol-1,2-diamin-trihydrochlorid (Zwischenprodukt 15) zugegeben und das Gemisch 48 Stunden unter Luftatmosphäre bei RT gerührt. Das Reaktionsgemisch wurde i. vac. eingeengt und EtOAc zugegeben. Die org. Phase wurde mit ges. wässeriger Natriumbicarbonat-Lösung gewaschen, über Natriumsulfat getrocknet und i. vac. eingeengt. Das Rohprodukt wurde mittels Säulenchromatographie (Kieselgel, EtOAc / MeOH / ges. wässeriger Ammoniak 90 / 10 / 1 v/v/v) gereinigt und mit Diisopropylether verrieben.
Ausbeute: 130 mg (25% der Theorie)
R_{f} = 0.17 (Kieselgel, EtOAc / MeOH / ges. wässeriger Ammoniak 90 / 10 / 1 v/vlv) - ESI-MS: (M+H)⁺ = 736/738 (Cl)

### Beispiel 62

### 3-(1-{4-(4-Amino-3-chlor-5-trifluormethyl-phenyl)-3-[1-(3-imidazol-1-yl-propyl)-1H-benzimidazol-2-yl]-butyryl}-piperidin-4-yl)-1,3,4,5-tetrahydro-1,3-benzodiazepin-2-on

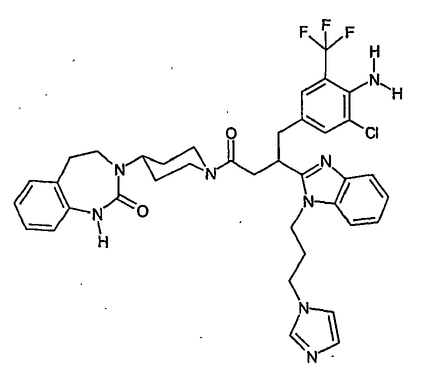

Zu einer Lösung von 537 mg (1.000 mmol) 2-(4-Amino-3-chlor-5-trifluormethylbenzyl)-4-oxo-4-[4-(2-oxo-1,2,4,5-tetrahydro-1,3-benzodiazepin-3-yl)-piperidin-1-yl]-butyraldehyd (Zwischenprodukt 36) in 10 mL DMF wurde 391 mg (1.200 mmol) *N*-(3-Imidazol-1-yl-propyl)-benzol-1,2-diamin-trihydrochlorid (Zwischenprodukt 28) und das Gemisch unter Luftatmosphäre 16 Stunden gerührt. Das Reaktionsgemisch wurde in Eiswasser gegossen, der gebildete Niederschlag abfiltriert und mit Wasser gewaschen. Das Rohprodukt wurde mittels HPLC-MS (Zorbax Bonus C18 Amid-Phase 5 µm, Gradient 0.15% Ameisensäure in Wasser / Acetonitril 10 /90 → 90 / 10 v/v) gereinigt und lyophilisiert.
Ausbeute: 470 mg (64% der Theorie)
ESI-MS: (M+H)⁺ = 733/735 (Br₂)

### Beispiel 63

### 3-(1-{4-(4-Amino-3-chlor-5-trifluormethyl-phenyl)-3-[5-(3-dimethylamino-propyl)-1-ethyl-1H-benzimidazol-2-yl]-butyryl}-piperidin-4-yl)-1,3,4,5-tetrahydro-1,3-benzodiazepin-2-on

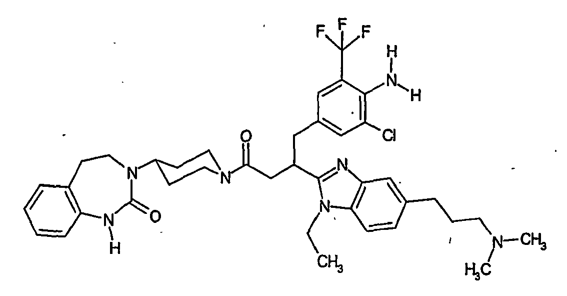

Zu einer Lösung von 860. mg (1.602 mmol) 2-(4-Amino-3-chlor-5-trifluormethylbenzyl)-4-oxo-4-[4-(2-oxo-1,2,4,5-tetrahydro-1,3-benzodiazepin-3-yl)-piperidin-1-yl]-butyraldehyd (Zwischenprodukt 36) in 15 mL DMF wurde 391 mg (1.200 mmol) 4-(3-Dimethylamino-propyl)-*N*¹-ethyl-benzol-1,2-diamin (Zwischenprodukt 29) zugegeben, das Gemisch mit halbkonz. wässeriger HCl auf pH 4-5 angesäuert und unter Luftatmosphäre 3 Stunden gerührt. Das Reaktionsgemisch wurde auf ges. wässerige Natriumbicarbonat-Lösung gegossen und der gebildete Niederschlag abfiltriert. Das Rohprodukt wurde mittels Säulenchromatographie (Kieselgel, Dichlormethan / MeOH / konz. wässeriger Ammoniak 90 / 10 / 3 v/v/v) gereinigt.
Ausbeute: 280 mg (24% der Theorie)
R_{f} = 0.37 (Kieselgel, Dichlormethan / Cyclohexan / MeOH / ges. wässeriger Ammoniak 70 / 15 / 15 / 2 v/v/v/v)
ESI-MS: (M+H)⁺ = 738/740 (Cl)

### Beispiel 64

### 3-(1-{4-(4-Amino-3-chlor-5-trifluormethyl-phenyl)-3-[1-(3-diethylamino-propyl)-1H-benzimidazol-2-yl]-butyryl}-piperidin-4-yl)-1,3,4,5-tetrahydro-1,3-benzodiazepin-2-on

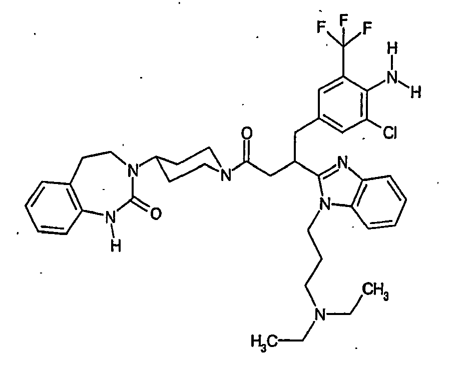

Das Produkt wurde analog zu Beispiel 62 ausgehend von 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-butyraldehyd (Zwischenprodukt 36) und *N*-(3-Diethylamino-propyl)-benzol-1,2-diamin-trihydrochlorid (Zwischenprodukt 39) erhalten.
Ausbeute: 65% der Theorie
ESI-MS: (M+H)⁺ = 738/740 (Cl)

### Beispiel 65

### 3-(1-{4-(4'-Amino-3-chlor-5-trifluormethyl-phenyl)-3-[1-(4-hydroxy-butyl)-1H-benzimidazol-2-yl]-butyryl}-piperidin-4-yl)-1,3,4,5-tetrahydro-1,3-benzodiazepin-2-on

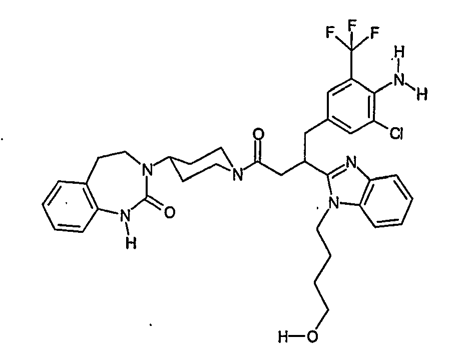

Das Produkt wurde analog zu Beispiel 62 ausgehend von 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-butyraldehyd (Zwischenprodukt 36) und 4-(2-Amino-phenylamino)-butan-1-ol-dihydrochlorid (Zwischenprodukt 30) erhalten.
Ausbeute: 72% der Theorie
ESI-MS: (M+H)⁺ = 697/699 (Cl)

### Beispiel 66

### 3-(1-{4-(4-Amino-3-chlor-5-trifluormethyl-pheny-1)-3-[1-(1-methyl-piperidin-3-ylmethyl)-1H-benzimidazol-2-yl]-butyryl}-piperidin-4-yl)-1,3,4,5-tetrahydro-1,3-benzodiazepin-2-on

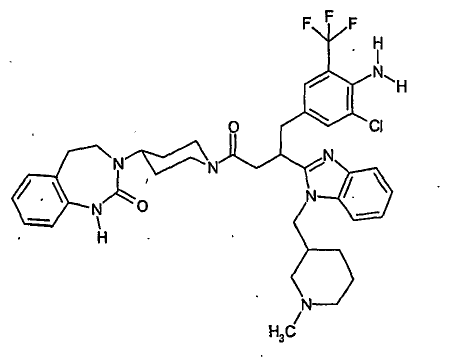

Das Produkt wurde analog zu Beispiel 62 ausgehend von 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-butyraldehyd (Zwischenprodukt 36) und *N*-(1-Methyl-piperidin-3-ylmethyl)-benzol-1,2-diamin-trihydrochlorid (Zwischenprodukt 31) erhalten.
Ausbeute: 64% der Theorie
ESI-MS: (M+H)⁺ = 736/738 (CI)

### Beispiel 67

### 3-[1-(4-(4-Amino-3-chlor-5-trifluormethyl-phenyl)-3-{1-[2-(4-methyl-piperazin-1-yl)-ethyl]-1H-benzimidazol-2-yl}-butyryl)-piperidin-4-yl]-1,3,4,5-tetrahydro-1,3-benzodiazepin-2-on

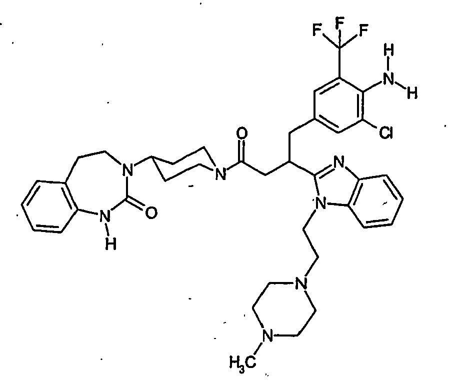

Das Produkt wurde analog zu Beispiel 62 ausgehend von 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-butyraldehyd (Zwischenprodukt 36) und *N*-[2-(4-Methyl-piperazin-1-yl)-ethyl]-benzol-1,2-diamin-tetrahydrochlorid (Zwischenprodukt 32) erhalten.
Ausbeute: 71 % der Theorie
ESI-MS: (M+H)⁺ = 751/753 (CI)

### Beispiel 68

### 3-{1-[4-(4-Amino-3-chlor-5-trifluormethyl-phenyl)-3-(1-pyridin-4-ylmethyl-1H-benzimidazol-2-yl)-butyryl]-piperidin-4-yl}-1,3,4,5-tetrahydro-1,3-benzodiazepin-2-on

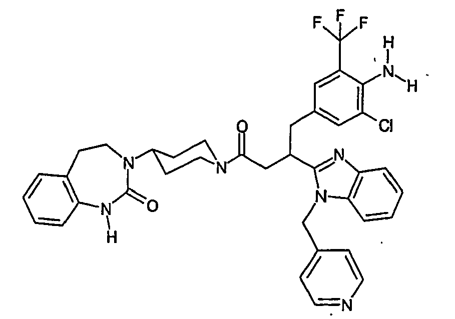

Das Produkt wurde analog zu Beispiel 62 ausgehend von 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-butyraldehyd (Zwischenprodukt 36) und *N*-Pyridin-4-ylmethyl-benzol-1,2-diamin-trihydrochlorid (Zwischenprodukt 33) erhalten.
Ausbeute: 82% der Theorie
ESI-MS: (M+H)⁺ = 716/718 (Cl)

### Beispiel 69

### 3-{1-[4-(4-Amino-3-chlor-5-trifluormethyl-phenyl)-3-(1 -piperidin-4-ylmethyl-1H-benzimidazol-2-yl)-butyryl]-piperidin-4-yl}-1,3,4,5-tetrahydro-1,3-benzodiazepin-2-on

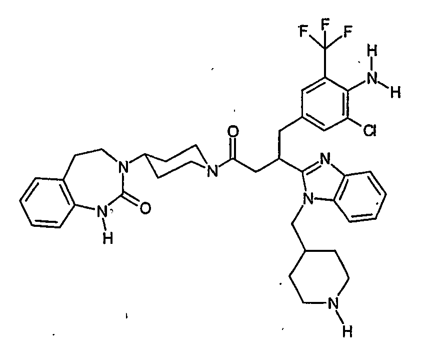

Zu einer Lösung von 537 mg (1.000 mmol) 2-(4-Amino-3-chlor-5-trifluormethylbenzyl)-4-oxo-4-[4-(2-oxo-1,2,4,5-tetrahydro-1,3-benzodiazepin-3-yl)-piperidin-1-yl]-butyraldehyd (Zwischenprodukt 36) in 10 mL DMF wurde 4-[(2-Amino-phenylamino)-methyl]-piperidin-1-carbonsäure-*tert*.-butylester (Zwischenprodukt 34) zugegeben und das Gemisch 16 Stunden unter Luftatmosphäre bei RT gerührt. Das Reaktionsgemisch wurde auf Eiswasser gegossen, der gebildeter Niederschlag abfiltriert, mit Wasser gewaschen und i. vac. getrocknet (Ausbeute: 790 mg, 96% der Theorie). Zu einer Lösung von 580 mg (0.705 mmol) des Rohprodukts in 10 mL Dichlormethan wurde 1 mL TFA zugegeben und das Gemisch 16 Stunden bei RT gerührt. Das Reaktionsgemisch wurde mit Dichlormethan verdünnt, die org. Phase mit ges. wässeriger Natriumbicarbonat-Lösung gewaschen, über Natriumsulfat getrocknet und i. vac. eingeengt. Der Rückstand wurde in 3.5 mL DMF gelöst und mittels HPLC-MS (Zorbax Bonus C18 Amid-Phase 5 µm, Gradient 0.15% Ameisensäure in Wasser / Acetonitril 10 / 90 → 90 / 10 v/v) gereinigt und anschließend lyophilisiert.
Ausbeute: 150 mg (30% der Theorie)
ESI-MS: (M+H)⁺ = 722/724 (Cl)

### Beispiel 70

### 3-(1-{4-(4-Amino-3-chlor-5-trifluormethyl-phenyl)-3-[1-(3-dimethylamino-2,2-dimethylpropyl)-1H-benzimidazol-2-yl]-butyryl}-piperidin-4-yl)-1,3,4,5-tetrahydro-1,3-benzodiazepin-2-on

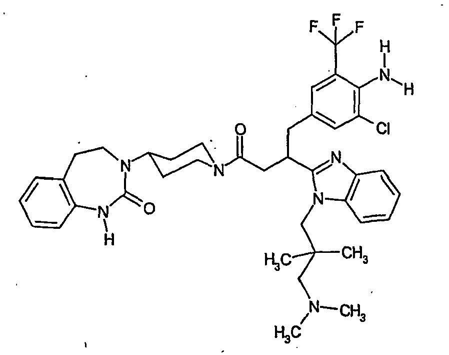

Zu einer Lösung von 320 mg (0.596 mmol) 2-(4-Amino-3-chlor-5-trifluormethylbenzyl)-4-oxo-4-[4-(2-oxo-1,2,4,5-tetrahydro-1,3-benzodiazepin-3-yl)-piperidin-1-yl]-butyraldehyd (Zwischenprodukt 36) in 7 mL DMF wurde 230 mg (0.695 mmol) N-(3-Dimethylamino-2,2-dimethyl-propyl)-benzol-1,2-diamin-trihydrochlorid (Zwischenprodukt 37) zugegeben und unter Luftatmosphäre 16 Stunden gerührt. Das Reaktionsgemisch wurde i. vac. eingeengt und der Rückstand mit EtOAc versetzt. Die org. Phase wurde mit ges. wässeriger Natriumbicarbonat-Lösung gewaschen, über Natriumsulfat getrocknet und i. vac. eingeengt. Das Rohprodukt wurde mittels Säulenchromatographie (Kieselgel, Gradient Dichlormethan / MeOH 20/1 → 10/1 v/v) dann (Alox, neutral, Aktivität II-III, Gradient EtOAc / MeOH 100 /0 → 40 /1) und HPLC-MS (Zorbax Bonus C18 Amid-Phase 5 µm, Gradient 0.15% Ameisensäure in Wasser / Acetonitril 10 / 90 → 90 /10 v/v) gereinigt.
Ausbeute: 71 mg (16% der Theorie)
ESI-MS: (M+H)⁺ = 738/740 (Cl)

### Beispiel 71

### 3-(1-{4-(4-Amino-3-chlor-5-trifluormethyl-phenyl)-3-[7-(3-dimethylamino-propoxy)-1H-benzimidazol-2-yl]-butyryl}-piperidin-4-yl)-1,3,4,5-tetrahydro-1,3-benzodiazepin-2-on

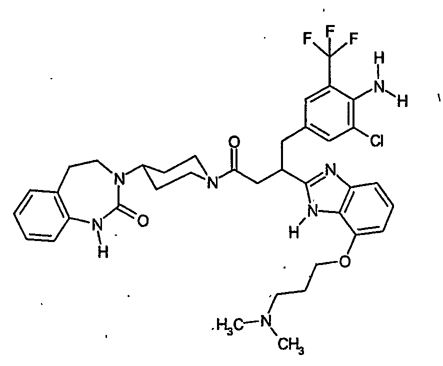

Das Produkt wurde analog zu Beispiel 70 ausgehend von 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-butyraldehyd (Zwischenprodukt 36) und 3-(3-Dimethylamino-propoxy)-benzol-1,2-diamin-trihydrochlorid (Zwischenprodukt 38) erhalten.
Ausbeute: 19% der Theorie
ESI-MS: (M+H)⁺ = 726/728 (Cl)

### Beispiel 72

### 3-{1-[4-(4-Amino-3-chlor-5-trifluormethyl-phenyl)-3-(1-piperidin-4-ylmethyl-1H-imidazo[4,5-c]pyridin-2-yl)-butyryl]-piperidin-4-yl}-1,3,4,5-tetrahydro-1,3-benzodiazepin-2-on

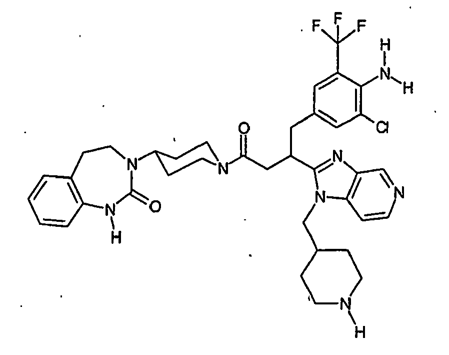

Zu einer Lösung von 537 mg (1.000 mmol) 2-(4-Amino-3-chlor-5-trifluormethylbenzyl)-4-oxo-4-[4-(2-oxo-1,2,4,5-tetrahydro-1,3-benzodiazepin-3-yl)-piperidin-1-yl]-butyraldehyd (Zwischenprodukt 36) in 10 mL DMF wurde 4-[(3-Amino-pyridin-4-yl-amino)-methyl]-piperidin-1-carbonsäure-tert.-butylester (Zwischenprodukt 40) zugegeben und das Gemisch 16 Stunden unter Luftatmosphäre bei 100 °C gerührt. Das Reaktionsgemisch wurde auf Eiswasser gegossen, der gebildeter Niederschlag abfiltriert, mit Wasser gewaschen und i. vac. getrocknet (Ausbeute: 720 mg, 80% der Theorie).
Zu einer Lösung von 690 mg (0.838 mmol) des Rohprodukts in 10 mL Dichlormethan wurde 1 mL TFA zugegeben und das Gemisch 16 Stunden bei RT gerührt. Das Reaktionsgemisch wurde mit Dichlormethan verdünnt, die org. Phase mit ges. wässeriger Natriumbicarbonat-Lösung gewaschen, über Natriumsulfat getrocknet und i. vac. eingeengt. Der Rückstand wurde in DMF gelöst und mittels HPLC-MS (Zorbax Bonus C18 Amid-Phase 5 µm, Gradient 0.15% Ameisensäure in Wasser /Acetonitril 10/90 → 90 / 10 v/v) gereinigt und anschließend lyophilisiert.
Ausbeute: 22 mg (4% der Theorie)
ESI-MS: (M+H)⁺ = 723/725 (Cl)

### Beispiel 73

### 3-(1-{4-(4-Amino-3-chlor-5-trifluormethyl-phenyl)-3-[3-(3-pyrrolidin-1-yl-propyl)-3H-imidazo[4,5-c]pyridin-2-yl]-butyryl}-piperidin-4-yl)-1,3,4,5-tetrahydro-1,3-benzodiazepin-2-on

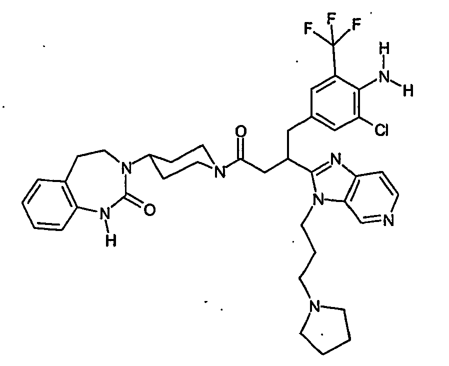

Das Produkt wurde analog zu Beispiel 62 ausgehend von 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-butyraldehyd (Zwischenprodukt 36) und *N*³-(3-Pyrrolidin-1-yl-propyl)-pyridine-3,4-diamin (Zwischenprodukt 41) bei 100 °C hergestellt und mittels HPLC-MS (Zorbax Bonus C18 Amid-Phase 5 µm, Gradient 0.15% Ameisensäure in Wasser / Acetonitril 10 / 90 → 90 / 10 v/v) gereinigt. Lyophilisierung einer 1 N wässerigen Lösung des Produkts ergab das entsprechende Hydrochlorid.
Ausbeute: 11 % der Theorie
ESI-MS: (M+H)⁺ = 737/739 (Cl)

### Beispiel 74

### 3-{1-[4-(4-Amino-3-chlor-5-trifluormethyl-phenyl)-3-(1-methyl-6-pyrrolidin-1-ylmethyl-1H-benzimidazol-2-yl)-butyryl]-piperidin-4-yl}-1,3,4,5-tetrahydro-1,3-benzodiazepin-2-on

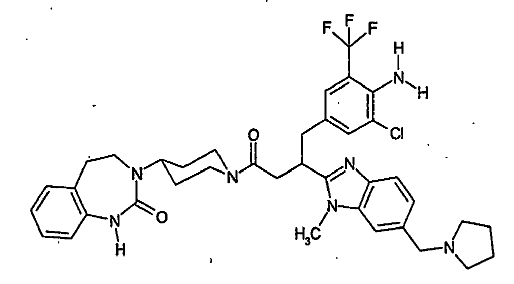

Das Produkt wurde analog zu Beispiel 62 ausgehend von 2-(4-Amino-3-chlor-5-trifluormethyl-benzyl)-4-oxo-4-[4-(2-oxo-1,2,4,5-tetrahydro-1,3-benzodiazepin-3-yl)-piperidin-1-yl]-butyraldehyd (Zwischenprodukt 36) und *N*²-Methyl-4-pyrrolidin-1-ylmethyl-benzol-1,2-diamin (Zwischenprodukt 42) bei 100 °C hergestellt und mittels HPLC-MS (Zorbax Bonus C18 Amid-Phase 5 µm, Gradient 0.15% Ameisensäure in Wasser / Acetonitril 10 / 90 → 90 / 10 v/v) gereinigt.
Ausbeute: 11 % der Theorie
HPLC-MS: Rₜ = 5,622 Min. (Zorbax C18, Gradient 0.1% Ameisensäure in Wasser /Acetonitril / Ameisensäure 10 / 90 → 90 / 10 v/v/v)
ESI-MS: (M+H)⁺ = 722/724 (Cl)

### Beispiel 75

### Enantiomere von 3-(1-{4-(4-Amino-3-chlor-5-trifluormethyl-phenyl)-3-[1-(3-diethylamino-propyl)-1H-benzimidazol-2-yl]-butyryl}-piperidin-4-yl)-1,3,4,5-tetrahydro-1,3-benzodiazepin-2-on

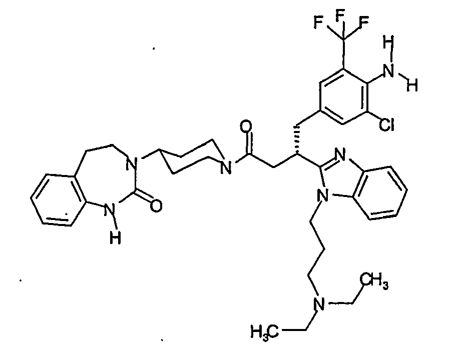

Die Enantiomere wurde ausgehend von Beispiel 64 mittels HPLC (Chiralcel OD 250^{*}4.6, Hexan / EtOH / DEA 70 / 30 / 0.1 v/v/v), Rₜ(1) = 14.25 Min., Rₜ(2) = 17.17 Min.) erhalten.
ESI-MS: (M+H)⁺ = 738/740 (Cl)

### Beispiel 76

### Enantiomere von 3-[1-(4-(4-Amino-3-chlor-5-trifluormethyl-phenyl)-3-{1-[2-(4-methylpiperazin-1-yl)-ethyl]-1H-benzimidazol-2-yl}-butyryl)-piperidin-4-yl]-1,3,4,5-tetrahydro-1,3-benzodiazepin-2-on

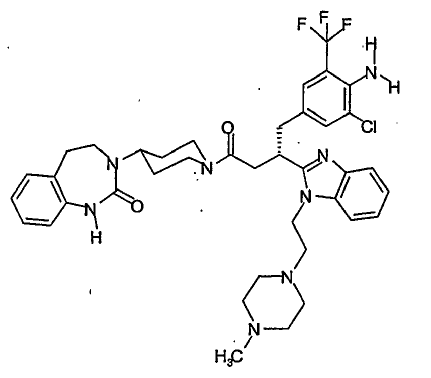

Die Enantiomere wurde ausgehend von Beispiel 67 mittels HPLC (Chiralcel OD 250*4.6, Hexan / EtOH / DEA 70 / 30 / 0.1 v/v/v), Rₜ(1) = 21.75 Min., Rₜ(2) = 24.86 Min.) erhalten.
ESI-MS: (M+H)⁺ = 751/753 (Cl)

Die nachfolgenden Beispiele beschreiben die Herstellung pharmazeutischer Anwendungsformen, die als Wirkstoff eine beliebige Verbindung der allgemeinen Formel (I) enthalten:

### Beispiel 77

### Kapseln zur Pulverinhalation mit 1 mg Wirkstoff

| Zusammensetzung: | |
|---|---|
| 1 Kapsel zur Pulverinhalation enthält: | |
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel 78

### Inhalationslösung für Respimat® mit 1 mg Wirkstoff

| Zusammensetzung: | |
|---|---|
| 1 Hub enthält: | |
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel 79

### Inhalationslösung für Vernebler mit 1 mg Wirkstoff

| Zusammensetzung: | |
|---|---|
| 1 Fläschchen enthält: | |
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

Wirkstoff, Natriumchlorid und Benzalkoniumchlorid werden in Wasser gelöst.

### Beispiel 80

### Treibqas-Dosieraerosol mit 1 mg Wirkstoff

| Zusammensetzung: | |
|---|---|
| 1 Hub enthält: | |
| Wirkstoff | 1.0 mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel 81

### Nasalspray mit 1 mg Wirkstoff

| Zusammensetzung: | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel 82

### Injektionslösung mit 5 mg Wirksubstanz pro 5 ml

| Zusammensetzung: | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel 83

### Injektionslösung mit 100 mg Wirksubstanz pro 20 ml

| Zusammensetzung: | |
|---|---|
| Wirksubstanz | 100 mg |
| Monokaliumdihydrogenphosphat = KH₂PO₄ | 12 mg |
| Dinatriumhydrogenphosphat = Na₂HPO₄**·**2H₂O | 2 mg |
| Natriumchlorid | 180 mg |
| Human-Serum-Albumin | 50 mg |
| Polysorbat 80 | 20 mg |
| Wasser für Injektionszwecke ad | 20 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel 84

### Lyophilisat mit 10 mg Wirksubstanz

| Zusammensetzung: | |
|---|---|
| Wirksubstanz | 10 mg |
| Mannit | 300 mg |
| Human-Serum-Albumin | 20 mg |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

| Lösungsmittel für Lyophilisat: | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel 85

### Tabletten mit 20 mg Wirksubstanz

| Zusammensetzung: | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

### Beispiel 86

### Kapseln mit 20 mg Wirksubstanz

| Zusammensetzung: | |
|---|---|
| Wirksubstanz | 20 mg |
| Maisstärke | 80 mg |
| Kieselsäure. hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Grösse 3 abfüllen.

### Beispiel 87

### Zäpfchen mit 50 mg Wirksubstanz

| Zusammensetzung: | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgiessen.

### Beispiel 88

### Injektionslösung mit 10 mg Wirksubstanz pro 1 ml

| Zusammensetzung: | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. Verbindungen der allgemeinen Formel 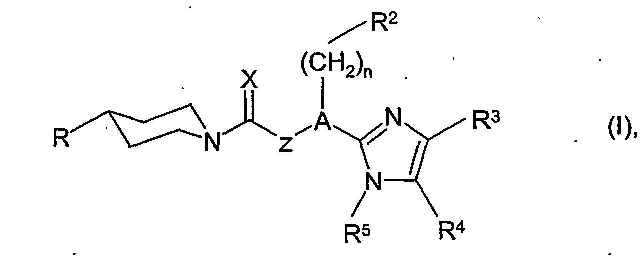 in der
R einen gesättigten, einfach ungesättigten oder, im Fall der 6- und 7-gliedrigen Heterocyclen, auch zweifach ungesättigten 5- bis 7-gliedrigen Aza-, Diaza-, Triaza-, Oxaza-; Thiaza-, Thiadiaza- oder S,S-Dioxido-thiadiaza-Heterocyclus,
wobei die vorstehend erwähnten Heterocyclen über ein Kohlenstoff- oder Stickstoffatom verknüpft sind und
ein oder zwei Carbonylgruppen enthalten, die mit mindestens einem Stickstoffatom verknüpft sind,
an einem der Stickstoffatome durch eine Alkylgruppe substituiert sein können,
an einem oder an zwei Kohlenstoffatomen jeweils durch eine Alkyl-, Phenyl-, Phenylmethyl-, Naphthyl-, Biphenylyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolylgruppe substituiert sein können, wobei die Substituenten gleich oder verschieden sein können,
und wobei eine Doppelbindung eines der vorstehend erwähnten ungesättigten Heterocyclen mit einem Benzol-, Pyridin-, Diazin-, 1,3-Oxazol-, Thiophen-, Furan-, Thiazol-, Pyrrol-, *N*-Methyl-pyrrol- oder Chinolin-Ring, mit einem gegebenenfalls am Stickstoffatom durch eine Alkylgruppe substituierten 2(1*H*)-Oxochinolin-Ring oder mit einem Imidazol- oder *N*-Methyl-imidazol-Ring kondensiert sein kann oder auch zwei olefinische Doppelbindungen eines der vorstehend erwähnten ungesättigten Heterocyclen jeweils mit einem Benzolring kondensiert sein können,
wobei die in R enthaltenen Phenyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolylgruppen sowie benzo-, thieno-, pyrido- und diazinokondensierten Heterocyclen im Kohlenstoffgerüst zusätzlich durch Fluor-, Chlor-, Brom- oder lodatome, durch Alkyl-, Alkoxy-, Nitro-, Alkylthio-, Alkylsulfinyl-, Alkylsulfonyl-, Alkylsulfonylamino-, Phenyl-, Trifluormethyl-, Difluormethyl-, Difluormethoxy-, Alkoxycarbonyl-, Carboxy-, Dialkylamino-, Hydroxy-, Amino-, Acetylamino-, Propionylamino-, Aminocarbonyl-, Alkylaminocarbonyl-, Dialkylaminocarbonyl-, (4-Morpholinyl)carbonyl-, (1-Pyrrolidinyl)carbonyl-, (1-Piperidinyl)carbonyl-, (Hexahydro-1-azepinyl)carbonyl-, (4-Methyl-1-piperazinyl)carbonyl-, Methylendioxy-, Aminocarbonylamino-, Alkanoyl-, Cyan-, Trifluormethoxy-, Trifluormethylthio-, Trifluormethylsulfinyl-oder Trifluormethylsulfonylgruppen mono-, di- oder trisubstituiert sein können, wobei die Substituenten gleich oder verschieden sein können,
X ein Sauerstoffatom oder, sofern Z die Gruppe -NR¹- bedeutet, auch eine der Gruppen =N-CN oder =N-SO₂-R⁶, worin
R⁶ einen Alkylrest mit 1 bis 4 Kohlenstoffatomen oder einen gegebenenfalls durch ein Halogenatom, eine Methyl- oder eine Methoxygruppe substituierten Phenylrest darstellt,
Z eine der Gruppen -CH₂-, in der die Wasserstoffatome unabhängig voneinander durch ein Fluoratom oder eine C₁₋₃-Alkylgruppe ersetzt sein können, oder -NR¹-, in der
R¹ das Wasserstoffatom, eine Alkylgruppe, die im Alkylteil mit Ausnahme der Position 1 durch eine Amino-, C₁₋₃-Alkyl-amino- oder Di-(C₁₋₃-alkyl)-aminogruppe substituiert sein kann, oder eine Phenylalkylgruppe, die im Phenylteil durch Fluor-, Chlor-, Brom- oder lodatome, durch C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe mono- oder disubstituiert sein kann, wobei die Substituenten gleich oder verschieden sein können, darstellt,
A ein durch ein Wasserstoffatom oder durch eine C₁₋₃-Alkylgruppe substituiertes Kohlenstoffatom
n die Zahl 1 oder 2,
R² die Gruppe 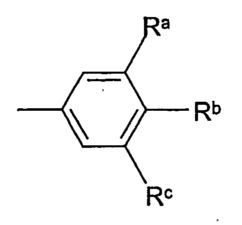 in der
eine der Gruppen R^{a}, R^{b} und R^{c} das Wasserstoff-, Fluor-, Chlor-, Brom- oder lodatom, eine verzweigte oder unverzweigte Alkylgruppe, eine Hydroxy-, Alkoxy-, Trifluormethyl-, Difluormethyl-, Trifluormethoxy-, Difluormethoxy-, Trifluormethylthio-, Amino-, Acetylamino-, Dialkylaminoalkyl-, Dialkylaminoalkoxy-, Nitro-, Methylsulfonyloxy-, Aminocarbonyl-, Alkylaminocarbonyl-, Dialkylaminocarbonyl-, Alkanoyl-, Cyan-, Trifluormethylsulfinyl- oder. Trifluormethylsulfonylgruppe,
eine zweite der Gruppen R^{a}, R^{b} und R^{c} das Wasserstoff-, Fluor-, Chlor-, Brom-oder lodatom, eine verzweigte oder unverzweigte Alkylgruppe, eine Hydroxy-, Alkoxy-, Trifluormethyl-, Difluormethyl-, Trifluormethoxy-, Difluormethoxy-, Trifluormethylthio-, Amino-, Acetylamino-, Alkanoyl-, Aminocarbonyl-, Alkylaminocarbonyl- oder Dialkylaminocarbonylgruppe und
die dritte der Gruppen R^{a}, R^{b} und R^{c} das Wasserstoff-, Fluor-, Chlor-, Brom-oder lodatom, eine verzweigte oder unverzweigte Alkylgruppe, eine Hydroxy-, Alkoxy-, Trifluormethyl-, Difluormethyl-, Difluormethoxy-, oder Trifluormethoxygruppe darstellen, wobei die Substituenten gleich oder verschieden sein können,
R³ und R⁴, die gleich oder verschieden sein können, jeweils das Wasserstoffatom, eine C₁₋₄-Alkyl-, Amino-C₁₋₃-alkyl-, C₁₋₃-Alkyl-amino-C₁₋₃-alkyl oder Di-(C₁₋₃-alkyl)-amino-C₁₋₃-alkylgruppe,
eine Phenyl- oder Naphthylgruppe, die jeweils durch R^{d} mono- oder disubstituiert sein können, wobei die Substituenten gleich oder verschieden sein können und R^{d} ein Fluor-, Chlor-, Brom- oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe oder eine 4- bis 7-gliedrige Cycloalkylenimino-carbonylgruppe, in der
ein oder zwei Wasserstoffatome jeweils durch eine C₁₋₃-Alkylgruppe ersetzt sein können oder/und
jeweils die Methylengruppe in Position 4 eines 6- oder 7-gliedrigen Cycloalkyleniminoteils durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)-gruppe ersetzt sein kann, darstellt,
eine im Kohlenstoffgerüst gegebenenfalls durch eine C₁₋₃-Alkyl-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte monocyclische 5- oder 6-gliedrige Heteroarylgruppe, wobei
die 6-gliedrige Heteroarylgruppe ein, zwei oder drei Stickstoffatome enthält und gegebenenfalls zusätzlich durch ein Fluor-, Chlor-, Brom- oder lodatom, eine C₁₋₃-Alkyl-, C₁₋₃-Alkoxy-, Amino-, C₁₋₃-Alkylamino- oder Di-(C₁₋₃-alkyl)aminogruppe substituiert sein kann und
die 5-gliedrige Heteroarylgruppe eine gegebenenfalls durch eine C₁₋₃-Alkyl-, Acetyl-, Trifluoracetyl-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe, ein Sauerstoff- oder Schwefelatom oder
eine gegebenenfalls durch eine C₁₋₃-Alkyl-, Acetyl-, Trifluoracetyl-, C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl-, Acetyl-, Trifluoracetyl-, C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe und zwei Stickstoffatome enthält,
oder R³ und R⁴ zusammen eine 1,3-Butadien-1,4-ylengruppe, in der
eine, zwei oder drei Methingruppen jeweils durch ein Stickstoffatom ersetzt sein können,
ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Di-(C₁₋₃-alkyl)-amino-C₂₋₃-alkyl-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe oder durch eine 4- bis 7-gliedrige Cycloalkylenimino-carbonylgruppe, in der
ein oder zwei Wasserstoffatome jeweils durch eine C₁₋₃-Alkylgruppe ersetzt sein können oder/und
jeweils die Methylengruppe in Position 4 eines 6- oder 7-gliedrigen Cycloalkyleniminoteils durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)-gruppe ersetzt sein kann,
und gegebenenfalls zusätzlich ein zweites Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy- oder Trifluormethoxygruppe ersetzt sein kann, und
R⁵ die Gruppe -(CH₂)ₘ-R^{e}, in der
m die Zahl 0 und
R^{e} ein Wasserstoffatom,
eine Phenyl- oder Naphthylgruppe, die jeweils durch R^{f} mono- oder disubstituiert sein können, wobei die Substituenten gleich oder verschieden sein können und R^{f} ein Fluor-, Chlor-, Brom- oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt, oder
eine im Kohlenstoffgerüst gegebenenfalls durch eine C₁₋₃-Alkylgruppe substituierte Pyridinyl-, Pyrazinyl-, Pyrimidinyl-, Pyridazinyl-, Pyrrolyl-, Furyl-, Thienyl-, Pyrazolyl-, Imidazolyl-, Oxazolyl-, Isoxazolyl-, Thiazolyl- oder Isothiazolylgruppe, in denen ein an ein Stickstoffatom gebundenes Wasserstoffatom durch eine C₁₋₃-Alkyl- oder Acetylgruppe ersetzt sein kann, oder
eine C₃₋₇-Cycloalkylgruppe, wobei
ein Wasserstoffatom der C₃₋₇-Cycloalkylgruppe durch eine Amino-, C₁₋₃-Alkyl-amino-, Di-(C₁₋₃-alkyl)-amino-, Amino-C₁₋₃-alkyl-, C₁₋₃-Alkyl-amino-C₁₋₃-alkyl- oder Di-(C₁₋₃-alkyl)-amino-C₁₋₃-alkylgruppe ersetzt sein kann oder/und
die Methylengruppe in Position 3 der Cyclopentylgruppe und die Methylengruppe in Position 4 der Cyclohexyl- und Cycloheptylgruppe jeweils durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)-gruppe ersetzt sein kann,
oder m eine der Zahlen 1 bis 5 und
R^{e} ein Wasserstoffatom, eine Aminomethylen-, C₁₋₃-Alkylaminomethylen-, Di-(C₁₋₃-alkyl)-aminomethylen-, Aminocarbonyl-, C₁₋₃-Alkylaminocarbonyl-, Di-(C₁₋₃-alkyl)-aminocarbonyl- oder C₃₋₇-Cycloalkylgruppe, wobei
ein Wasserstoffatom der C₃₋₇-Cycloalkylgruppe durch eine Amino-, C₁₋₃-Alkyl-amino-, Di-(C₁₋₃-alkyl)-amino-, Amino-C₁-₃-alkyl-, C₁₋₃-Alkyl-amino-C₁₋₃-alkyl- oder Di-(C₁₋₃-alkyl)-amino-C₁₋₃-alkylgruppe ersetzt sein kann oder/und
die Methylengruppe in Position 3 der Cyclopentylgruppe und die Methylengruppe in Position 4 der Cyclohexyl- und Cycloheptylgruppe jeweils durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)-gruppe ersetzt sein kann,
eine 4- bis 7-gliedrige Cycloalkyleniminogruppe, in der
ein oder zwei Wasserstoffatome jeweils durch eine C₁₋₃-Alkylgruppe ersetzt sein können oder/und
jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)-gruppe ersetzt sein kann,
eine Phenyl- oder Naphthylgruppe, die jeweils unabhängig voneinander durch R⁹ mono- oder disubstituiert sein können, wobei die Substituenten gleich oder verschieden sein können und R⁹ ein Fluor-, Chlor-, Brom- oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt,
oder eine im Kohlenstoffgerüst gegebenenfalls durch eine C₁₋₃-Alkyl-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte monocyclische 5- oder 6-gliedrige Heteroarylgruppe, wobei
die 6-gliedrige Heteroarylgruppe ein, zwei oder drei Stickstoffatome und gegebenenfalls zusätzlich durch ein Fluor-, Chlor-, Brom- oder lodatom, eine C₁₋₃-Alkyl-, C₁₋₃-Alkoxy-, Amino-, C₁₋₃-Alkylamino- oder Di-(C₁₋₃-alkyl)aminogruppe substituiert sein kann und
die 5-gliedrige Heteroarylgruppe eine gegebenenfalls durch eine C₁₋₃-Alkyl-, Acetyl-, Trifluoracetyl-, C₁₋₃-Alkoxy-carbonyl-, Aminocarbonyl-, C₁₋₃-Alkylaminocarbonyl-, Di-(C₁₋₃-alkyl)-aminocarbonyl-, Amino-C₁₋₃-alkyl-, C₁₋₃-AlKylamino-C₁₋₃-alkyl oder Di-(C₁₋₃-alkyl)-amino-C₁₋₃-alkylgruppe substituierte Iminogruppe, ein Sauerstoff- oder Schwefelatom oder
eine gegebenenfalls durch eine C₁₋₃-Alkyl-, Acetyl-, Trifluoracetyl-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe oder ein Sauerstoff-oder Schwefelatom und zusätzlich ein Stickstoffatom oder
eine gegebenenfalls durch eine C₁₋₃-Alkyl-, Acetyl-, Trifluoracetyl-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(Di₁₋₃-alkyl)-amino-C₁₋₃-alkylgruppe substituierte Iminogruppe und zwei Stickstoffatome enthält,
darstellen, wobei die vorstehend genannten Alkylgruppen oder die in den vorstehend genannten Resten enthaltenen Alkylgruppen, sofern nichts anderes angegeben wurde, 1 bis 5 Kohlenstoffatome enthalten und verzweigt oder unverzweigt sein können,
deren Tautomere, deren Diastereomere, deren Enantiomere, deren Gemische und deren Salze.

2. Verbindungen der allgemeinen Formel I gemäß Anspruch 1, in der
R einen gesättigten oder einfach ungesättigten 5- bis 7-gliedrigen Aza-, Diaza-, Oxaza- oder Thiaza-Heterocyclus,
wobei die vorstehend erwähnten Heterocyclen über ein Stickstoffatom verknüpft sind und
eine mit einem oder zwei Stickstoffatomen des Heterocyclus verknüpfte Carbonylgruppe enthalten,
an einem der Stickstoffatome durch eine Alkylgruppe substituiert sein können,
an einem der Kohlenstoffatome durch eine Alkyl-, Phenyl-, Pyridinyl-, Furyl-, Thienyl- oder Pyrrolylgruppe substituiert sein können,
und wobei eine Doppelbindung eines der vorstehend erwähnten ungesättigten Heterocyclen mit einem Benzol-, Pyridin- oder Thiophen-Ring kondensiert sein kann,
wobei die in R enthaltenen Phenyl-, Pyridinyl-, Furyl-, Thienyl-, Pyrrolylgruppen sowie die benzo-, thieno- und pyridokondensierten Heterocyclen im Kohlenstoffgerüst zusätzlich durch Fluor-, Chlor-, Brom- oder lodatome, durch Alkyl-, Alkoxy-, Nitro-, Alkylthio-, Trifluormethyl-, Difluormethyl-, Alkoxycarbonyl-, Carboxy-, Dialkylamino-, Hydroxy-, Amino-, Acetylamino-, Aminocarbonyl-, Alkylaminocarbonyl-, Alkanoyl-, Cyan- oder Trifluormethoxygruppen mono- oder disubstituiert sein können, wobei die Substituenten gleich oder verschieden sein können,
X ein Sauerstoffatom oder, sofern Z die Gruppe -NR¹- bedeutet, auch die Gruppe =N-CN,
Z eine der Gruppen -CH₂- oder-NR¹-, in der
R¹ das Wasserstoffatom oder eine Alkylgruppe darstellt,
A ein durch ein Wasserstoffatom oder durch eine C₁₋₃-Alkylgruppe substituiertes Kohlenstoffatom
n die Zahl 1 oder 2,
R² die Gruppe 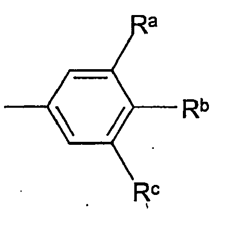 in der
eine der Gruppen R^{a}, R^{b} und R^{c} das Wasserstoff-, Fluor-, Chlor-, Brom- oder lodatom, eine verzweigte oder unverzweigte Alkylgruppe, eine Hydroxy-, Alkoxy-, Trifluormethyl-, Difluormethyl-, Trifluormethoxy-, Amino-, Acetylamino-, Dialkylaminoalkyl-, Dialkylaminoalkoxy-, Aminocarbonyl-, Alkylaminocarbonyl-, Dialkylaminocarbonyl-, Alkanoyl-, Cyan- oder Trifluormethylsulfonylgruppe,
eine zweite der Gruppen R^{a}, R^{b} und R^{c} das Wasserstoff-, Fluor-, Chlor-, Brom-oder lodatom, eine verzweigte oder unverzweigte Alkylgruppe, eine Alkoxy-, Trifluormethyl-, Amino-, Acetylamino- oder Alkanoylgruppe und
die dritte der Gruppen R^{a}, R^{b} und R^{c} das Wasserstoff-, Fluor-, Chlor-, Brom- oder lodatom oder eine verzweigte oder unverzweigte Alkylgruppe darstellen, wobei die Substituenten gleich oder verschieden sein können,
R³ das Wasserstoffatom oder eine C₁₋₃-Alkylgruppe,
R⁴ das Wasserstoffatom, eine C₁₋₄-Alkyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe,
eine Phenylgruppe, die durch R^{d} mono- oder disubstituiert sein kann, wobei die Substituenten gleich oder verschieden sein können und R^{d} ein Fluor-, Chlor-, Brom-oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt, oder
eine im Kohlenstoffgerüst gegebenenfalls durch eine C₁₋₃-Alkylgruppe substituierte Pyridinyl-, Pyrazinyl-, Pyrimidinyl-, Pyridazinyl-, Pyrrolyl-, Furyl-, Thienyl-, Pyrazolyl-, Imidazolyl-, Oxazolyl-, Isoxazolyl-, Thiazolyl- oder Isothiazolylgruppe, in denen ein an ein Stickstoffatom gebundenes Wasserstoffatom durch eine C₁₋₃-Alkyl- oder Acetylgruppe ersetzt sein kann,
oder R³ und R⁴ zusammen eine 1,3-Butadien-1,4-ylengruppe, in der
ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Di-(C₁₋₃-alkyl)-amino-C₂₋₃-alkyl-aminocarbonyl-, Amino-C₁₋₃-alkyl-, C₁₋₃-Alkyl-amino-C₁₋₃-alkyl-, Di-(C₁₋₃-alkyl)-amino-C₁₋₃-alkylgruppe oder durch eine 5- bis 7-gliedrige Cycloalkylenimino-carbonylgruppe, in der
ein oder zwei Wasserstoffatome jeweils durch eine C₁₋₃-Alkylgruppe ersetzt sein können oder/und
jeweils die Methylengruppe in Position 4 eines 6- oder 7-gliedrigen Cycloalkyleniminoteils durch ein Sauerstoff- oder Schwefelatom, durch eine -NH-, -N(C₁₋₃-Alkyl)- oder-N(C₁₋₃-Alkyl-carbonyl)- Gruppe ersetzt sein kann,
und gegebenenfalls zusätzlich ein zweites Wasserstoffatom durch ein Fluor-, Chlor-, Brom- oder lodatom, durch eine C₁₋₃-Alkyl- oder Trifluormethylgruppe ersetzt sein kann, und
R⁵ die Gruppe -(CH₂)ₘ-R^{e}, in der
m die Zahl 0 und
R^{e} ein Wasserstoffatom oder eine C₅₋₆-Cycloalkylgruppe, wobei
die Methylengruppe in Position 3 der Cyclopentylgruppe und die Methylengruppe in Position 4 der Cyclohexylgruppe jeweils durch eine -NH-, -N(C₁₋₃-Alkyl)- oder-N(C₁₋₃-Alkyl-carbonyl)-gruppe ersetzt sein kann,
oder m eine der Zahlen 1 bis 5 und
R^{e} ein Wasserstoffatom, eine Aminomethylen-, C₁₋₃-Alkylaminomethylen-, Di-(C₁₋₃-alkyl)-aminomethylen-, Aminocarbonyl-, C₁₋₃-Alkyl-aminocarbonyl-, Di-(C₁₋₃-alkyl)-aminocarbonyl- oder C₅₋₆-Cycloalkylgruppe, wobei
die Methylengruppe in Position 3 der Cyclopentylgruppe und die Methylengruppe in Position 4 der Cyclohexylgruppe jeweils durch eine -NH-, -N(C₁₋₃-Alkyl)- oder -N(C₁₋₃-Alkyl-carbonyl)-gruppe ersetzt sein kann,
eine 5- bis 6-gliedrige Cycloalkyleniminogruppe, wobei
die Methylengruppe in Position 4 einer 6-gliedrigen Cycloalkyleniminogruppe durch eine -NH-, -N(C₁₋₃-Alkyl)- oder -N(C₁₋₃-Alkyl-carbonyl)-gruppe ersetzt sein kann,
eine Phenylgruppe, die durch R^{g} mono- oder disubstituiert sein kann, wobei die Substituenten gleich oder verschieden sein können und R^{g} ein Fluor-, Chlor-, Brom- oder lodatom, eine C₁₋₃-Alkyl-, Trifluormethyl-, Difluormethyl-, Hydroxy-, C₁₋₃-Alkoxy-, Amino-, C₁₋₃-Alkyl-amino-, Di-(C₁₋₃-alkyl)-amino-, Aminocarbonyl-C₁₋₃-Alkyl-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt,
oder eine im Kohlenstoffgerüst gegebenenfalls durch eine C₁₋₃-Alkylgruppe substituierte Pyridinyl-, Pyrazinyl-, Pyrimidinyl-, Pyridazinyl-, Pyrrolyl-, Furyl-, Thienyl-, Pyrazolyl-, Imidazolyl-, Oxazolyl-, Isoxazolyl-, Thiazolyl- oder lsothiazolylgruppe, in denen ein an ein Stickstoffatom gebundenes Wasserstoffatom durch eine C₁₋₃-Alkyl- oder Acetylgruppe ersetzt sein kann, darstellen,
bedeuten, wobei die vorstehend genannten Alkylgruppen oder die in den vorstehend genannten Resten enthaltenen Alkylgruppen, sofern nichts anderes angegeben wurde, 1 bis 4 Kohlenstoffatome enthalten und verzweigt oder unverzweigt sein können,
deren Tautomere, deren Diastereomere, deren Enantiomere, deren Gemische und deren Salze.

3. Verbindungen der allgemeinen Formel I gemäß Anspruch 2, in denen
R einen einfach ungesättigten, über ein Stickstoffatom verknüpften 5- bis 7-gliedrigen Diaza-Heterocyclus,
wobei die vorstehend erwähnten Heterocyclen eine mit beiden Stickstoffatomen des Heterocyclus verknüpfte Carbonylgruppe enthalten,
an einem der Stickstoffatome und einem der gesättigten Kohlenstoffatome unabhängig voneinander jeweils durch eine C₁₋₃-Alkylgruppe substituiert sein können,
und wobei die Doppelbindung der vorstehend erwähnten ungesättigten Heterocyclen mit einem gegebenenfalls durch ein Fluor-, Chlor-, Brom- oder lodatom, durch eine C₁₋₃-Alkyl-, C₁₋₃-Alkoxy-, Trifluormethyl-, Carboxy-, C₁₋₃-Alkoxycarbonyl-, Amino-, Acetylamino-, Hydroxy-, Aminocarbonyl- oder Alkanoylgruppe substituierten Benzolring kondensiert ist,
X ein Sauerstoffatom,
Z die Gruppe -NR¹-, in der
R¹ das Wasserstoffatom oder eine C₁₋₃-Alkylgruppe darstellt,
A ein durch ein Wasserstoffatom oder durch eine C₁₋₃-Alkylgruppe substituiertes Kohlenstoffatom,
n die Zahl 1,
R² die Gruppe 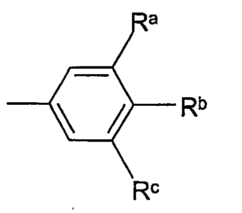 in der
eine der Gruppen R^{a}, R^{b} und R^{c} das Wasserstoff-, Fluor-, Chlor-, Brom- oder lodatom, eine C₁₋₃-Alkyl-, Trifluormethyl-, Hydroxy-, Alkoxy- oder Aminogruppe,
eine zweite der Gruppen R^{a}, R^{b} und R^{c} das Wasserstoff-, Fluor-, Chlor-, Brom-oder lodatom, eine C₁₋₃-Alkyl- oder Trifluormethylgruppe und
die dritte der Gruppen R^{a}, R^{b} und R^{c} das Wasserstoff-, Fluor-, Chlor-, Brom- oder lodatom darstellen, wobei die Substituenten gleich oder verschieden sein können,
R³ das Wasserstoffatom oder eine C₁₋₃-Alkylgruppe,
R⁴ eine gegebenenfalls durch ein Fluor-, Chlor-, Brom- oder lodatom, durch eine C₁₋₃-Alkyl-, Trifluormethyl-, Hydroxy-, C₁₋₃-Alkoxy-, Amino-, C₁₋₃-Alkylamino-, Di-(C₁₋₃-alkyl)-amino-, Carboxy- oder C₁₋₃-Alkoxy-carbonylgruppe substitiuierte Phenylgruppe,
oder R³ und R⁴ zusammen eine 1,3-Butadien-1,4-ylengruppe, in der
ein Wasserstoffatom durch ein Fluor-, Chlor-, Brom- oder lodatom, durch eine C₁₋₃-Alkyl-, Trifluormethyl-, Hydroxy-, C₁₋₃-Alkoxy-, Carboxy-, C₁₋₃-Alkoxycarbonyl-, Aminocarbonyl-, C₁₋₃-Alkyl-aminocarbonyl-, Di-(C₁₋₃-alkyl)-aminocarbonyl- oder Di-(C₁₋₃-alkyl)-amino-C₂₋₃-alkyl-aminocarbonylgruppe oder durch eine 5- bis 7-gliedrige Cycloalkylenimino-carbonylgruppe, wobei
die Methylengruppe in Position 4 des 6-gliedrigen Cycloalkyleniminoteils durch eine -NH-, -N(C₁₋₃-Alkyl)- oder -N(C₁₋₃-Alkyl-carbonyl)- Gruppe ersetzt sein kann,
und gegebenenfalls zusätzlich ein zweites Wasserstoffatom durch ein Fluor-, Chlor-, Brom- oder lodatom oder durch eine C₁₋₃-Alkylgruppe ersetzt sein kann, und
R⁵ die Gruppe -(CH₂)ₘ,-R^{e}, in der
m die Zahl 0 und
R^{e} ein Wasserstoffatom,
oder m eine der Zahlen 1 bis 3 und
R^{e} ein Wasserstoffatom, eine Aminomethylen-, C₁₋₃-Alkylaminomethylen- oder Di-(C₁₋₃-alkyl)-aminomethylengruppe darstellen,
bedeuten, wobei die vorstehend genannten Alkylgruppen oder die in den vorstehend genannten Resten enthaltenen Alkylgruppen, sofern nichts anderes angegeben wurde, verzweigt oder unverzweigt sein können,
deren Tautomere, deren Diastereomere, deren Enantiomere, deren Gemische und deren Salze.

4. Verbindungen der allgemeinen Formel I gemäß Anspruch 1, in der
R, X, Z, A, n, R², und R⁵ wie in Anspruch 1 erwähnt definiert sind und
R³ das Wasserstoffatom oder eine C₁₋₄-Alkylgruppe und
R⁴ das Wasserstoffatom, eine C₁₋₄-Alkyl-, Amino-C₁₋₃-alkyl-, C₁₋₃-Alkyl-amino-C₁₋₃-alkyl- oder Di-(C₁₋₃-alkl)-amino-C₁₋₃-alkylgruppe,
eine Phenyl- oder Naphthylgruppe, die jeweils unabhängig voneinander durch R^{d} mono- oder disubstituiert sein können, wobei die Substituenten gleich oder verschieden sein können und R^{d} ein Fluor-, Chlor-, Brom- oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt, oder
eine im Kohlenstoffgerüst gegebenenfalls durch eine C₁₋₃-Alkyl-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte monocyclische 5- oder 6-gliedrige Heteroarylgruppe, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Acetyl-, Trifluoracetyl-, C₁₋₃-Alkoxy-carbonyl-, Aminocarbonyl-, C₁₋₃-Alkylaminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe, ein Sauerstoff- oder Schwefelatom oder
eine gegebenenfalls durch eine C₁₋₃-Alkyl-, Acetyl-, Trifluoracetyl- C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl-, Acetyl-, Trifluoracetyl- C₁₋₃-Alkoxycarbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe substituierte Iminogruppe und zwei Stickstoffatome enthält,
bedeuten, deren Tautomere, deren Diastereomere, deren Enantiomere, deren Gemische und deren Salze.

5. Verbindungen der allgemeinen Formel I gemäß Anspruch 2, in denen
R, X, Z, A, n, R², und R⁵ wie in Anspruch 2 erwähnt definiert sind und
R³ das Wasserstoffatom oder eine C₁₋₃-Alkylgruppe und
R⁴ eine Phenylgruppe, die durch R^{d} mono- oder disubstituiert sein kann, wobei die Substituenten gleich oder verschieden sein können und R^{d} ein Fluor-, Chlor-, Brom-oder lodatom, eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Amino-, C₁-₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe darstellt, oder
eine im Kohlenstoffgerüst gegebenenfalls durch eine C₁₋₃-Alkylgruppe substituierte Pyridinyl-, Pyrazinyl-, Pyrimidinyl-, Pyridazinyl-, Pyrrolyl-, Furyl-, Thienyl-, Pyrazolyl-, Imidazolyl-, Oxazolyl-, Isoxazolyl-, Thiazolyl- oder Isothiazolylgruppe, in denen ein an ein Stickstoffatom gebundenes Wasserstoffatom durch eine C₁₋₃-Alkyl- oder Acetylgruppe ersetzt sein kann, bedeuten,
deren Tautomere, deren Diastereomere, deren Enantiomere, deren Gemische und deren Salze.

6. Verbindungen der allgemeinen Formel l gemäß Anspruch 3, in denen
R, X, Z, A, n, R², und R⁵ wie in Anspruch 3 erwähnt definiert sind und
R³ das Wasserstoffatom oder eine C₁₋₃-Alkylgruppe und
R⁴ eine gegebenenfalls durch ein Fluor-, Chlor-, Brom- oder lodatom, durch eine C₁₋₃-Alkyl-, Trifluormethyl-, Hydroxy-, C₁₋₃-Alkoxy-, Amino-, C₁₋₃-Alkylamino-, Di-(C₁₋₃-alkyl)-amino-, Carboxy- oder C₁₋₃-Alkoxy-carbonylgruppe substitiuierte Phenylgruppe bedeuten,
deren Tautomere, deren Diastereomere, deren Enantiomere, deren Gemische und deren Salze.

7. Verbindungen der allgemeinen Formel I gemäß Anspruch 1, in denen
R, X, Z, A, n, R², und R⁵ wie in Anspruch 1 erwähnt definiert sind und
R³ und R⁴ zusammen eine 1,3-Butadien-1,4-ylengruppe, in der
eine, zwei oder drei Methingruppen jeweils durch ein Stickstoffatom ersetzt sein können,
ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Di-(C₁₋₃-alkyl)-amino-C₂₋₃-alkyl-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe oder durch eine 4- bis 7-gliedrige Cycloalkylenimino-carbonylgruppe, in der
ein oder zwei Wasserstoffatome jeweils durch eine C₁₋₃-Alkylgruppe ersetzt sein können oder/und
jeweils die Methylengruppe in Position 4 eines 6- oder 7-gliedrigen Cycloalkyleniminoteils durch ein Sauerstoff- oder Schwefelatom, durch eine Sulfinyl-, Sulfonyl-, -NH-, -N(C₁₋₃-Alkyl)- oder-N(C₁₋₃-Alkyl-carbonyl)-Gruppe ersetzt sein kann,
und gegebenenfalls zusätzlich ein zweites Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy- oder Trifluormethoxygruppe ersetzt sein kann,
bedeuten, deren Tautomere, deren Diastereomere, deren Enantiomere, deren Gemische und deren Salze.

8. Verbindungen der allgemeinen Formel I gemäß Anspruch 2, in denen
R, X, Z, A, n, R², und R⁵ wie in Anspruch 2 erwähnt definiert sind und
R³ und R⁴ zusammen eine 1,3-Butadien-1,4-ylengruppe, in der
ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl- oder Di-(C₁₋₃-alkyl)-amino-C₂₋₃-alkyl-aminocarbonylgruppe oder durch eine 5- bis 7-gliedrige Cycloalkylenimino-carbonylgruppe, in der
ein oder zwei Wasserstoffatome jeweils durch eine C₁₋₃-Alkylgruppe ersetzt sein können oder/und
jeweils die Methylengruppe in Position 4 eines 6- oder 7-gliedrigen Cycloalkyleniminoteils durch ein Sauerstoff- oder Schwefelatom, durch eine -NH-, -N(C₁₋₃-Alkyl)- oder-N(C₁₋₃-Alkyl-carbonyl)- gruppe ersetzt sein kann,
und gegebenenfalls zusätzlich ein zweites Wasserstoffatom durch ein Fluor-, Chlor-, Brom- oder lodatom, durch eine C₁₋₃-Alkyl- oder Trifluormethylgruppe ersetzt sein kann, bedeuten,
deren Tautomere, deren Diastereomere, deren Enantiomere, deren Gemische und deren Salze.

9. Verbindungen der allgemeinen Formel I gemäß Anspruch 3, in denen
R, X, Z, A, n, R², und R⁵ wie in Anspruch 3 erwähnt definiert sind und
R³ und R⁴ zusammen eine 1,3-Butadien-1,4-ylengruppe, in der
ein Wasserstoffatom durch ein Fluor-, Chlor-, Brom- oder lodatom, durch eine C₁₋₃-Alkyl-, Trifluormethyl-, Hydroxy-, C₁₋₃-Alkoxy-, Carboxy-, C₁₋₃-Alkoxycarbonyl-, Aminocarbonyl-, C₁₋₃-Alkyl-aminocarbonyl-, Di-(C₁₋₃-alkyl)-aminocarbonyl- oder Di-(C₁₋₃-alkyl)-amino-C₂₋₃-alkyl-aminocarbonylgruppe oder durch eine 5- bis 7-gliedrige Cycloalkylenimino-carbonylgruppe, wobei
die Methylengruppe in Position 4 des 6-gliedrigen Cycloalkyleniminoteils durch eine -NH-, -N(C₁₋₃-Alkyl)- oder-N(C₁₋₃-Alkyl-carbonyl)- gruppe ersetzt sein kann,
und gegebenenfalls zusätzlich ein zweites Wasserstoffatom durch ein Fluor-, Chlor-, Brom- oder lodatom oder durch eine C₁₋₃-Alkylgruppe ersetzt sein kann,
bedeuten, deren Tautomere, deren Diastereomere, deren Enantiomere, deren Gemische und deren Salze.

10. Folgende Verbindungen der allgemeinen Formel I gemäß Anspruch 1:
(1) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(3-dimethylamino-propyl)-1*H-*benzimidazol-2-yl]-ethyl}-amid,
(2) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(2-dimethylamino-ethyl)-1*H-*benzimidazol-2-yl]-ethyl}-amid,
(3) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-methyl-1*H*-benzimidazol-2-yl)-ethyl]-amid,
(4) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1*H*-benzimidazol-2-yl)-ethyl]-amid,
(5) 2-((*R*)-2-(4-Amino-3,5-dibrom-phenyl)-1-{[4-(2-oxo-1 ,4-dihydro-3*H*-chinazolin-3-yl)-piperidin-1-carbonyl]-amino}-ethyl)-1H-benzimidazol-5-carbonsäuremethylester,
(6) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1H-benzimidazol-5-carbonsäuremethylester,
(7) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimidazol-5-carbonsäure,
(8) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[6-(4-methyl-piperazin-1-carbonyl)-1*H-*benzimidazol-2-yl]-ethyl}-amid,
(9) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimidazol-5-carbonsäure-(3-dimethylamino-propyl)-amid,
(10) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1*H*-benzimidazol-5-carbonsäure-(2-dimethylamino-ethyl)-amid,
(11) 4-(2-Oxo-1,4-dihydro-2*H*-chinazolin-3-yl)-piperidin-1-carbonsäure-{(*R*)-2-(4-amino-3,5-dibrom-phenyl)-1-[1-(3-dimethylamino-propyl)-5-phenyl-1*H*-imidazol-2-yl]-ethyl}-amid,
(12) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-butyl-1*H*-benzimidazol-2-yl)-ethyl]-amid,
(13) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(3-pyrrolidin-1-yl-propyl)-1*H-*benzimidazol-2-yl]-ethyl}-amid,
(14) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-pyridin-3-ylmethyl-1*H*-benzimidazol-2-yl)-ethyl]-amid,
(15) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-benzyl-1*H*-benzimidazol-2-yl)-ethyl]-amid,
(16) 4-(2-Oxo-1,2,4,5-tetrahydro-1,3-benzodiazepin-3-yl)-piperidin-1-carbonsäure {(*R*)-2-(4-amino-3,5-dibrom-phenyl)-1-[1-(1-methyl-piperidin-4-ylmethyl)-1*H-*benzimidazol-2-yl]-ethyl}-amid,
(17) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(4-dimethylamino-butyl)-1*H-*benzimidazol-2-yl]-ethyl}-amid,
(18) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(1-methyl-piperidin-4-yl)-1*H-*benzimidazol-2-yl]-ethyl}-amid,
(19) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-cyclohexyl-1*H*-benzimidazol-2-yl)-ethyl]-amid,
(20) 4-(2-Oxo-1,2,4,5-tetrahydro-1,3-benzodiazepin-3-yl)-piperidin-1-carbonsäure-[(*R*)-2-(4-amino-3,5-dibrom-phenyl)-1-(1-cyclopentyl-1*H*-benzimidazol-2-yl)-ethyl]-amid,
(21) 4-(2-Oxo-1,2,4,5-tetrahydro-1,3-benzodiazepin-3-yl)-piperidin-1-carbonsäure{(*R*)-2-(4-amino-3,5-dibrom-phenyl)-1-[1-(5-dimethylamino-pentyl)-1*H*-benzimidazol-2-yl]-ethyl}-amid,
(22) 4-[2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-benzimidazol-1-yl]-buttersäure-methylester,
(23) 4-(2-Oxo-1,2,4,5-tetrahydro-1,3-benzodiazepin-3-yl)-piperidin-1 -carbonsäure-{(*R*)-2-(4-amino-3,5-dibrom-phenyl)-1-[6-chlor-1-(3-dimethylamino-propyl)-1*H-*benzimidazol-2-yl]-ethyl}-amid,
(24) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(3-dimethylamino-propyl)-6-fluor-1*H-*benzimidazol-2-yl]-ethyl}-amid,
(25) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[1-(3-dimethylamino-propyl)-5-fluor-1*H-*benzimidazol-2-yl]-ethyl}-amid,
(26) 4-(2-Oxo-1,2,4,5-tetrahydro-1,3-benzodiazepin-3-yl)-piperidin-1-carbonsäure-{(*R*)-2-(4-amino-3,5-dibrom-phenyl)-1-[5,6-dichlor-1-(3-dimethylamino-propyl)-1*H*-benzimidazol-2-yl]-ethyl}-amid,
(27) 2-((*R*)-2-(4-Amino-3,5-dibrom-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1H-benzimidazol-5-carbonsäure-methylester,
(28) 2-(2-(4-Amino-3-chlor-5-triflüormethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazole-5-carbonsäure,
(29) 4-(2-Oxo-1,2,4,5-tetrahydro-1,3-benzodiazepin-3-yl)-piperidin-1-carbonsäure-{(*R*)-2-(3,5-dibrom-4-hydroxy-phenyl)-1-[1-(1-methyl-piperidin-4-ylmethyl)-1*H-*benzimidazol-2-yl]-ethyl}-amid,
(30) 4-(2-Oxo-1,2,4,5-tetrahydro-1,3-benzodiazepin-3-yl)-piperidin-1-carbonsäure-{(*R*)-2-(3,5-dibrom-4-hydroxy-phenyl)-1-[1-(1-methyl-piperidin-4-yl)-1*H-*benzimidazol-2-yl]-ethyl}-amid,
(31) 4-(2-Oxo-1,2,4,5-tetrahydro-1,3-benzodiazepin-3-yl)-piperidin-1-carbonsäure-{(*R*)-2-(3,5-dibrom-4-hydroxy-phenyl)-1-[1-(3-dimethylamino-propyl)-1*H-*benzimidazol-2-yl]-ethyl}-amid,
(32) 4-(2-Oxo-1,2,4,5-tetrahydro-1,3-benzodiazepin-3-yl)-piperidin-1-carbonsäure-{(*R*)-2-(3,5-dibrom-4-hydroxy-phenyl)-1-[1-(3-pyrrolidin-1-yl-propyl)-1*H-*benzimidazol-2-yl]-ethyl}-amid,
(33) 4-(2-Oxo-1,2,4,5-tetrahydro-1,3-benzodiazepin-3-yl)-piperidin-1-carbonsäure-[1-[6-chlor-1-(3-dimethylamino-propyl)-1*H*-benzimidazol-2-yl]-2-(3,4-dibromphenyl)-ethyl]-amid,
(34) 4-(2-Oxo-1,2,4,5-tetrahydro-1,3-benzodiazepin-3-yl)-piperidin-1-carbonsäure-{2-(3,4-dibrom-phenyl)-1-[5,6-dichlor-1-(3-dimethylamino-propyl)-1*H-*benzimidazol-2-yl]-ethyl}-amid,
(35) 4-(2-Oxo-1,2,4,5-tetrahydro-1,3-benzodiazepin-3-yl)-piperidin-1-carbonsäure-[1-[7-chlor-1-(3-dimethylamino-propyl)-1*H*-benzimidazol-2-yl]-2-(3,4-dibromphenyl)-ethyl]-amid,
(36) 4-(2-Oxo-1,2,4,5-tetrahydro-1,3-benzodiazepin-3-yl)-piperidin-1-carbonsäure-{2-(3,4-dibrom-phenyl)-1-[1-(3-dimethylamino-propyl)-6-fluor-1*H*-benzimidazol-2-yl]-ethyl}-amid,
(37) 4-(2-Oxo-1,2,4,5-tetrahydro-1,3-benzodiazepin-3-yl)-piperidin-1-carbonsäure-{2-(3,4-dibrom-phenyl)-1-[1-(1-methyl-piperidin-4-yl)-1*H*-benzimidazol-2-yl]-ethyl}-amid,
(38) 4-(2-Oxo-1,2,4,5-tetrahydro-1,3-benzodiazepin-3-yl)-piperidin-1 -carbonsäure-{2-(3,4-dibrom-phenyl)-1-[1-(3-pyrrolidin-1-yl-propyl)-1*H* benzimidazol-2-yl]-ethyl}-amid,
(39) 4-(2-Oxo-1,2,4,5-tetrahydro-1,3-benzodiazepin-3-yl)-piperidin-1-carbonsäure-{2-(3,4-dibrom-phenyl)-1-[1-(1-methyl-piperidin-4-ylmethyl)-1*H*-benzimidazol-2-yl]-ethyl}-amid,
(40) 4-(2-Oxo-1,2,4,5-tetrahydro-1,3-benzodiazepin-3-yl)-piperidin-1-carbonsäure-[2-(3,4-dibrom-phenyl)-1-(1-pyridin-3-ylmethyl-1*H*-benzimidazol-2-yl)-ethyl]-amid,
(41) 4-(2-Oxo-1,2,4,5-tetrahydro-1,3-benzodiazepin-3-yl)-piperidin-1-carbonsäure-{2-(3,4-dibrom-phenyl)-1-[1-(3-dimethylamino-propyl)-1*H*-benzimidazol-2-yl]-ethyl}-amid,
(42) 4-(2-Oxo-1,2,4,5-tetrahydro-1,3-benzodiazepin-3-yl)-piperidin-1-carbonsäure-{2-(3,4-dibrom-phenyl)-1-[1-(4-dimethylamino-butyl)-1*H*-benzimidazol-2-yl]-ethyl}-amid,
(43) 4-(2-Oxo-1,2,4,5-tetrahydro-1,3-benzodiazepin-3-yl)-piperidin-1-carbonsäure-{2-(3,4-diethyl-phenyl)-1-[1-(3-dimethylamino-propyl)-1*H*-benzimidazol-2-yl]-ethyl}-amid,
(44) 4-(2-Oxo-1,2,4,5-tetrahydro-1,3-benzodiazepin-3-yl)-piperidin-1-carbonsäure-{2-(3,4-diethyl-phenyl)-1-[1-(4-dimethylamino-butyl)-1*H* benzimidazol-2-yl]-ethyl}-amid,
(45) 4-(2-Oxo-1,2,4,5-tetrahydro-1,3-benzodiazepin-3-yl)-piperidin-1-carbonsäure-{2-(3,4-diethyl-phenyl)-1-[1-(1-methyl-piperidin-4-yl)-1*H*-benzimidazol-2-yl]-ethyl}-amid,
(46) 4-(2-Oxo-1,2,4,5-tetrahydro-1,3-benzodiazepin-3-yl)-piperidin-1-carbonsäure-{2-(3,4-diethyl-phenyl)-1-[1-(3-pyrrolidin-1-yl-propyl)-1*H*-benzimidazol-2-yl]-ethyl}-amid,
(47) 4-(2-Oxo-1,2,4,5-tetrahydro-1,3-benzodiazepin-3-yl)-piperidin-1-carbonsäure-{2-(3,4-diethyl-phenyl)-1-[1-(1-methyl-piperidin-4-ylmethyl)-1*H*-benzimidazol-2-yl]-ethyl}-amid,
(48) 4-(2-Oxo-1,2,4,5-tetrahydro-1,3-benzodiazepin-3-yl)-piperidin-1-carbonsäure-[2-(3,4-diethyl-phenyl)-1-(1-pyridin-3-ylmethyl-1*H*-benzimidazol-2-yl)-ethyl]-amid,
(49) 4-(2-Oxo-1,2,4,5-tetrahydro-1,3-benzodiazepin-3-yl)-piperidin-1-carbonsäure-{2-(3,5-bis-trifluormethyl-phenyl)-1-[1-(1-methyl-piperidin-4-yl)-1*H-*benzimidazol-2-yl]-ethyl}-amid,
(50) 4-(2-Oxo-1,2,4,5-tetrahydro-1,3-benzodiazepin-3-yl)-piperidin-1-carbonsäure-{2-(3,5-bis-trifluormethyl-phenyl)-1-[1-(3-pyrrolidin-1-yl-propyl)-1*H-*benzimidazol-2-yl]-ethyl}-amid,
(51) 4-(2-Oxo-1,2,4,5-tetrahydro-1,3-benzodiazepin-3-yi)-piperidin-1-carbonsäure-{2-(3,5-bis-trifluormethyl-phenyl)-1-[1-(1-methyl-piperidin-4-ylmethyl)-1H-benzimidazol-2-yl]-ethyl}-amid,
(52) 4-(2-Oxo-1,2,4,5-tetrahydro-1,3-benzodiazepin-3-yl)-piperidin-1-carbonsäure-{2-(3,5-bis-trifluormethyl-phenyl)-1-[1-(3-dimethy(amino-propyl)-1*H-*benzimidazol-2-yl]-ethyl}-amid,
(53) 2-(2-(4-Amino-3-chlor-5-trifluormethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazole-5-carbonsäure-methylester,
(54) 2-(2-(4-Amino-3-chlor-5-trifluormethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazole-5-carbonsäure,
(55) 4-(2-0xo-1 ,2,4,5-tetrahydro-1,3-benzodiazepin-3-yl)-piperidin-1-carbonsäure-{2-(4-amino-3-chlor-5-trifluormethyl-phenyl)-1-[1-(1-methyl-piperidin-4-yl)-1*H-*benzimidazol-2-yl]-ethyl}-amid,
(56) 4-(2-Oxo-1,2,4,5-tetrahydro-1,3-benzodiazepin-3-yl)-piperidin-1-carbonsäure-{2-(4-amino-3-chlor-5-trifluormethyl-phenyl)-1-[1-(3-pyrrolidin-1-yl-propyl)-1*H-*benzimidazol-2-yl]-ethyl}-amid,
(57) 4-(2-Oxo-1,2,4,5-tetrahydro-1,3-benzodiazepin-3-yl)-piperidin-1-carbonsäure-{2-( 4-amino-3-chlor-5-tritluormethyl-phenyl)-1-[1-(1-methyl-piperidin-4-ylmethyl)-1*H*-benzimidazol-2-yl]-ethyl}-amid,
(58) 4-(2-Oxo-1,2,4,5-tetrahydro-1,3-benzodiazepin-3-yl)-piperidin-1-carbonsäure-[2-(4-amino-3-chlor-5-tritluormethyl-phenyl)-1-(1-pyridin-3-ylmethyl-1*H-*benzimidazol-2-yl)-ethyl]-amid,
(59) 4-(2-Oxo-1,2,4,5-tetrahydro-1,3-benzodiazepin-3-yl)-piperidin-1-carbonsäure-{2-(4-amino-3-chlor-5-trifluormethyl-phenyl)-1-[1-(3-dimethylamino-propyl)-1*H-*benzimidazol-2-yl]-ethyl}-amid,
(60) 3-(1-{4-(3,4-Diethyl-phenyl)-3-[1-(3-dimethylamino-propyl)-1*H*-benzimidazol-2-yl]-butyryl}-piperidin-4-yl)-1,3,4,5-tetrahydro-1,3-benzodiazepin-2-on,
(61) 3-(1-{4-(4-Amino-3-chlor-5-trifluormethyl-phenyl)-3-[1-(3-pyrrolidin-1-yl-propyl)-1*H*-benzimidazol-2-yl]-butyryl}-piperidin-4-yl)-1,3,4,5-tetrahydro-1,3-benzodiazepin-2-on,
(62) 3-(1-{4-(4-Amino-3-chlor-5-trifluormethyl-phenyl)-3-[1-(3-imidazol-1-yl-propyl)-1*H*-benzimidazol-2-yl]-butyryl}-piperidin-4-yl)-1,3,4,5-tetrahydro-1,3-benzodiazepin-2-on,
(63) 3-(1-{4-(4-Amino-3-chlor-5-trifluormethyl-phenyl)-3-[5-(3-dimethylaminopropyl)-1-ethyl-1*H*-benzimidazol-2-yl]-butyryl}-piperidin-4-yl)-1,3,4,5-tetrahydro-1,3-benzodiazepin-2-on,
(64) 3-(1-{4-(4-Amino-3-chlor-5-trifluormethyl-phenyl)-3-[1-(3-diethylamino-propyl)-1*H*-benzimidazol-2-yl]-butyryl}-piperidin-4-yl)-1,3,4,5-tetrahydro-1,3-benzodiazepin-2-on,
(65) 3-(1-{4-(4-Amino-3-chlor-5-trifluormethyl-phenyl)-3-[1-(4-hydroxy-butyl)-1*H-*benzimidazol-2-yl]-butyryl}-piperidin-4-yl)-1,3,4,5-tetrahydro-1,3-benzodiazepin-2-on,
(66) 3-(1-{4-(4-Amino-3-chlor-5-trifluormethyl-phenyl)-3-[1-(1-methyl-piperidin-3-ylmethyl)-1*H*-benzimidazol-2-yl]-butyryl}-piperidin-4-yl)-1,3,4,5-tetrahydro-1,3-benzodiazepin-2-on,
(67) 3-[1-(4-(4-Amino-3-chlor-5-trifluormethyl-phenyl)-3-{1-[2-(4-methyl-piperazin-1-yl)-ethyl]-1*H*-benzimidazol-2-yl}-butyryl)-piperidin-4-yl]-1,3,4,5-tetrahydro-1,3-benzodiazepin-2-on,
(68) 3-{1-[4-(4-Amino-3-chlor-5-trifluormethyl-phenyl)-3-(1-pyridin-4-ylmethyl- 1*H-*benzimidazol-2-yl)-butyryl]-piperidin-4-yl}-1,3,4,5-tetrahydro-1,3-benzodiazepin-2-on,
(69) 3-{1-[4-(4-Amino-3-chlor-5-trifluormethyl-phenyl)-3-(1-piperidin-4-ylmethyl-1*H-*benzimidazol-2-yl)-butyryl]-piperidin-4-yl}-1,3,4,5-tetrahydro-1,3-benzodiazepin-2-on,
(70) 3-(1-{4-(4-Amino-3-chlor-5-trifluormethyl-phenyl)-3-[1-(3-dimethylamino-2,2-dimethyl-propyl)-1*H*-benzimidazol-2-yl]-butyryl}-piperidin-4-yl)-1,3,4,5-tetrahydro-1,3-benzodiazepin-2-on,
(71) 3-(1-{4-(4-Amino-3-chlor-5-trifluormethyl-phenyl)-3-[7-(3-dimethylamino-propoxy)-1*H*-benzimidazol-2-yl]-butyryl}-piperidin-4-yl)-1,3,4,5-tetrahydro-1,3-benzodiazepin-2-on,
(72) 3-{1-[4-(4-Amino-3-chlor-5-trifluormethyl-phenyl)-3-(1-piperidin-4-ylmethyl-1*H-*imidazo[4,5-c]pyridin-2-yl)-butyryl]-piperidin-4-yl}-1,3,4,5-tetrahydro-1,3-benzodiazepin-2-on,
(73) 3-(1-{4-(4-Amino-3-chlor-5-trifluormethyl-phenyl)-3-[3-(3-pyrrolidin-1-yl-propyl)-3*H*-imidazo[4,5-c]pyridin-2-yl]-butyryl}-piperidin-4-yl)-1,3,4,5-tetrahydro-1,3-benzodiazepin-2-on,
(74) 3-{1-[4-(4-Amino-3-chlor-5-trifluormethyl-phenyl)-3-(1-methyl-6-pyrrolidin-1-ylmethyl-1*H*-benzimidazol-2-yl)-butyryl]-piperidin-4-yl}-1,3,4,5-tetrahydro-1,3-benzodiazepin-2-on,
deren Stereoisomere und Salze.

11. Physiologisch verträgliche Salze der Verbindungen nach mindestens einem der Ansprüche 1 bis 10 mit anorganischen oder organischen Säuren oder Basen.

12. Arzneimittel enthaltend eine Verbindung nach mindestens einem der Ansprüche 1 bis 10 oder ein physiologisch verträgliches Salz gemäß Anspruch 11 neben gegebenenfalls einem oder mehreren inerten Trägerstoffen und/oder Verdünnungsmitteln.

13. Verwendung einer Verbindung nach mindestens einem der Ansprüche 1 bis 11 zur Herstellung eines Arzneimittels zur akuten und prophylaktischen Behandlung von Kopfschmerzen, insbesondere Migräne- bzw. Cluster-Kopfschmerz.

14. Verfahren zur Herstellung eines Arzneimittels gemäß Anspruch 12, **dadurch gekennzeichnet, daß** auf nichtchemischen Weg eine Verbindung nach mindestens einem der Ansprüche 1 bis 11 in einen oder mehrere inerte Trägerstoffe und/oder Verdünnungsmittel eingearbeitet wird.

15. Verfahren zu Herstellung der Verbindungen gemäß den Ansprüchen 1 bis 11, **dadurch gekennzeichnet, daß** man
a) zur Herstellung von Verbindungen der allgemeinen Formel 1, in der X das Sauerstoffatom und Z die -NR¹-Gruppe bedeuten und A, n, R, R¹, R², R³ und R⁴ und A wie in den Ansprüchen 1 bis 10 erwähnt definiert sind,
ein Amin der allgemeinen Formel II 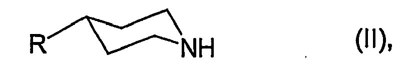 in der
R wie wie in den Ansprüchen 1 bis 10 erwähnt definiert ist, mit einem Kohlensäurederivat der allgemeinen Formel III 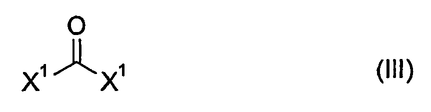 in der
X¹ eine nucleofuge Gruppe bedeutet,
und mit einer Verbindung der allgemeinen Formel IV 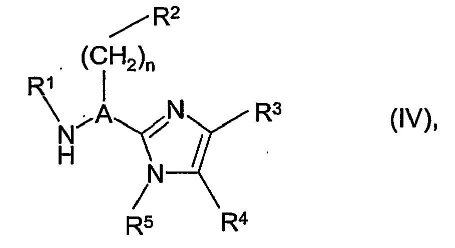 in der
R¹, R², R³, R⁴, R⁵, A und n wie wie in den Ansprüchen 1 bis 10 erwähnt definiert sind, umsetzt
und, falls nötig, anschließend Schutzgruppen abspaltet oder Präcursor-Funktionen umwandelt, oder
b) zur Herstellung von Verbindungen der allgemeinen Formel I, in der A, X, Z, n, R, R² und R⁵ wie in den Ansprüchen 1 bis 10 erwähnt definiert sind und R³ und R⁴ zusammen eine 1;3-Butadien-1,4-ylengruppe bedeuten, in der
eine, zwei oder drei Methingruppen jeweils durch ein Stickstoffatom ersetzt sein können,
ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Di-(C₁₋₃-alkyl)-amino-C₂₋₃-alkyl-aminocarbonyl-, Amino-C₁₋₃-alkyl-, C₁₋₃-Alkyl-amino-C₁₋₃-3-alkyl- oder Di-(C₁₋₃-alkyl)-amino-C₁₋₃-alkylgruppe oder durch eine 4- bis 7-gliedrige Cycloalkylenimino-carbonylgruppe, in der
ein oder zwei Wasserstoffatome jeweils durch eine C₁₋₃-Alkylgruppe ersetzt sein können oder/und
jeweils die Methylengruppe in Position 4 eines 6- oder 7-gliedrigen Cycloalkyleniminoteils durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)- Gruppe ersetzt sein kann,
und gegebenenfalls zusätzlich ein zweites Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy- oder Trifluormethoxygruppe ersetzt sein kann,
ein Amid der allgemeinen Formel 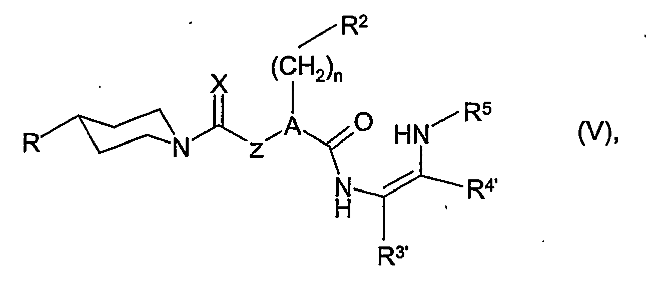 in der R, X, Z, A, n, R² und R⁵ wie in den Ansprüchen 1 bis 10 erwähnt definiert sind, R^{3'} und R^{4'} zusammen eine 1,3-Butadien-1,4-ylengruppe bedeuten, in der
eine, zwei oder drei Methingruppen jeweils durch ein Stickstoffatom ersetzt sein können,
ein Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy-, Trifluormethoxy-, Cyan-, Nitro-, Amino-, C₁₋₃-Alkyl-amino-, Di-(C₁₋₃-alkyl)-amino-, Carboxy-, C₁₋₃-Alkoxy-carbonyl-, Aminocarbonyl-, C₁₋₃-Alkyl-aminocarbonyl-, Di-(C₁₋₃-alkyl)-aminocarbonyl-, Di-(C₁₋₃-alkyl)-amino-C₂₋₃-alkyl-aminocarbonyl-, Amino-C₁₋₃-alkyl-, C₁₋₃-Alkyl-amino-C₁₋₃-alkyl- oder Di-(C₁₋₃-alkyl)-amino-C₁₋₃-alkylgruppe oder durch eine 4- bis 7-gliedrige Cycloalkyleniminocarbonylgruppe, in der
ein oder zwei Wasserstoffatome jeweils durch eine C₁₋₃-Alkylgruppe ersetzt sein können oder/und
jeweils die Methylengruppe in Position 4 eines 6- oder 7-gliedrigen Cyclo alkyleniminoteils durch ein Sauerstoff- oder Schwefelatom, durch eine Sulfinyl-, Sulfonyl-, -NH-, -N(C₁₋₃-Alkyl)- oder -N(C₁₋₃-Alkyl-carbonyl)- Gruppe ersetzt sein kann,
und gegebenenfalls zusätzlich ein zweites Wasserstoffatom durch ein Fluor-, Chlor-, Brom- oder lodatom, durch eine C₁₋₃-Alkyl-, Trifluormethyl-, Difluormethyl-, Cyclopropyl-, Hydroxy-, C₁₋₃-Alkoxy-, Difluormethoxy- oder Trifluormethoxygruppe ersetzt sein kann, zum Ringschluß kondensiert oder
c) zur Herstellung von Verbindungen der allgemeinen Formel I, in der R, A, n sowie R² bis R⁵ wie in den Ansprüchen 1 bis 10 erwähnt definiert sind und X das Sauerstoffatom und Z die -CH₂-Gruppe darstellt:
eine Carbonsäure der allgemeinen Formel 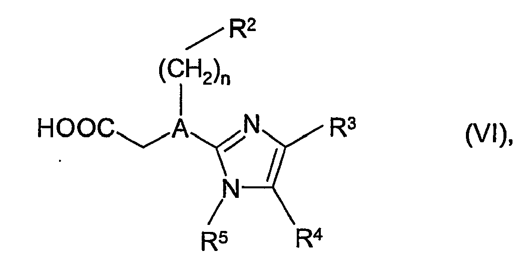 in der
R² bis R⁵, A und n wie in den Ansprüchen 1 bis 10 erwähnt definiert sind,
mit einer Verbindung der allgemeinen Formel 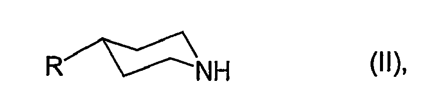 in der
R wie in den Ansprüchen 1 bis 10 erwähnt definiert ist, kuppelt oder
d) zur Herstellung von Verbindungen der allgemeinen Formel I, in der in der R, A, n sowie R² bis R⁵ wie in den Ansprüchen 1 bis 10 erwähnt definiert sind und X das Sauerstoffatom und Z die -CH₂-Gruppe darstellt:
eine Verbindung der allgemeinen Formel 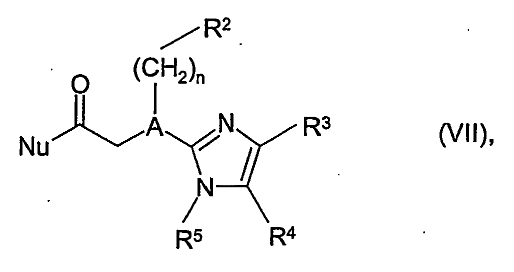 in der
A, n, und R² bis R⁵ wie in den Ansprüchen 1 bis 10 erwähnt definiert sind und Nu eine Austrittsgruppe bedeutet,
mit einer Verbindung der allgemeinen Formel 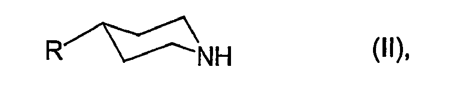 in der
R wie in den Ansprüchen 1 bis 10 erwähnt definiert ist kuppelt oder
e) zur Herstellung von Verbindungen der allgemeinen Formel I, in der in der R, A, n sowie R² bis R⁵ wie in den Ansprüchen 1 bis 10 erwähnt definiert sind und X das Sauerstoffatom und Z die Gruppe -NH- darstellt:
ein lsocyanat der allgemeinen Formel VIII 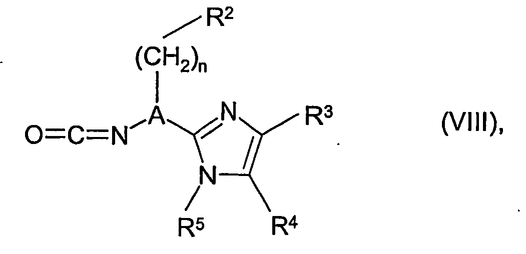 in der
R² bis R⁵, A und n wie in den Ansprüchen 1 bis 10 definiert sind und bedeutet,
mit Aminen der allgemeinen Formel II 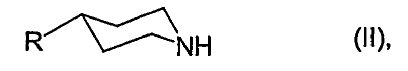 in der
R wie in den Ansprüchen 1 bis 10 erwähnt definiert ist, umsetzt und, falls nötig, anschließend Schutzgruppen abspaltet oder Präcursor-Funktionen umwandelt oder
f) zur Herstellung von Verbindungen der allgemeinen Formel I, in der in der R, A, n sowie R² bis R⁵ wie in den Ansprüchen 1 bis 10 erwähnt definiert sind und X eine der Gruppen =N-CN oder =N-SO₂-R⁶ und Z die Gruppe -NR¹- bedeutet, wobei R¹ und R⁶ wie in den Ansprüchen 1 bis 10 erwähnt definiert sind:
eine Verbindung der allgemeinen Formel 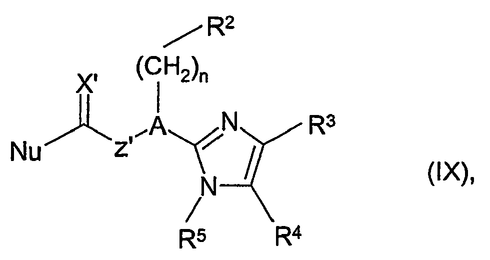
in der A, n und R² bis R⁵ wie in den Ansprüchen 1 bis 10 definiert sind, X' eine der Gruppen =N-CN oder =N-SO₂-R⁶, Z' die Gruppe -NR¹-, wobei R¹ und R⁶ wie in den Ansprüchen 1 bis 10 erwähnt definiert sind, bedeutet und Nu eine Austrittsgruppe ist,
mit sekundären Aminen der allgemeinen Formel 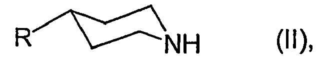
in der R wie in den Ansprüchen 1 bis 10 definiert ist, umsetzt und
gegebenenfalls vorhandene Schutzgruppen von reaktive Gruppen wie Hydroxy-, Carboxy-, Amino- oder Iminogruppen abspaltet und / oder
eine so erhaltene Verbindung der allgemeinen Formel I in ihre Stereoisomere auftrennt und / oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base überführt.

16. Verwendung einer Verbindung nach mindestens einem der Ansprüche 1 bis 11 zur Herstellung eines Arzneimittels zur Bekämpfung von nicht-insulinabhängigem Diabetes-mellitus (NIDDM).

17. Verwendung einer Verbindung nach mindestens einem der Ansprüche 1 bis 11 zur Herstellung eines Arzneimittels zur Behandlung von cardiovaskulären Erkrankungen, von Morphintoleranz, von Erkrankungen der Haut, insbesondere von thermischen und strahlungsbedingten Schäden inclusive Sonnenbrand, von entzündlichen Erkrankungen wie insbesondere entzündlicher Gelenkerkrankungen wie Arthritis, von entzündlichen Lungenerkrankungen, von allergischer Rhinitis, von Asthma, von Erkrankungen, die mit einer überschießenden Gefäßerweiterung und dadurch bedingter verringerter Gefäßdurchblutung, wie insbesondere Schock oder Sepsis, einhergeht, zur Linderung von Schmerzzuständen im allgemeinen oder zu präventiven oder akut therapeutischen Beeinflussung der durch Gefäßerweiterung und erhöhten Blutfluss verursachten Symptomatik von Hitzewallungen menopausaler, östrogendefizienter Frauen sowie hormonbehandelter Prostatakarzinompatienten.

## Claims

1. Compounds of general formula 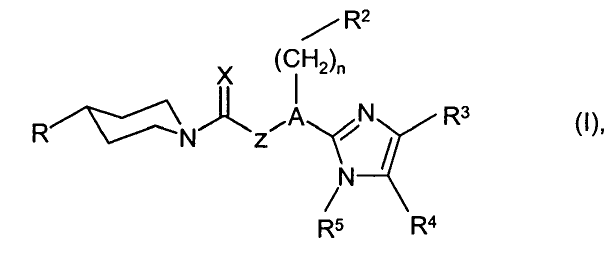 wherein
R denotes a saturated, monounsaturated or, in the case of the 6- and 7-membered heterocyclic groups, a diunsaturated 5- to 7-membered aza, diaza, triaza, oxaza, thiaza, thiadiaza or S,S-dioxido-thiadiaza-heterocyclic group,
while the abovementioned heterocyclic groups are linked via a carbon or nitrogen atom and
contain one or two carbonyl groups, which are linked to at least one nitrogen atom,
may be substituted by an alkyl group at one of the nitrogen atoms,
may be substituted at one or two carbon atoms in each case by an alkyl, phenyl, phenylmethyl, naphthyl, biphenylyl, pyridinyl, diazinyl, furyl, thienyl, pyrrolyl, 1,3-oxazolyl, 1,3-thiazolyl, isoxazolyl, pyrazolyl, 1-methylpyrazolyl, imidazolyl or 1-methylimidazolyl group, while the substituents may be identical or different,
and wherein a double bond of one of the abovementioned unsaturated heterocyclic groups may be fused to a benzene, pyridine, diazine, 1,3-oxazole, thiophene, furan, thiazole, pyrrole, *N*-methyl-pyrrole or quinoline ring, to a 2(1*H*)-oxoquinoline ring optionally substituted by an alkyl group at the nitrogen atom or to an imidazole or *N*-methyl-imidazole ring, or also two olefinic double bonds of one of the abovementioned unsaturated heterocyclic groups may be fused to a benzene ring in each case,
while the phenyl, pyridinyl, diazinyl, furyl, thienyl, pyrrolyl, 1,3-oxazolyl, 1,3-thiazolyl, isoxazolyl, pyrazolyl, 1-methylpyrazolyl, imidazolyl or 1-methylimidazolyl groups contained in R as well as benzo-, thieno-, pyrido- and diazino-fused heterocyclic groups in the carbon skeleton may additionally be mono-, di- or trisubstituted by fluorine, chlorine, bromine or iodine atoms, by alkyl, alkoxy, nitro, alkylthio, alkylsulphinyl, alkylsulphonyl, alkylsulphonylamino, phenyl, trifluoromethyl, difluoromethyl, difluoromethoxy, alkoxycarbonyl, carboxy, dialkylamino, hydroxy, amino, acetylamino, propionylamino, aminocarbonyl, alkylaminocarbonyl, dialkylaminocarbonyl, (4-morpholinyl)carbonyl, (1-pyrrolidinyl)carbonyl, (1-piperidinyl)carbonyl, (hexahydro-1-azepinyl)carbonyl, (4-methyl-1-piperazinyl)carbonyl, methylenedioxy, aminocarbonylamino, alkanoyl, cyano, trifluoromethoxy, trifluoromethylthio, trifluoromethylsulphinyl or trifluoromethylsulphonyl groups, while the substituents may be identical or different,
X denotes an oxygen atom or, if Z denotes the group -NR¹, it may also denote one of the groups =N-CN or =N-SO₂-R⁶, wherein
R⁶ denotes an alkyl group with 1 to 4 carbon atoms or a phenyl group optionally substituted by a halogen atom, a methyl or a methoxy group, Z denotes one of the groups -CH₂, wherein the hydrogen atoms may be replaced independently of one another by a fluorine atom or a C₁₋₃-alkyl group, or -NR¹, wherein
R¹ denotes the hydrogen atom, an alkyl group which may be substituted in the alkyl moiety with the exception of position 1 by an amino, C₁₋₃-alkylamino or di-(C₁₋₃-alkyl)-amino group, or a phenylalkyl group which may be mono- or disubstituted in the phenyl moiety by fluorine, chlorine, bromine or iodine atoms, by a C₁₋₃-alkyl, trifluoromethyl, difluoromethyl, cyclopropyl, hydroxy, C₁₋₃-alkoxy, difluoromethoxy, trifluoromethoxy, cyano, nitro, amino, C₁₋₃-alkyl-amino, di-(C₁₋₃-alkyl)-amino, carboxy, C₁₋₃-alkoxycarbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group, while the substituents may be identical or different,
A denotes a carbon atom substituted by a hydrogen atom or by a C₁₋₃-alkyl group
n denotes the number 1 or 2,
R² denotes the group 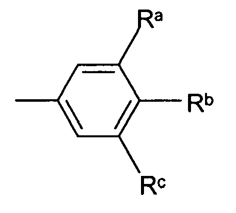 wherein
one of the groups R^{a}, R^{b} and R^{c} denotes the hydrogen, fluorine, chlorine, bromine or iodine atom, a branched or unbranched alkyl group, a hydroxy, alkoxy, trifluoromethyl, difluoromethyl, trifluoromethoxy, difluoromethoxy, trifluoromethylthio, amino, acetylamino, dialkylaminoalkyl, dialkylaminoalkoxy, nitro, methylsulphonyloxy, aminocarbonyl, alkylaminocarbonyl, dialkylaminocarbonyl, alkanoyl, cyano, trifluoromethylsulphinyl or trifluoromethylsulphonyl group,
a second of the groups R^{a}, R^{b} and R^{c} denotes the hydrogen, fluorine, chlorine, bromine or iodine atom, a branched or unbranched alkyl group, a hydroxy, alkoxy, trifluoromethyl, difluoromethyl, trifluoromethoxy, difluoromethoxy, trifluoromethylthio, amino, acetylamino, alkanoyl, aminocarbonyl, alkylaminocarbonyl or dialkylaminocarbonyl group and
the third of the groups R^{a}, R^{b} and R^{c} denotes the hydrogen, fluorine, chlorine, bromine or iodine atom, a branched or unbranched alkyl group, a hydroxy, alkoxy, trifluoromethyl, difluoromethyl, difluoromethoxy, or trifluoromethoxy group, while the substituents may be identical or different,
R³ and R⁴, which may be identical or different, in each case denote the hydrogen atom, a C₁₋₄-alkyl, amino-C₁₋₃-alkyl, C₁₋₃-alkyl-amino-C₁₋₃-alkyl or di-(C₁₋₃-alkyl)-amino-C₁₋₃-alkyl group,
a phenyl or naphthyl group which in each case may be mono- or disubstituted by R^{d}, while the substituents may be identical or different and R^{d} denotes a fluorine, chlorine, bromine or iodine atom, a C₁₋₃-alkyl, trifluoromethyl, difluoromethyl, cyclopropyl, hydroxy, C₁₋₃-alkoxy, difluoromethoxy, trifluoromethoxy, cyano, nitro, amino, C₁₋₃-alkyl-amino, di-(C₁₋₃-alkyl)-amino, carboxy, C₁₋₃-alkoxy-carbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group or a 4- to 7-membered cycloalkyleneimino-carbonyl group wherein
one or two hydrogen atoms may each be replaced by a C₁₋₃-alkyl group and/or
in each case the methylene group in the 4 position of a 6- or 7-membered cycloalkyleneimino moiety may be replaced by an oxygen or sulphur atom, by a sulphinyl, sulphonyl, -NH, -N(C₁₋₃-alkyl) or -N(C₁₋₃-alkyl-carbonyl) group,
a monocyclic 5- or 6-membered heteroaryl group optionally substituted in the carbon skeleton by a C₁₋₃-alkyl, amino, C₁₋₃-alkyl-amino, di-(C₁₋₃-alkyl)-amino, carboxy, C₁₋₃-alkoxy-carbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group, while
the 6-membered heteroaryl group contains one, two or three nitrogen atoms and may optionally additionally be substituted by a fluorine, chlorine, bromine or iodine atom, a C₁₋₃-alkyl, C₁₋₃-alkoxy, amino, C₁₋₃-alkylamino or di-(C₁₋₃-alkyl)amino group and
the 5-membered heteroaryl group contains an imino group optionally substituted by a C₁₋₃-alkyl, acetyl, trifluoroacetyl, C₁₋₃-alkoxy-carbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group, an oxygen or sulphur atom or
an imino group optionally substituted by a C₁₋₃-alkyl, acetyl, trifluoroacetyl, C₁₋₃-alkoxy-carbonyl, aminocarbonyl, C₁₋₃-alkylaminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group or an oxygen or sulphur atom and additionally a nitrogen atom or
an imino group optionally substituted by a C₁₋₃-alkyl, acetyl, trifluoroacetyl, C₁₋₃-alkoxy-carbonyl, aminocarbonyl, C₁₋₃-alkylaminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group and two nitrogen atoms,
or R³ and R⁴ together (denote) a 1,3-butadien-1,4-ylene group wherein
one, two or three methyne groups may each be replaced by a nitrogen atom,
a hydrogen atom may be replaced by a fluorine, chlorine, bromine or iodine atom, by a C₁₋₃-alkyl, trifluoromethyl, difluoromethyl, cyclopropyl, hydroxy, C₁₋₃-alkoxy, difluoromethoxy, trifluoromethoxy, cyano, nitro, amino, C₁₋₃-alkyl-amino, di-(C₁₋₃-alkyl)-amino, carboxy, C₁₋₃-alkoxycarbonyl, aminocarbonyl, C₁₋₃-alkyl-aminocarbonyl, di-(C₁₋₃-alkyl)-aminocarbonyl, di-(C₁₋₃-alkyl)-amino-C₂₋₃-alkyl-aminocarbonyl, amino-C₁₋₃-alkyl, C₁₋₃-alkyl-amino-C₁₋₃-alkyl or di-(C₁₋₃-alkyl)-amino-C₁₋₃-alkyl group or by a 4- to 7-membered cycloalkyleneimino-carbonyl group wherein
one or two hydrogen atoms may each be replaced by a C₁₋₃-alkyl group and/or
in each case the methylene group in the 4 position of a 6- or 7-membered cycloalkyleneimino moiety may be replaced by an oxygen or sulphur atom, by a sulphinyl, sulphonyl, -NH, -N(C₁₋₃-alkyl) or -N(C₁₋₃-alkyl-carbonyl)- group,
and optionally additionally a second hydrogen atom may be replaced by a fluorine, chlorine, bromine or iodine atom, by a C₁₋₃-alkyl, trifluoromethyl, difluoromethyl, cyclopropyl, hydroxy, C₁₋₃-alkoxy, difluoromethoxy or trifluoromethoxy group, and
R⁵ denotes the group -(CH₂)ₘ-R^{e}, wherein
m denotes the number 0 and
R^{e} denotes a hydrogen atom,
a phenyl or naphthyl group which in each case may be mono- or disubstituted by R^{f}, while the substituents may be identical or different and R^{f} denotes a fluorine, chlorine, bromine or iodine atom, a C₁₋₃-alkyl, trifluoromethyl, difluoromethyl, cyclopropyl, hydroxy, C₁₋₃-alkoxy, difluoromethoxy, trifluoromethoxy, cyano, nitro, amino, C₁₋₃-alkyl-amino, di-(C₁₋₃-alkyl)-amino, carboxy, C₁₋₃-alkoxy-carbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group, or
a pyridinyl, pyrazinyl, pyrimidinyl, pyridazinyl, pyrrolyl, furyl, thienyl, pyrazolyl, imidazolyl, oxazolyl, isoxazolyl, thiazolyl or isothiazolyl group optionally substituted in the carbon skeleton by a C₁₋₃-alkyl group, wherein a hydrogen atom bound to a nitrogen atom may be replaced by a C₁₋₃-alkyl or acetyl group, or
a C₃₋₇-cycloalkyl group, while
a hydrogen atom of the C₃₋₇-cycloalkyl group may be replaced by an amino, C₁₋₃-alkyl-amino, di-(C₁₋₃-alkyl)-amino, amino-C₁₋₃-alkyl, C₁₋₃-alkyl-amino-C₁₋₃-alkyl or di-(C₁₋₃-alkyl)-amino-C₁₋₃-alkyl group and/or
the methylene group in the 3 position of the cyclopentyl group and the methylene group in the 4 position of the cyclohexyl and cycloheptyl group may each be replaced by an oxygen or sulphur atom, by a sulphinyl, sulphonyl, -NH, -N(C₁₋₃-alkyl) or -N(C₁₋₃-alkyl-carbonyl)- group,
or m denotes one of the numbers 1 to 5 and
R^{e} denotes a hydrogen atom, an aminomethylene, C₁₋₃-alkylaminomethylene, di-(C₁₋₃-alkyl)-aminomethylene, aminocarbonyl, C₁₋₃-alkylaminocarbonyl, di-(C₁₋₃-alkyl)-aminocarbonyl or C₃₋₇-cycloalkyl group, while
a hydrogen atom of the C₃₋₇-cycloalkyl group may be replaced by an amino, C₁₋₃-alkyl-amino, di-(C₁₋₃-alkyl)-amino, amino-C₁₋₃-alkyl, C₁₋₃-alkyl-amino-C₁₋₃-alkyl or di-(C₁₋₃-alkyl)-amino-C₁₋₃-alkyl group and/or
the methylene group in the 3 position of the cyclopentyl group and the methylene group in the 4 position of the cyclohexyl and cycloheptyl group may each be replaced by an oxygen or sulphur atom, by a sulphinyl, sulphonyl, -NH, -N(C₁₋₃-alkyl) or -N(C₁₋₃-alkyl-carbonyl)- group,
a 4- to 7-membered cycloalkyleneimino group wherein
one or two hydrogen atoms may each be replaced by a C₁₋₃-alkyl group and/or
in each case the methylene group in the 4 position of a 6 or 7-membered cycloalkyleneimino group may be replaced by an oxygen or sulphur atom, by a sulphinyl, sulphonyl, -NH, -N(C₁-₃-alkyl) or -N(C₁-₃-alkyl-carbonyl)- group,
a phenyl or naphthyl group which may be mono- or disubstituted by R^{g} in each case independently of one another, while the substituents may be identical or different and R^{g} denotes a fluorine, chlorine, bromine or iodine atom, a C₁₋₃-alkyl, trifluoromethyl, difluoromethyl, cyclopropyl, hydroxy, C₁₋₃-alkoxy, difluoromethoxy, trifluoromethoxy, cyano, nitro, amino, C₁₋₃-alkyl-amino, di-(C₁₋₃-alkyl)-amino, carboxy, C₁₋₃-alkoxycarbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group,
or a monocyclic 5- or 6-membered heteroaryl group optionally substituted in the carbon skeleton by a C₁₋₃-alkyl, amino, C₁₋₃-alkyl-amino, di-(C₁₋₃-alkyl)-amino, carboxy, C₁₋₃-alkoxy-carbonyl, aminocarbonyl, C₁₋₃-alkylaminocarbonyl, di-(C₁₋₃-alkyl)-aminocarbonyl, amino-C₁₋₃-alkyl, C₁₋₃-alkylamino-C₁₋₃-alkyl or di-(C₁₋₃-alkyl)-amino-C₁₋₃-alkyl group, while
the 6-membered heteroaryl group contains one, two or three nitrogen atoms and may optionally additionally be substituted by a fluorine, chlorine, bromine or iodine atom, a C₁₋₃-alkyl, C₁₋₃-alkoxy, amino, C₁₋₃-alkylamino or di-(C₁₋₃-alkyl)amino group and
the 5-membered heteroaryl group contains an imino group optionally substituted by a C₁₋₃-alkyl, acetyl, trifluoroacetyl, C₁₋₃-alkoxy-carbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group, an oxygen or sulphur atom or
an imino group optionally substituted by a C₁₋₃-alkyl, acetyl, trifluoroacetyl, C₁₋₃-alkoxy-carbonyl, aminocarbonyl, C₁₋₃-alkylaminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group or an oxygen or sulphur atom and additionally a nitrogen atom or
an imino group optionally substituted by a C₁₋₃-alkyl, acetyl, trifluoroacetyl, C₁₋₃-alkoxy-carbonyl, aminocarbonyl, C₁₋₃-alkylaminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group and two nitrogen atoms,
while the abovementioned alkyl groups or the alkyl groups contained in the abovementioned groups, unless otherwise stated, contain 1 to 5 carbon atoms and may be branched or unbranched,
the tautomers, diastereomers, enantiomers, mixtures and salts thereof.

2. Compounds of general formula I according to claim 1, wherein
R denotes a saturated or monounsaturated 5- to 7-membered aza, diaza, oxaza or thiaza heterocyclic group,
while the abovementioned heterocyclic groups are linked via a nitrogen atom and
contain a carbonyl group linked to one or two nitrogen atoms of the heterocyclic group,
may be substituted by an alkyl group at one of the nitrogen atoms,
may be substituted at one of the carbon atoms by an alkyl, phenyl, pyridinyl, furyl, thienyl or pyrrolyl group,
and wherein a double bond of one of the abovementioned unsaturated heterocyclic groups may be fused to a benzene, pyridine or thiophene ring,
while the phenyl, pyridinyl, furyl, thienyl, pyrrolyl groups contained in R as well as the benzo-, thieno- and pyrido-condensed heterocyclic groups in the carbon skeleton may additionally be mono- or disubstituted by fluorine, chlorine, bromine or iodine atoms, by alkyl, alkoxy, nitro, alkylthio, trifluoromethyl, difluoromethyl, alkoxycarbonyl, carboxy, dialkylamino, hydroxy, amino, acetylamino, aminocarbonyl, alkylaminocarbonyl, alkanoyl, cyano or trifluoromethoxy groups, while the substituents may be identical or different,
X denotes an oxygen atom or, if Z the group -NR¹- denotes, it may also denote the group =N-CN,
Z denotes one of the groups -CH₂ or -NR¹, wherein
R¹ denotes the hydrogen atom or an alkyl group,
A denotes a carbon atom substituted by a hydrogen atom or by a C₁-₃-alkyl group,
n denotes the number 1 or 2,
R² denotes the group 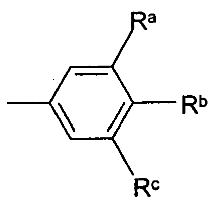 wherein
one of the groups R^{a}, R^{b} and R^{c} denotes the hydrogen, fluorine, chlorine, bromine or iodine atom, a branched or unbranched alkyl group, a hydroxy, alkoxy, trifluoromethyl, difluoromethyl, trifluoromethoxy, amino, acetylamino, dialkylaminoalkyl, dialkylaminoalkoxy, aminocarbonyl, alkylaminocarbonyl, dialkylaminocarbonyl, alkanoyl, cyano or trifluoromethylsulphonyl group,
a second of the groups R^{a}, R^{b} and R^{c} denotes the hydrogen, fluorine, chlorine, bromine or iodine atom, a branched or unbranched alkyl group, an alkoxy, trifluoromethyl, amino, acetylamino or alkanoyl group and
the third of the groups R^{a}, R^{b} and R^{c} denotes the hydrogen, fluorine, chlorine, bromine or iodine atom or a branched or unbranched alkyl group, while the substituents may be identical or different,
R³ denotes the hydrogen atom or a C₁₋₃-alkyl group,
R⁴ denotes the hydrogen atom, a C₁₋₄-alkyl, amino-C₁₋₃-alkyl, C₁₋₃-alkylamino-C₁₋₃-alkyl or di-(C₁₋₃-alkyl)-amino-C₁₋₃-alkyl group,
a phenyl group which may be mono- or disubstituted by R^{d}, while the substituents may be identical or different and R^{d} denotes a fluorine, chlorine, bromine or iodine atom, a C₁₋₃-alkyl, trifluoromethyl, difluoromethyl, cyclopropyl, hydroxy, C₁₋₃-alkoxy, difluoromethoxy, trifluoromethoxy, amino, C₁₋₃-alkylamino, di-(C₁₋₃-alkyl)-amino, carboxy, C₁₋₃-alkoxy-carbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group, or
a pyridinyl, pyrazinyl, pyrimidinyl, pyridazinyl, pyrrolyl, furyl, thienyl, pyrazolyl, imidazolyl, oxazolyl, isoxazolyl, thiazolyl or isothiazolyl group optionally substituted in the carbon skeleton by a C₁₋₃-alkyl group, wherein a hydrogen atom bound to a nitrogen atom may be replaced by a C₁₋₃-alkyl or acetyl group,
or R³ and R⁴ together denote a 1,3-butadien-1,4-ylene group wherein
a hydrogen atom may be replaced by a fluorine, chlorine, bromine or iodine atom, by a C₁₋₃-alkyl, trifluoromethyl, difluoromethyl, cyclopropyl, hydroxy, C₁₋₃-alkoxy, amino, C₁₋₃-alkyl-amino, di-(C₁₋₃-alkyl)-amino, carboxy, C₁₋₃-alkoxy-carbonyl, aminocarbonyl, C₁₋₃-alkyl-aminocarbonyl, di-(C₁₋₃-alkyl)-aminocarbonyl, di-(C₁₋₃-alkyl)-amino-C₂₋₃-alkylaminocarbonyl, amino-C₁₋₃-alkyl, C₁₋₃-alkyl-amino-C₁₋₃-alkyl, di-(C₁₋₃-alkyl)-amino-C₁₋₃-alkyl group or by a 5- to 7-membered cycloalkyleneimino-carbonyl group wherein
one or two hydrogen atoms may each be replaced by a C₁₋₃-alkyl group and/or
in each case the methylene group in the 4 position of a 6- or 7-membered cycloalkyleneimino moiety by an oxygen or sulphur atom, may be replaced by a -NH, -N(C₁₋₃-alkyl) or-N(C₁₋₃-alkyl-carbonyl)-group,
and optionally a second hydrogen atom may additionally be replaced by a fluorine, chlorine, bromine or iodine atom, by a C₁₋₃-alkyl or trifluoromethyl group, and
R⁵ denotes the group -(CH₂)m-R^{e}, wherein
m denotes the number 0 and
R^{e} denotes a hydrogen atom or a C₅₋₆-cycloalkyl group, while
the methylene group in the 3 position of the cyclopentyl group and the methylene group in the 4 position of the cyclohexyl group may each be replaced by a -NH, -N(C₁₋₃-alkyl) or -N(C₁₋₃-alkyl-carbonyl) group,
or m denotes one of the numbers 1 to 5 and
R^{e} denotes a hydrogen atom, an aminomethylene, C₁₋₃-alkylaminomethylene, di-(C₁₋₃-alkyl)-aminomethylene, aminocarbonyl, C₁₋₃-alkyl-aminocarbonyl, di-(C₁₋₃-alkyl)-amino carbonyl or C₅₋₆-cycloalkyl group, while
the methylene group in the 3 position of the cyclopentyl group and the methylene group in the 4 position of the cyclohexyl group may each be replaced by a -NH, -N(C₁₋₃-alkyl) or -N(C₁₋₃-alkyl-carbonyl) group,
a 5- to 6-membered cycloalkyleneimino group, while
the methylene group in the 4 position of a 6-membered cycloalkyleneimino group may be replaced by a -NH, -N(C₁₋₃-alkyl) or -N(C₁₋₃-alkyl-carbonyl)- group,
a phenyl group which may be mono- or disubstituted by R⁹, while the substituents may be identical or different and R⁹ denotes a fluorine, chlorine, bromine or iodine atom, a C₁₋₃-alkyl, trifluoromethyl, difluoromethyl, hydroxy, C₁₋₃-alkoxy, amino, C₁₋₃-alkyl-amino, di-(C₁₋₃-alkyl)-amino, aminocarbonyl- C₁₋₃-alkyl-aminocarbonyl, amino-C₁₋₃-alkyl, C₁₋₃-alkyl-amino-C₁₋₃-alkyl or di-(C₁₋₃-alkyl)-amino-C₁₋₃-alkyl group,
or a pyridinyl, pyrazinyl, pyrimidinyl, pyridazinyl, pyrrolyl, furyl, thienyl, pyrazolyl, imidazolyl, oxazolyl, isoxazolyl, thiazolyl or isothiazolyl group optionally substituted in the carbon skeleton by a C₁₋₃-alkyl group, wherein a hydrogen atom bound to a nitrogen atom by a C₁₋₃-alkyl or acetyl group may be replaced,
while the abovementioned alkyl groups or the alkyl groups contained in the abovementioned groups, unless otherwise stated, contain 1 to 4 carbon atoms and may be branched or unbranched,
the tautomers, diastereomers, enantiomers, mixtures and salts thereof.

3. Compounds of general formula I according to claim 2, wherein
R denotes a monounsaturated, 5- to 7-membered diaza heterocyclic group linked via a nitrogen atom,
while the abovementioned heterocyclic groups contain a carbonyl group linked to both nitrogen atoms of the heterocyclic group,
may each be substituted independently of one another at one of the nitrogen atoms and one of the saturated carbon atoms by a C₁₋₃-alkyl group,
and wherein the double bond of the abovementioned unsaturated heterocyclic groups is fused to a benzene ring optionally substituted by a fluorine, chlorine, bromine or iodine atom, or by a C₁₋₃-alkyl, C₁₋₃-alkoxy, trifluoromethyl, carboxy, C₁₋₃-alkoxy-carbonyl, amino, acetylamino, hydroxy, aminocarbonyl or alkanoyl group,
X denotes an oxygen atom,
Z denotes the group -NR¹, wherein
R¹ denotes the hydrogen atom or a C₁₋₃-alkyl group,
A denotes a carbon atom substituted by a hydrogen atom or by a C₁₋₃-alkyl group,
n denotes the number 1,
R² denotes the group 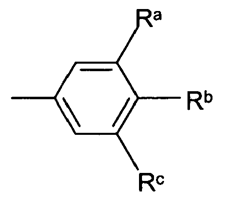 wherein
one of the groups R^{a}, R^{b} and R^{c} denotes the hydrogen, fluorine, chlorine, bromine or iodine atom, a C₁₋₃-alkyl, trifluoromethyl, hydroxy, alkoxy or amino group,
a second of the groups R^{a}, R^{b} and R^{c} denotes the hydrogen, fluorine, chlorine, bromine or iodine atom, a C₁₋₃-alkyl or trifluoromethyl group and
the third of the groups R^{a}, R^{b} and R^{c} denotes the hydrogen, fluorine, chlorine, bromine or iodine atom, while the substituents may be identical or different,
R³ denotes the hydrogen atom or a C₁₋₃-alkyl group,
R⁴ denotes a phenyl group optionally substituted by a fluorine, chlorine, bromine or iodine atom, by a C₁₋₃-alkyl, trifluoromethyl, hydroxy, C₁₋₃-alkoxy, amino, C₁₋₃-alkylamino, di-(C₁₋₃-alkyl)-amino, carboxy or C₁₋₃-alkoxy-carbonyl group,
or R³ and R⁴ together denote a 1,3-butadien-1,4-ylene group wherein
a hydrogen atom may be replaced by a fluorine, chlorine, bromine or iodine atom, by a C₁₋₃-alkyl, trifluoromethyl, hydroxy, C₁₋₃-alkoxy, carboxy, C₁₋₃-alkoxy-carbonyl, aminocarbonyl, C₁₋₃-alkyl-aminocarbonyl, di-(C₁₋₃-alkyl)-aminocarbonyl or di-(C₁₋₃-alkyl)-amino-C₂₋₃-alkyl-aminocarbonyl group or by a 5- to 7-membered cycloalkyleneimino-carbonyl group, while the methylene group in the 4 position of the 6-membered cycloalkyleneimino moiety may be replaced by a -NH, -N(C₁₋₃-alkyl) or -N(C₁₋₃-alkyl-carbonyl)- group,
and optionally a second hydrogen atom may additionally be replaced by a fluorine, chlorine, bromine or iodine atom or by a C₁₋₃-alkyl group, and
R⁵ denotes the group -(CH₂)ₘ-R^{e} , wherein
m denotes the number 0 and
R^{e} denotes a hydrogen atom,
or m denotes one of the numbers 1 to 3 and
R^{e} denotes a hydrogen atom, an aminomethylene, C₁₋₃-alkylaminomethylene or di-(C₁₋₃-alkyl)-aminomethylene group,
while the abovementioned alkyl groups or the alkyl groups contained in the abovementioned groups, unless otherwise stated, may be branched or unbranched,
the tautomers, diastereomers, enantiomers, mixtures and salts thereof.

4. Compounds of general formula I according to claim 1, wherein
R, X, Z, A, n, R², and R⁵ are defined as in claim 1 and
R³ denotes the hydrogen atom or a C₁₋₄-alkyl group and
R⁴ denotes the hydrogen atom, a C₁₋₄-alkyl, amino-C₁₋₃-alkyl, C₁₋₃-alkylamino-C₁₋₃-alkyl or di-(C₁₋₃-alkyl)-amino-C₁₋₃-alkyl group,
a phenyl or naphthyl group which may be mono- or disubstituted by R^{d} in each case independently of one another, while the substituents may be identical or different and R^{d} denotes a fluorine, chlorine, bromine or iodine atom, a C₁₋₃-alkyl, trifluoromethyl, difluoromethyl, cyclopropyl, hydroxy, C₁₋₃-alkoxy, difluoromethoxy, trifluoromethoxy, cyano, nitro, amino, C₁₋₃-alkylamino, di-(C₁₋₃-alkyl)amino, carboxy, C₁₋₃-alkoxy-carbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group, or
a monocyclic 5- or 6-membered heteroaryl group optionally substituted in the carbon skeleton by a C₁₋₃-alkyl, amino, C₁₋₃-alkyl-amino, di-(C₁₋₃-alkyl)-amino, carboxy, C₁₋₃-alkoxy-carbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group, while
the 6-membered heteroaryl group contains one, two or three nitrogen atoms and
die 5-membered heteroaryl group contains an imino group optionally substituted by a C₁₋₃-alkyl, acetyl, trifluoroacetyl, C₁₋₃-alkoxy-carbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group, an oxygen or sulphur atom or
an imino group optionally substituted by a C₁₋₃-alkyl, acetyl, trifluoroacetyl- C₁₋₃-alkoxy-carbonyl, aminocarbonyl, C₁₋₃-alkylaminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group or an oxygen or sulphur atom and additionally a nitrogen atom or
an imino group optionally substituted by a C₁₋₃-alkyl, acetyl, trifluoroacetyl- C₁₋₃-alkoxy-carbonyl, aminocarbonyl, C₁₋₃-alkylaminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group and two nitrogen atoms,
the tautomers, diastereomers, enantiomers, mixtures and salts thereof.

5. Compounds of general formula I according to claim 2, wherein
R, X, Z, A, n, R², and R⁵ are defined as in claim 2 and
R³ denotes the hydrogen atom or a C₁₋₃-alkyl group and
R⁴ denotes a phenyl group which may be mono- or disubstituted by R^{d}, while the substituents may be identical or different and R^{d} denotes a fluorine, chlorine, bromine or iodine atom, a C₁₋₃-alkyl, trifluoromethyl, difluoromethyl, cyclopropyl, hydroxy, C₁₋₃-alkoxy, difluoromethoxy, trifluoromethoxy, amino, C₁₋₃-alkyl-amino, di-(C₁₋₃-alkyl)-amino, carboxy, C₁₋₃-alkoxy-carbonyl, aminocarbonyl, C₁₋₃-alkyl-aminocarbonyl, di-(C₁₋₃-alkyl)-aminocarbonyl, amino-C₁₋₃-alkyl, C₁₋₃-alkyl-amino-C₁₋₃-alkyl or di-(C₁₋₃-alkyl)-amino-C₁₋₃-alkyl group, or
a pyridinyl, pyrazinyl, pyrimidinyl, pyridazinyl, pyrrolyl, furyl, thienyl, pyrazolyl, imidazolyl, oxazolyl, isoxazolyl, thiazolyl or isothiazolyl group optionally substituted in the carbon skeleton by a C₁₋₃-alkyl group, wherein a hydrogen atom bound to a nitrogen atom may be replaced by a C₁₋₃-alkyl or acetyl group,
the tautomers, diastereomers, enantiomers, mixtures and salts thereof.

6. Compounds of general formula l according to claim 3, wherein
R, X, Z, A, n, R², and R⁵ are defined as in claim 3 and
R³ denotes the hydrogen atom or a C₁₋₃-alkyl group and
R⁴ denotes a phenyl group optionally substituted by a fluorine, chlorine, bromine or iodine atom, by a C₁₋₃-alkyl, trifluoromethyl, hydroxy, C₁₋₃-alkoxy, amino, C₁₋₃-alkylamino, di-(C₁₋₃-alkyl)-amino, carboxy or C₁₋₃-alkoxy-carbonyl group,
the tautomers, diastereomers, enantiomers, mixtures and salts thereof.

7. Compounds of general formula I according to claim 1, wherein
R, X, Z, A, n, R², and R⁵ are defined as in claim 1 and
R³ and R⁴ together denote a 1,3-butadien-1,4-ylene group wherein
one, two or three methyne groups may each be replaced by a nitrogen atom,
a hydrogen atom may be replaced by a fluorine, chlorine, bromine or iodine atom, by a C₁₋₃-alkyl, trifluoromethyl, difluoromethyl, cyclopropyl, hydroxy, C₁₋₃-alkoxy, difluoromethoxy, trifluoromethoxy, cyano, nitro, amino, C₁₋₃-alkyl-amino, di-(C₁₋₃-alkyl)-amino, carboxy, C₁₋₃-alkoxycarbonyl, aminocarbonyl, C₁₋₃-alkyl-aminocarbonyl, di-(C₁₋₃-alkyl)-aminocarbonyl, di-(C₁₋₃-alkyl)-amino-C₂₋₃-alkyl-aminocarbonyl, amino-C₁₋₃-alkyl, C₁₋₃-alkyl-amino-C₁₋₃-alkyl or di-(C₁₋₃-alkyl)-amino-C₁₋₃-alkyl group or by a 4- to 7-membered cycloalkyleneimino-carbonyl group wherein
one or two hydrogen atoms may each be replaced by a C₁₋₃-alkyl group and/or
in each case the methylene group in the 4 position of a 6- or 7-membered cycloalkyleneimino moiety may be replaced by an oxygen or sulphur atom, by a sulphinyl, sulphonyl, -NH, -N(C₁₋₃-alkyl) or-N(C₁₋₃-alkyl-carbonyl)- group,
and a second hydrogen atom may optionally additionally be replaced by a fluorine, chlorine, bromine or iodine atom, by a C₁₋₃-alkyl, trifluoromethyl, difluoromethyl, cyclopropyl, hydroxy, C₁₋₃-alkoxy, difluoromethoxy or trifluoromethoxy group,
the tautomers, diastereomers, enantiomers, mixtures and salts thereof.

8. Compounds of general formula I according to claim 2, wherein
R, X, Z, A, n, R², and R⁵ are defined as in claim 2 and
R³ and R⁴ together denote a 1,3-butadien-1,4-ylene group wherein
a hydrogen atom may be replaced by a fluorine, chlorine, bromine or iodine atom, by a C₁₋₃-alkyl, trifluoromethyl, difluoromethyl, cyclopropyl, hydroxy, C₁₋₃-alkoxy, amino, C₁₋₃-alkyl-amino, di-(C₁₋₃-alkyl)-amino, carboxy, C₁₋₃-alkoxy-carbonyl, aminocarbonyl, C₁₋₃-alkyl-aminocarbonyl, di-(C₁₋₃-alkyl)-aminocarbonyl or di-(C₁₋₃-alkyl)-amino-C₂₋₃-alkylaminocarbonyl group or by a 5- to 7-membered cycloalkyleneimino-carbonyl group wherein
one or two hydrogen atoms may each be replaced by a C₁₋₃-alkyl group and/or
in each case the methylene group in the 4 position of a 6- or 7-membered cycloalkyleneimino moiety may be replaced by an oxygen or sulphur atom, by a -NH, -N(C₁₋₃-alkyl) or-N(C₁₋₃-alkyl-carbonyl)-group,
and a second hydrogen atom may optionally additionally be replaced by a fluorine, chlorine, bromine or iodine atom, by a C₁₋₃-alkyl or trifluoromethyl group,
the tautomers, diastereomers, enantiomers, mixtures and salts thereof.

9. Compounds of general formula I according to claim 3, wherein
R, X, Z, A, n, R², and R⁵ are defined as in claim 3 and
R³ and R⁴ together denote a 1,3-butadien-1,4-ylene group wherein
a hydrogen atom may be replaced by a fluorine, chlorine, bromine or iodine atom, by a C₁₋₃-alkyl, trifluoromethyl, hydroxy, C₁₋₃-alkoxy, carboxy, C₁₋₃-alkoxy-carbonyl, aminocarbonyl, C₁₋₃-alkyl-aminocarbonyl, di-(C₁₋₃-alkyl)-aminocarbonyl or di-(C₁₋₃-alkyl)-amino-C₂₋₃-alkyl-aminocarbonyl group or by a 5- to 7-membered cycloalkyleneimino-carbonyl group, while
the methylene group in the 4 position of the 6-membered cycloalkyleneimino moiety may be replaced by a -NH, -N(C₁₋₃-alkyl) or-N(C₁₋₃-alkyl-carbonyl) group,
and a second hydrogen atom may optionally additionally be replaced by a fluorine, chlorine, bromine or iodine atom or by a C₁₋₃-alkyl group,
the tautomers, diastereomers, enantiomers, mixtures and salts thereof.

10. The following compounds of general formula I according to claim 1:
(1) 4-(2-oxo-1,2,4,5-tetrahydro-1,3-benzodiazepin-3-yl)-piperidin-1-carboxylic acid-{(*R*)-2-(4-amino-3,5-dibromo-phenyl)-1-[1-(3-dimethylamino-propyl)-1*H*-benzimidazol-2-yl]-ethyl}-amide,
(2) 4-(2-oxo-1,2,4,5-tetrahydro-1 ,3-benzodiazepin-3-yl)-piperidin-1-carboxylic acid-{(*R*)-2-(4-amino-3,5-dibromo-phenyl)-1-[1-(2-dimethylamino-ethyl)-1*H*-benzimidazol-2-yl]-ethyl}-amide,
(3) 4-(2-oxo-1,2,4,5-tetrahydro-1,3-benzodiazepin-3-yl)-piperidin-1-carboxylic acid-[(*R*)-2-(4-amino-3,5-dibromo-phenyl)-1-(1-methyl-1*H-*benzimidazol-2-yl)-ethyl]-amide,
(4) 4-(2-oxo-1,2,4,5-tetrahydro-1,3-benzodiazepin-3-yl)-piperidin-1-carboxylic acid-[(*R*)-2-(4-amino-3,5-dibromo-phenyl)-1-(1*H-*benzimidazol-2-yl)-ethyl]-amide,
(5) methyl 2-((*R*)-2-(4-amino-3,5-dibromo-phenyl)-1-{[4-(2-oxo-1,4-dihydro-3*H*-quinazolin-3-yl)-piperidin-1-carbonyl]-amino}-ethyl)-1H-benzimidazole-5-carboxylate,
(6) methyl 2-((*R*)-2-(4-amino-3,5-dibromo-phenyl)-1-{[4-(2-oxo-1,2,4,5-tetrahydro-1,3-benzodiazepin-3-yl)-piperidin-1-carbonyl]-amino}-ethyl)-1H-benzimidazole-5-carboxylate,
(7) 2-((*R*)-2-(4-amino-3,5-dibromo-phenyl)-1-{[4-(2-oxo-1,2,4,5-tetrahydro-1,3-benzodiazepin-3-yl)-piperidin-1-carbonyl]-amino}-ethyl)-1*H-*benzimidazole-5-carboxylic acid,
(8) 4-(2-oxo-1,2,4,5-tetrahydro-1,3-benzodiazepin-3-yl)-piperidin-1-carboxylic acid-{(*R*)-2-(4-amino-3,5-dibromo-phenyl)-1-[6-(4-methylpiperazin-1-carbonyl)-1*H*-benzimidazol-2-yl]-ethyl}-amide,
(9) 2-((*R*)-2-(4-amino-3,5-dibromo-phenyl)-1-{[4-(2-oxo-1,2,4,5-tetrahydro-1,3-benzodiazepin-3-yl)-piperidin-1-carbonyl]-amino}-ethyl)-1*H-*benzimidazole-5-carboxylic acid-(3-dimethylamino-propyl)-amide,
(10) 2-((*R*)-2-(4-amino-3,5-dibromo-phenyl)-1-{[4-(2-oxo-1,2,4,5-tetrahydro-1,3-benzodiazepin-3-yl)-piperidin-1-carbonyl]-amino}-ethyl)-1*H-*benzimidazole-5-carboxylic acid-(2-dimethylamino-ethyl)-amide,
(11) 4-(2-oxo-1,4-dihydro-2*H*-quinazolin-3-yl)-piperidin-1-carboxylic acid-{(*R*)-2-(4-amino-3,5-dibromo-phenyl)-1-[1-(3-dimethylamino-propyl)-5-phenyl-1*H*-imidazol-2-yl]-ethyl}-amide,
(12) 4-(2-oxo-1,2,4,5-tetrahydro-1,3-benzodiazepin-3-yl)-piperidin-1-carboxylic acid-[(*R*)-2-(4-amino-3,5-dibromo-phenyl)-1-(1-butyl-1*H-*benzimidazol-2-yl)-ethyl]-amide,
(13) 4-(2-oxo-1,2,4,5-tetrahydro-1,3-benzodiazepin-3-yl)-piperidin-1-carboxylic acid-{(*R*)-2-(4-amino-3,5-dibromo-phenyl)-1-[1-(3-pyrrolidin-1-yl-propyl)-1*H*-benzimidazol-2-yl]-ethyl}-amide,
(14) 4-(2-oxo-1,2,4,5-tetrahydro-1,3-benzodiazepin-3-yl)-piperidin-1-carboxylic acid-[(*R*)-2-(4-amino-3,5-dibromo-phenyl)-1-(1-pyridin-3-ylmethyl-1*H*-benzimidazol-2-yl)-ethyl]-amide,
(15) 4-(2-oxo-1,2,4,5-tetrahydro-1,3-benzodiazepin-3-yl)-piperidin-1-carboxylic acid-[(*R*)-2-(4-amino-3,5-dibromo-phenyl)-1-(1-benzyl-1*H-*benzimidazol-2-yl)-ethyl]-amide,
(16) 4-(2-oxo-1,2,4,5-tetrahydro-1,3-benzodiazepin-3-yl)-piperidin-1-carboxylic acid {(*R*)-2-(4-amino-3,5-dibromo-phenyl)-1-[1-(1-methyl-piperidin-4-ylmethyl)-1*H*-benzimidazol-2-yl]-ethyl}-amide,
(17) 4-(2-oxo-1,2,4,5-tetrahydro-1 ,3-benzodiazepin-3-yl)-piperidin-1-carboxylic acid-{(*R*)-2-(4-amino-3,5-dibromo-phenyl)-1-[1-(4-dimethylamino-butyl)-1*H*-benzimidazol-2-yl]-ethyl}-amide,
(18) 4-(2-oxo-1,2,4,5-tetrahydro-1 ,3-benzodiazepin-3-yl)-piperidin-1-carboxylic acid-{(*R*)-2-(4-amino-3,5-dibromo-phenyl)-1-[1-(1-methyl-piperidin-4-yl)-1*H*-benzimidazol-2-yl]-ethyl}-amide,
(19) 4-(2-oxo-1,2,4,5-tetrahydro-1 ,3-benzodiazepin-3-yl)-piperidin-1-carboxylic acid-[(R)-2-(4-amino-3,5-dibromo-phenyl)-1-(1-cyclohexyl-1*H*-benzimidazol-2-yl)-ethyl]-amide,
(20) 4-(2-oxo-1,2,4,5-tetrahydro-1,3-benzodiazepin-3-yl)-piperidin-1-carboxylic acid-[(*R*)-2-(4-amino-3,5-dibromo-phenyl)-1-(1-cyclopentyl-1*H*-benzimidazol-2-yl)-ethyl]-amide,
(21) 4-(2-oxo-1,2,4,5-tetrahydro-1,3-benzodiazepin-3-yl)-piperidin-1-carboxylic acid{(*R*)-2-(4-amino-3,5-dibromo-phenyl)-1-[1-(5-dimethylamino-pentyl)-1 *H*-benzimidazol-2-yl]-ethyl}-amide,
(22) methyl 4-[2-((*R*)-2-(4-amino-3,5-dibromo-phenyl)-1-{[4-(2-oxo-1,2,4,5-tetrahydro-1,3-benzodiazepin-3-yl)-piperidin-1-carbonyl]-amino}-ethyl)-benzimidazol-1-yl]-butyrate,
(23) 4-(2-oxo-1,2,4,5-tetrahydro-1,3-benzodiazepin-3-yl)-piperidin-1-carboxylic acid-{(*R*)-2-(4-amino-3,5-dibromo-phenyl)-1-[6-chloro-1-(3-dimethylamino-propyl)-1*H*-benzimidazol-2-yl]-ethyl}-amide,
(24) 4-(2-oxo-1,2,4,5-tetrahydro-1 ,3-benzodiazepin-3-yl)-piperidin-1-carboxylic acid-{(*R*)-2-(4-amino-3,5-dibromo-phenyl)-1-[1-(3-dimethylamino-propyl)-6-fluoro-1*H*-benzimidazol-2-yl]-ethyl}-amide,
(25) 4-(2-oxo-1,2,4,5-tetrahydro-1,3-benzodiazepin-3-yl)-piperidin-1-carboxylic acid-{(*R*)-2-(4-amino-3,5-dibromo-phenyl)-1-[1-(3-dimethylamino-propyl)-5-fluoro-1*H*-benzimidazol-2-yl]-ethyl}-amide,
(26) 4-(2-oxo-1,2,4,5-tetrahydro-1,3-benzodiazepin-3-yl)-piperidin-1-carboxylic acid-{(*R*)-2-(4-amino-3,5-dibromo-phenyl)-1-[5,6-dichloro-1-(3-dimethylamino-propyl)-1*H*-benzimidazol-2-yl]-ethyl}-amide,
(27) methyl 2-((*R*)-2-(4-amino-3,5-dibromo-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1H-benzimidazole-5-carboxylate,
(28) 2-(2-(4-amino-3-chloro-5-trifluoromethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazole-5-carboxylic acid,
(29) 4-(2-oxo-1,2,4,5-tetrahydro-1,3-benzodiazepin-3-yl)-piperidin-1-carboxylic acid-{(R)-2-(3,5-dibromo-4-hydroxy-phenyl)-1-[1-(1-methylpiperidin-4-ylmethyl)-1 *H*-benzimidazol-2-yl]-ethyl}-amide,
(30) 4-(2-oxo-1,2,4,5-tetrahydro-1,3-benzodiazepin-3-y)-piperidin-1-carboxylic acid-{(*R*)-2-(3,5-dibromo-4-hydroxy-phenyl)-1-[1-(1-methyl-piperidin-4-yl)-1 *H*-benzimidazol-2-yl]-ethyl}-amide,
(31) 4-(2-oxo-1,2,4,5-tetrahydro-1,3-benzodiazepin-3-yl)-piperidin-1-carboxylic acid-{(*R*)-2-(3,5-dibromo-4-hydroxy-phenyl)-1-[1-(3-dimethylamino-propyl)-1*H*-benzimidazol-2-yl]-ethyl}-amide,
(32) 4-(2-oxo-1,2,4,5-tetrahydro-1,3-benzodiazepin-3-yl)-piperidin-1-carboxylic acid-{(*R*)-2-(3,5-dibromo-4-hydroxy-phenyl)-1-[1-(3-pyrrolidin-1-yl-propyl)-1*H*-benzimidazol-2-yl]-ethyl}-amide,
(33) 4-(2-oxo-1,2,4,5-tetrahydro-1,3-benzodiazepin-3-yl)-piperidin-1-carboxylic acid-[1-[6-chloro-1-(3-dimethylamino-propyl)-1*H-*benzimidazol-2-yl]-2-(3,4-dibromo-phenyl)-ethyl]-amide,
(34) 4-(2-oxo-1,2,4,5-tetrahydro-1,3-benzodiazepin-3-yl)-piperidin-1-carboxylic acid-{2-(3,4-dibromo-phenyl)-1-[5,6-dichloro-1-(3-dimethylamino-propyl)-1 *H*-benzimidazol-2-yl]-ethyl}-amide,
(35) 4-(2-oxo-1,2,4,5-tetrahydro-1,3-benzodiazepin-3-yl)-piperidin-1-carboxylic acid-[1-[7-chloro-1-(3-dimethylamino-propyl)-1*H-*benzimidazol-2-yl]-2-(3,4-dibromo-phenyl)-ethyl]-amide,
(36) 4-(2-oxo-1,2,4,5-tetrahydro-1,3-benzodiazepin-3-yl)-piperidin-1-carboxylic acid-{2-(3,4-dibromo-phenyl)1-[1-(3-dimethylamino-propyl)-6-fluoro-1*H*-benzimidazol-2-yl]-ethyl}-amide,
(37) 4-(2-oxo-1,2,4,5-tetrahydro-1,3-benzodiazepin-3-yl)-piperidin-1-carboxylic acid-{2-(3,4-dibromo-phenyl)-1-[1-(1-methyl-piperidin-4-yl)-1*H*-benzimidazol-2-yl]-ethyl}-amide,
(38) 4-(2-oxo-1,2,4,5-tetrahydro-1,3-benzodiazepin-3-yl)-piperidin-1-carboxylic acid-{2-(3,4-dibromo-phenyl)-1-[1-(3-pyrrolidin-1-yl-propyl)-1*H*-benzimidazol-2-yl]-ethyl}-amide,
(39) 4-(2-oxo-1,2,4,5-tetrahydro-1,3-benzodiazepin-3-yl)-piperidin-1-carboxylic acid-{2-(3,4-dibromo-phenyl)-1-[1-(1-methyl-piperidin-4-ylmethyl)-1*H*-benzimidazol-2-yl]-ethyl}-amide,
(40) 4-(2-oxo-1,2,4,5-tetrahydro-1,3-benzodiazepin-3-yl)-piperidin-1-carboxylic acid-[2-(3,4-dibromo-phenyl)-1-(1-pyridin-3-ylmethyl-1*H-*benzimidazol-2-yl)-ethyl]-amide,
(41) 4-(2-oxo-1 ,2,4,5-tetrahydro-1 ,3-benzodiazepin-3-yl)-piperidin-1-carboxylic acid-{2-(3,4-dibromo-phenyl)-1-[1-(3-dimethylamino-propyl)-1*H*-benzimidazol-2-yl]-ethyl}-amide,
(42) 4-(2-oxo-1,2,4,5-tetrahydro-1,3-benzodiazepin-3-yl)-piperidin-1-carboxylic acid-{2-(3,4-dibromo-phenyl)-1-[1-(4-dimethylamino-butyl)-1*H*-benzimidazol-2-yl]-ethyl}-amide,
(43) 4-(2-oxo-1,2,4,5-tetrahydro-1 ,3-benzodiazepin-3-yl)-piperidin-1-carboxylic acid-{2-(3,4-diethyl-phenyl)-1-[1-(3-dimethylamino-propyl)-1*H*-benzimidazol-2-yl]-ethyl}-amide,
(44) 4-(2-oxo-1,2,4,5-tetrahydro-1,3-benzodiazepin-3-yl)-piperidin-1-carboxylic acid-{2-(3,4-diethyl-phenyl)-1-[1-(4-dimethylamino-butyl)-1*H*-benzimidazol-2-yl]-ethyl}-amide,
(45) 4-(2-oxo-1,2,4,5-tetrahydro-1,3-benzodiazepin-3-yl)-piperidin-1-carboxylic acid-{2-(3,4-diethyl-phenyl)-1-[1-(1-methyl-piperidin-4-yl)-1*H*-benzimidazol-2-yl]-ethyl}-amide,
(46) 4-(2-oxo-1,2,4,5-tetrahydro-1,3-benzodiazepin-3-yl)-piperidin-1-carboxylic acid-{2-(3,4-diethyl-phenyl)-1-[1-(3-pyrrolidin-1-yl-propyl)-1*H*-benzimidazol-2-yl]-ethyl}-amide,
(47) 4-(2-oxo-1,2,4,5-tetrahydro-1,3-benzodiazepin-3-yl)-piperidin-1-carboxylic acid-{2-(3,4-diethyl-phenyl)-1-[1-(1-methyl-piperidin-4-ylmethyl)-1*H*-benzimidazol-2-yl]-ethyl}-amide,
(48) 4-(2-oxo-1,2,4,5-tetrahydro-1,3-benzodiazepin-3-yl)-piperidin-1-carboxylic acid-[2-(3,4-diethyl-phenyl)-1-(1-pyridin-3-ylmethyl-1*H-*benzimidazol-2-yl)-ethyl]-amide,
(49) 4-(2-oxo-1,2,4,5-tetrahydro-1,3-benzodiazepin-3-yl)-piperidin-1-carboxylic acid-{2-(3,5-bis-trifluoromethyl-phenyl)-1-[1-(1-methyl-piperidin-4-yl)-1*H*-benzimidazol-2-yl]-ethyl}-amide,
(50) 4-(2-oxo-1,2,4,5-tetrahydro-1,3-benzodiazepin-3-yl)-piperidin-1-carboxylic acid-{2-(3,5-bis-trifluoromethyl-phenyl)-1-[1-(3-pyrrolidin-1-yl*-*propyl)-1*H*-benzimidazol-2-yl]-ethyl}-amide,
(51) 4-(2-oxo-1,2,4,5-tetrahydro-1,3-benzodiazepin-3-yl)-piperidin-1-carboxylic acid-{2-(3,5-bis-trifluoromethyl-phenyl)-1-[1-(1-methyl-piperidin-4-ylmethyl)-1*H*-benzimidazol-2-yl]-ethyl}-amide,
(52) 4-(2-oxo-1,2,4,5-tetrahydro-1,3-benzodiazepin-3-yl)-piperidin-1-carboxylic acid-{2-(3,5-bis-trifluoromethyl-phenyl)-1-[1-(3-dimethylamino-propyl)-1*H*-benzimidazol-2-yl]-ethyl}-amide,
(53) methyl 2-(2-(4-amino-3-chloro-5-trifluoromethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazole-5-carboxylate,
(54) 2-(2-(4-amino-3-chloro-5-trifluoromethyl-phenyl)-1-{[4-(2-oxo-1,2,4,5-tetrahydro-1,3-benzodiazepin-3-yl)-piperidin-1-carbonyl]-amino}-ethyl)-1-(3-pyrrolidin-1-yl-propyl)-1*H*-benzimidazole-5-carboxylic acid,
(55) 4-(2-oxo-1,2,4,5-tetrahydro-1,3-benzodiazepin-3-yl)-piperidin-1-carboxylic acid-{2-(4-amino-3-chloro-5-trifluoromethyl-phenyl)-1-[1-(1-methyl-piperidin-4-yl)-1*H*-benzimidazol-2-yl]-ethyl}-amide,
(56) 4-(2-oxo-1,2,4,5-tetrahydro-1,3-benzodiazepin-3-yl)-piperidin-1-carboxylic acid-{2-(4-amino-3-chloro-5-trifluoromethyl-phenyl)-1-[1-(3-pyrrolidin-1-yl-propyl)-1*H*-benzimidazol-2-yl]-ethyl}-amide,
(57) 4-(2-oxo-1,2,4,5-tetrahydro-1,3-benzodiazepin-3-yl)-piperidin-1-carboxylic acid-{2-(4-amino-3-chloro-5-trifluoromethyl-phenyl)-1-[1-(1-methyl-piperidin-4-ylmethyl)-1*H*-benzimidazol-2-yl]-ethyl}-amide,
(58) 4-(2-oxo-1,2,4,5-tetrahydro-1,3-benzodiazepin-3-yl)-piperidin-1-carboxylic acid-[2-(4-amino-3-chloro-5-trifluoromethyl-phenyl)-1-(1-pyridin-3-ylmethyl-1*H*-benzimidazol-2-yl)-ethyl]-amide,
(59) 4-(2-oxo-1,2,4,5-tetrahydro-1,3-benzodiazepin-3-yl)-piperidin-1-carboxylic acid-{2-(4-amino-3-chloro-5-trifluoromethyl-phenyl)-1-[1-(3-dimethylamino-propyl)-1*H*-benzimidazol-2-yl]-ethyl}-amide,
(60) 3-(1-{4-(3,4-diethyl-phenyl)-3-[1-(3-dimethylamino-propyl)-1*H-*benzimidazol-2-yl]-butyryl}-piperidin-4-yl)-1,3,4,5-tetrahydro-1,3-benzodiazepin-2-one,
(61) 3-(1-{4-(4-amino-3-chloro-5-trifluoromethyl-phenyl)-3-[1-(3-pyrrolidin-1-yl-propyl)-1*H*-benzimidazol-2-yl]-butyryl}-piperidin-4-yl)-1,3,4,5-tetrahydro-1,3-benzodiazepin-2-one,
(62) 3-(1-{4-(4-amino-3-chloro-5-trifluoromethyl-phenyl)-3-[1-(3-imidazol-1-yl-propyl)-1*H*-benzimidazol-2-yl]-butyryl}-piperidin-4-yl)-1,3,4,5-tetrahydro-1,3-benzodiazepin-2-one,
(63) 3-(1-{4-(4-amino-3-chloro-5-trifluoromethyl-phenyl)-3-[5-(3-dimethylamino-propyl)-1-ethyl-1*H*-benzimidazol-2-yl]-butyryl}-piperidin-4-yl)-1,3,4,5-tetrahydro-1,3-benzodiazepin-2-one,
(64) 3-(1-{4-(4-amino-3-chloro-5-trifluoromethyl-phenyl)-3-[1-(3-diethylamino-propyl)-1*H*-benzimidazol-2-yl]-butyryl}-piperidin-4-yl)-1,3,4,5-tetrahydro-1,3-benzodiazepin-2-one,
(65) 3-(1-{4-(4-amino-3-chloro-5-trifluoromethyl-phenyl)-3-[1-(4-hydroxybutyl)-1*H*-benzimidazol-2-yl]-butyryl}-piperidin-4-yl)-1,3,4,5-tetrahydro-1,3-benzodiazepin-2-one,
(66) 3-(1-{4-(4-amino-3-chloro-5-trifluoromethyl-phenyl)-3-[1-(1-methyl-piperidin-3-ylmethyl)-1*H*-benzimidazol-2-yl]-butyryl}-piperidin-4-yl)-1,3,4,5-tetrahydro-1,3-benzodiazepin-2-one,
(67) 3-[1-(4-(4-amino-3-chloro-5-trifluoromethyl-phenyl)-3-{1-[2-(4-methylpiperazin-1-yl)-ethyl]-1*H*-benzimidazol-2-yl}-butyryl)-piperidin-4-yl]-1,3,4,5-tetrahydro-1,3-benzodiazepin-2-one,
(68) 3-{1-[4-(4-amino-3-chloro-5-trifluoromethyl-phenyl)-3-(1-pyridin-4-ylmethyl-1*H*-benzimidazol-2-yl)-butyryl]-piperidin-4-yl}-1,3,4,5-tetrahydro-1,3-benzodiazepin-2-one,
(69) 3-{1-[4-(4-amino-3-chloro-5-trifluoromethyl-phenyl)-3-(1-piperidin-4-ylmethyl-1*H*-benzimidazol-2-yl)-butyryl]-piperidin-4-yl}-1,3,4,5-tetrahydro-1,3-benzodiazepin-2-one,
(70) 3-(1-{4-(4-amino-3-chloro-5-trifluoromethyl-phenyl)-3-[1-(3-dimethylamino-2,2-dimethyl-propyl)-1*H*-benzimidazol-2-yl]-butyryl}-piperidin-4-yl)-1,3,4,5-tetrahydro-1,3-benzodiazepin-2-one,
(71) 3-(1-{4-(4-amino-3-chloro-5-trifluoromethyl-phenyl)-3-[7-(3-dimethylamino-propoxy)-1*H*-benzimidazol-2-yl]-butyryl}-piperidin-4-yl)-1,3,4,5-tetrahydro-1,3-benzodiazepin-2-one,
(72) 3-{1-[4-(4-amino-3-chloro-5-trifluoromethyl-phenyl)-3-(1-piperidin-4-ylmethyl-1*H*-imidazo[4,5-c]pyridin-2-yl)-butyryl]-piperidin-4-yl}-1,3,4,5-tetrahydro-1,3-benzodiazepin-2-one,
(73) 3-(1-{4-(4-amino-3-chloro-5-trifluoromethyl-phenyl)-3-[3-(3-pyrrolidin-1-yl-propyl)-3*H*-imidazo[4,5-c]pyridin-2-yl]-butyryl}-piperidin-4-yl)-1,3,4,5-tetrahydro-1,3-benzodiazepin-2-one,
(74) 3-{1-[4-(4-amino-3-chloro-5-trifluoromethyl-phenyl)-3-(1-methyl-6-pyrrolidin-1-ylmethyl-1*H*-benzimidazol-2-yl)-butyryl]-piperidin-4-yl}-1,3,4,5-tetrahydro-1,3-benzodiazepin-2-one,
their stereoisomers and salts.

11. Physiologically acceptable salts of the compounds according to at least one of claims 1 to 10 with inorganic or organic acids or bases.

12. Pharmaceutical compositions containing a compound according to at least one of claims 1 to 10 or a physiologically acceptable salt according to claim 11 optionally together with one or more inert carriers and/or diluents.

13. Use of a compound according to at least one of claims 1 to 11 for preparing a pharmaceutical composition for the acute and prophylactic treatment of headaches, particularly migraine or cluster headaches.

14. Process for preparing a pharmaceutical composition according to claim 12, **characterised in that** a compound according to at least one of claims 1 to 11 is incorporated in one or more inert carriers and/or diluents by a non-chemical method.

15. Process for preparing the compounds according to claims 1 to 11, **characterised in that**
a) in order to prepare compounds of general formula I wherein X denotes the oxygen atom and Z denotes the -NR¹ group, and wherein A, n, R, R¹, R², R³ and R⁴ and A are defined as in claims 1 to 10,
an amine of general formula II, 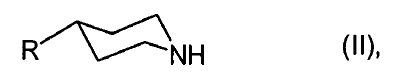 wherein
R is defined as in claims 1 to 10, is reacted with a carbonic acid derivative of general formula III, 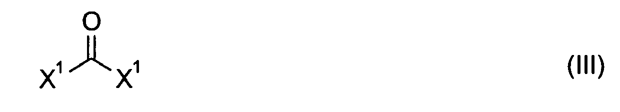 wherein
X¹ denotes a nucleofugic group,
and with a compound of general formula IV, 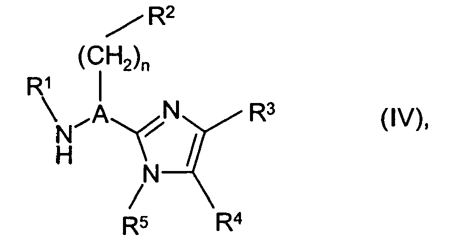 wherein
R¹, R², R³, R⁴, R⁵, A and n are defined as in claims 1 to 10,
and subsequently, if necessary, any protecting groups are cleaved or precursor functions are converted, or
b) In order to prepare compounds of general formula I wherein A, n, R, R¹, R², R³ and R⁴ and A are defined as in claims 1 to 10 and R³ and R⁴ together represent a 1,3-butadien-1,4-ylene group wherein
one, two or three methyne groups may each be replaced by a nitrogen atom,
a hydrogen atom may be replaced by a fluorine, chlorine, bromine or iodine atom, by a C₁₋₃-alkyl, trifluoromethyl, difluoromethyl, cyclopropyl, hydroxy, C₁₋₃-alkoxy, difluoromethoxy, trifluoromethoxy, cyano, nitro, amino, C₁₋₃-alkyl-amino, di-(C₁₋₃-alkyl)-amino, carboxy, C₁₋₃-alkoxycarbonyl, aminocarbonyl, C₁₋₃-alkyl-aminocarbonyl, di-(C₁₋₃-alkyl)-aminocarbonyl, di-(C₁₋₃-alkyl)-amino-C₂₋₃-alkyl-aminocarbonyl, amino-C₁₋₃-alkyl, C₁₋₃-alkyl-amino-C₁₋₃-alkyl or di-(C₁₋₃-alkyl)-amino-C₁₋₃-alkyl group or by a 4- to 7-membered cycloalkyleneimino-carbonyl group wherein
one or two hydrogen atoms may each be replaced by a C₁₋₃-alkyl group and/or
in each case the methylene group in the 4 position of a 6- or 7-membered cycloalkyleneimino moiety may be replaced by an oxygen or sulphur atom, by a sulphinyl, sulphonyl, -NH, -N(C₁₋₃-alkyl) or -*N*(C₁₋₃-alkylcarbonyl)- group,
and a second hydrogen atom may optionally additionally be replaced by a fluorine, chlorine, bromine or iodine atom, by a C₁₋₃-alkyl, trifluoromethyl, difluoromethyl, cyclopropyl, hydroxy, C₁₋₃-alkoxy, difluoromethoxy or trifluoromethoxy group,
an amide of general formula 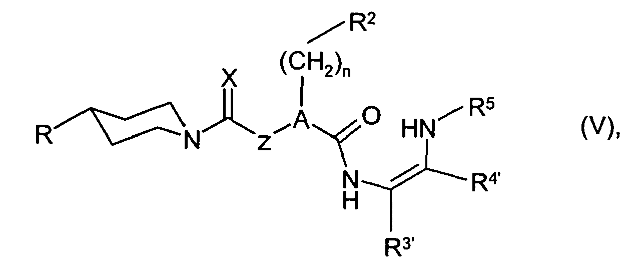 wherein R, X, Z, A, n, R² and R⁵ are defined as in claims 1 to 10, R^{3'} and R^{4'} together represent a 1,3-butadien-1,4-ylene group wherein
one, two or three methyne groups may each be replaced by a nitrogen atom,
a hydrogen atom may be replaced by a fluorine, chlorine, bromine or iodine atom, by a C₁₋₃-alkyl, trifluoromethyl, difluoromethyl, cyclopropyl, hydroxy, C₁₋₃-alkoxy, difluoromethoxy, trifluoromethoxy, cyano, nitro, amino, C₁₋₃-alkyl-amino, di-(C₁₋₃-alkyl)-amino, carboxy, C₁₋₃-alkoxycarbonyl, aminocarbonyl, C₁₋₃-alkyl-aminocarbonyl, di-(C₁₋₃-alkyl)-aminocarbonyl, di-(C₁₋₃-alkyl)-amino-C₂₋₃-alkyl-aminocarbonyl, amino-C₁₋₃-alkyl, C₁₋₃-alkyl-amino-C₁₋₃-alkyl or di-(C₁₋₃-alkyl)-amino-C₁₋₃-alkyl group or by a 4- to 7-membered cycloalkyleneiminocarbonyl group wherein
one or two hydrogen atoms may each be replaced by a C₁₋₃-alkyl group and/or
in each case the methylene group in the 4 position of a 6- or 7-membered cycloalkyleneimino moiety may be replaced by an oxygen or sulphur atom, by a sulphinyl, sulphonyl, -NH, -N(C₁₋₃-alkyl) or -N(C₁₋₃-alkyl-carbonyl) group,
and a second hydrogen atom may optionally additionally be replaced by a fluorine, chlorine or bromine atom, by a C₁₋₃-alkyl, trifluoromethyl, difluoromethyl, cyclopropyl, hydroxy, C₁₋₃-alkoxy, difluoromethoxy or trifluoromethoxy group,
is anellated, or
c) In order to prepare compounds of general formula I wherein R, A, n and R² to R⁵ are defined as in claims 1 to 10 and X denotes the oxygen atom and Z denotes the -CH₂ group:
A carboxylic acid of general formula 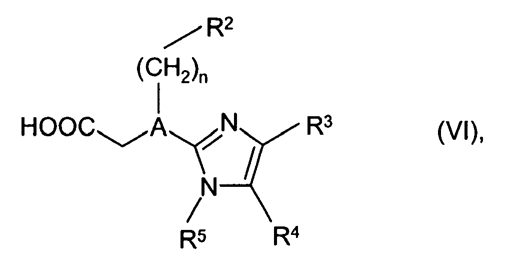
wherein
R² to R⁵, A and n are defined as in claims 1 to 10,
is coupled with a compound of general formula 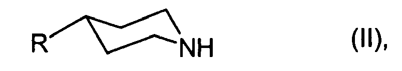 wherein
R is defined as in claims 1 to 10, or
d) In order to prepare compounds of general formula I wherein R, A, n and R² to R⁵ are defined as in claims 1 to 10 and X denotes the oxygen atom and Z denotes the -CH₂ group:
a compound of general formula 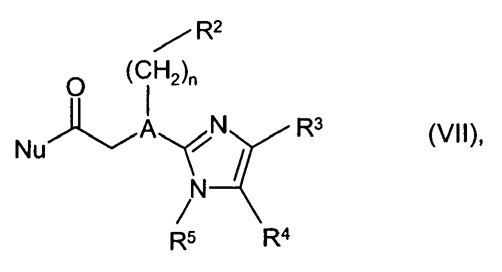
wherein
A, n, and R² to R⁵ are defined as in claims 1 to 10 and Nu denotes a leaving group,
is coupled with a compound of general formula 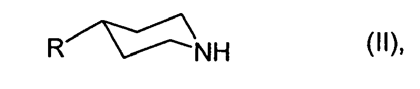 wherein
R is defined as in claims 1 to 10, or
e) In order to prepare compounds of general formula I wherein R, A, n and R² to R⁵ are defined as in claims 1 to 10 and X denotes the oxygen atom and Z denotes the group -NH-:
an isocyanate of general formula VIII, 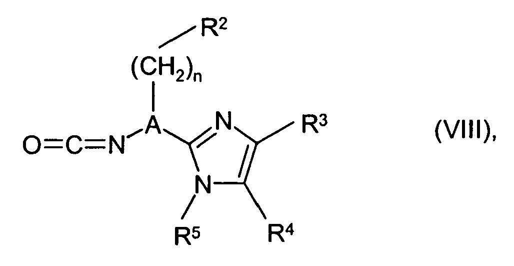
wherein
R² to R⁵, A and n are defined as in claims 1 to 10
is reacted with amines of general formula II, 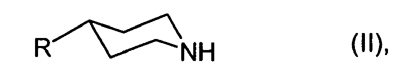 wherein
R is defined as in claims 1 to 10, and subsequently, if necessary, protecting groups are cleaved or precursor functions are converted, or
f) In order to prepare compounds of general formula I wherein R, A, n and R² to R⁵ are defined as in claims 1 to 10 and X denotes one of the groups =N-CN or =N-SO₂-R⁶ and Z denotes the group -NR¹, where R¹ and R⁶ are defined as in claims 1 to 10:
a compound of general formula 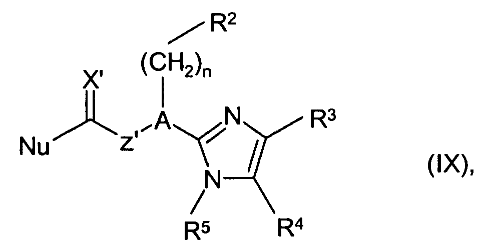
wherein A, n and R² to R⁵ are defined as in claims 1 to 10, X' denotes one of the groups =N-CN or =N-SO₂-R⁶, Z' denotes the group -NR¹, while R¹ and R⁶ are defined as in claims 1 to 10, and Nu is a leaving group,
is reacted with secondary amines of general formula 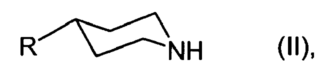 wherein R is defined as in claims 1 to 10, and
any protecting groups for reactive groups such as hydroxy, carboxy, amino or imino groups are cleaved and / or
a compound of general formula I thus obtained is resolved into the stereoisomers thereof and / or
a compound of general formula I thus obtained is converted into the salts thereof, particularly for pharmaceutical use into the physiologically acceptable salts thereof with an inorganic or organic acid or base.

16. Use of a compound according to at least one of claims 1 to 11 for preparing a pharmaceutical composition for combating non-insulin-dependent diabetes mellitus (NIDDM).

17. Use of a compound according to at least one of claims 1 to 11 for preparing a pharmaceutical composition for the treatment of cardiovascular diseases, morphine tolerance, skin diseases, particularly thermal and radiation-induced skin damage including sunburn, inflammatory diseases such as in particular inflammatory diseases of the joints such as arthritis, inflammatory lung diseases, allergic rhinitis, asthma, diseases accompanied by excessive vasodilatation and consequent reduced circulation of blood through the tissues, such as particularly shock or sepsis, for relieving pain in general or for preventive or acute-therapeutic influence on the symptoms of hot flushes caused by vasodilatation and increased blood flow in menopausal oestrogen-deficient women and hormone-treated patients with prostate carcinoma.

## Revendications

1. Composés de la formule générale 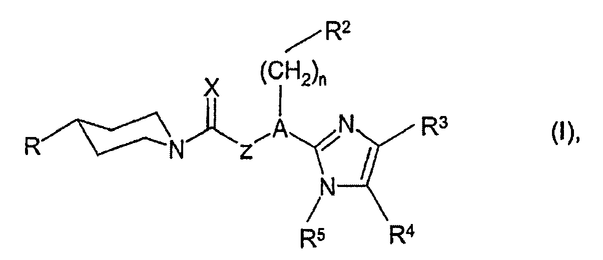 dans laquelle
R représente un hétérocycle aza, diaza, triaza, oxaza, thiaza, thiadiaza ou S,S-dioxydo-thiadiaza saturé, simplement insaturé ou, dans le cas d'hétérocycles à 6 ou 7 chaînons, aussi doublement insaturé,
dans lequel les hétérocycles mentionnés ci-avant sont liés par l'intermédiaire d'un atome de carbone ou d'un atome d'azote et
contiennent un ou deux groupes carbonyles qui sont reliés par au moins un atome d'azote,
qui peut porter sur un des atomes d'azote un substituant alkyle,
qui peut porter sur un ou sur deux des atomes de carbone un substituant alkyle, phényle, phénylméthyle, naphthyle, biphénylyle; pyridinyle, diazinyle, furyle, thiényle, pyrrolyle, 1,3-oxazolyle, 1,3-thiazolyle, isoxazolyle, pyrazolyle, 1-méthylpyrazolyle, imidazolyle ou 1-méthylimidazolyle, les substituants pouvant être identiques ou différents,
une double liaison d'un des hétérocycles insaturés cités ci-avant pouvant être condensée avec un noyau de benzène, de pyridine, de diazine, de 1,3-oxazole, de thiophène, de furanne, de thiazole, de pyrrole, de n-méthyl-pyrrole ou de quinoléine, avec un noyau 2(1H)-oxoquinoléine substitué le cas échéant par un groupe alkyle sur l'atome d'azote ou peut être condensée avec un noyau imidazole ou N-méthyl-imidazole, ou bien deux doubles liaisons oléfiniques d'un des hétérocycles insaturés mentionnés ci-avant pouvant être condensées chacun avec un noyau benzénique,
les groupes phényle, pyridinyle, diazinyle, furyle, thiényle, pyrrolyle, 1,3-oxazolyle, 1,3-thiazolyle, isoxazolyle, pyrazolyle, 1-méthyl-pyrazolyle, imidazolyle ou 1-méthylimidazolyle, ainsi que les benzo-, thiéno-, pyrido- et diazino-hétérocycles condensés contenus dans R pouvant être en plus mono-, di- ou trisubstitués dans le squelette carboné par des atomes de fluor, de chlore, de brome ou d'iode, par des groupes alkyle, alkoxyle, nitro, alkylthiol, alkylsulfinyle, alkylsulfonyle, alkylsulfonylamine, phényle, trifluorométhyle, difluorométhyle, difluorométhoxyle, alkoxycarbonyle, carboxyle, dialkylamino, hydroxyle, amino, acétylamino, propionylamino, aminocarbonyle, alkylaminocarbonyle, dialkylaminocarbonyle, (4-morpholinyl)carbonyle, (1-pyrrolidinyl)carbonyle, (1-pipéridinyl)carbonyle, (hexahydro-1-azépinyl)carbonyle, (4-méthyl-1-pipérazinyl)carbonyle, méthylène-dioxy, aminocarbonylamino, alkanoyle, cyano, trifluorométhoxy, trifluorométhylthio, trifluorométhylsulfinyle ou trifluorométhylsulfonyle, les substituants pouvant être identiques ou différents,
X représente un atome d'oxygène ou, dans la mesure où Z représente le groupe - NR¹-, également un groupe =N-CN ou =N-SO₂-R⁶, dans lequel
R⁶ représente un radical alkyle avec 1 à 4 atomes de carbone ou un radical phényle, substitué éventuellement par un atome d'halogène, un groupe méthyle ou un groupe méthoxyle,
Z représente un des groupes -CH₂-, dans lequel les atomes d'hydrogène peuvent être remplacés, indépendamment l'un de l'autre par un atome de fluor ou un groupe C₁₋₃-alkyle, ou -NR¹-, dans lequel
R¹ représente un atome d'hydrogène, un groupe alkyle, qui peut être substitué dans la partie alkyle, à l'exception de la position 1, par un groupe amino, C₁₋₃-alkylamino ou di-(C₁₋₃-alkyl)-amino, ou un groupe phénylalkyle, qui peut être mono- ou disubstitué dans la partie phényle par des atomes de fluor, de chlore, de brome ou d'iode, par des radicaux C₁₋₃-alkyle, trifluorométhyle, difluorométhyle, cyclopropyle, hydroxyle, C₁₋₃-alcoxyle, difluorométhoxyle, trifluorométhoxyle, cyano, nitro, amino, C₁₋₃-alkylamino, di-(C₁₋₃-alkyl)amino, carboxyle, C₁₋₃-alkoxycarbonyle, aminocarbonyle, C₁₋₃-alkylaminocarbonyle, di-(C₁₋₃-alkyl)aminocarbonyle, amino-C₁₋₃-alkyle, C₁₋₃-alkylamino-C₁₋₃-alkyle ou di-(C₁₋₃-alkyl)-amino-C₁₋₃-alkyle, les substituants pouvant être identiques ou différents,
A représente un atome de carbone substitué par un atome d'hydrogène ou par un groupe C₁₋₃-alkyle
n représente le nombre 1 ou 2,
R² représente le groupe 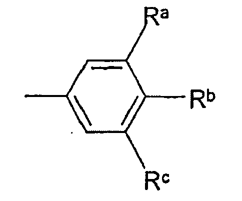 dans lequel
un des groupes R^{a}, R^{b} et R^{c} représente un atome d'hydrogène, de fluor, de chlore, de brome ou d'iode, un groupe alkyle ramifié ou linéaire, un groupe hydroxyle, alkoxyle, trifluorométhyle, difluorométhyle, trifluorométhoxyle, difluorométhoxyle, trifluorométhylthiol, amino, acétylamino, dialkylaminoalkyle, dialkylaminoalkoxyle, nitro, méthylsulfonyloxyle, aminocarbonyle, alkylaminocarbonyle, dialkylaminocarbonyle, alkanoyle, cyano, trifluorométhylsulfinyle ou trifluorométhylsulfonyle,
un deuxième des groupes R^{a}, R^{b} et R^{c} représente un atome d'hydrogène, de fluor, de chlore, de brome ou d'iode, un groupe alkyle ramifié ou linéaire, un groupe hydroxyle, alkoxyle, trifluorométhyle, difluorométhyle, trifluorométhoxyle, difluorométhoxyle, trifluorométhylthiol, amino, acétylamino, alkanoyle, aminocarbonyle, alkylaminocarbonyle ou dialkylaminocarbonyle et
le troisième des groupes R^{a}, R^{b} et R^{c} représente un atome d'hydrogène, de fluor, de chlore, de brome ou d'iode, un groupe alkyle ramifié ou linéaire, un groupe hydroxyle, alkoxyle, trifluorométhyle, difluorométhyle, difluorométhoxyle, ou trifluorométhoxyle, les substituants pouvant être identiques ou différents,
R³ et R⁴, qui peuvent être identiques ou différents, représentent chacun un atome d'hydrogène, un C₁₋₄-alkyle, un amino-C₁₋₃-alkyle, un C₁₋₃-alkyl-amino-C₁₋₃-alkyle ou un di-(C₁₋₃-alkyl)-amino-C₁₋₃-alkyle,
un groupe phényle ou naphthyle, éventuellement chacun mono- ou disubstitués par R^{d}, les substituants pouvant être identiques ou différents, et R^{d} représente un atome de fluor, de chlore, de brome ou d'iode, un groupe C₁₋₃-alkyle, trifluorométhyle, difluorométhyle, cyclopropyle, hydroxyle, C₁₋₃-alkoxyle, difluorométhoxyle, trifluorométhoxyle, cyano, nitro, amino, C₁₋₃-alkyl-amino, di-(C₁₋₃-alkyl)-amino, carboxyle, C₁₋₃-alkoxy-carbonyle, aminocarbonyle, C₁₋₃-alkyl-aminocarbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle ou un groupe cycloalkylène-imino-carbonyle à 4 à 7 chaînons, dans lequel
un ou deux atomes d'hydrogène peuvent être remplacés chacun par un groupe C₁₋₃-alkyle ou/et
le groupe méthylène en position 4 d'un fragment cycloalkylènimine à 6 ou 7 chaînons peut être remplacé par un atome d'oxygène ou par un atome de soufre, par un groupe sulfinyle, sulfonyle, -NH-, -N(C₁₋₃-alkyl)- ou -N(C₁₋₃-alkyl-carbonyl),
un groupe hétéroaryle monocyclique à 5 ou 6 chaînons éventuellement substitué sur le squelette de carbone par un groupe C₁₋₃-alkyle, amino, C₁₋₃-alkylamino, di-(C₁₋₃-alkyl)-amino, carboxyle, C₁₋₃-alkoxy-carbonyle, aminocarbonyle, C₁₋₃-alkyl-aminocarbonyle, di-(C₁₋₃-alkyl)-aminocarbonyle, amino-C₁₋₃-alkyle, C₁₋₃-alkylamino-C₁₋₃-alkyle ou di-(C₁₋₃-alkyl)-amino-C₁₋₃-alkyle, dans lequel
le groupe hétéroaryle à 6 chaînons contient un, deux ou trois atomes d'azote et peut être substitué le cas échéant en plus par un atome de fluor, de chlore, de brome ou d'iode, un groupe C₁₋₃-alkyle, C₁₋₃-alkoxyle, amino, C₁₋₃-alkylamino-ou di-(C₁₋₃-alkyl)amino et
le groupe hétéroaryle à 5 chaînons contient un groupe imine substitué éventuellement par un groupe C₁₋₃-alkyle, acétyle, trifluoracétyle, C₁₋₃-alkoxycarbonyle, aminocarbonyle, C₁₋₃-alkyl-amino-carbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle, un atome d'oxygène ou un atome de soufre ou
un groupe imine substitué éventuellement par un groupe C₁₋₃-alkyle, acétyle, trifluoracétyle, C₁₋₃-alkoxy-carbonyle, aminocarbonyle, C₁₋₃-alkyl-amino-carbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle, ou un atome d'oxygène ou un atome de soufre et en plus un atome d'azote ou
un groupe imine substitué éventuellement par un groupe C₁₋₃-alkyle, acétyle, trifluoracétyle, C₁₋₃-alkoxy-carbonyle, aminocarbonyle, C₁₋₃-alkyl-amino-carbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle, et deux atomes d'azote,
ou R³ et R⁴ représentent ensemble un groupe 1,3-butadién-1,4-ylène, dans lequel un, deux ou trois groupes méthine peuvent être remplacés chacun par un atome d'azote,
un atome d'hydrogène peut être remplacé par un atome de fluor, de chlore, de brome ou d'iode, par un groupe C₁₋₃-alkyle, trifluorométhyle, difluorométhyle, cyclopropyle, hydroxyle, C₁₋₃-alkoxyle, difluorométhoxyle, trifluorométhoxyle-, cyano, nitro, amino, C₁₋₃-alkyl-amino, di-(C₁₋₃-alkyl)-amino, carboxyle, C₁₋₃-alkoxy-carbonyle, aminocarbonyle, C₁₋₃-alkyl-aminocarbonyle, di-(C₁₋₃-alkyl)-aminocarbonyle, di-(C₁₋₃-alkyl)-amino-C₂₋₃-alkyl-aminocarbonyle, amino-C₁₋₃-alkyle, C₁₋₃-alkyl-amino-C₁₋₃-alkyle ou di-(C₁₋₃-alkyl)-amino-C₁₋₃-alkyle ou par
un groupe cycloalkylène-imino-carbonyle à 4 à 7 chaînons, dans lequel
un ou deux atomes d'hydrogène peuvent être remplacés chacun par un groupe C₁₋₃-alkyle ou/et
le groupe méthylène en position 4 d'un fragment cycloalkylènimine à 6 ou 7 chaînons peut être remplacé par un atome d'oxygène ou de soufre, par un groupe sulfinyle, sulfonyle, -NH-, -N(C₁₋₃-alkyl)- ou -N(C₁₋₃-alkyl-carbonyl),
et le cas échéant en plus un deuxième atome d'hydrogène peut être remplacé par un atome de fluor, de chlore, de brome ou d'iode, par un groupe C₁₋₃-alkyle, trifluorométhyle, difluorométhyle, cyclopropyle, hydroxyle, C₁₋₃-alkoxyle, difluorométhoxyle ou trifluorométhoxyle, et
R⁵ représente le groupe -(CH₂)ₘ-R^{e}, dans lequel
m représente le nombre 0 et
R^{e} représente un atome d'hydrogène,
un groupe phényle ou naphthyle, qui peuvent être chacun mono- ou disubstitués par R^{f}, les substituants pouvant être identiques ou différents et que R^{f} représente un atome de fluor, de chlore, de brome ou d'iode, un groupe C₁₋₃-alkyle, trifluorométhyle, difluorométhyle, cyclopropyle, hydroxyle, C₁₋₃-alkoxyle, difluorométhoxyle, trifluorométhoxyle, cyano, nitro, amino, C₁₋₃-alkyl-amino, di-(C₁₋₃-alkyle)-amino, carboxyle, C₁₋₃-alkoxy-carbonyle, aminocarbonyle, C₁₋₃-alkyl-aminocarbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyl, ou
un groupe pyridinyle, pyrazinyle, pyrimidinyle, pyridazinyle, pyrrolyle, furyle, thiényle, pyrazolyle, imidazolyle, oxazolyle, isoxazolyle, thiazolyle ou isothiazolyle, dans lesquels un atome d'hydrogène lié à un atome d'azote peut être remplacé par un groupe C₁₋₃-alkyle ou acétyle, substitué le cas échéant dans le squelette de carbone par un groupe C₁₋₃-alkyle, ou
un groupe C₃₋₇-cycloalkyle, dans lequel
un atome d'hydrogène du groupe C₃₋₇-cycloalkyle peut être remplacé par un groupe amino, C₁₋₃-alkyl-amino, di-(C₁₋₃- alkyl)-amino, amino-C₁₋₃-alkyle, C₁₋₃-alkyl-amino-C₁₋₃-alkyle ou di-(C₁₋₃-alkyl)-amino-C₁₋₃-alkyle ou/et le groupe méthylène en position 3 du groupe cyclopentyle et le groupe méthylène en position 4 du groupe cyclohexyle et cycloheptyle peuvent être remplacés chacun par un atome d'oxygène ou un atome de soufre, par un groupe sulfinyle, sulfonyle, -NH-, -N(C₁₋₃-alkyl)- ou -N(C₁₋₃-alkyl-carbonyl),
ou m représente un des nombre 1 à 5 et
R^{e} représente un atome d'hydrogène, un groupe aminométhylène, C₁₋₃-alkylaminométhylène, di-(C₁₋₃-alkyl)-aminométhylène, aminocarbonyle, C₁₋₃-alkylaminocarbonyle, di-(C₁₋₃-alkyl)-aminocarbonyle ou un groupe C₃₋₇-cycloalkyle
dans lequel
un atome d'hydrogène du groupe C₃₋₇-cycloalkyle peut être remplacé par un groupe amino, C₁₋₃-alkylamino, di-(C₁₋₃-alkyl)-amino, amino-C₁₋₃-alkyle, C₁₋₃-alkyl-amino-C₁₋₃-alkyle ou di-(C₁₋₃-alkyl)-amino-C₁₋₃-alkyle ou/et
le groupe méthylène en position 3 du groupe cyclopentyle et le groupe méthylène en position 4 des groupes cyclohexyle et cycloheptyle peuvent être remplacés chacun par un atome d'oxygène ou un atome de soufre, par un groupe sulfinyle, sulfonyle, -NH-, -N(C₁₋₃-alkyl)- ou -N(C₁₋₃-alkyl-carbonyl),
un groupe cycloalkylèneimine à 4 à 7 chaînons, dans lequel
un ou deux atomes d'hydrogène peuvent être remplacés chacun par un groupe C₁₋₃-alkyle ou/et
le groupe méthylène en position 4 d'un groupe cycloalkylèneimine à 6 ou 7 chaînons peut être remplacé par un atome d'oxygène ou de soufre, par un groupe sulfinyle, sulfonyle, -NH-, -N(C₁₋₃-alkyl)- ou -N(C₁₋₃-alkyl-carbonyl),
un groupe phényle ou naphthyle, qui peuvent être chacun mono- ou disubstitués, indépendamment l'un de l'autre par R^{g}, sachant que les substituants peuvent être identiques ou différents et que R^{g} représente un atome de fluor, de chlore, de brome ou d'iode, un groupe C₁₋₃- alkyle, trifluorométhyle, difluorométhyle, cyclopropyle, hydroxyle, C₁₋₃-alkoxyle, difluorométhoxyle, trifluorométhoxyle, cyano, nitro, amino, C₁₋₃-alkyl-amino-, di-(C₁₋₃-alkyl)-amino, carboxyle, C₁₋₃-alkoxy-carbonyle, aminocarbonyle, C₁₋₃-alkyl-aminocarbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle,
ou un groupe hétéroaryle monocyclique à 5 ou 6 chaînons substitué le cas échéant sur le squelette de carbone par un groupe C₁₋₃-alkyle, amino, C₁₋₃-alkyl-amino, di-(C₁₋₃-alkyl)-amino-, carboxyle, C₁₋₃-alkoxy-carbonyle, amino-carbonyle, C₁₋₃-alkyl-aminocarbonyle-, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle, sachant que
le groupe hétéroaryle à 6 chaînons contient un, deux ou trois atomes d'azote et peut être substitué le cas échéant en plus par un atome de fluor, de chlore, de brome ou d'iode, un groupe C₁₋₃-alkyle, C₁₋₃-alkoxyle, amino, C₁₋₃-alkylamino ou di-(C₁₋₃-alkyl)amino et que
le groupe hétéroaryle à 5 chaînons contient un groupe imino, substitué le cas échéant par un groupe C₁₋₃-alkyle, acétyle, trifluoracétyle, C₁₋₃-alkoxycarbonyle, aminocarbonyle, C₁₋₃-alkyl-aminocarbonyle, di-(C₁₋₃-alkyl)-aminocarbonyle, C₁₋₃-alkyl-amino-C₁₋₃-alkyle ou di-(C₁₋₃-alkyl)-amino-C₁₋₃-alkyle, un atome d'oxygène ou de soufre ou
un groupe imino, substitué le cas échéant par un groupe C₁₋₃-alkyle, acétyle, trifluoracétyle, C₁₋₃-alkoxy-carbonyle, aminocarbonyle, C₁₋₃-alkyl-aminocarbonyle, di-(C₁₋₃-alkyl)-aminocarbonyle, C₁₋₃-alkyl-amino-C₁₋₃-alkyle ou di-(C₁₋₃-alkyl)-amino-C₁₋₃-alkyle ou un atome d'oxygène ou de soufre et en plus un atome d'azote ou
un groupe imine, substitué le cas échéant par un groupe C₁₋₃-alkyle, acétyle, trifluoracétyle, C₁₋₃-alkoxy-carbonyle, aminocarbonyle, C₁₋₃-alkyl-aminocarbonyle, di-(C₁₋₃-alkyl)-aminocarbonyle, C₁₋₃-alkyl-amino-C₁₋₃-alkyle ou di-(C₁₋₃-alkyl)-amino-C₁₋₃-alkyle, et deux atomes d'azote,
sachant que les groupes alkyle susmentionnés ou les groupes alkyle contenus dans les radicaux susmentionnés, si rien d'autre n'a été spécifié, contiennent 1 à 5 atomes de carbone et peuvent être ramifiés ou linéaires,
leurs tautomères, leurs diastéréo-isomères, leurs énantiomères, leurs mélanges et leurs sels.

2. Composés de la formule générale I selon la revendication 1, dans laquelle
R représente un hétérocycle aza, diaza, oxaza ou thiaza saturé ou simplement insaturé à 5 à 7 chaînons,
les hétérocycles susmentionnés étant liés par un atome d'azote et
contenant un groupe carbonyle lié à un ou deux atomes d'azote de l'hétérocycle, pouvant être substitués sur un des atomes d'azote par un groupe alkyle,
pouvant être substitués sur un des atomes de carbone par un groupe alkyle, phényle, pyridinyle, furyle, thiényle ou pyrrolyle,
et sachant qu'une double liaison d'un des hétérocycles insaturés susmentionnés peut être condensée avec un noyau benzénique, pyridinique ou thiophénique,
sachant que les groupes phényle, pyridinyle, furyle, thiényle, pyrrolyle contenus dans R ainsi que les benzo-, thiéno- et pyrido-hétérocycles condensés peuvent être mono- ou disubstitués en plus sur le squelette de carbone par des atomes de fluor, de chlore, de brome ou d'iode, par des groupes alkyle, alkoxyle, nitro, alkylthiol, trifluorométhyle, difluorométhyle, alkoxycarbonyle, carboxyle, dialkylamino, hydroxyle, amino, acétylamino, aminocarbonyle, alkylaminocarbonyle, alkanoyle, cyano ou trifluorométhoxyle, sachant que les substituants peuvent être identiques ou différents,
X représente un atome d'oxygène ou, lorsque Z représente le groupe -NR¹-, aussi le groupe =N-CN,
Z représente un groupe -CH₂- ou -NR¹-, dans lequel
R¹ représente un atome d'hydrogène ou un groupe alkyle,
A représente un atome de carbone substitué par un atome d'hydrogène ou par un groupe C₁₋₃-alkyle
n représente le nombre 1 ou 2,
R² représente le groupe 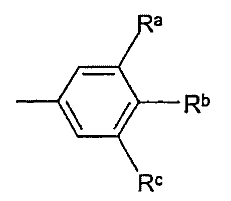 dans lequel
un des groupes R^{a}, R^{b} et R^{c} représente un atome d'hydrogène, de fluor, de chlore, de brome ou d'iode, un groupe alkyle ramifié ou linéaire, un groupe hydroxyle, alkoxyle, trifluorométhyle, difluorométhyle, trifluorométhoxyle, amino, acétylamino, dialkylaminoalkyle, dialkylaminoalkoxyle, aminocarbonyle, alkylaminocarbonyle, dialkylaminocarbonyle, alkanoyle, cyano ou trifluorométhylsulfonyle,
un deuxième des groupes R^{a}, R^{b} et R^{c} représente un atome d'hydrogène, de fluor, de chlore, de brome ou d'iode, un groupe alkyle ramifié ou linéaire, un groupe alkoxyle, trifluorométhyle, amino, acétylamino ou alkanoyle et
le troisième des groupes R^{a}, R^{b} et R^{c} représente un atome d'hydrogène, de fluor, de chlore, de brome ou d'iode, un groupe alkyle ramifié ou linéaire, sachant que les substituants peuvent être identiques ou différents,
R³ représente un atome d'hydrogène ou un groupe C₁₋₃-alkyle,
R⁴ représente un atome d'hydrogène, un groupe C₁₋₄-alkyle, amino-C₁₋₃-alkyl-, C ₁₋₃-alkyl-amino-C₁₋₃-alkyle ou di-(C ₁₋₃-alkyl)-amino-C₁₋₃-alkyle,
un groupe phényle, qui peut être mono- ou disubstitué par R^{d}, sachant que les substituants peuvent être identiques ou différents et que R^{d} représente un atome de fluor, de chlore, de brome ou d'iode, un groupe C₁₋₃-alkyle, trifluorométhyle, difluorométhyle, cyclopropyle, hydroxyle, C₁₋₃-alkoxyle, difluorométhoxyle, trifluorométhoxyle, amino, C₁₋₃-alkyl-amino, di-(C₁₋₃-alkyl)-amino, carboxyle-, C₁₋₃-alkoxy-carbonyle, aminocarbonyle, C₁₋₃-alkyl-amino-carbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle, ou
un groupe pyridinyle, pyrazinyle, pyrimidinyle, pyridazinyle, pyrrolyle, furyle, thiényle, pyrazolyle, imidazolyle, oxazolyle, isoxazolyle, thiazolyle ou isothiazolyle, substitué le cas échéant sur le squelette de carbone par un groupe C₁₋₃-alkyle, dans lesquels un atome d'hydrogène lié à un atome d'azote peut être remplacé par un groupe C₁₋₃-alkyle ou acétyle,
ou R³ et R⁴ représentent ensemble un groupe 1,3-butadién-1,4-ylène, dans lequel
un atome d'hydrogène peut être remplacé par un atome de fluor, de chlore, de brome ou d'iode, par un groupe C₁₋₃-alkyle, trifluorométhyle, difluorométhyle, cyclopropyle, hydroxyle, C₁₋₃-alkoxyle, amino, C₁₋₃-alkyl-amino, di-(C₁₋₃-alkyl)-amino, carboxyle, C₁₋₃-alkoxy-carbonyle, aminocarbonyle, C₁₋₃-alkyl-aminocarbonyle, di-(C₁₋₃-alkyl}-aminocarbonyle, di-(C₁₋₃-alkyl)-amino-C₂₋₃-alkyl-aminocarbonyle, amino-C₁₋₃-alkyle, C₁₋₃-alkyl-amino-C₁₋₃-alkyle, di-(C₁₋₃-alkyl)-amino-C₁₋₃-alkyle ou par un groupe cycloalkylène-imino-carbonyle à 5 à 7 chaînons, dans lequel
un ou deux atomes d'hydrogène peuvent être remplacés chacun par un groupe C₁₋₃-alkyle ou/et
le groupe méthylène en position 4 d'un fragment cycloalkylèneimine à 6 ou 7 chaînons peut être remplacé par un atome d'oxygène ou de soufre, par un groupe -NH-, -N(C₁₋₃-alkyl)- ou -N(C₁₋₃-alkyl-carbonyl),
et le cas échéant en plus un deuxième atome d'hydrogène peut être remplacé par un atome de fluor, de chlore, de brome ou d'iode, par un groupe C₁₋₃-alkyle, trifluorométhyle, et
R⁵ représente un groupe -(CH₂)ₘ-R^{e}, dans lequel
m représente le nombre 0 et
R^{e} représente un atome d'hydrogène ou un groupe C₅₋₆-cycloalkyle, dans lequel
le groupe méthylène en position 3 du groupe cyclopentyle et le groupe méthylène en position 4 du groupe cyclohexyle peut être remplacé par un groupe -NH-, -N(C₁₋₃-alkyl)- ou -N(C₁₋₃-alkyl-carbonyl),
ou m représente un des nombres 1 à 5 et
R^{e} représente un atome d'hydrogène, un groupe aminométhylène, C₁₋₃-alkylaminométhylène, di-(C₁₋₃-alkyl)-aminométhylène, aminocarbonyle, C₁₋₃-alkylaminocarbonyle, di-(C₁₋₃-alkyl)-aminocarbonyle ou un groupe C₅₋₆-cycloalkyle dans lequel
le groupe méthylène en position 3 du groupe cyclopentyle et le groupe méthylène en position 4 du groupe cyclohexyle peut être remplacé par un groupe -NH-, -N(C₁₋₃-alkyl)- ou -N(C₁₋₃-alkyl-carbonyl)-,
un groupe cycloalkylèneimine à 5 à 6 chaînons, dans lequel
le groupe méthylène en position 4 d'un groupe cycloalkylèneimine à 6 chaînons peut être remplacé par un groupe -NH-, -N(C₁₋₃-alkyl)- ou - N(C₁₋₃-alkyl-carbonyl)-,
un groupe phényle, qui peut être mono- ou disubstitué par R^{g}, sachant que les substituants peuvent être identiques ou différents et R^{g} représente un atome de fluor, de chlore, de brome ou d'iode, un groupe C₁₋₃-alkyle, trifluorométhyle, difluorométhyle, hydroxyle, C₁₋₃-alkoxyle, amino, C₁₋₃-alkyl-amino, di-(C₁₋₃-alkyl)-amino, aminocarbonyl-C₁₋₃-alkyl-aminocarbonyle, amino-C₁₋₃ -alkyle C₁₋₃-alkyl-amino-C₁₋₃-alkyle ou di-(C₁₋₃-alkyl)-amino-C₁₋₃-alkyle,
ou un groupe pyridinyle, pyrazinyle, pyrimidinyle, pyridazinyle, pyrrolyle, furyle, thiényle, pyrazolyle, imidazolyle, oxazolyle, isoxazolyle, thiazolyle ou isothiazolyle, dans lesquels un atome d'hydrogène lié à un atome d'azote peut être remplacé par un groupe C₁₋₃-alkyle ou acétyle, substitué le cas échéant sur le squelette de carbone par un groupe C₁₋₃-alkyle,
sachant que les groupes alkyle susmentionnés ou les groupes alkyle contenus dans les radicaux susmentionnés, si rien d'autre n'a été spécifié, contiennent 1 à 4 atomes de carbone et peuvent être ramifiés ou linéaires, leurs tautomères, leurs diastéréo-isomères, leurs énantiomères, leurs mélanges et leurs sels.

3. Composés de la formule générale I selon la revendication 2, dans lesquels
R représente un hétérocycle diaza simplement insaturé à 5 à 7 chaînons, lié par un atome d'azote,
sachant que les hétérocycles mentionnés ci-avant contiennent un groupe carbonyle attaché aux deux atomes d'azote de l'hétérocycle,
qu'ils peuvent être substitués sur un des atomes d'azote et un des atomes de carbone saturés, indépendamment l'un de l'autre par un groupe C₁₋₃-alkyle,
et sachant que la double liaison des hétérocycles insaturés mentionnés ci-avant est condensée avec un noyau benzénique substitué le cas échéant par un atome de fluor, de chlore, de brome ou d'iode, par un groupe C₁₋₃-alkyle, C₁₋₃-alkoxyle, trifluorométhyle, carboxyle, C₁₋₃-alkoxy-carbonyle, amino, acétylamino, hydroxyle, aminocarbonyle ou alkanoyle,
X représente un atome d'oxygène,
Z représente le groupe -NR¹-, dans lequel
R¹ représente atome d'hydrogène ou un groupe C₁₋₃-alkyle,
A représente un atome de carbone substitué par un atome d'hydrogène ou par un groupe C₁₋₃-alkyle,
n représente le nombre 1,
R² représente le groupe 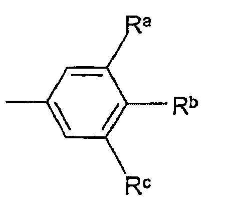 dans lequel
un des groupes R^{a}, R^{b} et R^{c} représente un atome d'hydrogène, de fluor, de chlore, de brome ou d'iode, un groupe C₁₋₃-alkyle, trifluorométhyle, hydroxyle, alkoxyle ou amino,
un deuxième des groupes R^{a}, R^{b} et R^{c} représente un atome d'hydrogène, de fluor, de chlore, de brome ou d'iode, un groupe C₁₋₃-alkyle ou trifluorométhyle et
le troisième des groupes R^{a}, R^{b} et R^{c} représente un atome d'hydrogène, de fluor, de chlore, de brome ou d'iode, sachant que les substituants peuvent être identiques ou différents,
R³ représente un atome d'hydrogène ou un groupe C₁₋₃-alkyle,
R⁴ représente un groupe phényle substitué le cas échéant par un atome de fluor, de chlore, de brome ou d'iode, par un groupe C₁₋₃-alkyle, trifluorométhyle, hydroxyle, C₁₋₃-alkoxyle, amino, C₁₋₃-alkylamino, di-(C₁₋₃-alkyl)-amino, carboxyle ou C₁₋₃-alkoxy-carbonyle,
ou R³ et R⁴ représentent ensemble un groupe 1,3-butadién-1,4-ylène, dans lequel
un atome d'hydrogène peut être remplacé par un atome de fluor, de chlore, de brome ou d'iode, par un groupe C₁₋₃-alkyle, trifluorométhyle, hydroxyle, C₁₋₃-alkoxyle, carboxyle, C₁₋₃-alkoxy-carbonyle, aminocarbonyle, C₁₋₃-alkyl-aminocarbonyle, di-(C₁₋₃-alkyl)-amino-carbonyle ou di-(C₁₋₃-alkyl)-amino-C₂₋₃-alkyl-aminocarbonyle ou par un groupe cycloalkylène-imino-carbonyle 5 à 7 chaînons, sachant que
le groupe méthylène en position 4 du fragment cycloalkylène-imino à 6 chaînons peut être remplacé par un groupe -NH-, -N(C₁₋₃-alkyl)- ou - N(C₁₋₃-alkyl-carbonyl)-,
et le cas échéant en plus un deuxième atome d'hydrogène peut être remplacé par un atome de fluor, de chlore, de brome ou d'iode ou par un groupe C₁₋₃-alkyle, et
R⁵ représente le groupe (CH₂)ₘ-R^{e}, dans lequel
m représente le nombre 0 et
R^{e} représente un atome d'hydrogène,
ou m représente un des nombre 1 à 3 et
R^{e} représente un atome d'hydrogène, un groupe aminométhylène, C₁₋₃-alkylaminométhylène ou di-(C₁₋₃-alkyl)-aminométhylène,
sachant que, si rien d'autre n'a été spécifié, les groupes alkyle susmentionnés ou les groupes alkyle contenus dans les radicaux susmentionnés peuvent être ramifiés ou linéaires,
leurs tautomères, leurs diastéréo-isomères, leurs énantiomères, leurs mélanges et leurs sels.

4. Composés de la formule générale I selon la revendication 1, dans laquelle
R, X, Z, A, n, R², et R⁵ sont définis comme mentionnés dans la revendication 1 et
R³ représente un atome d'hydrogène ou un groupe C₁₋₄-alkyle et
R⁴ représente un atome d'hydrogène, un groupe C₁₋₄-alkyle, amino-C₁₋₃-alkyle, C₁₋₃-alkyl-amino-C₁₋₃-alkyle ou di-(C₁₋₃-alkyl)-amino-C₁₋₃-alkyle,
un groupe phényle ou naphthyle, qui peuvent être mono- ou disubstitués indépendamment l'un de l'autre par R^{d}, sachant que les substituants peuvent être identiques ou différents et que R^{d} représente un atome de fluor, de chlore, de brome
ou d'iode, un groupe C₁₋₃-alkyle, trifluorométhyle, difluorométhyle, cyclopropyle, hydroxyle, C₁₋₃-alkoxyle, difluorométhoxyle, trifluorométhoxyle, cyano, nitro, amino, C₁₋₃-alkyl-amino, di-(C₁₋₃-alkyl)-amino, carboxyle, C₁₋₃-alkoxy-carbonyle, aminocarbonyle, C₁₋₃-alkyl-aminocarbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle, ou
un groupe hétéroaryle monocyclique à 5 ou 6 chaînons substitué le cas échéant sur le squelette de carbone par un groupe C₁₋₃-alkyle, amino, C₁₋₃-alkylamino, di-(C₁₋₃-alkyl)-amino, carboxyle, C₁₋₃-alkoxy-carbonyle, aminocarbonyle, C₁₋₃-alkyl-aminocarbonyle, di-(C₁₋₃-alkyl)-aminocarbonyle, amino-C₁₋₃-alkyle, C₁₋₃-alkylamino-C₁₋₃-alkyle ou di-(C₁₋₃-alkyl)-amino-C₁₋₃-alkyle, dans lequel
le groupe hétéroaryle à 6 chaînons contient un, deux ou trois atomes d'azote et
le groupe hétéroaryle à 5 chaînons contient un groupe imine substitué le cas échéant par un groupe C₁₋₃-alkyle, acétyle, trifluoracétyle, C₁₋₃-alkoxycarbonyle, aminocarbonyle, C₁₋₃ alkyl-aminocarbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle, un atome d'oxygène ou de soufre ou
un groupe imine substitué le cas échéant par un groupe C₁₋₃-alkyle, acétyle, trifluoracétyle, C₁₋₃-alkoxycarbonyle, aminocarbonyle, C₁₋₃-alkyl-aminocarbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle ou un atome d'oxygène ou de soufre et en plus par un atome d'azote ou
un groupe imine substitué le cas échéant par un groupe C₁₋₃-alkyle, acétyle, trifluoracétyle, C₁₋₃-alkoxycarbonyle, aminocarbonyle, C₁₋₃-alkyl-aminocarbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle et deux atomes d'azote,
leurs tautomères, leurs diastéréo-isomères, leurs énantiomères, leurs mélanges et leurs sels.

5. Composés de la formule générale I selon la revendication 2, dans lesquels
R, X, Z, A, n, R², et R⁵ sont définis comme dans la revendication 2 et
R³ représente un atome d'hydrogène ou un groupe C₁₋₃-alkyle et
R⁴ représente un groupe phényle, qui peut être mono- ou disubstitués par R^{d} sachant que les substituants peuvent être identiques ou différents et que R^{d} représente un atome de fluor, de chlore, de brome ou d'iode, un groupe C₁₋₃-alkyle, trifluorométhyle, difluorométhyle, cyclopropyle, hydroxyle, C₁₋₃-alkoxyle, difluorométhoxyle, trifluorométhoxyle, amino, C₁₋₃-alkyl-amino, di-(C₁₋₃-alkyl)-amino, carboxyle, C₁₋₃-alkoxy-carbonyle, aminocarbonyle, C₁₋₃-alkyl-amino-carbonyle, di-(C₁₋₃-alkyl)-aminocarbonyle, amino-C₁₋₃-alkyle, C₁₋₃-alkyl-amino-C₁₋₃-alkyle ou di-(C₁₋₃-alkyl)-amino-C₁₋₃-alkyle, ou
un groupe pyridinyle, pyrazinyle, pyrimidinyle, pyridazinyle, pyrrolyle, furyle, thiényle, pyrazolyle, imidazolyle, oxazolyle, isoxazolyle, thiazolyle ou isothiazolyle, substitué le cas échéant sur le squelette de carbone par un groupe C₁₋₃-alkyle, dans lesquels un atome d'hydrogène lié à un atome d'azote peut être remplacé par un groupe C₁₋₃-alkyle ou acétyle, leurs tautomères, leurs diastéréo-isomères, leurs énantiomères, leurs mélanges et leurs sels.

6. Composés de la formule générale I selon la revendication 3, dans lesquels
R, X, Z, A, n, R², et R⁵ sont définis comme mentionné dans la revendication 3 et
R³ représente un atome d'hydrogène ou un groupe C₁₋₃-alkyle et
R⁴ représente un groupe phényle substitué éventuellement par un atome de fluor, de chlore, de brome ou d'iode, par un groupe C₁₋₃-alkyle, trifluorométhyle, hydroxyle,
C₁₋₃-alkoxyle, amino, C₁₋₃-alkylamino, di-(C₁₋₃-alkyl)-amino, carboxyle ou C₁₋₃-alkoxy-carbonyle,
leurs tautomères, leurs diastéréo-isomères, leurs énantiomères, leurs mélanges et leurs sels.

7. Composés de la formule générale I selon la revendication 1, dans lesquels
R, X, Z, A, n, R², et R⁵ sont définis comme dans la revendication 1 et
R³ et R⁴ représentent ensemble un groupe 1,3-butadién-1,4-ylène, dans lequel
un, deux ou trois groupes méthine peuvent être remplacés chacun par un atome d'azote,
un atome d'hydrogène peut être remplacé par un atome de fluor, chlore, de brome ou d'iode, par un groupe C₁₋₃-alkyle, trifluorométhyle, difluorométhyle, cyclopropyle, hydroxyle, C₁₋₃-alkoxyle, difluorométhoxyle, trifluorométhoxyle, cyano, nitro, amino, C₁₋₃-alkyl-amino, di-(C₁₋₃-alkyl)-amino, carboxyle, C₁₋₃-alkoxy-carbonyle, aminocarbonyle, C-₁₋₃-alkyl-aminocarbonyle, di-(C₁₋₃-alkyl)-aminocarbonyle, di-(C₁₋₃-alkyl)-amino-C₁₋₃- alkyl-aminocarbonyle, amino-C₁₋₃-alkyle, C₁₋₃-alkyl-amino-C₁₋₃-alkyle ou di-(C₁₋₃-alkyl)-amino-C₁₋₃-alkyle ou par un groupe cycloalkylène-imino-carbonyle à 4 à 7 chaînons, dans lequel
un ou deux atomes d'hydrogène peuvent être remplacés chacun par un groupe C₁₋₃-alkyle ou/et
le groupe méthylène en position 4 d'un fragment cycloalkylèneimine à 6 ou 7 chaînons peut être remplacé par un atome d'oxygène ou de soufre, par un groupe sulfinyle, sulfonyle, -NH-, -N(C₁₋₃-alkyl)- ou -N(C₁₋₃-alkyl-carbonyl)-,
et le cas échéant en plus un deuxième atome d'hydrogène peut être remplacé par un atome de fluor, de chlore, de brome ou d'iode, par un groupe C₁₋₃-alkyle, trifluorométhyle, difluorométhyle, cyclopropyle, hydroxyle, C₁₋₃-alkoxyle, difluorométhoxyle ou trifluorométhoxyle,
leurs tautomères, leurs diastéréo-isomères, leurs énantiomères, leurs mélanges et leurs sels.

8. Composés de la formule générale I selon la revendication 3, dans lesquels
R, X, 2, A, n, R², et R⁵ sont définis comme mentionné dans la revendication 2 et
R³ et R⁴ représentent ensemble un groupe 1,3-butadién-1,4-ylène, dans lequel
un atome d'hydrogène peut être remplacé par un atome de fluor, de chlore, de brome ou d'iode, par un groupe C₁₋₃-alkyle, trifluorométhyle, difluorométhyle, cyclopropyle, hydroxyle, C₁₋₃-alkoxyle, amino, C₁₋₃-alkyl-amino, di-(C₁₋₃-alkyl)-amino, carboxyle, C₁₋₃-alkoxy-carbonyle, aminocarbonyle, C₁₋₃-alkyl-aminocarbonyle, di-(C₁₋₃-alkyl)-aminocarbonyle ou di-(C₁₋₃-alkyl)-amino-C₂₋₃-alkyl-aminocarbonyle ou par un groupe cycloalkylène-imino-carbonyle à 5 à 7 chaînons, dans lequel
un ou deux atomes d'hydrogène peuvent être remplacés chacun par un groupe C₁₋₃-alkyle ou/et
le groupe méthylène en position 4 d'un fragment cycloalkylèneimine à 6 ou 7 chaînons peut être remplacé par un atome d'oxygène ou de soufre, par un groupe -NH-, N(C₁₋₃-alkyl)- ou -N(C₁₋₃-alkyl-carbonyl)-,
et le cas échéant en plus un deuxième atome d'hydrogène peut être remplacé par un atome de fluor, de chlore, de brome ou d'iode, par un groupe C₁₋₃-alkyle ou trifluorométhyle,
leurs tautomères, leurs diastéréo-isomères, leurs énantiomères, leurs mélanges et leurs sels.

9. Composés de la formule générale 1 selon la revendication 3, dans lesquels
R, X, Z, A, n, R², et R⁵ sont définis comme mentionné dans la revendication 3 et
R³ et R⁴ représentent ensemble un groupe 1,3-butadién-1,4-ylène, dans lequel
un atome d'hydrogène peut être remplacé par un atome de fluor, de chlore, de brome ou d'iode, par un groupe C₁₋₃-alkyle, trifluorométhyle, hydroxyle, C₁₋₃-alkoxyle, carboxyle, C₁₋₃-alkoxycarbonyle, aminocarbonyle, C₁₋₃-alkyl-aminocarbonyle, di-(C₁₋₃-alkyl)-aminocarbonyle ou di-(C₁₋₃-alkyl)-amino-C₂₋₃-alkyl-aminocarbonyle ou par un groupe cycloalkylène-imino-carbonyle à 5 à 7 chaînons, sachant que
le groupe méthylène en position 4 du fragment cycloalkylèneimine à 6 chaînons peut être remplacé par un groupe -NH-, -N(C₁₋₃-alkyl)- ou - N(C ₁₋₃-alkyl-carbonyl)-,
et le cas échéant en plus un deuxième atome d'hydrogène peut être remplacé par un atome de fluor, de chlore, de brome ou d'iode, ou par un groupe C₁₋₃-alkyle,
leurs tautomères, leurs diastéréo-isomères, leurs énantiomères, leurs mélanges et leurs sels.

10. Composés suivants de la formule générale I selon la revendication 1
(1) 4-(2-oxo-1,2,4,5-tétrahydro-1,3-benzodiazépin-3-yl)-pipéridin-1-carboxy-{(R)-2-(4-amino-3,5-dibromo-phényl)-1-[1-(3-diméthylamino-propyl)-1H-benzimidazol-2-yl)-éthyl} -amide,
(2) 4-(2-oxo-1,2,4,5-tétrahydro-1,3-benzodiazépin-3-yl)-pipéridin-1 -carboxy-{(R)-2-(4-amino-3, 5-dibromo-phényl)-1-[1-(2-diméthylamino-éthyl)-1H-benzimidazol-2-yl]-éthyl)-amide,
(3) 4-(2-oxo-1,2,4,5-tétrahydro-1,3-benzodiazépin-3-yl)-pipéridin-1-carboxy-[(R)-2-(4-amino-3,5-dibromo-phényl)-1-(1-méthyl-1H-benzimidazol-2-yl)-éthyl]-amide,
(4) 4-(2-oxo-1,2,4,5-tétrahydro-1,3-benzodiazépin-3-yl)-pipéridin-1-carboxy-[(R)-2-(4-amino-3,5-dibromo-phényl)-1-(1 H-benzimidazol-2-yl)-éthyl]-amide,
(5) 2-((R)-2-(4-amino-3,5-dibromo-phényl)-1-{[4-(2-oxo-1,4-dihydro-3H-quinazolin-3-yl)-pipéridin-1-carbonyl]-amino}-éthyl)-1H-benzimidazol-5-carboxylate de méthyle,
(6) 2-((R)-2-(4-amino-3,5-dibromo-phényl)-1-{[4-(2-oxo-1,2,4,5-tétrahydro-1,3-benzodiazépin-3-yl)-pipéridin-1-carbonyl]-amino}-éthyl)-1H-benzimidazol-5-carboxylate de méthyle,
(7) acide 2-((R)-2-(4-amino-3,5-dibromo-phényl)-1-{[4-(2-oxo-1,2,4,5-tétrahydro- 1,3-benzodiazépin-3-yl)-pipéridin-1-carbonyl]-amino}-éthyl)-1H-benzimidazol-5-carboxylique,
(8) 4-(2-oxo-1,2,4,5-tétrahydro-1,3 -benzodiazépin-3-yl)-pipéridin- 1 -carboxy-{(R)-2-(4-amino-3,5-dibromo-phényl)-1-[6-(4-méthyl-pipérazin-1-carbonyl)-1H-benzimidazol-2-yl]-éthyl} -amide,
(9) 2-((R)-2-(4-amino-3,5-dibromo-phényl)-1 - {[4-(2-oxo-1,2,4,5-tétrahydro-1,3-benzodiazépin-3-yl)-pipéridin-1-carbonyl]-amino} -éthyl)-1H-benzimidazol-5-carboxy-(3-diméthylamino-propyl)-amide,
(10) 2-((R)-2-(4-amino-3,5-dibromo-phényl)-1 - {[4-(2-oxo-1,2,4,5-tétrahydro-1,3-benzodiazépin-3-yl)-pipéridin-1-carbonyl]-amino}-éthyl)-1 H-benzimidazol-5-carboxy-(2-diméthylamino-éthyl)-amide,
(11) 4-(2-oxo-1,4-dihydro-2H-quinazolin-3-yl)-pipéridin-1-carboxy-{(R)-2-(4-amino-3,5-dibromo-phényl)-1-[1-(3-diméthylamino-propyl)-5-phényl-1H-imidazol-2-yl]-éthyl}-amide,
(12) 4-(2-oxo-1,2,4,5-tétrahydro-1,3-benzodiazépin-3-yl)-pipéridin-1-carboxy-[(R)-2-(4-amino-3,5-dibromo-phényl)-1-(1-butyl-1H-benzimidazol-2-yl)-éthyl)-amide,
(13) 4-(2-oxo-1,2,4,5-tetrahydro-1,3-benzodiazépin-3-yl)-pipéridin-1-carboxy-{(R)-2-(4-amino-3,5-dibromo-phényl)-1-[1-(3-pyrrolidin-1-yl-propyl)-1H-benzimidazol-2-yl]-éthyl}-amide,
(14) 4-(2-oxo-1,2,4,5-tétrahydro-1,3-benzodiazépin-3-yl)-pipéridin-1-carboxy-[(R)-2-(4-amino-3,5-dibromo-phényl)-1-(1-pyridin-3-ylméthyl-1 H-benzimidazol-2-yl)-éthyl]-amide,
(15) 4-(2-oxo-1,2,4,5-tétrahydro-1,3-benzodiazépin-3-yl)-pipéridin-1-carboxy-[(R)-2-(4-amino-3,5-dibromo-phényl)-1-(1-benzyl-1H-benzimidazol-2-yl)-éthyl]-amide,
(16) 4-(2-oxo-1,2,4,5-tétrahydro-1,3-benzodiazépin-3-yl)-pipéridin-1-carboxy-{(R)-2-(4-amino-3,5-dibromo-phényl)-1-[1-(1-méthyl-pipéridin-4-ylméthyl)-1H-benzimidazol-2-yl]-éthyl}-amide,
(17) 4-(2-oxo-1,2,4,5-tétrahydro-1,3-benzodiazépin-3-yl)-pipéridin-1-carboxy-{(R)-2-(4-amino-3,5-dibromo-phényl)-1-[1-(4-diméthylamino-butyl)-1H-benzimidazol-2-yl]-éthyl}-amide,
(18) 4-(2-oxo-1,2,4,5-tétrahydro-1,3-benzodiazépin-3-yl)-pipéridin-1-carboxy-{(R)-2-(4-amino-3,5-dibromo-phényl)-1-[1-(1-méthyl-pipéridin-4-yl)-1H-benzimidazol-2-yl]-éthyl}-amide,
(19) 4-(2-oxo-1,2,4,5-tétrahydro-1,3-benzodiazépin-3-yl)-pipéridin-1-carboxy-[(R)-2-(4-amino-3,5-dibromo-phényl)-1-(1-cyclohexyl-1 H-benzimidazol-2-yl)-éthyl]-amide,
(20) 4-(2-oxo-1,2,4,5-tétrahydro-1,3-benzodiazépin-3-yl)-pipéridin-1-carboxy-[(R)-2-(4-amino-3,5-dibromo-phényl)-1-(1-cyclopentyl-1H-benzimidazol-2-yl)-éthyl]-amide,
(21) 4-(2-oxo-1,2,4,5-tétrahydro-1,3-benzodiazépin-3-yl)-pipéridin-1-carboxy-{(R)-2-(4-amino-3,5-dibromo-phényl)-1-[1-(5-diméthylamino-pentyl)-1H-benzimidazol-2-yl]-éthyl)-amide,
(22) 4-[2-((R)-2-(4-amino-3,5-dibromo-phényl)-1-{[4-(2-oxo-1,2,4,5-tétrahydro-1,3-benzodiazépin-3-yl)-pipéridin-1-carbonyl]-amino }-éthyl)-benzimidazol-1-yl]-butanoate de méthyle,
(23) 4-(2-oxo-1,2,4,5-tétrahydro-1,3-benzodiazépin-3-yl)-pipéridin-1-carboxy-{(R)-2-(4-amino-3,5-dibromo-phényl)-1-[6-chloro-1-(3-diméthylamino-propyl)- 1H-benzimidazol-2-yl]-éthyl}-amide,
(24) 4-(2-oxo-1,2,4,5-tétrahydro-1,3-benzodiazépin-3-yl)-pipéridin-1-carboxy-{(R)-2-(4-amino-3,5-dibromo-phényl)-1-[1-(3-diméthylamino-propyl)-6-fluoro-1H-benzimidazol-2-yl]-éthyl} -amide,
(25) 4-(2-oxo-1,2,4,5-tétrahydro-1,3-benzodiazépin-3-yl)-pipéridin-1-carboxy-{(R)-2-(4-amino-3,5-dibromo-phényl)-1-[1-(3-diméthylamino-propyl)-5-fluoro-1H-benzimidazol-2-yl]-éthyl}-amide,
(26) 4-(2-oxo-1,2,4,5-tétrahydro-1,3-benzodiazépin-3-yl)-pipéridin-1-carboxy-{(R)-2-(4-amino-3,5-dibromo-phényl)-1-[5,6-dichloro-1-(3-diméthylamino-propyl)-1H-benzimidazol-2-yl)-éthyl)-amide,
(27) 2-((R)-2-(4-amino-3,5-dibromo-phényl)-1-{[4-(2-oxo-1,2,4,5-tétrahydro-1,3-benzodiazépin-3-yl)-pipéridin-1-carbonyl]-amino)-éthyl)-1-(3-pyrrolidin-1-yl-propyl)-1H-benzimidazol-5-carboxylate de méthyle,
(28) acide 2-(2-(4-amino-3-chloro-5-trifluorométhyl-phényl)-1-{[4-(2-oxo-1,2,4,5-tétrahydro-1, 3-benzodiazépin-3-yl)-pipéridin-1-carbonyl]-amino }-éthyl)-1-(3-pyrrolidin-1-yl-propyle)-1H-benzimidazoie-5 -carboxylique,
(29) 4-(2-oxo-1,2,4,5-tétrahydro-1,3-benzodiazépin-3-yl)-pipéridin-1-carboxy-{(R)-2-(3,5-dibromo-4-hydroxy-phényl)-1-[1-(1-méthyl-pipéridin-4-ylméthyl)-1 H-benzimidazol-2-yl]-éthyl}-amide,
(30) 4-(2-oxo-1,2,4,5-tétrahydro-1,3-benzodiazépin-3-yl)-pipéridin-1-carboxy-{(R)-2-(3,5-dibromo-4-hydroxy-phényl)-1-[1-(1-méthyl-pipéridin-4-yl)-1 H-benzimidazol-2-yl]-éthyl} -amide,
(31) 4-(2-oxo-1,2,4,5-tétrahydro-1,3-benzodiazépin-3-yl)-pipéridin-1-carboxy-{(R)-2-(3,5-dibromo-4-hydroxy-phényl)-1-[1-(3-diméthylamino-propyl)- 1H-benzimidazol-2-yl]-éthyl} -amide,
(32) 4-(2-oxo-1,2,4,5-tétrahydro-1,3-benzodiazépin-3-yl)-pipéridin-1-carboxy-{(R)-2-(3,5-dibromo-4-hydroxy-phényl)-1-[1-(3-pyrrolidin-1-yl-propyl)-1 H-benzimidazol-2-yl]-éthyl}-amide,
(33) 4-(2-oxo-1,2,4,5-tétrahydro-1,3-benzodiazépin-3-yl)-pipéridin-1-carboxy-[1-[6-chloro-1-(3-diméthylamino-propyl)-1 H-benzimidazol-2-yl]-2-(3,4-dibromophényl)-éthyl]-amide,
(34) 4-(2-oxo-1,2,4,5-tétrahydro-1,3-benzodiazépin-3-yl)-pipéridin-1-carboxy-{2-(3,4-dibromo-phényl)-1-[5,6-dichloro-1-(3-diméthylamino-propyl)-1H-benzimidazol-2-yl]-éthyl }-amide,
(35) 4-(2-oxo-1,2,4,5-tétrahydro-1,3-benzodiazépin-3-yl)-pipéridin-1-carboxy-[1-[7-chloro-1-(3-diméthylamino-propyl)-1H-benzimidazol-2-yl]-2-(3,4-dibromophényl)-éthyl]-amide,
(36) 4-(2-oxo-1,2,4,5-tétrahydro-1,3-benzodiazépin-3-yl)-pipéridin-1-carboxy-{2-(3,4-dibromo-phényl)-1-[1-(3-diméthylamino-propyl)-6-fluoro-1H-benzimidazol-2-yl]-éthyl}-amide,
(37) 4-(2-oxo-1,2,4,5-tétrahydro-1,3-benzodiazépin-3-yl)-pipéridin-1-carboxy-{2-(3,4-dibromo-phényl)-1-[1-(1-méthyl-pipéridin-4-yl)-1H-benzimidazol-2-yl]-éthyl}-amide,
(38) 4-(2-oxo-1,2,4,5-tétrahydro-1,3-benzodiazépin-3-yl)-pipéridin-1-carboxy-{2-(3,4-dibromo-phényl)-1-[1-(3-pyrrolidin-1-yl-propyl)-1H-benzimidazol-2-yl]-éthyl}-amide,
(39) 4-(2-oxo-1,2,4,5-tétrahydro-1,3-benzodiazépin-3-yl)-pipéridin-1-carboxy-{2-(3,4-dibromo-phényl)-1-[1-(1-méthyl-pipéridin-4-ylméthyl)-1H-benzimidazol-2-yl]-éthyl}-amide,
(40) 4-(2-oxo-1,2,4,5-tétrahydro-1,3-benzodiazépin-3-yl)-pipéridin-1-carboxy-[2-(3,4-dibromo-phényl)-1-(1-pyridin-3-ylméthyl-1H-benzimidazol-2-yl)-éthyl]-amide,
(41) 4-(2-oxo-1,2,4,5-tétrahydro-1,3-benzodiazépin-3-yl)-pipéridin-1-carboxy-{2-(3,4-dibromo-phényl)-1-[1-(3-diméthylamino-propyl)-1H-benzimidazol-2-yl]-éthyl}-amide,
(42) 4-(2-oxo-1,2,4,5-tétrahydro-1,3-benzodiazépin-3-yl)-pipéridin-1-carboxy-{2-(3,4-dibromo-phényl)-1-[1-(4-diméthylamino-butyl)-1H-benzimidazol-2-yl]-éthyl}-amide,
(43) 4-(2-oxo-1,2,4,5-tétrahydro-1,3-benzodiazépin-3-yl)-pipéridin-1-carboxy-{2-(3,4-diéthyl-phényl)-1-[1-(3-diméthylamino-propyl)-1H-benzimidazol-2-yl]-éthyl}-amide,
(44) 4-(2-oxo-1,2,4,5-tétrahydro-1,3-benzodiazépin-3-yl)-pipéridin-1-carboxy-{2-(3,4diéthyl-phényl)-1-[1-(4-diméthylamino-butyl)-1H-benzimidazol-2-yl]-éthyl}-amide,
(45) 4-(2-oxo-1,2,4,5-tétrahydro-1,3-benzodiazépin-3-yl)-pipéridin-1-carboxy-{2-(3,4-diéthyl-phényl)-1-[1-(1-méthyl-pipéridin-4-yl)-1H-benzimidazol-2-yl)-éthyl}-amide,
(46) 4-(2-oxo-1,2,4,5-tétrahydro-1,3-benzodiazépin-3-yl)-pipéridin-1-carboxy- {2-(3,4-diéthyl-phényl)-1-[1-(3-pyrrolidin-1-yl-propyl)-1H-benzimidazol-2-yl]-éthyl}-amide,
(47) 4-(2-oxo-1,2,4,5-tétrahydro-1,3-benzodiazépin-3-yl)-pipéridin-1-carboxy-{2-(3,4-diéthyl-phényl)-1-[1-(1-méthyl-pipéridin-4-ylméthyl)-1H-benzimidazol-2-yl]-éthyl)-amide,
(48) 4-(2-oxo-1,2,4,5-tétrahydro-1,3-benzodiazépin-3-yl)-pipéridin-1-carboxy-[2-(3,4-diéthyl-phényl)-1-(1-pyridin-3-ylméthyl- 1H-benzimidazol-2-yl)-éthyl]-amide,
(49) 4-(2-oxo-1,2,4,5-tétrahydro-1,3-benzodiazépin-3-yl)-pipéridin-1-carboxy-{2-(3,5-bis-trifluorométhyl-phényl)-1-[1-(1-méthyl-piperidin-4-yl)-1H-benzimidazol-2-yl]-éthyl}-amide,
(50) 4-(2-oxo-1,2,4,5-tétrahydro-1,3-benzodiazépin-3-yl)-pipéridin-1-carboxy-{2-(3,5-bis-trifluorométhyl-phényl)-1-[1-(3-pyrrolidin-1-yl-propyl)-1H-benzimidazol-2-yl]-éthyl}-amide,
(51) 4-(2-oxo-1,2,4,5-tétrahydro-1,3-benzodiazépin-3-yl)-pipéridin-1-carboxy-{2-(3,5-bis-trifluorométhyl-phényl)-1-[1-(1-méthyl-piperidin-4-ylméthyl)-1H-benzimidazol-2-yl]-éthyl}-amide,
(52) 4-(2-oxo-1,2,4,5-tétrahydro-1,3-benzodiazépin-3-yl)-pipéridin-1-carboxy-{2-(3,5-bis-trifluorométhyl-phényl)-1-[1-(3-diméthylamino-propyl)-1H-benzimidazol-2-yl)-éthyl }-amide,
(53) 2-(2-(4-amino-3-chloro-5-trifluorométhyl-phényl)-1-{[4-(2-oxo-1,2,4,5 - tétrahydro-1,3-benzodiazépin-3-yl)-pipéridin-1-carbonyl]-amino}-éthyl)-1-(3-pyrrolidin-1-yl-propyl)-1 H-benzimidazole-5-carboxylate de méthyle,
(54) acide 2-(2-(4-amino-3-chloro-5-trifluorométhyl-phényl)-1-{[4-(2-oxo-1,2,4,5-tétrahydro-1,3-benzodiazépin-3-yl)-pipéridin-1-carbonyl]-amino}-éthyl)-1- (3-pyrrolidin-1-yl-propyl)-1 H-benzimidazole-5-carboxylique,
(55) 4-(2-oxo-1,2,4,5-tétrahydro-1,3-benzodiazépin-3-yl)-pipéridin-1-carboxy-{2-(4-amino-3-chloro-5-trifluorométhyl-phényl)-1-[1-(1-méthyl-pipéridin-4-yl)-1H-benzimidazol-2-yl]-éthyl -amide,
(56) 4-(2-oxo-1,2,4,5-tétrahydro-1,3-benzodiazépin-3-yl)-pipéridin-1-carboxy-{2-(4-amino-3-chloro-5-trifluorométhyl-phényl)-1-[1-(3-pyrrolidin-1-yl-propyl)-1H-benzimidazol-2-yl]-éthyl)-amide,
(57) 4-(2-oxo-1,2,4,5-tétrahydro-1,3-benzodiazépin-3-yl)-pipéridin-1-carboxy-{2-(4-amino-3-chloro-5-trifluorométhyl-phényl)-1-[1-(1-méthyl-pipéridin-4-ylméthyl)-1 H-benzimidazol-2-yl]-éthyl}-amide,
(58) 4-(2-oxo-1,2,4,5-tétrahydro-1,3-benzodiazépin-3-yl)-pipéridin-1-carboxy-[2-(4-amino-3-chloro-5-trifluorométhyl-phényl)-1-(1-pyridin-3-ylméthyl-1 H-benzimidazol-2-yl)-éthyl]-amide,
(59) 4-(2-oxo-1,2,4,5-tétrahydro-1,3-benzodiazépin-3-yl)-pipéridin-1-carboxy-{2-(4-amino-3-chloro-5-trifluorométhyl-phényl)-1-[1-(3-diméthylamino-propyl)- 1H-benzimidazol-2-yl]-éthyl}-amide,
(60) 3-(1-{4-(3,4-diéthyl-phényl)-3-(1-(3-diméthylamino-propyl)-1H-benzimidazol-2-yl]-butyryl}-pipéridin-4-yl)-1,3,4,5-tétrahydro-1,3-benzodiazépin-2-one,
(61) 3-(1-{4-(4-amino-3-chloro-5-trifluorométhyl-phényl)-3-[1-(3-pyrrolidin-1-yl-propyl)-1H-benzimidazol-2-yl]-butyryl)-pipéridin-4-yl)-1,3,4,5-tétrahydro-1,3-benzodiazépin-2-one,
(62) 3-(1-{4-(4-amino-3-chloro-5-trifluorométhyl-phényl)-3-[1-(3-imidazol-1-yl-propyl)-1H-benzimidazol-2-yl]-butyryl}-pipéridin-4-yl)-1,3,4,5-tétrahydro-1,3-benzodiazépin-2-one,
(63) 3-(1-(4-(4-amino-3-chloro-5-trifluorométhyl-phényl)-3-[5-(3-diméthylamino-propyl)-1-éthyl-1H-benzimidazol-2-yl]-butyryl}-piperidin-4-yl)-1,3,4,5-tétrahydro-1,3-benzodiazépin-2-one,
(64) 3-(1-{4-(4-amino-3-chloro-5-trifluorométhyl-phényl)-3-[1-(3-diéthylamino-propyl)-1H-benzimidazol-2-yl]-butyryl }-piperidin-4-yl)-1,3,4,5-tétrahydro-1,3-benzodiazépin-2-one,
(65) 3-(1-{4-(4-amino-3-chloro-5-trifluorométhyl-phényl)-3-[1-(4-hydroxy-butyl)-1H-benzimidazol-2-yl)-butyryl)-pipéridin-4-yl)-1,3,4,5-tétrahydro-1,3-benzodiazépin-2-one,
(66) 3-(1-{4-(-4-amino-3-chloro-5-trifluorométhyl-phényl)-3-(1-(1-méthyl pipéridin-3-ylméthyl)-1H-benzimidazol-2-yl]-butyryl }-pipéridin-4-yl)-1,3,4,5-tétrahydro-1,3-benzodiazépin-2-one,
(67) 3-[1-(4-(4-amino-3-chloro-5-trifluorométhyl-phényl)-3-{1-[2-(4-méthyl-pipérazin-1-yl)-éthyl]-1H-benzimidazol-2-yl}-butyryl)-pipéridin-4-yl]-1,3,4,5-tétrahydro-1,3-benzodiazépin-2-one,
(68) 3-{1-[4-(4-amino-3-chloro-5-trifluorométhyl-phényl)-3-(1-pyridin-4-ylméthyl-1H-benzimidazol-2-yl)-butyryl]-pipéridin-4-yl)-1,3,4, 5-tétrahydro-1,3-benzodiazépin-2-one,
(69) 3- {1-[4-(4-amino-3-chloro-5-trifluorométhyl-phényl)-3-(1-pipéridin-4-ylméthyl-1H-benzimidazol-2-yl)-butyryl]-pipéridin-4-yl)-1,3,4,5-tétrahydro-1,3-benzodiazépin-2-one,
(70) 3-(1-{4-(4-amino-3-chloro-5-trifluorométhyl-phényl)-3-[1-(3-diméthylamino-2,2-diméthyl-propyl)-1H-benzimidazol-2-yl]-butyryl}-pipéridin-4-yl)-1,3,4,5-tétrahydro-1,3-benzodiazépin-2-one,
(71) 3-(1-{4-(4-amino-3-chloro-5-trifluorométhyl-phényl)-3-[7-(3-diméthylamino-propoxy)-1H-benzimidazol-2-yl]-butyryl}-pipéridin-4-yl)-1,3,4,5-tétrahydro-1,3-benzodiazépin-2-one,
(72) 3-{1-[4-(4-amino-3-chloro-5-trifluorométhyl-phényl)-3-(1-pipéridin-4-ylméthyl-1 H-imidazo[4,5-c]pyridin-2-yl)-butyryl]-pipéridin-4-yl}-1,3,4,5-tétrahydro-1,3-benzodiazépin-2-one,
(73) 3-(1-{4-(4-amino-3-chloro-5-trifluorométhyl-phényl)-3-[3-(3-pyrrolidin-1-yl-propyl)-3H-imidazo[4,5-c]pyridin-2-yl]-butyryl}-pipéridin-4-yl)-1,3,4,5-tétrahydro-1,3-benzodiazépin-2-one,
(74) 3-{ 1-[4-(4-amino-3-chloro-5-trifluorométhyl-phényl)-3-(1-méthyl-6-pyrrolidin-1-ylméthyl-1H-benzimidazol-2-yl)-butyryl]-pipéridin-4-yl}-1,3,4,5-tétrahydro-1,3-benzodiazépin-2-one,
leurs stéréo-isomères et leurs sels.

11. Sels physiologiquement acceptables des composés selon au moins une des revendications 1 à 10 avec des acides ou des bases organiques ou inorganiques.

12. Médicament contenant un composé selon au moins une des revendications 1 à 10 ou un sel physiologiquement acceptable selon la revendication 11, et éventuellement un ou plusieurs excipients et/ou un ou plusieurs diluants inertes.

13. Utilisation d'un composé selon au moins une des revendications 1 à 11 pour la préparation d'un médicament pour le traitement aigu et prophylactique de céphalées, en particulier de la migraine ou de la céphalée vasculaire de Horton.

14. Procédé pour la préparation d'un médicament selon la revendication 12, **caractérisé en ce qu'**un composé selon au moins une des revendications 1 à 11 est incorporée par voie non chimique dans un ou plusieurs excipients et/ou un ou plusieurs diluants inertes.

15. Procédé pour la préparation d'un médicament selon l'une quelconque des revendications 1 à 11, **caractérisé en ce que**
a) pour la préparation de composés de la formule générale I, dans laquelle X représente un atome d'oxygène et Z le groupe -NR¹-, et A, n, R, R¹, R², R³ et R⁴ et A sont définis comme mentionné dans les revendications 1 à 10,
on fait réagir une amine de la formule générale II 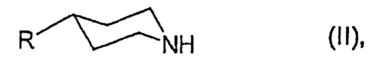 dans laquelle
R est défini comme mentionné dans les revendications 1 à 10, avec un dérivé de l'acide carbonique de la formule générale III 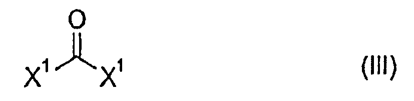 dans laquelle
X¹ représente un groupe nucléofuge,
et avec un composé de formule générale IV 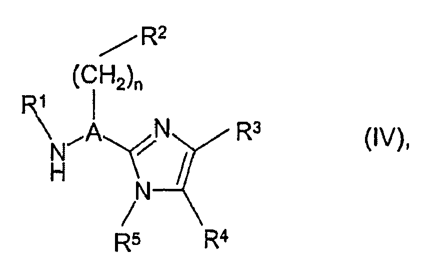 dans laquelle
R¹, R², R³, R⁴, R⁵, A et n sont définis comme mentionné dans les revendications 1 à 10
et, si nécessaire, **en ce qu'**on élimine ensuite les groupes de protection ou que l'on convertit les fonctions précurseurs, ou **en ce que**
b) pour la préparation de composés de la formule générale I, dans laquelle A, X, Z, n, R, R² et R⁵ sont définis comme mentionné dans les revendications 1 à 10 et R³ et R⁴ représentent ensemble un groupe 1,3-butadién-1,4-ylène, dans lequel
un, deux ou trois groupes méthine peuvent être remplacés chacun par un atome d'azote,
un atome d'hydrogène peut être remplacé par un atome de fluor, de chlore, de brome
ou d'iode, par un groupe C₁₋₃-alkyl, trifluorométhyle, difluorométhyle, cyclopropyle, hydroxyle, C₁₋₃-alkoxyle, difluorométhoxyle, trifluorométhoxyle, cyano, nitro, amino, C₁₋₃-alkyl-amino, di-(C₁₋₃-alkyl)-amino, carboxyle, C₁₋₃-alkoxy-carbonyle, aminocarbonyle, C₁₋₃-alkyl-aminocarbonyle, di-(C₁₋₃-alkyl)-aminocarbonyle, di-(C₁₋₃-alkyl)-amino-C₂₋₃-alkyl-aminocarbonyle, amino-C₁₋₃-alkyle, C₁₋₃-alkyl-amino-C₁₋₃-alkyle ou di-(C₁₋₃-alkyl)-amino-C₁₋₃-alkyle ou par un groupe cycloalkylène-imino-carbonyle à 4 à 7chaînons, dans lequel
un ou deux atomes d'hydrogène peuvent être remplacés chacun par un groupe C₁₋₃-alkyle ou/et
le groupe méthylène en position 4 d'un fragment cycloalkylène-imino à 6 ou 7 chaînons peut être remplacé par un atome d'oxygène ou de soufre, par un groupe sulfinyle, sulfonyle, -NH-, -N(C₁₋₃-alkyl)- ou -N(C₁₋₃-alkyl-carbonyl)-,
et en outre un deuxième atome d'hydrogène peut être remplacé par un atome de fluor, de chlore, de brome ou d'iode, par un groupe C₁₋₃-alkyle, trifluorométhyle,
difluorométhyle, cyclopropyle, hydroxyle, C₁₋₃-alkoxyle, difluorométhoxyle ou trifluorométhoxyle,
on soumet un amide de la formule générale (V) à une réaction de fermeture de cycle par condensation : 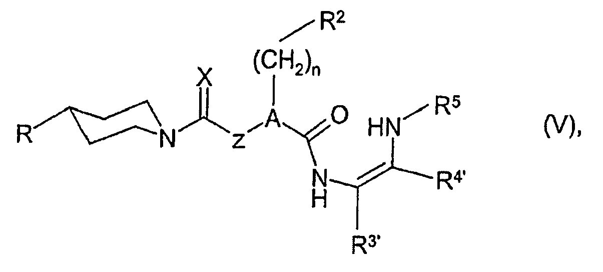 dans laquelle R, X, Z, A, n, R² et R⁵ sont définis comme mentionné dans les revendications 1 à 10, R^{3'} et R^{4'} représentent ensemble un groupe 1,3-butadién-1,4-ylène, dans lequel
un, deux ou trois groupes méthine peuvent être remplacés chacun par un atome d'azote,
un atome d'hydrogène peut être remplacé par un atome de fluor, de chlore, de brome
ou d'iode, par un groupe C₁₋₃-alkyle, trifluorométhyle, difluorométhyle, cyclopropyle, hydroxyle, C₁₋₃-alkoxyle, difluorométhoxyle, trifluorométhoxyle, cyano, nitro, amino, C₁₋₃-alkyl-amino, di-(C₁₋₃-alkyl)-amino, carboxyle, C₁₋₃-alkoxy-carbonyle, aminocarbonyle, C₁₋₃-alkyl-aminocarbonyle, di-(C₁₋₃-alkyl)-aminocarbonyle, di-(C₁₋₃-alkyl)-amino-C₂₋₃-alkyl-aminocarbonyle, amino-C₁₋₃-alkyle, C₁₋₃-alkyl-amino-C₁₋₃-alkyle ou di-(C₁₋₃-alkyl)-amino-C₁₋₃-alkyle ou par un groupe cycloalkylène-imino-carbonyle à 4 à 7 chaînons, dans lequel
un ou deux atomes d'hydrogène peuvent être remplacés chacun par un groupe C₁₋₃-alkyle ou/et
le groupe méthylène en position 4 d'un fragment cycloalkylèneimine à 6 ou 7 chaînons peut être remplacé par un atome d'oxygène ou de soufre, par un groupe sulfinyle, sulfonyle, -NH-, -N(C₁₋₃-alkyl)- ou -N(C₁₋₃-alkyl-carbonyl)-,
et en outre un deuxième atome d'hydrogène peut être remplacé par un atome de fluor, de chlore, de brome ou d'iode, par un groupe C₁₋₃-alkyle, trifluorométhyle, difluorométhyle, cyclopropyle, hydroxyle, C₁₋₃-alkoxyle, difluorométhoxyle ou trifluorométhoxyle, ou **en ce que**
c) pour la préparation de composés de la formule générale I, dans laquelle R, A, n ainsi que R² à R⁵ sont définis comme mentionné dans les revendications 1 à 10 et X représente un atome d'oxygène et Z le groupe -CH₂-:
on accouple un acide carboxylique de la formule générale 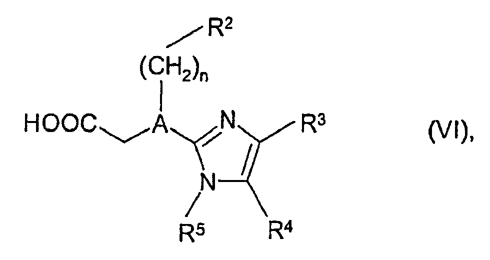
dans laquelle
R² à R⁵, A et n sont définis comme mentionné dans les revendications 1 à 10 avec un composé de la formule générale 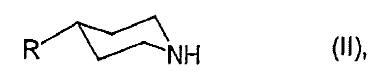 dans laquelle
R est défini comme mentionné dans les revendications 1 à 10, ou **en ce que**
d) pour la préparation de composés de la formule générale I, dans laquelle R, A, n ainsi que R² à R⁵ sont définis comme mentionné dans les revendications 1 à 10 et X représente un atome d'oxygène et Z le groupe -CH₂- :
on accouple un composé de la formule générale 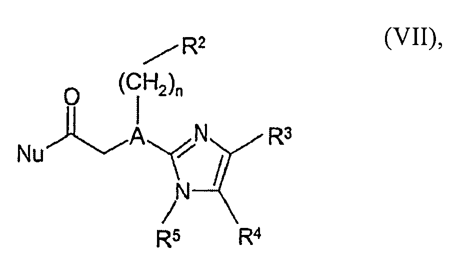
dans laquelle
A, ,n, et R² à R⁵ sont définis comme mentionné dans les revendications 1 à 10 et Nu représente un groupe partant,
avec un composé de la formule générale 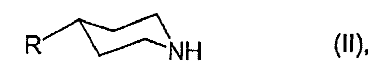 dans laquelle
R est défini comme mentionné dans les revendications 1 bis 10, ou **en ce que**
e) pour la préparation de composés de la formule générale 1, dans laquelle R, A, n ainsi que R² à R⁵ sont définis comme mentionné dans les revendications 1 à 10 et X représente un atome d'oxygène et Z le groupe -NH- :
on fait réagir un isocyanate de la formule générale VIII 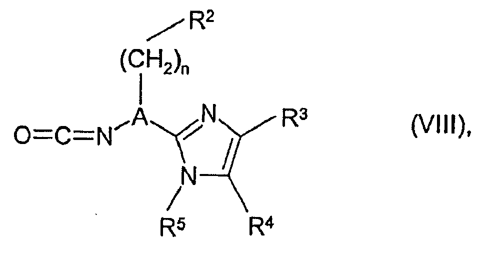
dans laquelle
R² à R⁵, A et n sont définis et ont la signification comme mentionné dans les revendications 1 à 10,
avec des amines de la formule générale II 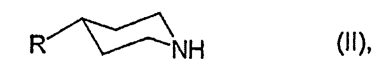 dans laquelle
R est défini comme mentionné dans les revendications 1 à 10, et, si nécessaire, on élimine ensuite les groupes de protection ou on convertit les fonctions précurseurs ou en ce que
f) pour la préparation de composés de la formule générale I, dans laquelle R, A, n ainsi que R² à R⁵ sont définis comme mentionné dans les revendications 1 bis 10 et X représente un groupe =N-CN ou =N-SO₂-R⁶, et Z le groupe -NR¹-, R¹ et R⁶ étant définis comme dans les revendications 1 à 10 :
on fait réagir un composé de la formule générale 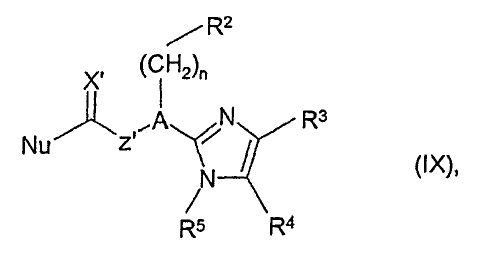
dans laquelle A, n et R² à R⁶ sont définis comme dans les revendications 1 à 10, X' représente un des groupes =N-CN ou =N-SO₂-R⁶, Z' le groupe -NR¹-, R¹ et R⁶ étant définis comme dans les revendications 1 à 10, et Nu est un groupe partant,
avec des amines secondaires de la formule générale 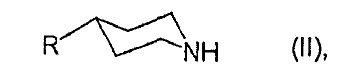 dans laquelle R est défini comme dans les revendications 1 à 10, et **en ce qu'**on élimine les groupes de protection des groupes réactifs comme les groupes hydroxyle, carboxyle, amino ou imine, éventuellement présents et/ou
on sépare un composé ainsi obtenu de formule générale I en ses stéréo-isomères et/ou on transforme un composé ainsi obtenu de formule générale I en sel, en particulier en vue de son utilisation pharmaceutique en un sel physiologiquement acceptable, par addition d'un acide ou d'une base organique ou minéral.

16. Utilisation d'un composé selon une au moins des revendications 1 à 11 pour la préparation d'un médicament pour la lutte contre le diabète sucré non insulino-dépendant (NIDDM),

17. Utilisation d'un composé selon une au moins des revendications 1 à 11 pour la préparation d'un médicament pour le traitement de maladies cardiovasculaires, de la tolérance à la morphine, d'affections de la peau, en particulier de lésions thermiques et dues à une irradiation, y compris les coups de soleil, d'affections inflammatoires telles que, en particulier, les affections inflammatoires articulaires comme l'arthrite, les affections pulmonaires inflammatoires, de rhinites allergiques, de l'asthme, d'affections accompagnées d'une dilatation excessive des vaisseaux et par là d'une réduction de l'irrigation des vaisseaux, comme en particulier le choc ou la septicémie, pour l'apaisement d'états douloureux en général ou pour l'action thérapeutique aiguë ou préventive sur les symptômes de bouffées de chaleur causés par la dilatation des vaisseaux et l'accroissement du débit sanguin chez des femmes ménopausées souffrant d'insuffisance oestrogénique, ainsi que chez les patients atteints de carcinome de la prostate subissant un traitement hormonal.
